(19) Europäisches Patentamt
European Patent Office
Office européen des brevets

(11) Publication number: 0 107 930 B1

(12) **EUROPEAN PATENT SPECIFICATION**

(45) Date of publication of patent specification: 28.11.90

(21) Application number: 83305964.5

(22) Date of filing: 30.09.83

(51) Int. Cl.[5]: C 07 D 267/14, C 07 D 281/10, C 07 D 498/04, C 07 D 513/04, C 07 D 413/06, C 07 D 417/06, A 61 K 31/55, C 07 D 205/04, C 07 D 207/12, C 07 D 401/12 // (C07D498/04, 267:00, 221:00),(C07D513/04, 281:00, 221:00)

(54) **Fused aromatic oxazepinones and sulphur analogues thereof and their preparation and use in counteracting histamine.**

(30) Priority: 30.09.82 US 431500
30.09.82 US 431998
29.08.83 US 527559
29.08.83 US 527558

(43) Date of publication of application: 09.05.84 Bulletin 84/19

(45) Publication of the grant of the patent: 28.11.90 Bulletin 90/48

(84) Designated Contracting States: AT BE CH DE FR GB IT LI LU NL SE

(56) References cited:
DE-A-1 912 546
DE-A-1 964 510
FR-A-2 496 653
US-A-4 307 091

(73) Proprietor: A.H. ROBINS COMPANY, INCORPORATED
1407 Cummings Drive P.O. Box 26609
Richmond Virginia 23261-6609 (US)

(72) Inventor: Cale, Albert Duncan, Jr.
Box 150, Route 8
Mechanicsville Virginia 23111 (US)
Inventor: Franko, Bernard Vincent
4012 Patterson Avenue
Richmond Virginia 23221 (US)
Inventor: Leonard, Charles Arthur
1601 Camelot Drive
Richmond Virginia 23229 (US)

(74) Representative: Sheard, Andrew Gregory et al
Kilburn & Strode 30, John Street
London WC1N 2DD (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

## Description

The present invention relates to novel aromatic 2,3-dihydro-1,4-oxazepin-5-(4H)-ones and sulphur analogues, thereof and is particularly concerned with aromatic-2,3-dihydro-1,4-oxazepin-5(4H)-ones and sulphur analogues thereof which have the aromatic component fused into the oxazepine or thiazepine ring and are substituted in the 2-position with short chain aminoalkyl radicals, having antihistaminic and anti-allergy utility and a novel process and novel intermediates for the preparation thereof.

3-Aryl-1,4-benzoxazepin-5(4H)-ones substituted on the oxazepine nitrogen by an aminoalkyl radical have been disclosed by Schenker, K. in Swiss Patent 505.850 (c.a. *75,* 98600s).

Conversion of flavanones into benzoxazepinones substituted in the 2-position by a phenyl radical has been disclosed by Levai, A. and Bognar, R., Top. Flavanoid Chem. Biochem. Proc. Hung. Bioflavonoid Symp. 4th Ed. 1973 (Pub. 1975) 119—23 (C.A. *75,* 79098n). Thione derivatives were obtained by treating with phosphorus pentasulphide.

Certain chemical intermediates, the 1-substituted-3-substituted phenoxypyrrolidines illustrated by
1-methyl-3-(2-carbamoylphenoxy)pyrrolidine,
1-benzyl-3-(2-carbamoylphenoxy)pyrrolidine, and
1-methyl-3-(2-carboxyphenoxy)pyrrolidine
in an otherwise novel class are disclosed in U.S. Patent 3,577,415.

According to a first aspect of the present invention, there is provided an oxazepine or thiazepine derivative having the formula:

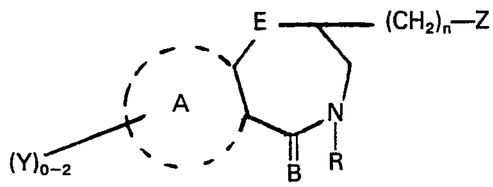

Formula I

wherein:

A represents an aromatic ring system having two of its carbon atoms held mutually with the oxazepine or thiazepine moiety selected from benzene, naphthalene or a pyridine in any of its four positions, any of the rings being optionally substituted by one or two Y radicals selected from halo, loweralkyl, loweralkoxy, nitro and trifluoromethyl;

B and E are selected from oxygen and sulphur and may be the same or different;

R is selected from hydrogen, loweralkyl, cycloalkyl or phenyl-loweralkyl, of which phenyl is optionally substituted by one or two radicals selected from halo, loweralkyl, loweralkoxy, nitro or trifluoromethyl;

n is 1, 2 or 3;

Z is selected from —$NR^1R^2$, 1H-pyrazol-1-yl and 1H-imidazol-1-yl;

each of $R^1$ and $R^2$ is selected from the group consisting of hydrogen, loweralkyl, cycloalkyl and phenyl-loweralkyl, of which phenyl is optionally substituted by 1 or 2 radicals selected from halo, loweralkyl, loweralkoxy, nitro, trifluoromethyl and cyano, or $R^1$ and $R^2$ taken together with the adjacent nitrogen atom may form a heterocyclic residue selected from 1-pyrrolidin-1-yl, 2,5-dimethylpyrrolidin-1-yl, 2-methylpyrrolidin-1-yl, 1-piperidinyl, 4-substituted piperidine-1-yl, 4-morpholinyl, 1-piperazinyl, 4-substituted piperazin-1-yl and 1,2,3,6-tetrahydropyridin-1-yl and the pharmaceutically acceptable salts thereof with the proviso that when R = H, Z is never a primary or secondary amine, and a further proviso that when n = 3, Z is not 1H-pyrazol-1-yl, 1H, imidazol-1-yl, 2,5-dimethylpyrrolidin-1-yl, 2-methylpyrrolidin-1-yl or 1,2,3,6-tetrahydropyridin-1-yl.

Preferred compounds of Formula 1 are those in which: the ring represented by A is substituted by one or more chlorine or bromine atoms or by a methoxy group; R represents a methyl, ethyl, benzyl or hexyl group; and/or Z represents a dimethylamino group.

According to a second aspect of the invention, there is provided a compound as defined above for use in medicine, which may be pharmaceutical or veterinary medicine, especially as an antihistamine.

In the further definition of symbols in the formulas hereof and where they appear elsewhere throughout this specification and the claims, the terms have the following significance.

The term "loweralkyl" as used herein incudes straight and branched chain radicals of up to eight carbons inclusive and is exemplified by such groups as methyl, ethyl, propyl, isopropyl, butyl, sec. butyl, tert.butyl, amyl, isoamyl, hexyl, heptyl and octyl radicals and the like. The term "loweralkoxy" has the formula —O-loweralkyl.

The term "cycloalkyl" as used herein includes primarily cyclic alkyl radicals containing 3—9 carbon atoms inclusive and includes such groups as cyclopropyl, cyclobutyl cyclopentyl, cyclohexyl, methylcyclohexyl, cycloheptyl and the like.

The terms "halo" or "halogen" when referred to herein include fluorine, chlorine, bromine and iodine unless otherwise stated.

"Pharmaceutically acceptable salts' include acid addition salts, hydrates, alcoholates and quaternary salts of the compounds of Formula I, which are physiologically compatible in warm-blooded animals. The

acid addition salt may be formed by either strong or weak acids. Representative of strong acids are hydrochloric, sulphuric and phosphoric acids. Representative of weak acids are fumaric, maleic, succinic, oxalic, citric, tartaric, cyclohexamic and the like.

Suitable quaternary salts include the loweralkyl halides and loweralkyl sulphates.

As used herein, the word "aromatic", when used to qualify such terms as "ring" and "ring system" should not be taken to imply that the ring or ring system as the case may be is necessarily composed entirely of carbon atoms. It will therefore be understood that certain heterocyclic rings and ring systems fall within the definition of "aromatic".

By sulphurizing agent" is meant any agent or mixture of agents which will convert ox- and thiazepinones to ox- and thiazepine-thiones, such as 2,4-bis(4-methoxyphenyl)-1,3,2,4-dithiadiphosphetane-2,4-disulphide or a mixture of phosphorus pentasulphide and alkalimetal sulphide or a mixture of phosphorus pentasulphide in pyridine.

The compounds of the present invention exhibit antihistaminic activity in guinea pigs. The method of testing is a modification of the procedure of Tozzi et al. (Agents and Actions, Vol. 4/4, 264—270, 1974) as follows: Guinea pigs are fasted 18—24 hrs in individual cages. Water is available *ad libitum.* On the test day, animals in groups of 3 are injected intraperitoneally with 30 mg/kg of the test compound prepared in an appropriate vehicle. Thirty minutes later histamine at a dosage level of 1.2 mg/kg (= 2 × the $LD_{99}$) is injected into a marginal ear vein. Survival of the guinea pigs for 24 hrs is positive evidence of antihistamine activity. If the vehicle used for the test compound is other than water, its effect is established by testing an equal amount as a control. The dose protecting 50% of the animals ($PD_{50}$) from death may be established from dose-response curves.

According to a third aspect of the invention there is provided a process for the preparation of a compound or salt in accordance with the first aspect, the process comprising either

i) reacting a compound of the formula

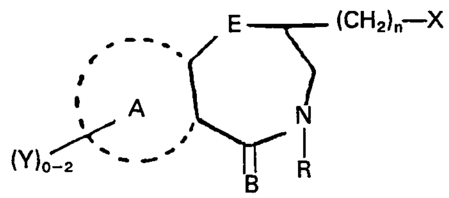

wherein A, B, E, R, Y and n are as defined for formula I and X represents chlorine, bromine or cyano with a compound of the formula

ZH

wherein Z is as defined for formula I; or

ii) reducing a compound of the formula

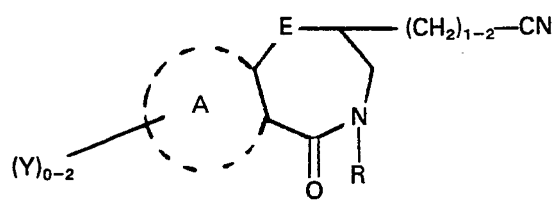

to give a compound of formula I in which B represents oxygen, Z represents $-NH_2$ and n is 2 or 3, and if necessary

iii) converting a compound of formula I so formed into another compound of formula I; and optionally

iv) preparing a pharmaceutically acceptable salt of a compound so formed.

The preferred novel process of this invention comprises the following steps:

Step 1) Halogenating a compound of the formula

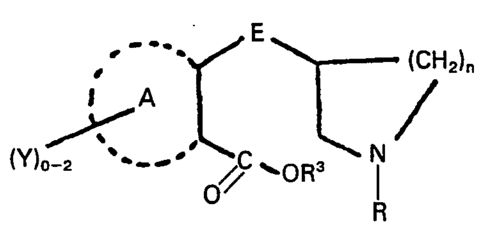

IVb

wherein:

A represents an aromatic ring selected from benzene, naphthalene, or a pyridine in any one of its four positions, optionally substituted by one or two Y-radicals selected from halo, loweralkyl, loweralkoxy, nitro and trifluoromethyl;

E is oxygen or sulphur;

R is selected from loweralkyl, cycloalkyl and phenyl-loweralkyl, of which phenyl may be optionally substituted by one or two radicals selected from halo, loweralkyl, loweralkoxy, nitro and trifluoromethyl;

$R^3$ is hydrogen or an acid neutralizing ion, and

n is one or two,

to give a compound of the formula

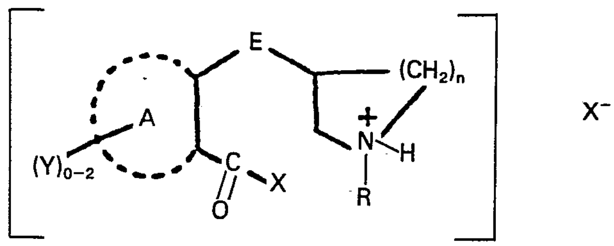

III

or its free base wherein X is chlorine or bromine and A, E, R, Y and n are the same as the starting values. Among suitable halogenating agents are

a) thionyl halides

b) triphenylphosphine and a carbon tetrahalide

c) phosphorus pentahalides

d) phosphorus trihalides, and

e) triphenylphosphine dihalide,

with the thionyl halides being preferred.

Step 2) Neutralizing, if necessary, and fusing the carboxylic acid halide derivative prepared in step 1 to give an oxazepinone or a thiazepinone of the formula

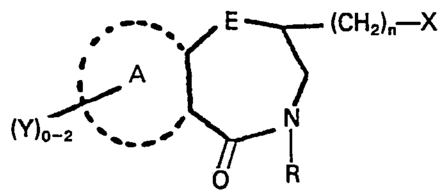

IIa

wherein A, E, R, X, Y and n are as defined above in step 1, and A now has two of its carbon atoms held mutually with the oxazepine or thiazepine moiety.

Step 3) Optionally reacting the compound prepared in step 2 with a sulphurizing agent to obtain an oxazepinethione or a thiazepinethione of the formula

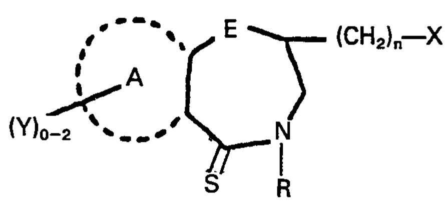

IIb

wherein A, E, R, X, Y and n are as defined above in step 2.

Step 4) When required, reacting a compound prepared in step 2 with an alkali-metal cyanide to obtain a compound of the formula

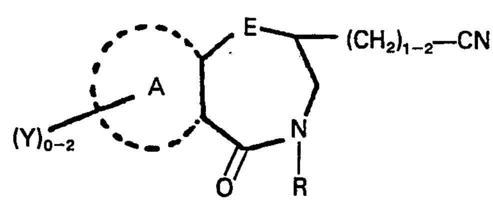

IIc

wherein A, E, Y, and R are as defined in step 2.

Step 5) Reacting a halogen compound prepared in step 2 or 3 with a compound of the formula

ZH

wherein Z is selected from —$NR^1R^2$, 1H-pyrazol-1-yl and 1H-imidazol-1-yl, and wherein $R^1$ and $R^2$ are

selected from hydrogen, loweralkyl, cycloalkyl and phenyl-loweralkyl, of which phenyl may be optionally substituted with 1 or 2 radicals selected from halo, loweralkyl, loweralkoxy, nitro, trifluoromethyl and cyano, or $R^1$ and $R^2$ taken together with the adjacent nitrogen atom may form a heterocyclic residue selected from 1-pyrrolidinyl, 2,5-dimethylpyrrolidin-1-yl, 2-methylpyrrolidin-1-yl, 1-piperidinyl, 4-substituted-piperidine-1-yl, 4-morpholinyl, 1-piperazinyl, 4-substituted-piperazin-1-yl and 1,2,3,6-tetrahydropyridine-1-yl, to give a compound of the formula

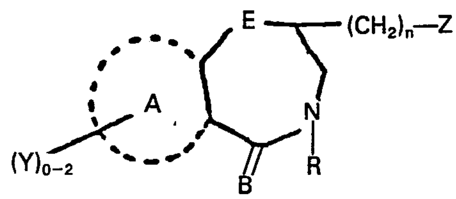

$I_a$

wherein A, E, R, n and Y are as defined above in step 2, Z is the same as in the ZH compound, and B is an oxygen or suphur atom.

Step 6) Optionally reacting a compound prepared in step 5 wherein B is an oxygen atom with a sulphurizing agent, preferably phosphorus pentasulphide in pyridine to obtain a compound of the formula

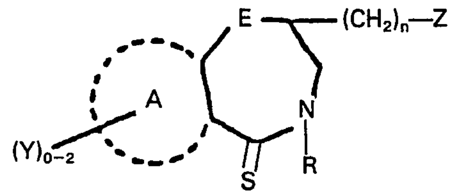

$I_b$

wherein A, E, R, n, Y and Z are as defined in step 5.

Step 7) Reducing a cyano compound (Formula IIc) prepared in step 4 to a primary amine of the formula

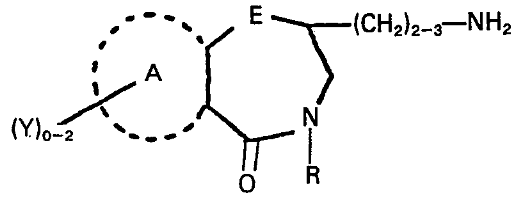

$I_{c-1a}$

wherein A, E, Y and R are defined in steps 2 and 4.

Step 8) When required, reacting a primary amine prepared in steps 5 or 7 of the formula

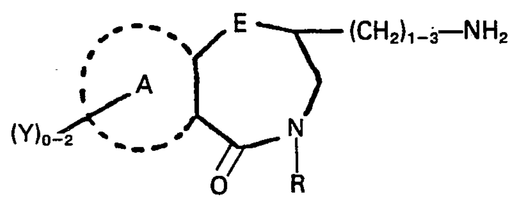

$I_{c-1}$

wherein A, E, Y and R are as defined in step 2 (R is not hydrogen) with one of the following reactants or sets of reactants

a) formaldehyde and formic acid to give a tertiary dimethylamine,

b) a dihalide to give a heterocyclic amine,

c) a dialdehyde and sodium cyanoborohydride to give a heterocyclic amine,

d) equal molar amounts of aldehyde or ketone, sodium cyanoborohydride with large excess of above primary amine to give a secondary amine,

e) equal molar amounts of the primary amine and sodium cyanoborohydride with at least two equivalents of aldehyde or ketone to give a tertiary amine,

f) in sequence trifluoroacetyl chloride, alkyl or phenylalkyl halide, potassium hydride and potassium hydroxide to give a secondary amine,

all products being encompassed by the formula

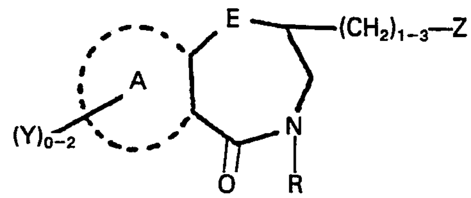

$I_d$

wherein A, E, Y and R are as defined in step 2 (note: R is not hydrogen) and Z is —$NR^1R^2$ wherein each of $R^1$ and $R^2$ is loweralkyl, cycloalkyl or phenyl-loweralkyl with phenyl being optionally substituted by halo, loweralkyl, loweralkoxy, nitro, trifluoromethyl or cyano or $R^1$ and $R^2$ taken together with the adjacent nitrogen may form a heterocyclic residue selected from 1-pyrrolidinyl, 1-piperidinyl, 4-substituted piperidin-1-yl, 4-morpholinyl, 1-piperazinyl-1-yl and 1,2,3,6-tetrahydropyridin-1-yl and optionally sulphurizing the azepinone or thiazepinone to give the corresponding thione as in step 6.

Step 9) When required, reacting a benzyl or substituted benzyl compound obtained in steps 5, 6 or 8 wherein Z is tertiary amino, pyrzolyl or imidazolyl of the formula

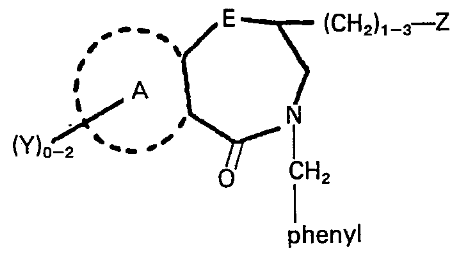

$I_e$

wherein A, E and Y are as defined in step 2, and Z is any radical taken from the definition of Z under Formula I, subject to the same provisos given thereunder, and with sodium and ammonia to give a compound of the formula

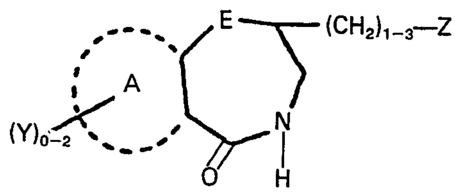

$I_f$

wherein A, E and Y are as defined in step 2, n is 1 to 3 and Z is as defined under Formula I with limitations given thereunder.

Step 10) Optionally reacting the free base of any compound prepared in steps 5 to 9 with a pharmaceutically acceptable acid or quaternary forming halide or sulphate to form a pharmaceutically acceptable salt thereof.

The compounds of Formula I wherein n is 2 are preferred for their antihistaminic activity. The process which includes steps 1 to 3, 5, 6 and 10 wherein compounds prepared have a methyl or ethyl side chain (n = 1 or 2) represents a preferred process corresponding to a succession of steps designated A to F as explained hereinbelow.

The compounds of Formulas $I_a$, $I_b$, $I_{c-1}$ to $I_{c-7}$, $I_{c-1a}$, $I_d$, $I_e$, $I_f$ are all encompassed by Formula I and the compounds of Formulas $II_a$, $II_b$, $II_c$, $II_d$ and $II_e$ are all encompassed by Formula II.

Steps 1 to 4 also represent a novel process for preparing compounds of Formula $II_a$, $II_b$, $II_c$, all encompassed by Formula II.

The present invention provides certain novel aromatic 2,3-dihydro-1,4-oxazepin-5(4H)-ones (and sulphur analogues thereof) substituted in the 2-position with short chain aminoalkyl radicals which have antihistaminic activity.

The invention also provides certain novel aromatic 2,3-dihydro-1,4-oxazepin-5(4H)-ones (and sulphur analogues thereof) substituted in the 2-position with alkyl halo or alkyl cyano radicals which are chemical intermediates.

The novel oxazepine and thiazepine precursors leading to compounds of Formula I constitute a fourth aspect of the invention and have the formula:

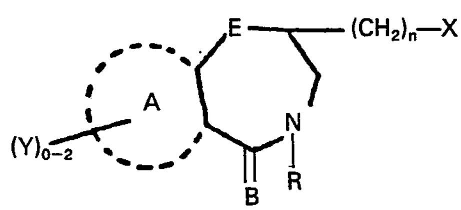

Formula II

wherein A, B, E, R and Y are as defined under Formula I above except R is not hydrogen, n is 1 or 2, and X is chlorine, bromine or cyano, and the acid addition salts thereof.

The invention further provides a novel route and process for preparation of aromatic 2,3-dihydro-1,4-oxazepin-5(4H)-ones (and sulphur analogues thereof) substituted in the 2-position with short chain alkylhalo or alkylcyano or alkylamino radicals.

According to a fifth aspect of the invention, there is provided a process for the preparation of a compound of the formula:

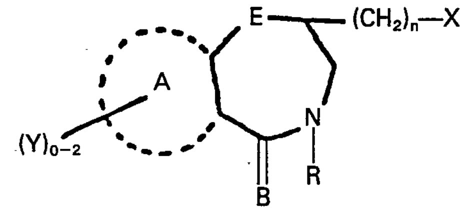

wherein:

A selected from an aromatic ring system having two of its carbon atoms held mutually with the oxazepine or thiazepine moiety selected from benzene, a naphthalene, a pyridine in any of its four positions, any of which ring systems is optionally substituted by one or two Y radicals selected from halo, loweralkyl, loweralkoxy, nitro or trifluoromethyl;

B and E are selected from oxygen and sulphur and may be the same or different;

R is selected from loweralkyl, cycloalky or phenylloweralkyl wherein phenyl is optionally substituted by one or two radicals selected from halo, loweralkyl, loweralkoxy, nitro or trifluoromethyl;

n is 1 or 2;

X is chlorine, bomide or cyano or an acid addition salt thereof, the process comprising

i)a) fusing a compound having the formula

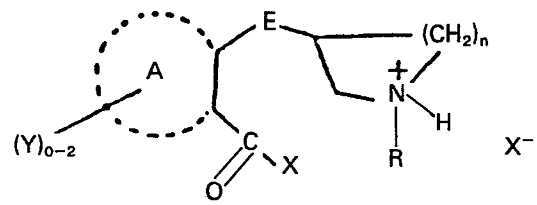

wherein x represents chlorine or bromine and A, E, n, R and Y are as defined above;

b) and when required reacting the compound so formed with a sulphurizing agent to obtain an oxazepinethione or a thiazepinethione; and

c) optionally reacting a compound obtained in step i)a) or i)b) with an alkali metal cyanide to obtain the corresponding compound in which X represents CN; or

ii) halogenating a compound obtained in step i)a), i)b) or i)c) having the formula

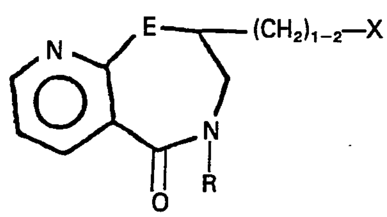

wherein E and R are as defined above and X is chlorine, bromine or cyano with sulphuryl chloride in a suitable solvent to obtain a compound having the formula

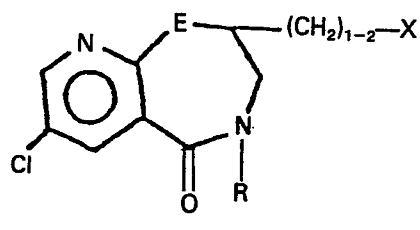

wherein E and R are as defined above X is chlorine, bromine or cyano.

In a preferred embodiment, the process of the fifth aspect comprises the steps of:

Step 1) halogenating a compound of the formula

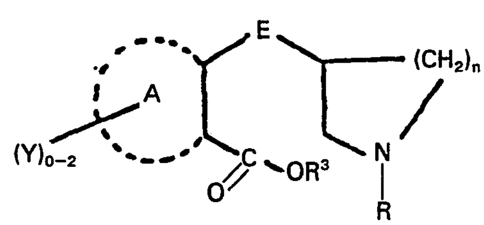

wherein A, E, R, n and Y are as defined above except the two carbon atoms of A are not bound by oxazepine or thiazepine rings and $R^3$ is hydrogen or an acid salt neutralizing ion, to give an acid halide of the formula

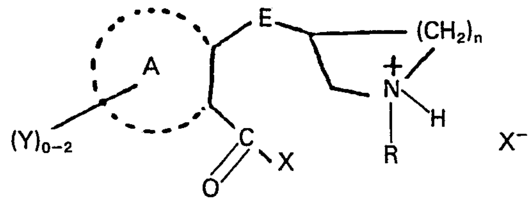

or its free base wherein X is chlorine or bromine and A, E, n, R and Y are as defined above.

Step 2) fusing the compound prepared in Step 1 to give an oxazepinone or thiazepinone of the formula

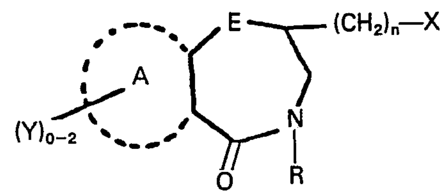

wherein A, E, R, Y, n and Y are as defined above in Step 1 and A now has 2 of its carbon atoms held mutually with the oxazepine or thiazepine moiety.

Step 3) When required, reacting the compound prepared in Step 2 with a sulphurizing agent to obtain an oxazepinethione or thiazepinethione of the formula

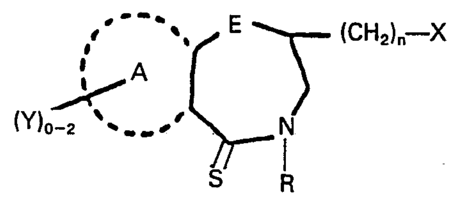

wherein A, E, R, X, Y and n are as defined above.

Step 4) when required, reacting a compound prepared in Step 2 with an alkali-metal cyanide to obtain a compound of the formula

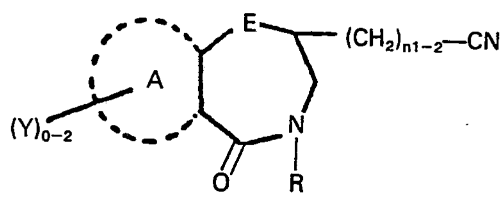

wherein A, E, Y and R are as defined in Step 2.

Step 5) in the alternate halogenating a compound prepared in Steps 2 or 4 having the formula

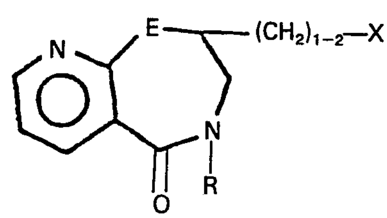

wherein E and R are as defined above and X is chlorine, bromine or cyano with sulfuryl chloride in a suitable solvent to give a compound having the formula

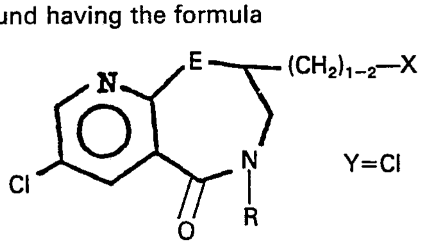

wherein E and R are as defined above and X is chlorine, bromine or cyano.

Other chemical intermediates in the preparation of compounds of Formula II are novel. They constitute a sixth aspect of the invention and have the formula:

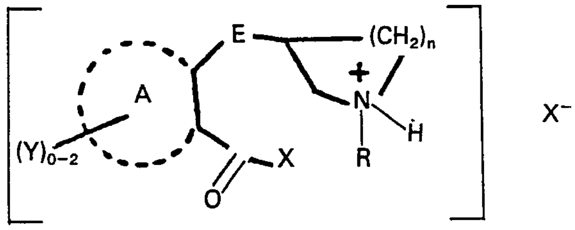

Formula III

wherein A, E, R, n and Y are as defined under Formula II above and X is chlorine or bromine.

Still other chemical intermediates leading to compounds of Formula IVb have the formula:

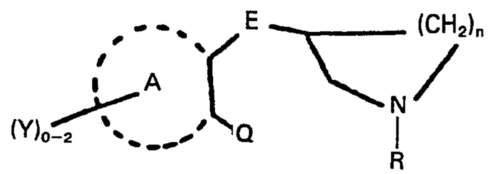

Formula IVa

wherein A, E, R, n and Y are as defined under Formula II, and Q is selected from

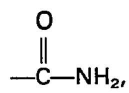 , —CN and 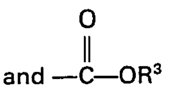

where $R^3$ is H, alkali metal ion or an esterifying radical. Compounds of Formula IVa are novel except wherein A is phenyl of substituted phenyl and E is oxygen. These novel compounds constitute a seventh aspect of the invention.

Additional objects and advantages of the present invention will be apparent to one skilled in the art and others will become apparent from the following description of preferred features of the present invention and from the appended claims.

The present invention encompasses the novel oxazepine and thiazepine derivatives set forth in Formulas I and II and certain novel compound of Formulas III, $IV_a$ and $IV_b$ as composition of matter and a process for the preparation of compounds of Formulas I, II and III.

Charts I and II illustrate the preparation of all intermediates. R is never hydrogen.

Chart III illustrates reaction sequence for preparing end-products wherein R is other than hydrogen and n is 1 or 2.

Chart IV illustrates preparation of compounds having 2-positioned ethyl and propyl radicals-omega substituted by primary amine (—$NH_2$). R is never hydrogen.

Chart V illustrates methods of converting the omega-$NH_2$-substituted ethyl and propyl compounds to secondary and tertiary amines. This is an alternative method for preparing the ethyl-secondary and tertiary amines. R is never hydrogen.

Chart VI illustrates preparation of compounds wherein R is hydrogen.

Preparations 1—23 illustrate syntheses of compounds of Formulas IVa, IVb or provide certain starting materials therefor. Intermediates 1—36 (see also Table 1) illustrate preparation of compounds encompassed by Formula II, which are aromatic-2,3-dihydro-1,4-oxazepin-5(4H)-ones (and sulphur analogues thereof) substituted in the 2-position with alkylhalo or alkylcyano radicals. The compounds of Formula III are formed in the reaction mixtures usually without isolation. Examples 1—71 (see also Table 2) illustrate preparation of compounds encompassed by Formula I. The scope of the invention is not limited by the preparations, intermediates and examples, however

CHART I

Preparation of Intermediates (a) (b)

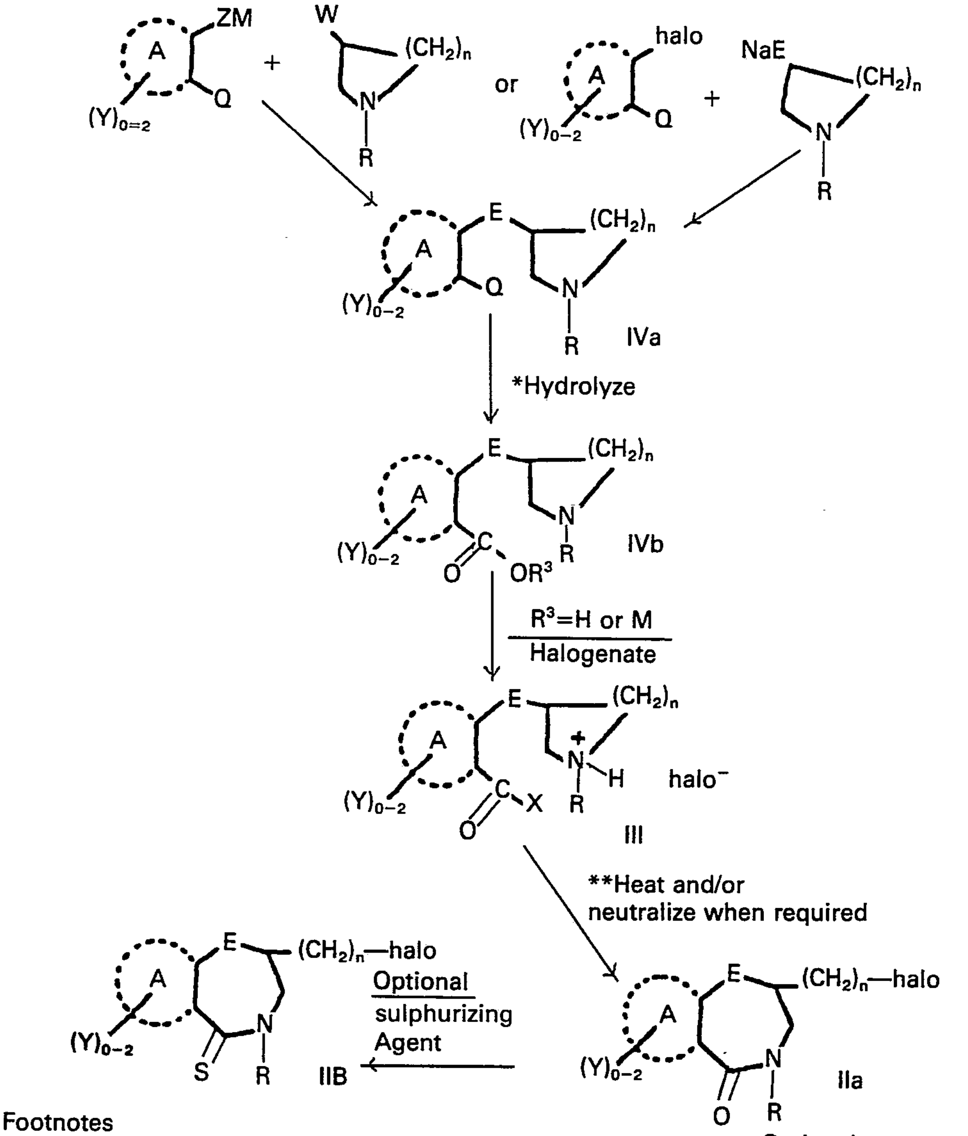


IVa

IVb

III

IIB

IIa

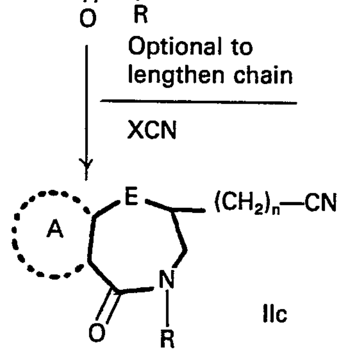


IIc

Footnotes

a) R is never hydrogen.

b) n = 1 or 2.

X = Halogen (Cl, Br)

W = Aryl sulphonate, alkyl sulfonate or X.

M = Acid neutralizing ion, e.g. alkali-metal

Q is $-\overset{O}{\overset{\|}{C}}-NH_2$, $-CN$, $-\overset{O}{\overset{\|}{C}}-OR^3$

where $R^3$ is H, M or esterifying radical.

*Hydrolyze when Q is other than

$-\overset{O}{\overset{\|}{C}}-OH$ or $\overset{O}{\overset{\|}{C}}-OM$.

**Diagram illustrating the suggested formation and cleavage of bonds to affect rearrangement.

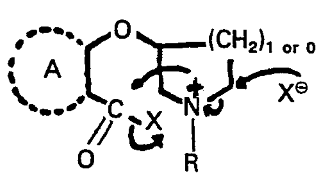

CHART II
Alternative Method of Preparing Pyrido Ring-
Halogenated Intermediates (a)

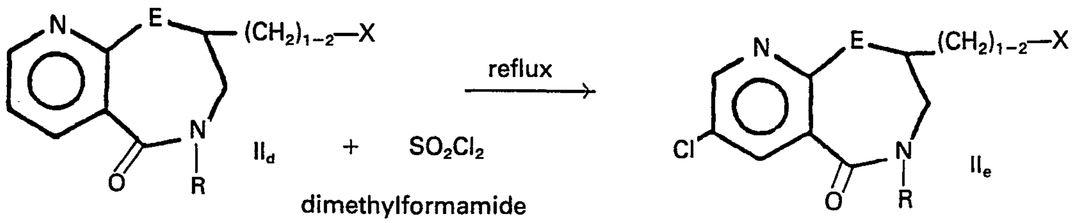


$II_d$ $II_e$

Footnootes:
X = Cl, Br, CN
E and R are as defined in Formula I
a) n = 1 or 2

CHART III
Preparation of End Products
(n = 1 or 2; R = other than H)

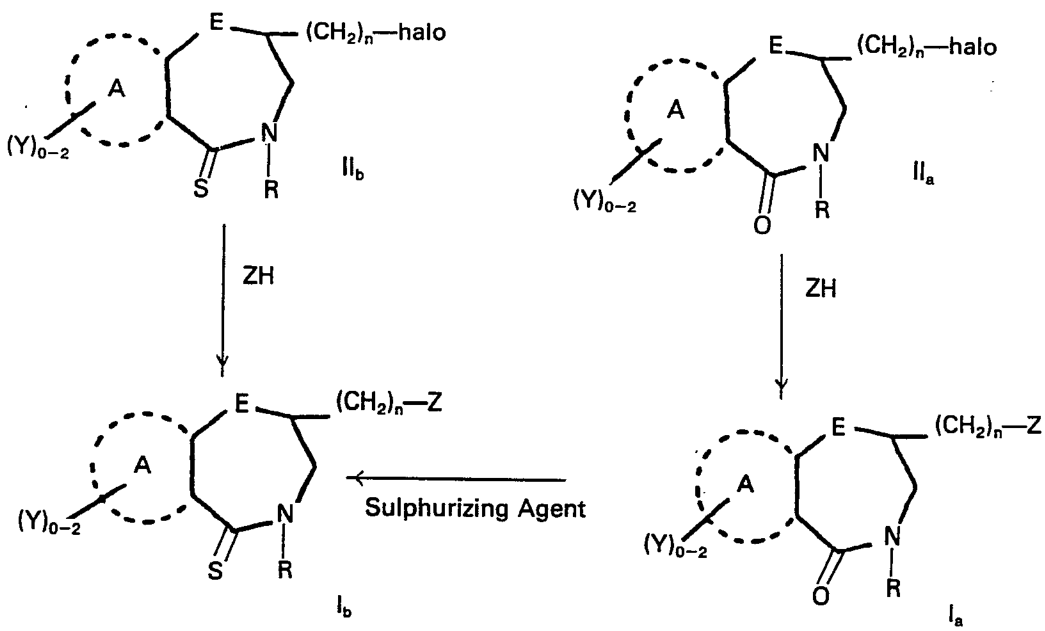


$II_b$ $II_a$ $I_b$ $I_a$

Footnote:
Z is as defined for n = 1 or 2
under Formula I.

CHART IV
Preparation of Primary Ethyl and Propyl Amino Compounds wherein R is other than Hydrogen from Cyano Intermediate

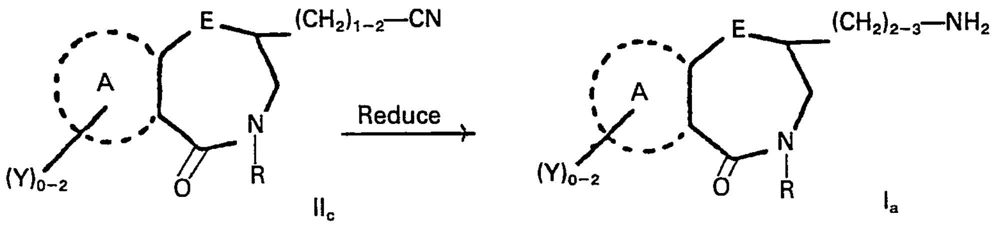


$II_c$ $I_a$

CHART VI
Preparation of Compounds Having Unsubstituted Azepine Nitrogen (R = H)

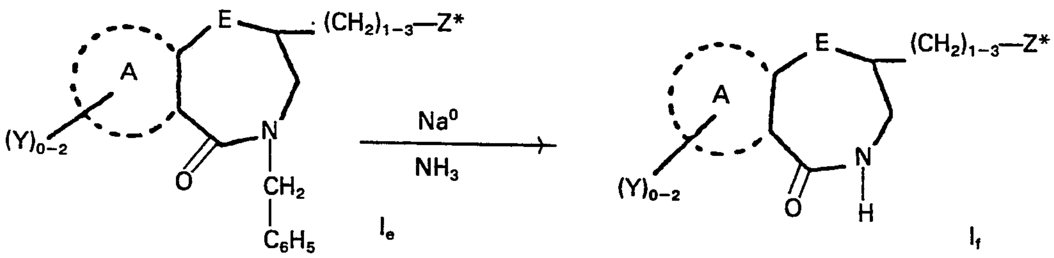


$I_e$ $I_f$

R = Benzyl R = H

Footnote:
*Z cannot be a primary or secondary amine.

CHART V
Additional Preparation of Secondary and Tertiary Amino-alkyl Compounds wherein R is other than H

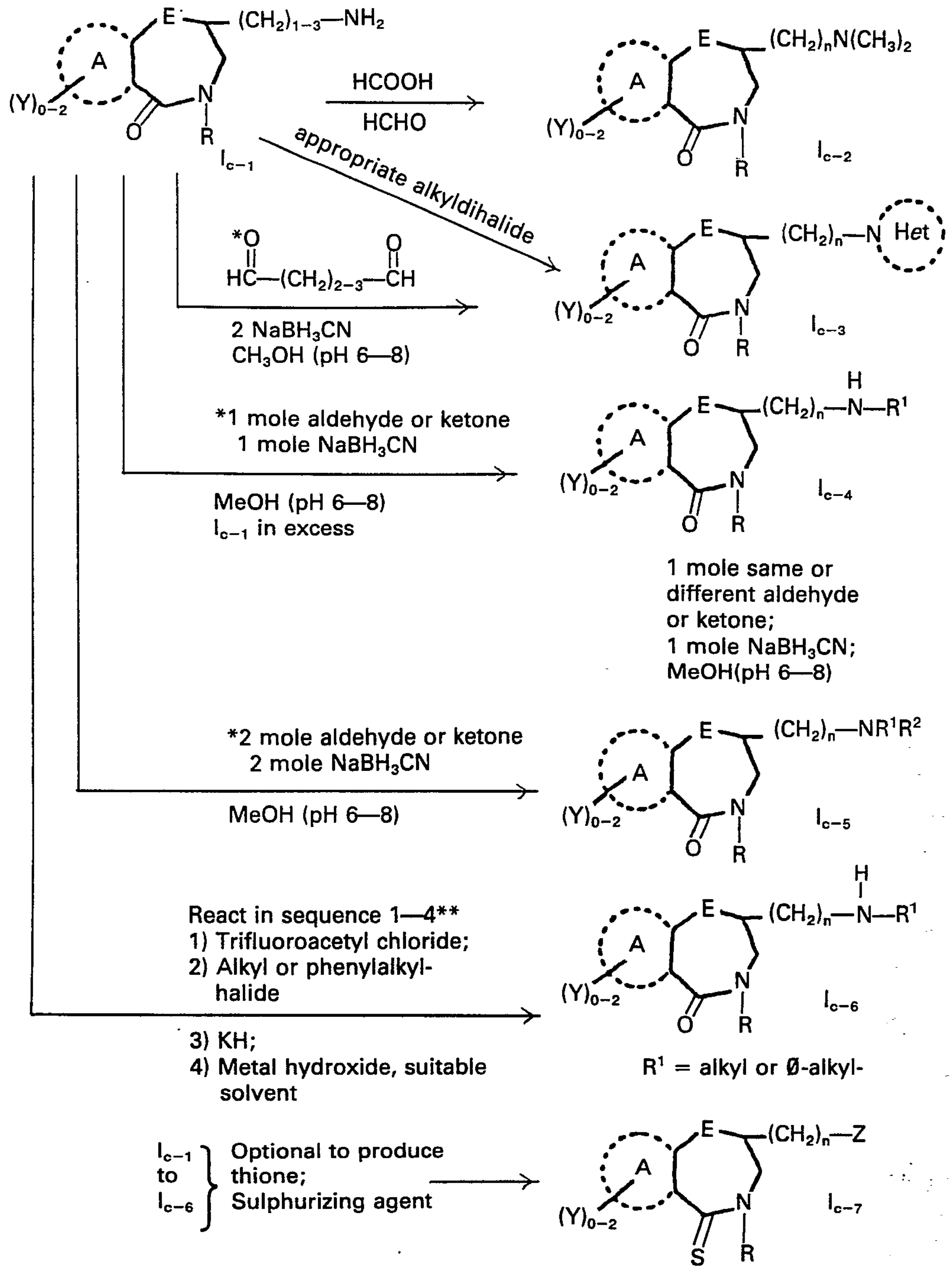


Footnotes:

n = 1—3

* Method described by R. F. Borch, et al., J. Amer. Chem. Soc. *93,* 2897(1971).

Illustration of reacton of a compound of Formula $I_{c-1}$ wherein Z is $NH_2$ with aldehydes and ketones follows:

| Reactant | Z-Radical Produced |
|---|---|
| 1) 1 mole acetaldehyde<br>1 mole $NaBH_3CN$<br>excess primary amine<br>(Z = $NH_2$) | $—NHC_2H_5$ |
| 2) 1 mole acetone<br>1 mole $NaBH_3CN$ | $—NHCH(CH_5)_2$ |
| 3) 1 mole benzaldehyde<br>1 mole $NaBH_3CN$<br>excess primary amine<br>(Z = $NH_2$) | $—NHCH_2C_6H_5$ |
| 4) 1 mole cyclohexanone<br>1 mole $NaBH_3CN$ | $—NHC_6H_{11}$ |
| 5) 2 mole acetaldehyde<br>2 mole $NaBH_3CN$ | $—N(C_2H_5)_2$ |
| 6) 1 mole acetaldehyde<br>1 mole $NaBH_3CN$<br>excessprimaryamine<br>(Z = H)<br>followed by<br>1 mole formaldehyde<br>1 mole $NaBH_3CN$ | $—N(CH_3)C_2H_5)$ |

** J. E. Norlander et al., Tetrahedron Letters 1978(50) pp 4987—4990.

In reference to the processes and the process steps of the invention summarized above as they apply to the preparation of compounds of Formulas I, II and III, the following further description is applicable.

In step 1, starting compounds of Formula $IV_b$ (See Chart I) bearing a carboxylic acid or an acid neutralizing ion such as an alkali-metal salt thereof on the A ring ortho to the ether linkage as a substantially pure entity or preferably derived in a reaction mixture resulting from hydrolysis of precursors being in the same ortho position, carbamoyl, cyano or carboxylic acid ester functions without substantial isolation of the carboxylic acid (or salt) compound from the reaction mixture, are treated with any suitable halogenating agent such as are described above, preferably thionyl chloride or triphenyl phosphine and carbon tetrachloride. The halogenation is conducted in any suitable organic solvent, preferably a refluxing organic solvent or a refluxing halogenating agent such as the preferred thionyl chloride. Temperatures for the chlorination over a wide range may be employed, for example, from room temperature to 100°C or above; however, temperatures of 50—80°C are preferred, which temperatures encompass that of refluxing chloroform or thionyl chloride. When excess halogenating agent such as thionyl chloride has been used as carrier, it is advantageously evaporated. When solvent such as chloroform is used, it may, but not necessarily, be evaporated away. In any event a solution comprising a solvent and compounds of Formula III or a residue comprised of Formula III compounds, all of which are confirmed by infra-red analysis is available for use in the next step.

In step 2, the halogenated compounds of Formula III, prepared in step 1, if not already in a solvent, are solubilized with organic solvent, preferably chloroform and usually neutralized or basified preferably with a tertiary amine such as triethyl amine, and then heated at a temperature and for a time sufficient to effect a fusion of the carbonyl with the basic nitrogen and cleavage of the cyclic amine and formation of the 2-(2-alylchloro or bromo) oxazepine or thiazepine compounds of Formula IIa, for example, in refluxing triethylamine. If the tendency to fuse is sufficiently great, the neutralization or basification may be eliminated. The Formula IIa compounds may be isolated by conventional means, for example, by partitioning between a suitable organic solvent or mixture of solvents and aqueous acid or base followed by drying and evaporating the organic layer and recrystallizing the residue from a suitable solvent.

In step 3, the compounds of Formula IIa may optionally be converted to the oxazepinethione or thiazepinethione (IIb) by heating together with a sulphurizing agent in a suitable organic solvent such as toluene. The thione (IIb) may be isolated by conventional means, preferably by partitioning between an organic solvent and dilute alkali-metal base and crystallizing from a suitable solvent such as ethanol.

In step 4, an oxazepinone or thiazepinone (IIa) is reacted with potassium cyanide in a hot protic solvent using a phase transfer catalyst such as tetrabutyl ammonium bromide. The resulting cyano compound is then extracted into a suitable solvent such as ethyl acetate, and the solution dried and evaporated. The

residue is then recrystallized from suitable solvent such as a mixture of ethyl acetate and isopropyl ether or ethyl acetate alone. As will be realized, the compounds produced have methyl and ethyl cyano side chains (n = 1 or 2) which lead to side chain lengthening to amino propyl n = 3 or as an alternate starting material for lengthening of a methyl chain to amino ethyl.

In step 5, the oxazepinone and thiazepinone (IIa) obtained in step 2 or the oxazepinethione and thiazepinethione (IIb) obtained in step 3 are reacted with pyrazole, imidazole or with an amine of the formula $NHR^1R^2$ wherein $R^1$ and $R^2$ have the value given under Formula I above to give compounds of Formulas Ia and Ib, respectively. The latter reaction is preferably conducted in excess amine as in the instance of volatile methylamines. The free bases of products of Formula Ia and Ib are isolated by conventional means by removing volatiles and partitioning between dilute aqueous alkali metal base and a solvent such as chloroform or methylene chloride followed by evaporation. The free base may be converted to a pharmaceutically acceptable salt with an appropriate acid and in the case of a quaternary salt with a loweralkyl halide or sulphate and recrystallized by conventional means. The free bases may be recovered from the acid addition salts, usually in a purer form, by again partitioning the salt between aqueous base and a suitable solvent followed by evaporation. As will be realized and as shown in Chart I, the side chain of the intermediate produced is limited to aminomethyl and aminoethyl (n = 2).

In step 6, when it is desirable, a compound prepared in step 5 wherein B is oxygen is sulphurized preferably by refluxing in dry pyridine with phosphorus pentasulphide for several hours. The resulting thione is isolated by cooling the solution and partitioning between a suitable solvent such as chloroform and an aqueous base and evaporating the organic phase and isolating by conventional means.

In step 7, a cyano compound (IIc) prepared in step 4 which is an oxazepinone and thiazepinone is reduced, preferably with hydrogen using Raney nickel catalyst at about 60°C. The primary aminoethyl or aminopropyl compound (n = 2 or 3) produced is isolated by conventional means, preferably as an acid addition salt which may be converted back to the free base by partitioning between a suitable solvent and aqueous base and thereafter drying and evaporating the organic layer.

In step 8 (see Chart V), a primary amine is converted to a secondary or tertiary amine by a choice of reactants. The method provides a route to secondary and tertiary amino compounds of Formula I having n = 3 not afforded by step 5 and, in addition, provides an alternate route to secondary and tertiary amino compounds of Formula I wherein n = 1 or 2. The preparation of dimethylamino derivatives by reaction of primary amine with formaldehyde and formic acid is a conventional method for preparing tertiary dimethyl amines as is reaction of a dihalide to give a heterocyclic amine such as 1-pyrrolidino, piperidino or 4-morpholino. The alternatives employing sodium cyanoborohydride follow the procedures described by R. F. Borch et al., J. Amer. Chem. Soc. *93*, 2897 (1971). The procedure which employs conversion to a trifluoroacetamide is described by J. E. Norlander et al., Tetrahydron Letters, 1978 (50) pp 4987—4990.

In step 9, a 4-benzyloxazepinone or thiazepinone derivative (R = Benzyl) under Formula I excluding primary or secondary amines is converted to the corresponding 4-unsubstituted (R = H) oxazepinone or thiazepinone by reaction with sodium and ammonia and may be isolated as illustrated in Example 68.

Step 10 is optional depending on whether the compound of Formula I is already in the form of a pharmaceutically acceptable salt or whether it is desirable to convert to another salt or whether the free base is desired. To obtain the free base from any addition slat of Formula I, the salt is partitioned between a suitable organic solvent such as chloroform and a dilute aqueous base. The organic layer is dried and condensed to give the free base which is then, if desired, reacted with an acid described above to give the desired salt.

As mentioned above, the preferred steps for reaching the preferred compounds having an ethyl side chain in the 2-position include steps 1 to 3, 5, 6 and 10 of the general process for preparing all the compounds of Formula I. Inasmuch as the compounds having a methyl side chain can be made by the same process, compounds wherein n = 1 are included in the preferred process. These steps of a preferred process are designated A to F corresponding to the numbered steps of the general process with the limitation that n = 1 or 2 and R is other than hydrogen as follows:

| Preferred Process Step Designaton | Corresponding General Step Number with Description Pertaining to n = 1 or 2 |
|---|---|
| A | 1 |
| B | 2 |
| C | 3 |
| D | 5 |
| E | 6 |
| F | 10 |

A preferred process for the preparation of a compound or salt in accordance with the invention therefore comprises the steps of

Step A) halogenating a compound of the formula

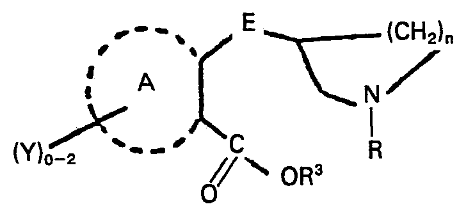

IVb

wherein A represents an aromatic ring system selected from benzene, naphthalene, and pyridine in any of its 4 positions, any of the rings being optionally substituted by one or two Y radicals selected from halo, loweralkyl, loweralkoxy, nitro and trifluoromethyl, and E, R, and n are as defined above and $R^3$ is hydrogen or an acid salt neutralizing ion to give an acid halide of the formula

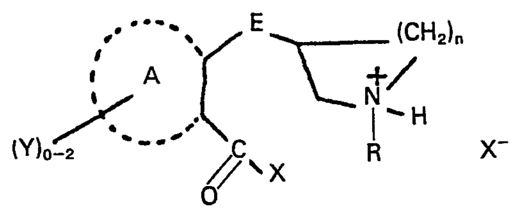

III

or its free base, wherein X is chlorine or bromine and A, E, R, n and Y are the same as the starting values.

Step B) fusing a compound prepared in Step A to give a compound of the formula

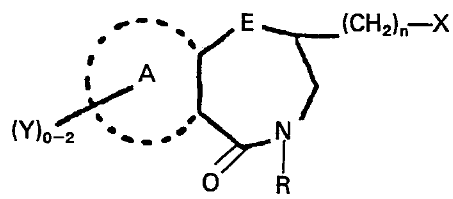

wherein A, E, R, n, X and Y are as defined above in Step A and A now has two of its carbon atoms held mutually with the oxazepine or thiazepine moiety.

Step C) optionally reacting a compound prepared in Step B with a sulphurizing agent to obtain an azepinethione of the formula

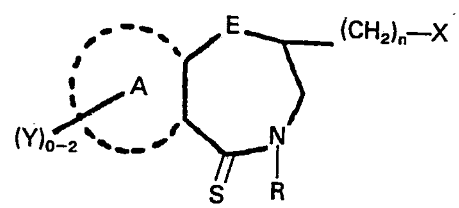

wherein A, E, R, n, X and Y are as defined in step 2,

Step D) reacting a halogen compound prepared in Steps B or C with a compound of the formula

ZH

wherein Z is selected from $-NR^1R^2$, 1H-pyrazol-1-yl and 1H-imidazol-1-yl wherein each of $R^1$ and $R^2$ is selected from hydrogen loweralkyl, cycloclkyl and phenyl-loweralkyl optionally substituted on phenyl with 1 or 2 radicals selected from halo, loweralkyl, loweralkoxy, nitro, trifluoromethyl and cyano, or $R^1$ and $R^2$ taken together with the adjacent nitrogen atom form a heterocyclic residue selected from pyrrolidin-1-yl, 2-methylpyrrolidin-1-yl, 1-pyperidinyl, 4-substituted piperidine-1-yl, 4-morpholinyl, 1-piperazinyl, 4-substituted piperazin-1-yl and 1,2,3,6-tetrahydropyridin-1-yl, to give a compound of the formula

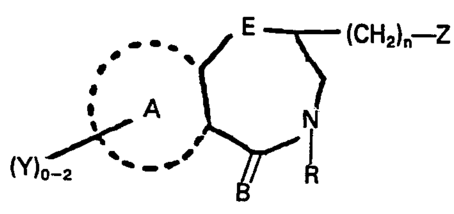

$I_a$

wherein A, E, B, R, n and Y are as defined in step B and Z is the same as in the ZH compound;

Step E) optionally reacting a compound prepared in Step D, wherein B is an oxygen atom with a sulphurizing agent to obtain a compound of the formula

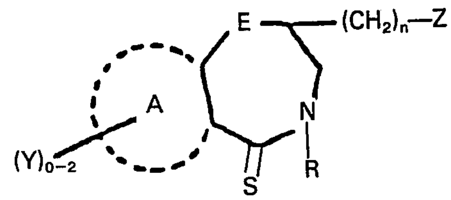

$I_b$

wherein A, E, R, Z, n and Y are as defined above, and

Step F) optionally reacting a free base of any compound prepared in Steps D or E with a) a pharmaceutically acceptable acid, b) a loweralkylhalide, or c) a loweralkyl sulphate to form a pharmaceutically acceptable salt thereof.

Preparation 1

2-(1-Benzyl-3-pyrrolidinyloxy)benzamide

To a suspension of 4.3 g (0.11 mole) of sodium amide in 60 ml of dry toluene was added 19.3 g (0.11 mole) of 1-benzyl-3-pyrrolidinol at a rate to maintain a temperature of 35°C. Stirring was continued at room temperature for 3 hours. To the mixture was added at rapid drop 19 g (0.1 mole) of o-toluenesulphonyl chloride with ice bath cooling to maintain a temperature of 20—30°C. Stirring was continued at room temperature for 2.5 hours and the mixture allowed to stand overnight. The toluene was washed twice with water, dried with sodium sulphate and concentrated.

To a suspension of 5.4 g (0.1 mole)] of sodium methoxide in 50 ml of dimethylformamide in another vessel was added 13.6 g (0.1 mole) of salicylamide in 75 ml of dimethylformamide at a rate to maintain a temperature of 50°C. After stirring 15 minutes, the above prepared sulphonate in 25 ml of dimethylformamide was added dropwise and the solution refluxed 5 hours. The material was partitioned between 500 ml of ethyl acetate and 500 ml of water. The ethyl acetate was extracted with dilute hdyrochloric acid, the acid basified with dilute sodium hydroxide and extracted with ethyl acetate. The organic layer was dried, concentrated, and the residue crystallized twice from isopropyl ether-ethyl acetate. Yield of product was 12.5 g (42%), m.p. 120.5—122°C.

Analysis:

| | | | |
|---|---|---|---|
| Calculated for $C_{18}H_2N_2O_2$: | C, 72.95; | H, 6.80; | N, 9.46 |
| Found: | C, 73.23; | H, 6.78; | N, 9.56 |

Preparation 2

2-(1-Methyl-3-pyrrolidinyloxy)benzamide

To 85.6 g (2.2 moles) of sodium amide in 1.5 litre of dry toluene was added 202 g (2 moles) of 1-methyl-3-pyrrolidinol so as not to exceed a temperature of 50°C. The mixture was then heated to 70°C for 4.5 hours. The mixture was cooled and 381 g (2 moles) of o-toluene-sulphonylchloride was added at a rapid drop while maintaining a temperature of 20—30°C with an ice bath. The mixture was stirred at room temperature for 2.5 hours and washed with water. The toluene solution was dried with sodium sulphate and concentrated. The residue, dissolved in 500 ml of dimethylformamide, was added to a reaction mixture prepared by adding 119 g (2.2 moles) of sodium methoxide and 274 g (2.0 moles) of salicylamide to one litre of dimethylformamide and the mixture was worked up as in preparation 1. Yield of product was 170 g (38%), m.p. 116—118°C.

Analysis:

| | | | |
|---|---|---|---|
| Calculated for $C_{12}H_{16}N_2O_2$: | C, 65.43; | H, 7.32; | N, 12.72 |
| Found: | C, 65.28; | H, 7.28; | N, 12.77 |

Preparation 3

2-[3-(1-Benzyl)pyrrolidinyloxy]benzoic acid

To a solution of 20.3 g (0.52 mole) of sodium hydroxide in 600 ml of ethanol and 400 ml of water was added 150 g (0.51 mole) of 2-[3-(1-benzyl)pyrrolidinyloxy] benzamide and the mixture was stirred at reflux for 48 hours. The mixture was concentrated on the rotary evaporator to one-half volume and the residue was extracted with ethyl acetate to remove unreacted amide. The water layer was filtered and the pH of the filtrate adjusted to 6.5 with hydrochloric acid. the filtrate was concentrated on the rotary evaporator. The residue was dissolved in isopropyl alcohol. The resulting mixture was filtered and the filtrate concentrated. The residue 85.7 g was comprised substantially of the title compound.

Preparation 4

3-[(1-Methyl-3-pyrrolidinyl)oxy]-2-naphthalenecarboxamide

To a cooled solution of 68 g (0.67 mole) of 1-methyl-3-pyrrolidinol and 74 g (0.73 mole) triethylamine in 700 ml of dry benzene was added dropwise 74 g (0.63 mole) of methanesulphonyl chloride. After stirring at

room temperature for 45 min, the mixture was filtered and the filtrate concentrated under reduced pressure and dissolved in 100 ml of dimethylformamide.

To a cooled suspension of 10.8 g (0.45 mole) of sodium hydride in 75 ml of dimethylformamide in another vessel, 84 g (0.45 mole) of 3-hydroxy-2-naphthalenecarboxamide dissolved in 400 ml of dimethylformamide was added dropwise. The above prepared sulphonate solution was added dropwise and the reaction mixture stirred and heated at reflux for 16 hr. The cooled solution was diluted with 1000 ml of water and extracted twice with 500 ml portions of chloroform. The chloroform was washed with water and extracted twice with 500 ml portions of 3N hydrochloric acid. The aqueous extracts were made alkaline with 50% sodium hydroxide and extracted thrice with 500 ml portions of chloroform. After drying over magnesium sulphate, the chloroform was evaporated under reduced pressure affording 27.4 g (22%) of a pale yellow solid. Recrystallized from ethyl acetate, m.p. = 128—130°C.

Analysis:

| | | | |
|---|---|---|---|
| Calculated for $C_{16}H_{18}N_2O_2$: | C, 71.09; | H, 6.71; | N, 10.36 |
| Found: | C, 70.88; | H, 6.68; | N, 10.37 |

Preparation 5

3-[(1-Methyl-3-pyrrolidinyl)oxy]-2-naphthalenecarboxylic acid oxalate [2:1]

To a solution of 21.6 g (0.54 mole) of sodium hydroxide in 500 ml of water was added 74 g (0.27 mole) of 3-[(1-methyl-3-pyrrolinyl)oxy]-2-naphthalenecarboxamide. The solution was heated at reflux for 16 hrs and on cooling, the pH was adjusted to 6.8 with concentrated hydrochloric acid. The resultant solid was separated by filtration and the pH of the filtrate was adjusted to 6.02. The filtrate was concentrated under reduced pressure and the residue boiled in 200 ml of isopropyl alcohol and filtered. The filtrate was again concentrated under reduced pressure to give 69 g (94%) of an amorphous solid. An aliquot was dissolved in isopropanol and treated with oxalic acid. The oxalate salt was recrystallized from ethanol/water, m.p. 209—212°C.

Analysis:

| | | | |
|---|---|---|---|
| Calculated for $C_{17}H_{18}NO_5$: | C, 64.55; | H, 5.74; | N, 4.43 |
| Found: | C, 63.86; | N, 5.68; | N, 4.37 |

Preparation 6

Sodium 2-[(1-methyl-3-pyrrolidinyl)oxy]-3-pyridinecarboxylate

To a stirred suspenson of 6.4 g (0.13 mole) of 50% sodium hydride (mineral oil) in 50 ml of dimethylsulphoxide was added dropwise 6.4 g (0.063 mole) of 1-methyl-3-pyrrolidinol. During addition, the temperature rose from 25°C to 31°C. After 10 minutes, a solution of 10 g (0.063 mole) of 2-chloronicotinic acid in 50 ml of dimethylsulphoxide was added dropwise causing the temperature to rise. When the temperature reached 55°C, it was maintained there by the intermittent use of an ice bath until addition was complete. The mixture was then heated to 55—60°C for 1.5 hr., cooled and filtered. The filter cake was suspended in 100 ml of ethyl acetate and filtered. The solid was recrystallized from ethyl acetate-methanol. Yield of product was 5 g, dec. 240°C. The NMR analysis showed that the compound contained 1/3 mole of sodium acetate as impurity.

Analysis:

| | | | |
|---|---|---|---|
| Calculated for $C_{11}H_{13}N_2O_3Na \cdot 1/3C_2H_3O_2Na$: | C, 51.62; | H, 5.20; | N, 10.32 |
| Found: | C, 51.81; | N, 5.15; | N, 10.39 |

Preparation 7

4-Chloro-2-[(1-methyl-3-pyrrolidinyl)oxy]benzamide

To a solution of 55.5 g (0.55 mole) of triethylamine in 500 ml of dry benzene was added dropwise 50.5 g (0.50 mole) of 1-methyl-3-pyrrolidinol at such a rate as to maintain a temperature of 25—35°C. To the mixture, maintained at 20—50°C, was added dropwise, 57 g (0.50 mole) of methanesulphonyl chloride. After stirring for 1 hr at room temperature, the mixture was filtered and the precipitate washed with 250 ml of hot benzene. The filtrate and wash were combined and concentrated under reduced pressure and the residue dissolved in 200 ml dimethylformamide.

To a cooled suspension of 19.6 g (0.14 mole) of sodium hydride in 100 ml of dimethylformamide in another vessel was added dropwise a solution of 70 g (0.41 mole) of 4-chlorosalicylamide in 200 ml dimethylformamide at a rate such as to maintain a temperature of 20°C. To the resulting reaction mixture was added dropwise the above-prepared sulphonate salt and the mixture was heated at reflux for 19 hrs. The reaction mixture was cooled and diluted with one liter of water. The diluted mixture was extracted three times with 300 ml portions of chloroform. The chloroform extracts were combined and extracted with two 500 ml portions of 3N hydrochloric acid. The combined aqueous extract was made alkaline with 50% sodium hydroxide and extracted three times with 500 ml portions of ethyl acetate. The combined ethyl acetate extract was dried over magnesium sulphate and concentrated under reduced pressure to give 46.5 g (45%) beige solid. The solid was recrystallized from ethyl acetate, m.p. 122—123°C.

Analysis:

| | | | |
|---|---|---|---|
| Calculated for $C_{12}H_{15}N_2ClO_2$: | C, 56.58; | H, 5.94; | N, 10.99 |
| Found: | C, 56.48; | H, 5.96; | N, 10.84 |

Preparation 8

5-Bromo-2-[(1-methyl-3-pyrrolidinyl)oxy]benzamide

To a cooled solution of 101 g (1.0 mole) 1-methyl-3-pyrrolidinol, 111 g (1.1 mole) triethylamine in 1000 ml of dry benzene was added dropwise 114 g (1.0 mole) of methanesulphonyl chloride. The reaction mixture was stirred at room temperature for 1 hour and filtered. The filtrate was concentrated under reduced pressure and dissolved in 100 ml dimethylformamide.

To a cooled suspension of 30 g (0.63 mole) sodium hydride in 100 ml dimethylformamide in another vessel was added dropwise 5-bromosalicylamide (137 g, 0.63 mole) dissolved in 750 ml of dimethylformamide. The above prepared sulphonate was added dropwise and the reaction mixture heated at reflux for 18 hrs. The cooled solution was diluted with 1000 ml of water and extracted thrice with 500 ml portions of chloroform. The chloroform extracts were washed with water and extracted four times with 500 ml portions of 3N hydrochloric acid. The aqueous layer was made alkaline with 50% sodium hydroxide and extracted with chloroform. The chloroform extracts were washed with water, dried over magnesium sulphate and evaporated under reduced pressure to give 52 g (28%) of a yellow solid. The solid was recrystallized from ethyl acetate/chloroform, m.p. 160—162°C.

| | | | |
|---|---|---|---|
| Analysis: Calculated for $C_{12}H_{15}N_2BrO_2$: | C, 48.18; | H, 5.05; | N, 9.36 |
| Found: | C, 48.02; | H, 5.01; | N, 9.22 |

Preparation 9

5-Chloro-2-[(1-methyl-3-pyrrolidinyl)oxy]benzamide hemihydrate

To a cooled suspension of 2.4 g (0.41 mole) sodium hydride in 50 ml of dimethylformamide was added dropwise 17 g (0.1 mole) of 5-chlorosalicylamide dissolved in 50 ml of dimethylformamide at a rate such that the temperature did not exceed 20°C. After addition of the salicylamide was complete, 16.7 g (0.1 mole) of 3-bromo-1-methylpyrrolidine dissolved in 50 ml of dimethylformamide was added dropwise. The reaction mixture was stirred and heated a reflux for 19 hr. The cooled solution was diluted with 250 ml of water and extracted twice with 250 ml portions of chloroform. The chloroform was extracted thrice with 500 ml portions of 3N hydrochloric acid. The aqueous extracts were made alkaline with 50% sodium hydroxide and extracted with ethyl acetate. Drying over magnesium sulphate and evaporation of the ethyl acetate under reduced pressure gave 6 g (23%) of product as a beige solid. The solid was recrystallized from ethyl acetate, m.p. 126—128°C.

Analysis:

| | | | |
|---|---|---|---|
| Calculated for $C_{24}H_{32}N_4Cl_2O_5$: | C, 54.65; | H, 6.11; | N, 10.62 |
| Found: | C, 54.87; | H, 6.12; | N, 10.69 |

Preparation 10

1-[(1-Methyl-3-pyrrolidinyl)oxy]naphthalenecarboxamide

A solution of 118 g (0.63 mole) of 1-hydroxy-2-naphthalenecarboxamide in 250 ml of dimethylsulphoxide was added dropwise to a suspension of 27.6 g (0.69 mole) of 50% sodium hydride (mineral oil) in 250 ml of dimethylsulphoxide. The reaction was exothermic and the temperature rose to 60°C.

In another vessel, 79 g (0.69 mole) of methanesulphonylchloride was added dropwise to a solution of 69.7 g (0.69 mole) of 1-methyl-3-pyrrolidinol and 77 g (0.76 mole) of triethylamine in 500 ml of dry benzene while cooling with an ice bath. The mixture was stirred 15 minutes and filtered. The filter cake was washed with 500 ml of benzene and the benzene filtrates were combined and concentrated on the rotary evaporator to about 200 ml. The residue was added dropwise to the above prepared dimethylsulphoxide solution containing the sodium salt of 1-hydroxy-2-naphthalenecarboxamide while stirring at 75°C. The temperature was maintained at 75°C for 18 hr with external heat. The resulting solution was cooled and an equal volume of water was added. The mixture was extracted with three portions of chloroform. The washes were combined and concentrated. The residue was partitioned between ethyl acetate and dilute hydrochloric acid. The acid layer was made basic with sodium hydroxide and extracted twice with ethyl acetate. The ethyl acetate washed were combined, dried over sodium sulphate and concentrated. The residue was crystallized from ethyl acetate-isooctane. Yield of solid was 55 g (32%). A portion was recrystallized twice from ethyl acetate-isooctane, m.p. 122—129°C.

Analysis:

| | | | |
|---|---|---|---|
| Calculated for $C_{16}H_{18}N_2O_2$: | C, 71.11; | H, 6.71; | N, 10.36 |
| Found: | C, 70.96; | H, 6.71 | N, 10.31 |

Preparation 11

5-Methoxy-2-[(1-methyl-3-pyrrolidinyl)oxy]benzamide

To a solution of 151 g (1.5 mole) 1-methyl-3-pyrrolidinol and 166 g (1.6 mole) triethylamine in 1500 ml of dry benzene was added dropwise 171 g (1.5 mole) of methanesulphonyl chloride with cooling. The reaction mixture was stirred at room temperature for one hour and filtered. The filtrate was concentrated under reduced pressure to give an orange-coloured oil.

In another vessel, to a suspension of 50% sodium hydride/mineral oil (72 g; 1.5 mole) in 150 ml dimethylformamide the sulphonate prepared above and 139 g (0.93 mole) of 5-methoxy salicylamide dissolved in 600 ml dimethylformamide were added dropwise with cooling. The reaction mixture was

heated at reflux for 14 hr. After cooling, the reaction was diluted with 1000 ml of water and extracted three times with 700 ml portions of chloroform. The combined chloroform extracts were washed thrice with water and extracted thrice with 500 ml portions of 3N hydrochloric acid. The aqueous layer was made alkaline and extracted with chloroform. The chloroform extracts were washed thrice with water, dried over magnesium sulphate and evaporated under reduced pressure to give a viscous brown oil. Vacuum distillation of this material yielded a viscous orange oil which was dissolved in chloroform, extracted in acid; made alkaline and extracted into chloroform again. Evaporation of the solvent gave a dark brown oil which solidified under reduced pressure. Three recrystallizations from ethyl acetate gave 10 g of white crystals (4%), m.p. 85—87°C.

Analysis:

| | | | |
|---|---|---|---|
| Calculated for $C_{15}H_{18}N_2O_3$: | C, 62.38; | H, 7.25; | N, 11.19 |
| Found: | C, 62.47; | H, 7.26; | N, 11.20 |

Preparation 12

3-[(1-Methyl-3-pyrrolidinyl)oxy]-4-pyridinecarbonitrile fumarate [1:2]

A solution of 55 g (0.55 mole) of 1-methyl-3-pyrrolidinol in 55 ml of dry dimethylformamide was added dropwise to a suspension of 22 g (0.58 mole) of 60% sodium hydride/40% mineral oil in 300 ml of dimethylformamide. The mixture was stirred at room temperature for one hour and 73 g (0.53 mole) of 3-chloro-4-cyanopyridine in 200 ml of dimethylformamide was added dropwise with mild cooling to maintain a temperature of 30—40°C. The solution was stirred 3 hours an equal volume of water added. The solution was made acidic with dilute hydrochloric acid and extracted with isopropyl ether. The aqueous layer was made basic with sodium hydroxide and extracted 5 times with chloroform. The extracts were combined, dried over sodium sulphate and concentrated. The residue was treated with 50 g of fumaric acid in 400 ml of isopropyl alcohol and 40 ml of water. The resulting crystals (51 g; 21%) were collected. A 2 g sample was recrystallized from methyl isobutyl ketone. Yield of product was 1.5 g, m.p. 172—174°C.

Analysis:

| | | | |
|---|---|---|---|
| Calculated for $C_{19}H_{21}N_5O_9$: | C, 52.42; | H, 4.86; | N, 9.65 |
| Found: | C, 52.40; | H, 4.90; | N, 9.68 |

Preparation 13

1-[(1-Methyl-3-pyrrolidinyl)oxy]-2-naphthalenecarbonitrile oxalate

A solution of 29 g (0.11 mole) of 1-[(1-methylpyrrolidinyl)oxy-2-naphthalenecarboxamide and 38 g (0.32 mole) of thionyl chloride in 150 ml of chloroform was heated to reflux for 6 hr. The solution was poured into ice and made basic with sodium hydroxide. The chloroform layer was separated, dried over sodium sulphate and concentrated. The residue was dissolved in hot isooctane. The solution was treated with charcoal, filtered and concentrated. The residue was dissolved in isopropyl alcohol and oxalic acid was added. The precipitate was recrystallized from isopropyl alcohol-water mixture. Yield of product was 11.5 g (31%), m.p. 176—184°C.

Analysis:

| | | | |
|---|---|---|---|
| Calculated for $C_{18}H_{18}N_2O_5$: | C, 63.15; | H, 5.30; | N, 8.18 |
| Found: | C, 63.00; | H, 5.29; | N, 8.15 |

Preparation 14

3,5-Diiodo-methylsalycilate

To one liter of absolute methanol was added 150 g (0.39 mole) of 3,5-diiodosalicylic acid. Hydrogen chloride was bubbled through the reaction mixture under agitation and refluxed for 3 hrs. The reaction mixture turned cloudy and suddenly a large volume of white crystals precipitated. The mixture was filtered to give, after drying, 136 g (83%) of product, m.p. 198—202°C.

Preparation 15

2-Hydroxy-3,5-diidobenzamide

A stainless steel bomb, cooled with dry ice acetone, was charged with excess liquid ammonia, 3,5-diiodomethylsalicylate and a catalytic amount of sodium hydride. The bomb was sealed and shaken at room temperature for 16 hrs. On cooling again, the contents of the bomb were poured out and excess ammonia allowed to evaporate at room temperature. The product melted 190—195°C with decomposition. Mass spec analysis confirmed molecular weight of the title compound.

Preparation 16

3,5-Diiodo-2-[(1-methyl-3-pyrrolidinyl)oxy]benzamide

Following the procedure of preparation 2, substituting 2-hydroxy-3,5-diiodobenzamide for sacicylamide, the title compound is prepared.

Preparation 17

Sodium-2-[(1-methyl-3-azetidinyl)oxy]-3-pyridinecarboxylate

The title compound is prepared by following the procedure of preparation 6 but substituting 1-methyl-3-azetidinol for 1-methyl-3-p-yrrolidinol.

Preparation 18

4-[(1-Methyl-3-pyrrolidinyl)oxy]-3-pyridine carboxylic acid sodium salt

4-Chloropyridine is reacted with diisopropyl lithium amide and carbon dioxide to give the lithium salt of 3-carboxy-4-chloropyridine which is then reacted with 1-methyl-3-pyrrolidinol as in Preparation 6.

Preparation 19

3-[(1-Methyl-3-pyrrolidinyl)oxy]-2-pyridinecarbonitrile fumarate

2-Carboxamido-3-hydroxypyridine is reacted with

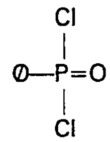

to give 2-cyano-3-chloropyridine which is then reacted with 1-methyl-3-pyrrolidinol as in preparation 12 to give the title compound.

Preparation 20

1-Methyl-3-pyrrolidinethiol acetate (ester) ethanedioate

To a solution of 101 g (1 mole) of 1-methyl-3-pyrrolidinol and 110 g (1.1 mole) of triethylamine in 700 ml of dry benzene was added dropwise 115 g (1 mole) of methanesulphonyl chloride while stirring and cooling with an ice bath. The resulting mixture was stirred for 0.5 hr. and filtered. The filtrate was concentrated on the rotary evaporator to about 200 ml being careful not to overheat. The residue was dissolved in about 150 ml of ethanol.

In a separate vessel, 25.3 g (1.1 mole) of sodium was dissolved in 800 ml of 200 proof ethanol under nitrogen gas sweep. After dissolution was complete, 83.6 g (1.1 mole) of thiolacetic acid was added slowly and the resulting solution was stirred an additional 10 min. The above prepared ethanolic solution of methane sulphonate was added and the resulting solution was heated to 60°C for 20 hrs. The mixture was cooled to 25°C and filtered and the filtrate was concentrated on a rotary evaporator. The residue was dissolved in isopropyl ether and the mixture filtered to remove a small amount of solid. The filtrate was concentrated and the residue distilled to yield 70 g of the free base title ester, b.p. 95—105/15 mm.

A 7 g portion of the free base was treated with 4 g of oxalic acid in isopropyl alcohol and the salt obtained was recrystallized from isopropyl alcohol to give 8.4 g of title product, m.p. 108—111°C.

Preparation 21

1-Methyl-3-pyrrolidinethiol oxalate

A solution of 62 g (0.39 mole) of 1-methyl-3-pyrrolidinethiol acetate (ester) in 200 ml of absolute methanol was treated with a 2 mm sphere of sodium and the resulting solution was distilled at 1 atm. pressure to a pot temperature of 100°C. Vacuum was applied and the pressure was slowly decreased to 100 mm. The residue was distilled to a pot temperature of 130°C, yielding 25 g (56%) of distillate with a boiling point of 95—100°C/100 mm which was the free base of the title compound. A 4 g sample was treated with oxalic and in isopropyl alcohol to give 5.5 g of oxalate salt, m.p. 80—82°C.

Preparation 22

2-[(1-Methyl-3-pyrrolidinyl)thio]-3-pyridinecarboxylic acid

To a stirred suspension of 80 g (2 mole) of 60% sodium hydride (in mineral oil) in 800 ml of dry dimethylformamide, all heated to 60°C and using nitrogen gas flow was added dropwise, a solution of 157.0 g (1 mole) of 2-chloronicotinic acid and 117 g (1 mole) of 1-methyl-3-pyrrolidinethiol in 300 ml of dimethylformamide at a rate which maintained a temperature of 60—67°C. The mixture was heated to 65°C for 6 hr and allowed to stand overnight at room temperature and then filtered. The collected solid was suspended in one litre of isopropyl alcohol and hydrogen chloride was bubbled into the suspension until a pH of 6.2 was reached. The mixture was brought to a boil and filtered. The solid was dissolved in 2 litre of water and extracted with isopropyl ether. The pH was adjusted to 6.0 and the solution was concentrated to a volume of 800 ml and placed in a refrigerator. The resulting solid (85 g) collected by filtration, was a mixture consisting of about 85% of the title compound and 15% sodium chloride. A sample portion of this was crystallized once from ethanol and twice from isopropyl alcohol-water. The recrystallized product decomposed at about 225°C.

Preparation 23

Sodium 2-[(1-Cyclohexyl-3-azetidinyl)oxy]-3-pyridine carboxylate

A solution of 105 g (0.68 mole) of 1-cyclohexyl-3-azetidinol and 106 g (0.68 mole) of 2-chloronicotinic acid in 400 ml of dry dimethylformamide was added at a rapid drop to 52 g (1.35 mole) of 60% sodium hydride/mineral oil suspended in 400 ml of dry dimethylformamide at 60°C. Mild exothermic reaction was noted. After stirring for 2 hr at 60°C, the mixture was filtered. The filter cake was washed with ethylacetate and dried at 80°C/2 mm to give 174 g (86%) of crude title compound.

Intermediate 1

2-(2-Chloroethyl)-2,3-dihydro-4-methyl-1,4-benzoxazepin-5(4H)-one

To 54 g (1.35 mole) of sodium hydroxide in 800 ml of water was added 148 g (0.67 mole) of 2-[(1-methyl-3-pyrrolidinyl)oxy]benzamide and the mixture brought to reflux for 18 hr. The pH was adjusted to 7 with hydrochloric acid and the solution filtered and concentrated. The residue was boiled with 400 ml of isopropanol and filtered. The filtrate was concentrated and the residue (which crystallized) was refluxed with 300 ml of thionyl chloride for 0.5 hr and concentrated *in vacuo*. The residue was dissolved in 300 ml of chloroform and the solvent boiled off *in vacuo*. The residue was redissolved in chloroform, 150 ml of triethyl amine added and the mixture refluxed 1 hr. The solution was concentrated *in vacuo* and the residue partitioned between 400 ml of ethyl acetate, 400 ml of isopropyl ether and 500 ml of dilute hydrochloric acid. The organic layer was washed twice with water and once with dilute sodium hydroxide, dried with sodium sulphate and concentrated. The residue was crystallized from isopropanol-water. Yield of product was 75 g (47%), m.p. 97—107°C.

Analysis:

| | | | |
|---|---|---|---|
| Calculated for $C_{12}H_{14}NO_2Cl$: | C, 60.13; | H, 5.89; | N, 5.84 |
| Found: | C, 60.35; | H, 5.91; | N, 5.65 |

Intermediate 2

4-Benzyl-2-(2-chloroethyl)-2,3-dihydro-1,4-benzoxazepin-5(4H)-one

To 85.7 g (0.29 mole) of 2-[(1-benzyl-3-pyrrolidinyl)oxy]-benzoic acid was added 150 ml of thionyl chloride. The solution stood for 15 min and was then refluxed 30 min. and concentrated *in vacuo*. The residue was twice treated with 250 ml of chloroform and concentrated *in vacuo*. The residue was dissolved in 500 ml of chloroform and 101 g (1 mole) of triethylamine added slowly with stirring. The solution was refluxed 1 hr. and concentrated *in vacuo*. The residue was partitioned between 50% ethyl acetate-50% isopropyl ether and dilute hydrochloric acid. The organic layer was washed with dilute sodium hydroxide and concentrated. The residue was crystallized 5 times from isopropyl ether-ethyl acetate. Yield of product was 23.8 g (26%), m.p. 145.0—147°C.

Analysis:

| | | | |
|---|---|---|---|
| Calculated for $C_{18}H_{18}NO_2Cl$: | C, 68.46; | H, 5.74; | N, 4.44 |
| Found: | C, 68.47; | H, 5.89; | N, 4.32 |

Intermediate 3

2-(2-(Chloroethyl)-2,3-dihydro-4-methylnaphth[2,3-f][1,4]oxazepin-5(4H)-one

To a solution of 21.6 g (0.54 mole) sodium hydroxide in 500 ml of water was added 74 g (0.27 mole) of 3-[1-methyl-3-pyrrolidinyl)oxy]-2-naphthalenecarboxamide and the mixture heated at reflux for 16 hr. The pH was adjusted to 6.8 with concentrated hydrochloric acid, the solution was filtered and concentrated. The residue was boiled with 200 ml of isopropyl alcohol and filtered. The filtrate was concentrated under reduced pressure and the residue dissolved in chloroform. Thionyl chloride (59 g, 0.50 mole) was added and the reaction mixture heated at reflux for 4 hr. After cooling (67 g, 0.67 mole) triethylamine was added dropwise. The mixture was washed sequentially; twice with 3N hydrochloric acid, twice with water, twice with 10% sodium hydroxide, twice with water and dried over magnesium sulphate. Evaporation of the chloroform under reduced pressure gave 44 g (58%) of a viscous dark brown oil. The material was purified by high pressure liquid chromatography (50/50 ethyl acetate/hexane) and recrystallized from isopropyl alcohol to yield brown crystals, m.p. = 101—102°C.

Analysis:

| | | | |
|---|---|---|---|
| Calculated for $C_{16}H_{16}NClO_2$: | C, 66.32; | H, 5.57; | N, 4.83 |
| Found: | C, 66.19; | H, 5.63; | N, 4.77 |

Intermediate 4

2-(2-Chloroethyl)-2,3-dihydro-4-methylpyrido[3,2-f][1,4]-oxazepin-5(4H)-one hydrochloride

Hydrogen chloride gas was bubbled into a suspension of 150 g (0.61 mole) of sodium 2-[(1-methyl-3-pyrrolidinyl)oxy]-3-pyridinecarboxylate in 1 litre of chloroform until a pH of 6 was reached. To the stirred mixture was added 350 g (1.34 mole) of triphenylphosphine and 350 g (2.3 mole) of carbon tetrachloride and the resulting cloudy solution was stirred at reflux for 1.5 hr. About 100 ml of ethanol was added and the heat removed. The solution was stirred for 1 hour while cooling and 200 ml of isooctane was added. The solution was extracted 4 times with a total of 800 ml of dilute hydrochloric acid. The acid extracts were combined, made basic with sodium hydroxide and extracted with chloroform. The chloroform layer was separated and dried over sodium sulphate and concentrated. The residue was dissolved in a mixture of 500 ml each of isopropyl alcohol and isopropyl ether and acidified with ethereal hydrogen chloride. The resulting crystals weighed 82 g (49%). A portion was recrystallized from isopropyl alcohol, m.p. 149—153°C.

Analysis:

| | | | |
|---|---|---|---|
| Calculated for $C_{11}H_{14}N_2O_2Cl_2$: | C, 47.67; | H, 5.09; | N, 10.11 |
| Found: | C, 47.57; | H, 5.18; | N, 10.00 |

Intermediate 5

8-Chloro-2-(2-chloroethyl)-2,3-dihydro-4-methyl-1,4-benzoxazepin-5(4H)-one

To a solution of 10.4 g (0.26 mole) of sodium hydroxide in 150 ml of water was added 32 g (0.13 mole) of 4-chloro-2-[(1-methyl-3-pyrrolidinyl)oxy]benzamide and the mixture was heated at reflux for 24 hr. The reaction mixture was adjusted to pH 6 with concentrated hydrochloric acid and filtered and the filtrate concentrated. The residue was boiled with 100 ml of isopropyl alcohol and the mixture filtered. The filtrate was concentrated and heated at reflux with 98 g (0.83 mole) of thionyl chloride for 1 hr. The excess thionyl chloride was evaporated under reduced pressure. The residue was dissolved in 70 ml of chloroform and the solvent evaporated under reduced pressure. The residue was redissolved in 75 ml of chloroform and 40 ml of triethylamine was added gradually. The mixture was heated at reflux for 1 hr. The solvent was evaporated under reduced pressure to give a dark-brown solid. The solid was dissolved in ethyl acetate and the resulting solution washed twice with 200 ml of water and twice with 250 ml of 20% sodium hydroxide. The organic layer was dried over magnesium sulphate and concentrated under reduced pressure to give 21 g (59%) of dark-brown solid. The solid was recrystallized from isopropyl alcohol to give the title compound, m.p. 85—87°C.

Analysis:

| | | | |
|---|---|---|---|
| Calculated for $C_{12}H_{13}NCl_2O_2$: | C, 52.57; | H, 4.78; | N, 5.11 |
| Found: | C, 52.57; | H, 4.77; | N, 5.04 |

Intermediate 6

7-Bromo-2-(2-chloroethyl)-2,3-dihydro-4-methyl-1,4-benzoxazepine-5(4H)-one

To a solution of 9.6 g (0.24 mole) of sodium hydroxide in 200 ml of water was added 37 g (0.12 mole) of 5-bromo-2-[(1-methyl-3-pyrrolidinyl)oxy]-benzamide and the mixture was heated at reflux for 18 hr. The pH of the mixture was adjusted to 6.7 with concentrated hydrochloric acid solution. The solution was concentrated under reduced pressure and the residue was boiled in 250 ml of isopropyl alcohol for 1 hr. The mixture was filtered and the filtrate was concentrated. The residue was dissolved in chloroform and to the solution was added 28.3 g (0.24 mole) of thionyl chloride. The mixture was heated at reflux for 0.5 hr and cooled to 15°C with an ice bath. To the mixture was added dropwise 26.6 g (0.26 mole) of triethylamine at such a rate that the temperature did not exceed 25°C. The reaction mixture was stirred at room temperature for 1 hr, then washed consecutively with 3N hydrochloric acid, 15% aqueous sodium hydroxide and water. The chloroform layer was dried over magnesium sulphate and concentrated under reduced pressure to give 23 g (60%) of brown solid. A portion of the solid was recrystallized from ethyl acetate-isopropyl ether, m.p. 92—94°C.

Analysis:

| | | | |
|---|---|---|---|
| Calculated for $C_{12}H_{13}NBrClO_2$: | C, 45.24; | H, 4.11 | N, 4.40 |
| Found: | C, 45.61; | H, 4.17; | N, 4.42 |

Intermediate 7

7-Chloro-2-(2-chloroethyl)-2,3-dihydro-4-methyl-1,4-benzoxazepin-5(4H)-one

Hydrogen chloride was bubbled through a solution of 113 g (0.44 mole) 5-chloro-2-[(1-methyl-3-pyrrolidinyl)oxy]benzamide dissolved in 500 ml of glacial acetic acid for 15 min while the reaction was cooled with an ice bath. Butylnitrile (142 g, 1.38 mole) was then added in one portion; the reaction was stirred at room temperature for 16 hr and heated at reflux for an additional 6 hr. The acetic acid was evaporated under reduced pressure, tetrachloroethane was added twice to the residue and evaporated.

The residue was dissolved in chloroform, treated with 163 g (1.38 mole) of thionyl chloride and heated at reflux for 22 hr. The reaction mixture was cooled with an ice bath and 152 g (1.5 mole) of triethylamine was added dropwise at such a rate that the temperature was kept at 25—30°C. The reaction mixture was diluted with 200 ml of chloroform and washed consecutively with 3N hydrochloric acid, water, 10% sodium hydroxide and water. The chloroform was evaporated under reduced pressure to give 40 g of a black, tar-like residue (33%).

An aliquot of this residue was purified on a silica gel column using ethyl acetate as the eluting solvent. Recrystallization from isopropyl alcohol gave beige crystals, m.p. 101—103°C.

Analysis:

| | | | |
|---|---|---|---|
| Calculated for $C_{12}H_{13}NCl_2O_2$: | C, 52.57; | H, 4.78; | N, 5.11 |
| Found: | C, 52.63; | H, 4.83; | N, 5.05 |

Intermediate 8

2-[(2-Chloroethyl)-2,3-dihydro-4-methylnaphth[2,1-f][1,4]oxazepin-5(4H)-one

Hydrogen chloride gas was bubbled into a solution of 8 g (0.03 mole) of 1-[(1-methyl-3-pyrrolidinyl)-oxy]-2-naphthalenecarboxamide in 40 ml of acetic acid for about 2 min. The solution was cooled with an ice bath and 6.1 g (0.06 mole) of n-butyl nitrite was added slowly beneath the surface of the liquid at 12—15°C (about 10 minutes required). The solution was stirred at 25°C for 18 hr and heated on the steam bath for 3 hr. The solution was concentrated on the rotary evaporator. The residue was dissolved in 60 ml of 1,1,2,2-tetrachloroethane which was removed on the rotary evaporator at 0.5 mm/steam temperature.

The residue was dissolved in 75 ml of chloroform and treated with 7 g (0.06 mole) of thionyl chloride

and brought to reflux for 12 hr. The solution was extracted with water (tested acidic) followed by dilute sodium hydroxide, dried over sodium sulphate and concentrated. The residue was crystallized twice from isopropyl ether-ethyl acetate. Yield of product was 3.2 g (37%), m.p. 109—111°C.

Analysis:

| | | | |
|---|---|---|---|
| Calculated for $C_{16}H_{13}NO_2Cl$: | C, 66.32; | H, 5.57; | N, 4.84 |
| Found: | C, 66.15; | H, 5.56; | N, 4.76 |

Intermediate 9

2-(2-Chloroethyl)-2,3-dihydro-7-methoxy-4-methyl-1,4-benzoxazepin-5(4H)-one

To a solution of 19.2 g (0.48 mole) of sodium hydroxide in 500 ml of water was added 60 g (0.24 mole) of 5-methoxy-2-[(1-methyl-3-pyrrolidinyl)oxy]-benzamide and the mixture was heated at reflux for 24 hr. The reaction mixture was cooled and the pH adjusted to 6.8 with concentrated hydrochloric acid. The mixture was concentrated under reduced pressure and the residue was boiled in isopropyl alcohol for 1 hour. The mixture was filtered and the filtrate was concentrated. The residue was dissolved in 500 ml of chloroform and to this solution was added 114 g (0.96 mole) of thionyl chloride. The mixture was heated at reflux for 48 hr, then cooled with an ice/acetone bath. To the mixture was added dropwise 97 g (0.96 mole) of triethylamine at such a rate that the temperature did not exceed 25°C. The reaction solution was washed in sequence with water, 3N hydrochloric acid solution, water, 15% aqueous sodium hydroxide and water and finally dried over magnesium sulphate. The solvent was evaporated under reduced pressure to give a black solid. The solid was purified on a silica gel column using ethyl acetate as the eluting solvent to give on isolation 15 g (23%) of beige colored product, m.p. 98—100°C.

Analysis:

| | | | |
|---|---|---|---|
| Calculated for $C_{13}H_{16}NClO_3$: | C, 57.89; | H, 5.98; | N, 5.19 |
| Found: | C, 57.53; | H, 6.00; | N, 5.16 |

Intermediate 10

2-(2-Chloroethyl)-2,3-dihydro-4-methyl-1,4-benzoxazepine-5(4H)-thione

A mixture of 18.5 g (0.0834 mole) of phosphorus pentasulphide and 18.5 g of potassium sulphide was ground together and added to a solution of 100 g (0.417 mole) of 2-(2-chloroethyl)-2,3-dihydro-4-methyl-1,4-benzoxazepin-5(4H)-one in dry toluene, the mixture refluxed 24 hr. and filtered. The filtrate was concentrated and partitioned between chloroform and dilute sodium hydroxide. The chloroform layer was concentrated and the residue was crystallized several times from ethanol. Yield of product was 55 g (52%), m.p. 105—108°C.

Analysis:

| | | | | |
|---|---|---|---|---|
| Calculated for $C_{12}H_{14}NSOCl$: | C, 56.35; | H, 5.52; | N, 5.48; | S, 12.54 |
| Found: | C, 56.55; | H, 5.47; | N, 5.49; | S, 12.55 |

Intermediate 11

2-(2-Chloroethyl)-2,3-dihydro-4-methylpyrido[3,2-f][1,4]-oxazepine-5(4H)-thione

To a solution of 59 g (0.25 mole) of 2-(2-chloroethyl)-2,3-dihydro-4-methylpyrido[3,2-f][1,4]oxazepin-5(4H)one hydrochloride in 1500 ml of chloroform was added 41.5 g (0.19 mole) of phosphorus pentasulphide and the mixture was heated to reflux for 18 hr. The mixture was filtered and the filtrate was extracted with dilute sodium hydroxide. The chloroform layer was concentrated and the residue was dissolved in 250 ml of boiling isopropyl alcohol. On cooling, 28 g (44%) of yellow solid precipitated. A portion was recrystallized from isopropyl alcohol, m.p. 134—136°C.

Analysis:

| | | | |
|---|---|---|---|
| Calculated for $C_{11}H_{13}N_2ClOS$: | C, 51.46; | H, 5.10; | N, 10.81 |
| Found: | C, 51.35; | H, 5.21; | N, 10.72 |

Intermediate 12

2-(2-Chloroethyl)-2,3-dihydro-4-methylnaphth[2,3-f][1,4]oxazepine-5(4H)-thione

To a solution of 16.6 g (0.06 mole) of 2-(2-chloroethyl)-2,3-dihydro-4-methylnaphth[2,3-f][1,4]oxazepin-5(4H)-one in 150 ml of dry toluene was added a mixture of 8.6 g (0.045 mole) of phosphorus pentasulphide and 8.6 g of potassium sulphide which had been ground together. The reaction mixture was stirred and heated at reflux for 24 hr. The mixture was filtered hot and the filtrate concentrated under reduced pressure. Yellow solid, 6.5 g (35%) was obtained which was recrystallized from ethanol, m.p. 166—168°C.

Analysis:

| | | | |
|---|---|---|---|
| Calculated for $C_{15}H_{15}NClOS$: | C, 62.84; | H, 5.27; | N, 4.58 |
| Found: | C, 62.29; | H, 5.48; | N, 4.47 |

Intermediate 13

8-Chloro-2-(2-chloroethyl)-2,3-dihydro-4-methyl-1,4-benzoxazepin-5(4H)-thione

To a solution of 43 g (0.16 mole) of 8-chloro-2-(2-chloroethyl)-2,3-dihydro-4-methyl-1,4-benzoxazepin-5(4H)-one in 400 ml of dry toluene was added a mixture of 23 g (0.12 mole) of phosphorus pentasulphide and 23 g of potassium sulphide which had been ground together. The reaction mixture was stirred and

heated at reflux for 24 hr. The mixture was filtered hot and the filtrate concentrated under reduced pressure to give 25.5 g (55%) of orange oil which solidified on standing at room temperature. The solid was recrystallized from ethanol, m.p. 105—106°C.

Analysis:

| | | | |
|---|---|---|---|
| Calculated for $C_{12}H_{13}NCl_2OS$: | C, 49.66; | H, 4.52; | N, 4.83 |
| Found: | C, 49.63; | H, 4.53; | N, 4.75 |

Intermediate 14

7-Bromo-2-(2-chloroethyl)-2,3-dihydro-4-methyl-1,4-benzoxazepine-5(4H)-thione

To a solution of 11.0 g (0.035 mole) of 7-bromo-2-(2-chloroethyl)-2,3-dihydro-4-methyl-1,4-benzoxazepin-5(4H)-one in 150 ml of dry toluene was added a mixture of 13.4 g (0.07 mole) of phosphorus pentasulphide and 13.4 g of potassium sulphate which had been ground together. The reaction mixture was heated at reflux for 5 hr under a nitrogen atmosphere. The mixture was filtered hot and the filtrate concentrated under reduced pressure. The residue was dissolved in chloroform. The chloroform solution was washed twice with dilute aqueous sodium hydroxide, dried over magnesium sulphate and concentrated under reduced pressure to give 8.5 g (72%) of yellow solid. The solid was recrystallized from ethanol, m.p. 118—120°C.

Analysis:

| | | | |
|---|---|---|---|
| Calculated for $C_{12}H_{15}NBrClOS$: | C, 43.07; | H, 3.92; | N, 4.18 |
| Found: | C, 43.08; | H, 3.88; | N, 4.12 |

Intermediate 15

2-(2-Chloroethyl)-2,3-dihydro-4-methylnaphth[2,1-f][1,4]oxazepine-5(4H)-thione

A mixture of 9.55 g of phosphorus pentasulphide and 9.5 g of potassium sulphide were ground together and added to a solution of 20.2 g (0.07 mole) of 2-(2-chloroethyl)-2,3-dihydro-4-methylnaphth[2,1-f][1,4]oxazepin-5(4H)-one in 200 ml of dry tolune. The mixture was stirred and heated at reflux for 7 hr. The hot reaction mixture was filtered and the product crystallized from the cooled filtrate. Recrystallization from chloroform gave 18 g (84%) of yellow crystals, m.p. 167—170°C.

Analysis:

| | | | |
|---|---|---|---|
| Calculated for $C_{16}H_{16}NClOS$: | C, 62.84; | H, 5.27; | N, 4.58 |
| Found: | C, 62.85; | H, 5.20; | N, 4.55 |

Intermediate 16

2-(2-Chloroethyl)-2,3-dihydro-4-methylpyrido[4,3-f][1,4]-oxazepin-5(4H)-one hydrochloride

A 49 g (0.11 mole) sample of 3-[(1-methyl-3-pyrrolidinyl)oxy]-4-pyridinecarbonitrile fumarate [1:2] was partitioned between chloroform and a saturated solution of potassium carbonate. The aqueous layer was extracted twice with chloroform. All chloroform extracts were combined, dried and concentrated. The residue was dissolved in 125 ml of *t*-butanol and added to 34 g (0.6 mole) of potassium hydroxide pellets. The mixture was stirred at room temperature for 88 hr and then diluted with 150 ml of toluene. This mixture was filtered and the filtrate concentrated. The residue was dissolved in chloroform, with cooling, and the pH adjusted to 6.0 with hydrogen chloride gas. The resulting mixture was concentrated and 400 ml of dry toluene was added to the residue. The toluene was removed on the rotary evaporator (steam heat/reduced pressure) to remove any water. The residue was dissolved in 400 ml of chloroform and 63 g of triphenylphosphine was added followed by 70 g of carbon tetrachloride. The solution was stirred at reflux for 2 hr and another 30 g of triphenylphosphine added. After an additional hour reflux, 70 g more carbon tetrachloride and 63 g more of triphenylphosphine were added and reflux was continued for 4 hr. The solution was extracted with dilute sodium hydroxide, then concentrated. The residue was partitioned between toluene and dilute hydrochloric acid. The toluene layer was extracted five times with dilute hydrochloric acid. The acid extracts were combined, basified with sodium hydroxide and extracted with chloroform. The chloroform layer was dried over sodium sulphate and concentrated. The residue was chromatographed on a 7 × 25 cm column of silica gel with acetone liquid phase. Free base of the title compound isolated after evaporation amounted to 5.8 g (20%). To a portion of the free base dissolved in isopropyl alcohol was added ethereal hydrogen chloride and isopropyl ether. The resulting crystals were collected and dried, m.p. 188—190°C.

Analysis:

| | | | |
|---|---|---|---|
| Calculated for $C_{11}H_{14}N_2O_2Cl_2$: | C, 47.67; | H, 5.09; | N, 10.11 |
| Found: | C, 48.33; | H, 5.22; | N, 9.73 |

Intermediate 17

2-(2-Chloroethyl)-2,3-dihydro-4-methylpyrido[3,4-f][1,4]oxazepin-5(4H)-one hydrochloride

In the procedure of Intermediate 4, equal molar amounts of sodium 4-[1-methyl-3-pyrrolidinyl)oxy]-3-pyridinecarboxylate was substituted for 2-[(1-methyl-3-pyrrolidinyl)oxy]-3-pyridinecarboxylic acid and the title compound was obtained.

Intermediate 18

2-(2-Chloroethyl)-2,3-dihydro-4-methylpyrido[2,3-f][1,4]oxazepin-5(4H)-one hydrochloride

When the procedure of Intermediate 16, 3-[(1-methyl-3-pyrrolidinyl)oxy]-2-pyridinecarbonitrile fumarate is substituted for 3-[(1-methyl-3-pyrrolidinyl)oxy]-4-pyridinecarbonitrile fumarate, the title compound is obtained.

Intermediate 19

7-Chloro-2-(2-chloroethyl)-2,3-dihydro-4-methyl-1,4-benzoxazepine-5(4H)-thione

To a solution of 20 g (0.07 mole) of 7-chloro-2-(2-chloroethyl)-2,3-dihydro-4-methyl-1,4-benzoxazepin-5(4H)-one in 200 ml of toluene was added a mixture of 9.55 g (0.05 mole) of phosphorus pentasulphide and 9.5 g of potassium sulphide which had been ground together. The reaction mixture was filtered and the filtrate concentrated under reduced pressure to give a yellow solid. Recrystallization from absolute ethanol gave 12.5 g (68%) of the product, m.p. 102—104°C.

Analysis:

| | | | |
|---|---|---|---|
| Calculated for $C_{12}H_{13}NCl_2OS$: | C, 49.66; | H, 4.52; | N, 4.83 |
| Found: | C, 49.62; | H, 4.55; | N, 4.76 |

Intermediate 20

2-(2-Chloroethyl)-7,9-diiodo-2,3-dihydro-4-methyl-1,4-benzoxazepin-5(4H)-one

When in the procedure of Intermediate 1, 3,5-diido-2-[(1-methyl-3-pyrrolidinyl)oxy]-benzamide is substituted for 2-[(1-methyl-3-pyrrolidinyl)oxy]benzamide, the title compound is prepared.

Intermediate 21

2-Chloromethyl-2,3-dihydro-4-methylpyrido[3,2-f][1,4]oxazepin-5(4H)-hydrochloride

When in the procedure of Intermediate 4, an equal molar amount of sodium 2-[(1-methyl-3-azetidinyl)-oxy]-3-pyridinecarboxylate sodium acetate is substituted for sodium 2-[(1-methyl-3-pyrrolidinyl)oxy]-3-pyridinecarboxylate, the title compound is prepared.

Intermediate 22

2-(2-Chloroethyl)-2,3-dihydro-4-methylpyrido[4,3-f][1,4]oxazepine-5(4H)-thione

A solution of 5 g (0.021 mole) of 2-(2-chloroethyl)-2,3-dihydro-4-methylpyrido[4,3-f][1,4]-oxazepin-5(4H)-one and 5.1 g (0.0126 mole) of 2,4-bis(4-methoxyphenyl)-1,3,2,4-dithiodiphosphetane-2,4-disulphide in 100 ml of dry toluene was stirred at reflux for 2.5 hr. The solution was cooled and extracted three times with sodium bicarbonate solution. The toluene layer was dried over sodium sulphate and concentrated. The residue was chromatographed (high pressure liquid chromatograph) using a silica column and ethyl acetate liquid phase. The fraction containing the product was concentrated by evaporation and the residue was crystallized from ethyl alcohol to give 0.6 g (11%) of the title compound.

Intermediate 23

2-(2-Chloroethyl)-2,3-dihydro-7-methoxy-4-methyl-1,4-benzoxazepine-5(4H)-thione

To a solution of 10.3 g (0.04 mole) of 2 - (2 - chloroethyl) - 2,3 - dihydro - 7 - methoxy - 4 - methyl - 1,4 - benzoxazepin - 5(4H) - one in 200 ml of chlorofrom was added a mixture of 5.7 g (0.03 mole) of phosphorus pentasulphide 5.7 g of potassium sulphide which had been ground together. The reaction mixture was stirred and heated at reflux under nitrogen atmosphere for 5 hr. The mixture was filtered hot and the filtrate concentrated under reduced pressure. The residue, an orange solid, was recrystallized from ethanol to give 7.4 g (65%) of product, m.p. 98—100°C.

Analysis:

| | | | |
|---|---|---|---|
| Calculated for $C_{13}H_{16}NClO_2S$: | C, 54.64; | H, 5.65; | N, 4.90 |
| Found: | C, 54.57; | H, 5.67; | N, 4.85 |

Intermediate 24

When in the procedure of Intermediate 2, equal molar amounts of the following are substituted for 2-(1-benzyl-3-pyrrolidinyloxy)benzoic acid:

2-[(1-cyclohexyl-3-pyrrolidinyl)oxy]benzoic acid,
2-[(1-ethyl-3-pyrrolidinyl)oxy]benzoic acid,
2-[(1-isopropyl-3-pyrrolidinyl)oxy]benzoic acid,
2-[[(1-(4-chlorobenzyl)-3-pyrrolidinyl]oxy]benzoic acid,
2-[[1-(4-methylbenzyl)-3-pyrrolidinyl]oxy]benzoic acid,
2-[[1-(3,5-dimethoxybenzyl)-3-pyrrolidinyl]oxy]benzoic acid,
2-[[1-(3-trifluoromethylbenzyl)-3-pyrrolidinyl]oxy]benzoic acid, and
2-[[1-(4-nitrobenzyl)-3-pyrrolidinyl]oxy]benzoic acid,

there are obtained:

a) 2-(2-chloroethyl)-4-cyclohexyl-2,3-dihydro-1,4-benzoxazepin-5(4H)-one,
b) 2-(2-chloroethyl)-2,3-dihydro-4-ethyl-1,4-benzoxazepin-5(4H)-one,
c) 2-(2-chloroethyl)-2,3-dihydro-4-isoprpyl-1,4-benzoxazepin-5(4H)-one,
d) 2-(2-chloroethyl)-4-(4-chlorobenzyl)-2,3-dihydro-1,4-benzoxazepin-5(4H)-one,

e) 2-(2-chloroethyl)-2,3-dihydro-4-(4-methylbenzyl)-1,4-benzoxazepin-5(4H)-one,
f) 2-(2-chloroethyl)-2,3-dihydro-4-(3,5-dimethoxybenzyl)-1,4-benzoxazepin-5(4H)-one,
g) 2-(2-chloroethyl)-,3-dihydro-4-(3-trifluoromethylbenzyl)-1,4-benzoxazepin-5(4H)-one,
h) 2-(2-chloroethyl)-2,3-dihydro-4-(4-nitrobenzyl)-1,4-benzoxazepin-5(4H)-one.

Intermediate 25

When in the procedure of Intermediate 4, equal molar amounts of the following are substituted for sodium 2-[(1-methyl-3-pyrrolidinyl)oxy]-3-pyridine carboxylate:
2-[(1-cyclohexyl-3-pyrrolidinyl)oxy]-3-pyridine carboxylate,
2-[(1-ethyl-3-pyrrolidinyl)oxy]-3-pyridine carboxylate,
2-[1-isopropyl-3-pyrrolidinyl)oxy]-3-pyridine carboxylate,
2-[[1-(4-chlorobenzyl)-3-pyrrolidinyl]oxy]-3-pyridine carboxylate,
2-[[1-(4-methylbenzyl)-3-pyrrolidinyl]oxy]-3-pyridine carboxylate,
2-[[-1-(4-methoxybenzyl)-3-pyrrolidinyl]oxy]-3-pyridine carboxylate,
2-[[1-(3-trifluoromethylbenzyl]-3-pyrrolidinyl]oxy]-3-pyridine carboxylate, and
2-[[1-(4-nitrobenzyl)-3-pyrrolidinyl]oxy]-3-pyridine carboxylate,
there are obtained:
a) 2-(2-chloroethyl)-4-cyclohexyl-2,3-dihydro-pyrido[3,2-f][1,4]oxazepin-5(4H)-one hydrochloride,
b) 2-(2-chloroethyl)-2,3-dihydro-4-ethylpyrido[3,2-f][1,4]-oxazepin-5(4H)one hydrochloride,
c) 2-(2-chloroethyl)-2,3-dihydro-4-isopropylpyrido[3,2-f][1,4]-oxazepin-5(4H)-one hydrochloride,
d) 2-(2-chloroethyl)-4-(4-chlorobenzyl)-2,3-dihydropyrido[3,2-f][1,4]-oxazepin-5(4H)-one hydrochloride,
e) 2-(2-chloroethyl)-2,3-dihydro-4-(4-methylbenzyl)pyrido[3,2-f][1,4]-oxazepin-5(4H)-one hydrochloride,
f) 2-(2-chloroethyl)-2,3-dihydro-4-(4-methoxybenzyl)pyrido[3,2-f][1,4]-oxazepin-5[4H]-one hydrochloride,
g) 2-(2-chloroethyl)-2,3-dihydro-4-(3-trifluoromethylbenzyl)pyrido[3,2-f][1,4]-5(4H)-one hydrochloride, and
h) 2-(2-chloroethyl)-2,3-dihydro-4-(4-nitrobenzyl)pyrido)[3,2-f][1,4]-oxazepin-5(4H)-one hydrochloride.

Intermediate 26

2-(2-Chloroethyl)-2,3-dihydro-4-methylpyrido[3,2-f][1,4]-thiazepin-5(4H)-one

A mixture of 80.75 g (0.34 mole) of 2-[(1-methyl-3-pyrrolidinyl)thio]-3-pyridinecarboxylic acid, 500 ml of chloroform, 200 g of carbon tetrachloride and 178 g (0.68 mole) of triphenylphosphine was stirred at reflux for 2.5 hr. The resulting solution was extracted with one 500 ml and three 125 ml portions of 1N hydrochloric acid. The acid extracts were combined and extracted with isopropyl ether. The aqueous layer was basified with sodium hydroxide and extracted three times with chloroform. The combined chloroform extract was dried over sodium sulphate and concentrated. A portion of the residue was chromatographed on the high pressure liquid chromatograph using a silica column and ethyl acetate. The compound obtained was crystallized from isopropyl ether-isopropyl alcohol, m.p. 97—100°C.

Analysis:

| | | | |
|---|---|---|---|
| Calculated for $C_{11}H_{13}N_2OSCl$: | C, 51.46; | H, 5.10; | N, 10.91 |
| Found: | C, 51.63; | H, 5.12; | N, 10.85 |

Intermediate 27

2-(2-Chloroethyl)-2,3-dihydro-4-methylpyrido[3,2-f][1,4]-thiazepine-5(4H)-thione

A mixture of 4.3 g (0.017 mole) of 2-(2-chloroethyl)-2,3-dihydro-4-methylpyrido[3,2-f][1,4]thiazepin-(4H)-one, 100 ml of toluene and 4.8 g (0.012 mole) of 2,4-bis(4-methoxyphenyl)-1,3,2,4-dithiadiphosphetane-2,4-disulphide was refluxed for 3 hr and then extracted twice with dilute sodium hydroxide. The organic layer was concentrated and the residue chromatographed on the high pressure liquid chromatograph using a silica column and 50% ethyl acetate-50% hexane. The yield of title compound was 2 g, m.p. 160—162°C.

Analysis:

| | | | |
|---|---|---|---|
| Calculated for $C_{11}H_{13}N_2S_2Cl$: | C, 48.43; | H, 4.80; | N, 10.27 |
| Found: | C, 48.46; | H, 4.81; | N, 10.51 |

Intermediate 28

2-(2-Chloroethyl)-2,3-dihydro-4-methylpyrido[3,4-f][1,4]oxazepin-5(4H)-one

A solution of 78 g (0.5 mole) of 4-chloronicotinic acid and 52 g (0.52 mole) of 1-methylpyrrolidinol in 150 ml of dimethylformamide was added to a suspension of 44 g (1.1 mole) of 60% sodium hydride/mineral oil in 800 ml of dimethylformamide at a rate so as to maintain a temperature of 55—70°C (preheated to 55°C). The resulting mixture was heated to 60°C for 4 hr and filtered while hot. The filtrate was concentrated on the rotary evaporator (5 mm/steam bath). The residue was dissolved in 600 ml of water and extracted with isopropyl ether. The pH of the aqueous layer was adjusted to 6 with hydrochloric acid and the solution was concentrated on the rotary evaporator (5 mm/steam bath). The residue was suspended in 800 ml of chloroform and 188 g (1.1 mole) of triphenylphosphine added followed by 250 ml of carbon tetrachloride. The mixture was gently heated to 60°C whereupon the reaction became exothermic and an ice bath was used to maintain a temperature of 60—65°C for about 20 minutes. The ice bath was removed and the mixture was heated to reflux for 3.5 hr and cooled. The solution was extracted with 600 ml of water

followed by two 200 ml portions 1N hydrochloric acid. The acid layer was made basic with sodium hydroxide and extracted three times with chloroform. The chloroform was concentrated and the residue was chromatographed by high pressure liquid chromatography using silica gel and eluting with ethyl acetate. Yield of product was 30 g (25%). The mass spectra and NMR are in agreement with the structure of the title compound.

Intermediate 29

2-(2-Chloroethyl)-2,3-dihydro-4-methylpyrido[3,4-f][1,4]oxazepin-5(4H)-thione monohydrochloride

2-(2-Chloroethyl)-2,3-dihydro-4-methylpyrido[3,4-f][1,4]oxazepin-5(4H)-one, 15 g (0.06 mole), was dissolved in 200 ml of dry toluene and 15 g (0.037 mole) of [2,4-bis(4-methoxyphenyl)-1,3,2,4-dithiaphosphetane -2,4-disulphide was added. The mixture was refluxed for 2.5 hr and the toluene solution decanted. The residue was partitioned between dilute sodium hydroxide and chloroform. The chloroform was dried and concentrated. The residue was chromatographed on a high pressure liquid chromatograph (Waters 500) using a silica column and eluting with ethyl acetate. The fraction containing material of molecular weight 257 was concentrated. The residue in isopropyl alcohol was treated with hydrogen chloride and the resulting crystals were collected. Yield of hydrochloride salt was 0.1 g (0.6%), m.p. 168—171°C.

Analysis:

| | | | |
|---|---|---|---|
| Calculated for $C_{11}H_{14}N_2OSCl_2$: | C, 45.06; | H, 4.81; | N, 9.55 |
| Found: | C, 45.15; | H, 4.98; | N, 9.26 |

Intermediate 30

2,3,4,5-Tetrahydro-4-methyl-5-oxopyrido[3,2-f][1,4]oxazepine-2-propanenitrile

2-(2-Chloroethyl)-2,3-dihydro-4-methylpyrido[3,2-f][1,4]oxazepin-5(4H)-one hydrochloride, 100 g (0.415 mole) was partitioned between dilute aqueous sodium hydroxide (200 ml) and chloroform (200 ml). The organic layer was saved and the aqueous layer extracted with chloroform (3 × 50 ml). The organic layer were combined, dried over sodium sulfate and concentrated by rotary evaporation (70°C, water aspirator). The residue, the free base of the starting hydrochloride, 89 g (0.37 mole) was dissolved in 150 ml of toluene and to the solution was added tetrabutyl ammonium bromide, 9 g (0.027 mole). Saturated aqueous potassium cyanide (100 ml) was then added and the mixture stirred mechanically at reflux. After 2 hr, additional tetrabutyl ammonium bromide, 3 g (0.009 mole) and saturated aqueous potassium cyanide (20 ml) were added and the mixture stirred for 0.75 hr at reflux. The contents of the reaction vessel were extracted with ethyl acetate (3 × 50 ml). (Note: chloroform should be used instead). The organic layer dried over sodium sulphate and concentrated by rotary evaporation (70°C, water aspirator) to $\frac{1}{3}$ the original volume. Upon cooling, crystallization ensued. The crystals were filtered and washed with several portions of ethyl acetate and isopropyl ether. Thirty g (35%) of off-white crystals were collected, m.p. 104—105°C. A sample was recrystallized from ethyl acetate, m.p. 104—105°C.

Analysis:

| | | | |
|---|---|---|---|
| Calculated for $C_{12}H_{15}N_9O_2$: | C, 62.33; | H, 5.67; | N, 18.17 |
| Found: | C 62.06; | H, 5.65 | N, 17.97 |

Intermediate 31

2-(2-Chloroethyl)-4-ethyl-2,3-dihydropyrido[3,2-f][1,4]oxazepin-5(4H)-one hydrochloride

To a stirred suspension of sodium hydride mineral oil (81.45 g of 60% dispersion 2.036 mole) in dimethylsulphoxide (500 ml) heated to 50°C was added dropwise a solution of 2-chloronicotinic acid (142 g, 0.905 mole) and N-ethyl-2-pyrrolidinol (99 g, 0.86 mole) in dimethylsulphoxide (500 ml) at a rate to maintain 55°—60°C (occasional cooling was necessary). After the addition was complete, the mixture was stirred at 50°—60°C for 1.5 hr and allowed to cool. The solid which precipitated was filtered, washed with ethyl acetate and dried.

The dry sodium salt (172.53 g, 0.62 mole) was suspended in chloroform (1 liter). Hydrogen chloride gas was bubbled through the suspension until the pH meter read 5.76. Triphenylphosphine (365.5 g, 1.395 mole) and $CCl_4$ (365.5 g) were added and the mixture stirred at reflux. After 45 minutes, IR showed 95% reaction. Additional triphenylphosphine (100 g, 0.38 mole) and $CCl_4$ (100 g) were added and the solution stirred at reflux an additional 45 min. IR showed >99% reaction. After cooling, the solution was extracted several times with dilute hydrochloric acid (1.5 liter total). The aqueous layer was then made basic with concentrated sodium hydroxide solution and extracted into chloroform (3 × 250 ml). The organic layer was dried over sodium sulphate and concentrated by rotary evaporation (70°C, water aspirator). The residual oil was dissolved in isopropyl alcohol (500 ml) and acidified with hydrogen chloride gas. Upon cooling, an oil was noted and the volume reduced to $\frac{1}{3}$ the original volume. Upon cooling, 70 g (.241 mole, 28%) of pale brown crystals were collected, m.p. 153—155°C.

Analysis:

| | | | |
|---|---|---|---|
| Calculated for $C_{12}H_{15}N_2O_2Cl_2$: | C, 49.50; | H, 5.53; | N, 9.62 |
| Found: | C, 49.64; | H, 5.62; | N, 9.32 |

Intermediate 32

2-(2-Chloroethyl)-4-ethyl-2,3-dihydropyrido[3,2-f][1,4]oxazepin-5(4H)-thione hydrochloride

2-(2-Chloroethyl)-4-ethyl-2,3-dihydropyrido(3,2-f][1,4]oxazepin-5(4H)-one hydrochloride, approximately 50 g, was partitioned between dilute aqueous sodium hydroxide (50 ml) and chloroform (50 m). The organic layer was saved and the aqueous layer extracted with additional methylene chloride (2 × 50 ml). The organic layers were combined, dried over sodium sulphate, filtered and concentrated by rotary evaporation (70°C, water aspirator) yielding 39 g (0.153 mole) of the free base. The free base thus obtained was dissolved in chloroform (1.2 l), and phosphorus pentasulphide (33.9 g, 0.153 mole) was added while stirring. The resulting mixture was heated to reflux for 16 hr. After cooling, the reaction mixture was filtered, washed with dilute aqueous sodium hydroxide (3 × 300 ml), dried over sodium sulphate and concentrated by rotary evaporation (70°C, water aspirator) to a yellow viscous oil. The oil was taken up in isopropyl alcohol (~200 ml) and made acidic with hydrogen chloride gas. Upon cooling, 20 g (43%) of crystals were collected, m.p. 133—135°C.

Analysis:

| | | | |
|---|---|---|---|
| Calculated for $C_{12}H_{16}N_2OSCl_2$: | C, 46.91; | H, 5.25; | N, 9.12 |
| Found: | C, 47.33; | H, 5.38; | N, 9.10 |

Intermediate 33

7-Chloro-2-(2-chloroethyl)-2,3-dihydro-4-methylpyrido[3,2-f][1,4]oxazepin-5-(4H]-one

A sample of 2 -(2 -chloroethyl) -2,3 -dihydro -4 -methylpyrido[3,2 -f][1,4] -oxazepin -5 -(4H) -one hydrochloride (10 g, 136 mole) was dissolved in dimethylformamide (150 ml) and heated to reflux. Sulphuryl chloride (20 g, 0.148 mole) was then added dropwise over a period of 40—50 minutes. The reaction was allowed to stir at reflux for 30 minutes following the addition of $SO_2Cl_2$. After cooling, the contents of the flask were partitioned between water (150 ml) and benzene (150 ml). The benzene layer was saved and the water layer extracted with an additional amount of benzene (2 × 50 ml). The benzene extracts were combined and washed with dilute aqueous potassium hydroxide (2 × 50 ml) followed by dilute aqueous hydrochloric acid (2 × 50 ml). The benzene layer was dried over sodium sulphate concentrated by rotary evaporation (~70°C, water aspirator) yielding 2.61 g of crude material. The crude material was recrystallized from isopropyl ether giving 1.25 g (12.6%) of off-white crystals, m.p. 78—79°C.

Analysis:

| | | | |
|---|---|---|---|
| Calculated for $C_{11}H_{12}N_2O_2Cl_2$: | C, 48.02; | H, 4.40; | N, 10.18 |
| Found: | C, 48.07; | H, 4.53; | N, 10.10 |

Intermediate 34

7-Chloro-2-(2-chloroethyl)-2,3-dihydro-4-methylpyrido[3,2-f][1,4]oxazepine-5(4H)thione

7-Chloro-2-(2-chloroethyl)-2,3-dihydro-4-methylpyrido[3,2-f][1,4]oxazepine-5(4H)-one, 6.0 g (0.22 mole) was suspended in 200 ml of toluene. To this supension was added 2,4-bis(4-methoxyphenyl)-1,3-dithia-2,4-diphosphetane-2,4-disulphide. The mixture was heated to reflux with vigorous stirring for 2 hours. Because the reaction was not complete, an additional amount (3.0 g) of 2,4-bis(4-methoxypenyl)1,3-dithia-2,4-diphosphetane-2,4-disulphide was added and the mixture stirred at reflux for 2 hr and left standing for 56 hr at room temperature. The toluene layer was decanted and washed with 50 ml of dilute aqueous sodium hydroxide and 50 ml of dilute hydrochloric acid. Toluene was removed by rotary evaporation (~80°C, water aspirator). The crude oil was recrystallized from isopropyl alcohol giving 3.5 g (54%) of pale yellow crystals, m.p. 125—127°C.

Analysis:

| | | | |
|---|---|---|---|
| Calculated for $C_{11}H_{12}N_2OSCl_2$: | C, 45.37; | H, 4.15; | N, 9.62 |
| Found: | C, 45.40; | H, 4.20; | N, 9.71 |

Intermediate 35

2-(Chloromethyl)-4-cyclohexyl-2,3-dihydropyrido[3,2-f][1,4]oxazepin-5(4H)-one

A 15 g (0.05 mole) sample of sodium 2-[(1-cyclohexyl-3-azetidinyl)oxy]-3-pyridine carboxylate obtained in Preparation 23 was suspended in 100 ml of chloroform and hydrogen chloride passed in until a pH of 5.8 remained stady. To the stirred mixture was added 18 g of thionyl chloride. The resulting solution was stirred at room temperature for 3 hr. An I.R. specturm showed a peak at 1770 $cm^1$ which is characteristic of acid chloride. Forty millilitres of triethylmamine was added dropwise while cooling to about 25°C with an ice bath. The chloroform solution was stirred an additional 0.5 hr and was extracted with water, dried over sodium sulphate and concentrated. The residue was chromatographed on a 7 × 20 cm silica column using ethanol as the eluent. The desired material was the first to be removed from the column. The ethanolic solution was concentrated and the residue crystallized once from ethyl acetate-isopropyl ether and once from isopropyl alcohol. Yield of title compound was 1 g (7%), m.p. 120—122°C.

Analysis:

| | | | |
|---|---|---|---|
| Calculated for $C_{15}H_{19}N_2O_2Cl$: | C, 61.12; | H, 6.50; | N, 9.50 |
| Found: | C, 61.11; | H, 6.62; | N, 9.32 |

Intermediate 36

2-(3-Chloroethyl)-2,3-dihydro-4-(phenylmethyl)pyrido[3,2-f][1,4]oxazepin-5(4H)one

The title compound was prepared in crude form in the first part of Example 67.

Example 1

2-[2-(Dimethylamino)ethyl]-2,3-dihydro-4-methyl-1,4-benzoxazepin-5(4H)-one hydrochloride

A solution of 9 g (0.2 mole) of dimethylamine in 250 ml of ethanol was added to 24 g (0.1 mole) of 2-(2-chloroethyl)-2,3-dihydro-4-methyl-1,4-benzoxazepin-5(4H)one in a steel bomb. The mixture was heated at 100°C for 18 hrs. The solution was concentrated *in vacuo* and the residue partitioned between ethyl acetate and dilute sodium hydroxide. The ethyl acetate layer was concentrated and the residue comprised substantially of the free base of the title compound was dissolved in methyl isobutyl ketone-isopropanol mixture. The solution was acidified with hydrogen chloride gas to give the title compound, m.p. 188—197°C.

Example 2

2-[2-(Dimethylamine)ethyl]-2,3-dihydro-4-methyl-1,4-benzoxazepine-5(4H)-one

All of the hydrochloride salt obtained in Example 1 was partitioned between chloroform and dilute sodium hydroxide and the chloroform layer concentrated. The residue was crystallized several times from isopropyl ether to give 6 g (21%) of the free base, m.p. 56—76°C.

Analysis:

| | | | |
|---|---|---|---|
| Calculated for $C_{14}H_{20}N_2O_2$: | C, 67.72; | H, 8.12; | N, 11.28 |
| Found: | C, 67.35; | H, 8.16; | N, 11.09 |

Example 3

2,3-Dihydro-4-methyl-2-[2-(4-morpholino)ethyl]-1,4-benzoxazepin-5(4H)-one fumarate (1:1]

To 50 ml of morpholine was added 20 g (0.084 mole) of 2-(2-chloroethyl)-2,3-dihydro-4-methyl-1,4-benzoxazepine-5(4H)-one. The solution was refluxed for 5 hrs and then concentrated *in vacuo*. The residue was dissolved in chloroform, and the solution was washed with dilute sodium hydroxide, dried over sodium sulphate and concentrated *in vacuo*. The residue comprised substantially of the free base of the title compound was reacted with 10.5 g (0.09 mole) of fumaric acid in isopropanol-water. The resulting solid was recrystallized from isopropanol-water to give 21.5 g (64%), m.p. 199—201°C.

Analysis:

| | | | |
|---|---|---|---|
| Calculated for $C_{20}H_{26}N_2O_7$: | C, 59.10; | H, 6.45; | N, 6.89 |
| Found: | C, 58.95; | H, 6.52; | N, 6.88 |

Example 4

4-Benzyl-2,3-dihydro-2-[2-(4-morpholino)ethyl]-1,4-benzoxazepin-5(4H)-one

To 200 ml of morpholine was added 30 g (0.095 mole) of 4-benzyl-2-(2-chloroethyl)-2,3-dihydro-1,4-benzoxazepin-5-(4H)-one. The solution was refluxed for 3 hrs and then concentrated *in vacuo*. The residue was partitioned between dilute sodium hydroxide and chloroform. The chloroform layer was dried over sodium sulphate and concentrated *in vacuo*. The solid obtained was recrystallized from isopropyl ether-ethyl acetate three times to give 15.2 g of solid (43%), m.p. 97—99°C.

Analysis:

| | | | |
|---|---|---|---|
| Calculated for $C_{22}H_{26}N_2O_5$: | C, 72.10; | H, 7.15; | N, 7.64 |
| Found: | C, 72.25; | H, 7.22; | N, 7.64 |

Example 5

4-Benzyl-2,3-dihydro-2-[2-(methylamino)-3-ethyl]-1,4-benzoxazepin-5(4H)-one fumarate [1:1]

A solution of 5.95 g (0.19 mole) of monomethylamine in 200 ml of ethanol was added to 30 g (0.095 mole) of 4 - benzyl - 2 - (2 - chloroethyl) - 2,3 - dihydro - 1,4 - benzoxazepin - 5(4H) - one in a steel bomb. The mixture was heated at 100°C for 16 hr. The solution was concentrated *in vacuo* and the residue partitioned between chloroform and dilute sodium hydroxide. The chloroform layer was concentrated and the residue comprised substantially of the free base of the title compound was dissolved in isopropanol and reacted with fumaric acid to give the fumarate. The salt was dried under vacuum at 100°C until entrapped isopropyl alcohol was removed, m.p. 178—81°C.

Analysis:

| | | | |
|---|---|---|---|
| Calculated for $C_{23}H_{26}N_2O_5$: | C, 64.77; | H, 6.15; | N, 6.57 |
| Found: | C, 64.87; | H, 6.20; | N, 6.62 |

Example 6

2-[2-(Dimethylamino)ethyl]-2,3-dihydro-4-methyl-1,4-benzoxazepine-5(4H)thione hydrochloride [1:1]

To a solution of 7.2 g (0.16 mole) of dimethylamine in 350 ml of absolute ethanol was added 20.4 g (0.08 mole) of 2-(2-chloroethyl)-2,3-dihydro-4-methyl-1,4-benzoxazepine-5(4H)thione. The solution was heated in a steel bomb for 18 hr at 100°C and then concentrated. The residue was partitioned between chloroform and dilute sodium hydroxide. The chloroform layer was dried over sodium sulphate and

concentrated. The solid comprised substantially of the free base of the title compound was reacted with hydrogen chloride gas in ethanol to give the hydrochloride salt. The salt was recrystallized from ethanol and dimethylformamide folloed by three rerystallizations from ethanol to give 7.5 g (28%), m.p. 233—236°C.

Analysis:

| | | | |
|---|---|---|---|
| Calculated for $C_{14}H_{21}N_2SOCl$: | C, 55.90; | H, 7.04; | N, 9.32 |
| Found: | C, 55.72; | H, 7.26; | N, 8.94 |

Example 7

4-Benzyl-2-[2-(dimethylamino)ethyl]-2,3-dihydro-1,4-benzoxazepin-5(4H)-one monohydrate

Following the procedure of Example 1, 4-benzyl-2-(2-chloroethyl)-2,3-dihydro-1,4-benzoxazepin-5(4H)-one and dimethylamine were reacted and the free base of the title compound was obtained in the concentrated residue. Recrystallization from ethanol-water gave the product, m.p.: 75—77°C.

Analysis:

| | | | |
|---|---|---|---|
| Calculated for $C_{20}H_{26}N_2O_5$: | C, 70.13; | H, 7.65; | N, 8.21 |
| Found: | C, 70.02; | H, 7.53; | N, 8.25 |

Example 8

2,3-Dihydro-4-methyl-2-[2-(4-morpholino)ethyl]-1,4-benzoxazepine-5(4H)-thione hydrochloride [1:1]

A solution of 20.4 g (0.08 mole) of 2,3-dihydro-4-methyl-2-(2-chloroethyl)-1,4-benzoxazepin-5(4H)-thione in 60 ml of morpholine was refluxed for 5 hr then concentrated. The residue was partitioned between dilute sodium hydroxide and chloroform. The chloroform layer was dried over sodium sulphate and concentrated to give a residue comprised substantially of the free base of the title compound. The hydrochloride salt was prepared in methyl isobutyl ketone-dimethylformamide solution with hydrogen chloride gas. The salt was recrystallized from ethanol-dimethylformamide to give 14 g solid (51%), m.p. 253—256°C.

Analysis:

| | | | |
|---|---|---|---|
| Calculated for $C_{15}H_{25}N_2SO_2Cl$: | C, 56.04; | H, 6.76; | N, 8.17 |
| Found: | C, 55.73; | H, 6.63; | N, 7.97 |

Example 9

2-[2-(Dimethylamino)ethyl]-2,3-dihydro-4-methylnaphth[2,3-f][1,4]-oxazepin-5(4H)-one oxalate [1:1]

A steel bomb was charged with 5.0 g (0.017 mole) of 2-(2-chloroethyl)-2,3-dihydro-4-methylnaphth[2,3-f][1,4]oxazepin-5(4H)-one, 50 ml of absolute ethanol and 3.78 g (0.034 mole) of dimethylamine as 40% aqueous solution. The bomb was heated at 100°C for 16 hr. Volatiles were removed under reduced pressure and the residue patitioned between chloroform and 15% aqueous sodium hydroxide. The chloroform layer was washed twice with water, dried over magnesium sulphate and concentrated under reduced pressure to give 2.7 g (54%) of viscous yellow oil comprised substantially of the free base of the title compound. The oil was dissolved in isopropyl alcohol and reacted with oxalic acid. The oxalate salt was recrystallized from ethanol-water, m.p. 192—194°C.

Analysis:

| | | | |
|---|---|---|---|
| Calculated for $C_{20}H_{24}NO_6$: | C, 61.84; | H, 6.23; | N, 7.21 |
| Found: | C, 61.41; | H, 6.27; | N, 7.09 |

Example 10

2-[2-(Dimethylamino)ethyl]-2,3-dihydro-4-methylpyrido[3,2-f][1,4]-oxazepin-5(4H)-one fumarate [2:3]

To 90 g (0.8 mole) of 40% aqueous dimethylamine in a steel bomb was added 25 g (0.09 mole) of 2 - (2 - chloroethyl) - 2,3 - dihydro - 4 - methylpyrido[3,2 - f][1,4] - oxazepin - 5(4H) - one hydrochloride. The mixture was heated to 100°C for 15 hr under mild agitation. The mixture was partitioned using dilute sodium hydroxide and two chloroform extractions. The chloroform layers were combined and concentrated. The residue comprised substantially of the free base of the title compound was dissolved in 200 ml of isopropyl alcohol and 9 g of oxalic acid added. The oxalate salt was recrystallized from 95% ethanol to give 18 g. The oxalate salt was then converted to the free base by partitioning between chloroform and dilute sodium hydroxide and evaporating the chloroform layer. The residue, the free base of the title compound, was dissolved in isopropyl alcohol and reacted with fumaric acid to give 13 g of white solid (34%), m.p. 146—148°c.

Analysis:

| | | | |
|---|---|---|---|
| Calculated for $C_{19}H_{25}N_5O_8$: | C, 53.90; | H, 5.90; | N, 9.92 |
| Found: | C, 53.,76; | H, 6.02; | N, 9.96 |

Example 11

2-[2-(Dimethylamino)ethyl]-2,3-dihydro-4-methylpyrido[3,2-f][1,4]-oxazepine-5(4H)-thione fumarate [1:1], ethanol [2:1]

To a solution of 32.8 g (0.29 mole) of 40% aqueous dimethylamine and 100 ml of ethanol in steel bomb was added 15 g (0.058 mole) of 2 - (2 - chloroethyl) - 2,3 - dihydro - 4 - methylpyrido[3,2 - f][1,4] -

oxazepine - 5(4H) - thione. The mixture was heated to 100°C for 18 hr under mild agitation. The solution was cooled and partitioned between chloroform and dilute sodium hydroxide. The chloroform layer was dried over sodium sulphate and concentrated. The residue comprised substantially of the free base of the title compound was dissolved in isopropyl alcohol and reacted with 7 g of fumaric acid. The fumarate salt was recrystallized from isopropyl alcohol to give 19 g (86%), m.p. 105—129°C. A 14 g sample of the salt was recrystallized from ethanol to give 10.5 g yellow solid, m.p. 103—118°C. The NMR spectra indicates the crystals contain ½ mole ethanol.

Analysis:

| | | | |
|---|---|---|---|
| Calculated for $C_{36}H_{52}N_6O_{11}S_2$: | C, 53.45; | H, 6.48; | N, 10.39 |
| Found: | C, 53.07; | H, 6.53; | N, 10.23 |

Example 12

2-[2-(Dimethylamino)ethyl]-2,3-dihydro-4-methylpyrido[3,2-f][1,4]-oxazepine-5(4H)-thione fumarate [1:1]

To a solution of 113 ml (1.0 mole) of 40% aqueous dimethylamine and 326 ml of ethanol in a steel bomb was added 48.4 g (0.189 mole) of 2 - (2 - chloroethyl) - 2,3 - dihydro - 4 - methylpyrido[3,2 - f[1,4] - oxazepine - 5(4H) - thione. The mixture was heated at 100°C for 14 hr. The ethanol was removed in a rotary evaporator leaving some water in the residue. The residue was dissolved in 200 ml of methylene chloride and washed with three 100 ml portions of 20% aqueous potassium carbonate solution. The combined aqueous layers were extracted with three 150 ml portions of methylene chloride. Methylene chloride solutions were combined and treated with charcoal. Charcoal was filtered off and the filtrate was evaporated to give an oil. The oil was dissolved in 215 ml isopropyl alcohol and the solution was heated to a slow boil. A solution of 21.9 g (0.19 mole) of fumaric acid in 150 ml of boiling methanol was added to the isopropyl alcohol solution. Crystalline solid was obtained weighing 63.4 g (88%). The solid was recrystallized from hot 200 proof ethyl alcohol. The crystals were filtered off and triturated in isopropyl ether at room temperature and again separated by filtering. After drying in a vacuum oven overnight at 85°C, crystals in the amount of 72.45 g (79%), m.p. 130—133°C, were obtained.

Analysis:

| | | | |
|---|---|---|---|
| Calculated for $C_{17}H_{23}N_3O_5S$: | C, 53.53; | H, 6.08; | N, 11.02 |
| Found: | C, 53.23; | H, 6.11; | N, 10.64 |

Example 13

4-Benzyl-2,3-dihydro-2-[2-(4-morpholino)ethyl]-1,4-benzoxazepine-5(4H)-thione

To a suspension of a finely ground mixture of 2.9 g (0.013 mole) of phosphorus pentasulphide and 2.9 g of potassium sulphide in 75 ml of dry toluene was added 12 g (0.033 mole) of 4-benzyl-2,3-dihydro-2-[2-(4-morpholino)ethyl]-1,4-benzoxazepine-5(4H)-one. The mixture was stirred at reflux for 10 hr and filtered. The filtrate was concentrated and the residue crystallized from isopropyl ether-toluene to give 2.54 g (20%), m.p. 236—238°C.

Analysis:

| | | | |
|---|---|---|---|
| Calculated for $C_{22}H_{26}N_2O_2S$: | C, 69.08; | H, 6.85; | N, 7.32 |
| Found: | C, 69.60; | H, 6.96; | N, 7.15 |

Example 14

2,3-Dihydro-4-methyl-2-[2-(methylamino)ethyl]-1,4-benzoxazepin-5(4H)-one fumarate [1:1]

Following the procedure of Example 5, 50 g (0.21 mole) of 2-(2-chloroethyl)-2,3-dihydro-4-methyl-1,4-benzoxazepin-5(4H)-one and 13.0 g (0.42 mole) of monomethylamine (in 400 ml ethanol) were reacted to give the free base of the title compound which was reacted with fumaric acid to give, after isolation and recrystallization from ethyl alcohol, 17 g (23%) of the title compound, m.p. 154—156°C.

Analysis:

| | | | |
|---|---|---|---|
| Calculated for $C_{17}H_{22}N_2O_6$: | C, 58.27; | H, 6.33; | N, 8.00 |
| Found: | C, 58.34; | H, 6.52; | N, 7.82 |

Example 15

2,3-Dihydro-4-methyl-2-[2-(methylamino)ethyl]-1,4-benzoxazepin-5(4H)-one

2,3-Dihydro-4-methyl-2-[2-(methylamino)ethyl]-1,4-benzoxazepin-5(4H)-one fumarate was converted back to the free base by partitioning in dilute sodium hydroxide and chloroform. Evaporation of the chloroform layer and distilling, b.p. 182°/0.2 mm, gave 4.3 g of the product.

Analysis:

| | | | |
|---|---|---|---|
| Calculated for $C_{13}H_{18}N_2O_2$: | C, 66.64; | H, 7.74; | N, 11.96 |
| Found: | C, 66.48; | H, 7.69; | N, 11.88 |

Example 16

2,3-Dihydro-2-[2-(4-hydroxy-4-phenyl)-piperidinylethyl]-4-methyl-1,4-benzoxazepine-5(4H)-thione (and hydrochloride salt)

A suspension of 10.7 g (0.078 mole) of potassium carbonate, 13.7 g (0.078 mole) of 4-hydroxy-4-phenypiperidine and 19.8 g (0.078 mole) of 2 - (2 - chloroethyl) - 2,3 - dihydro - 4 - methyl - 1,4 -

benzoxazepine - 5(4H) - thione in 200 ml of n-butanol was refluxed overnight. The mixture was filtered and the filtrate concentrated *in vacuo*. The residue was dissolved in ethanol-ligroin and reacted with hydrogen chloride gas to give the hydrochloride salt which was recrystallized from ethanol-dimethylformamide. The hydrochloride salt was converted back to the free base by partitioning in chloroform and dilute sodium hydroxide and evaporating the chloroform. Recrystallization twice from isopropyl alcohol gave 9.27 g (30%) product free base, m.p. 142—148°C.

Analysis:

| | | | |
|---|---|---|---|
| Calculated for $C_{23}N_{28}N_2O_2S$: | C, 69.66; | H, 7.12; | N, 7.07 |
| Found: | C, 69.78; | H, 7.18; | N, 7.00 |

Example 17

2-3-Dihydro-4-methyl-2-[2-[1-(4-phenyl-1,2,3,6-tetrahydro)pyridinyl]ethyl]-1,4-benzoxazepine-5(4H)thione

A suspension of 24.3 g (0.176 mole) of potassium carbonate, 11.5 g (0.059 mole) of 4-phenyl-3,4-tetrahydropyridine and 15 g (0.059 mole) of 2-(2-chloroethyl)-2,3-dihydro-4-methyl-1,4-benzoxazepine-5(4H)-thione and enough n-butanol to form a slurry were refluxed for 72 hr. The reaction mixture was filtered hot and the filtrate cooled to room temperature and refiltered. The last filtrate was concentrated and the residue dissolved in ethyl acetate. The crystals obtained on cooling were recrystallized from ethyl acetate to give 7 g of product (31%), m.p. 153—155°C.

Analysis:

| | | | |
|---|---|---|---|
| Calculated for $C_{23}H_{26}N_2OS$: | C, 72.98; | H, 6.92; | N, 7.40 |
| Found: | C, 73.36; | H, 7.01; | N, 7.47 |

Example 18

8-Chloro-2-[2-(dimethylamino)ethyl]-2,3-dihydro-4-methyl-1,4-benzoxazepine-5(4H)-thione hydrochloride [1:1]

A solution of 9.8 g (0.04 mole) of 8-chloro-2-(2-chloroethyl)-2,3-dihydro-4-methyl-1,4-benzoxazepine-5(4H)-thione in 50 ml of absolute ethanol and 10 ml of a 40% aqueous solution of dimethylamine were mixed and heated in a steel bomb at 100°C for 16 hr. The ethanol was evaporated under reduced pressure and the residue dissolved in chloroform and partitioned with 10% sodium hydroxide solution. The chloroform layer was evaporated under reduced pressure to give an amorphous solid. The solid was dissolved in 6N hydrochloric acid and the solution washed with ethyl acetate. The aqueous layer was basified with 50% sodium hydroxide and extracted with ethyl acetate. The ethyl acetate layer was evaporated under reduced pressure to give a viscous oil comprised substantially of the free base of the title compound which was dissolved in absolute ethanol and reacted with ethereal hydrogen chloride. The hydrochloride salt was recrystallized from ethanol to give 30 g (25%) product, m.p. 196—199°C.

Analysis:

| | | | |
|---|---|---|---|
| Calculated for $C_{14}H_{20}N_2Cl_2OS$: | C, 50.15; | H, 6.01; | N, 8.35 |
| Found: | C, 50.15; | H, 6.18; | N, 8.07 |

Example 19

8-Chloro-2-[2-(dimethylamino)ethyl]-2,3-dihydro-4-methyl-1,4-benzoxazepin-5(4H)-one oxalate ([1:1]

A solution of 10 g (0.037 mole) of 8-chloro-2-(2-chloroethyl)-2,3-dihydro-4-methyl-1,4-benzoxazepin-5(4H)-one in 50 ml of absolute ethanol and 10 ml of 40% aqueous solution of dimethylamine were mixed and heated in a steel bomb at 100°C for 16 hr. The solution was concentrated under reduced pressure and the residue dissolved in chloroform and partitioned with 15% sodium hydroxide (2 washes). The chloroform layer was dried over magnesium sulphate and evaporated under reduced presure to give an oil, comprises substantially of the free base of the title compound. The oil was dissolved in absolute ethanol and reacted with oxalic acid. The oxalate salt was recrystallized from ethanol in the amount of 4 g (38%), m.p. 198—201°C.

Analysis:

| | | | |
|---|---|---|---|
| Calculated for $C_{15}H_{21}N_2ClO_5$: | C, 51.55; | H, 5.68; | N, 7.51 |
| Found: | C, 51.07; | H, 5.69; | N, 7.43 |

Example 20

7-Bromo-2-[2-(dimethylamino)ethyl]-2,3-dihydro-4-methyl-1,4-benzoxazepin-5(4H)-one oxalate [1:1]

To a solution of 3.0 g (0.01 mole) of 7-bromo-2-(2-chloroethyl)-2,3-dihydro-4-methyl-1,4-benzoxazepin-5(4H)-one in 50 ml of absolute ethanol was added 2.2 ml of a 40% aqueous solution of dimethylamine. The reaction mixture was heated in a stainless steel bomb at 100°C for 16 hr and concentrated under reduced pressure. The residue was partitioned between chloroform and 15% sodium hydroxide solution. The chloroform layer was separated and extracted with 3N aqueous hydrochloric acid. The acid layer was basified with 50% aqueous sodium hydroxide and extracted with chloroform. The chloroform was evaporated under reduced pressure to give 2.4 g (73%) viscous brown oil, the free base of the title compound. The oil was dissolved in isopropyl alcohol and reacted with oxalic acid. The oxalate salt was recrystallized from isopropyl alcohol/water to give the title salt, m.p. 192—194°C.

Analysis:

| | | | |
|---|---|---|---|
| Calculated for $C_{15}H_{21}O_5BrN_2$: | C, 46.06; | H, 5.07; | N, 6.71 |
| Found: | C, 46.00; | H, 5.10; | N, 6.68 |

Example 21

2-[2-(Dimethylamino)ethyl]-2,3-dihydro-4-methylnaphth[2,1-f][1,4]oxazepin-5(4H)-one oxalate [1:1]

A solution of 8 g (0.028 mole) of 2 - (2 - chloroethyl) - 2,3 - dihydro - 4 - methylnaphth[2,1 - f][1,4]oxazepin - 5(4H) - one and 6.2 g of 40% dimethylamine (0.055 mole) in 100 ml of ethanol was heated in a steel bomb to 100°C for 18 hr. The resulting solution was partitioned between methylene chloride and dilute sodium hydroxide solution. The methylene chloride layer was dried over sodium sulphate and concentrated. The residue comprised substantially of the free base of the title compound was dissolved in isopropyl alcohol and reacted with 2.6 g oxalic acid. The oxalate salt obtained was recrystallized from isopropyl alcohol in water, m.p. 206—209°C.

Analysis:

| | | | |
|---|---|---|---|
| Calculated for $C_{20}H_{24}N_2O_6$: | C, 61.85; | H, 6.23; | N, 7.21 |
| Found: | C, 61.61; | H, 6.26; | N, 7.13 |

Example 22

When in the procedure of Example 10 equal molar amounts of the following are substituted for 2 - (2 - chloroethyl) - 2,3 - dihydro - 4 - methylpyrido[3,2 - f][1,4] - oxazepin - 5(4H) - one hydrochloride:
2-(2-chloroethyl)-2,3-dihydro-4-methylpyrido[4,3-f][1,4]-oxazepin-5(4H)-one hydrochloride,
2-(2-chloroethyl)-2,3-dihydro-4-methylpyrido[3,4-f][1,4]-oxazepin-5(4H)-one hydrochloride, and
2-(2-chloroethyl)-2,3-dihydro-4-methylpyrido[2,3-f][1,4]-oxazepin-5(4H)-one hydrochloride, there are obtained:
2-[2-(dimethylamino)ethyl]-2,3-dihydro-4-methylpyrido[4,3-f][1,4]-oxazepin-5(4H)-one fumarate,
2-[2-(dimethylamino)ethyl]-2,3-dihydro-4-methylpyrido[3,4-f][1,4]-oxazepin-5(4H)-one fumarate, and
2-[2-(dimethylamino)ethyl]-2,3-dihydro-4-methylpyrido[2,3-f][1,4]-oxazepin-5(4H)-one fumarate.

Example 23

2-[2-(Dimethylamino)ethyl]-2,3-dihydro-4-methylpyrido[4,3-f][1,4]-oxazepine-5(4H)-thione hydrochloride [2:3]

To a solution of 0.5 g (0.002 mole) of 2-(2-chloroethyl)-2,3-dihydro-4-methylpyrido[4,3-f][1,4]-oxazepine-5(4H)-thione in 20 ml of ethyl alcohol was added 2 ml of 40% aqueous dimethylamine. The mixture was heated in a steel bomb to 100°C for 14 hr. The resulting solution was filtered and concentrated. The residue was dissolved in isopropyl alcohol and a few drops of ethereal hydrogen chloride were added. The hydrochloride salt crystals were recrystallized by dissolving in ethyl alcohol and boiling while replacing the ethyl alcohol with isopropyl alcohol. The yield of product was 0.3 g (47%), m.p.: decomp. above 200°C.

Analysis:

| | | | |
|---|---|---|---|
| Calculated for $C_{25}H_{41}N_5O_2S_2Cl_5$: | C, 48.78; | H, 6.46; | N, 13.13 |
| Found: | C, 49.34; | H, 6.47; | N, 13.03 |

Example 24

2-[2-(Diethylamino)ethyl]-2,3-dihydro-4-methylpyrido[3,2-f][1,4]oxazepine-5(4H)-thione

2-(2-Chloroethyl)-2,3-dihydro-4-methylpyrido[3,2-f][1,4]-oxazepine-5(4H]-thione and diethylamine in ethanol are heated together to obtain the title compound.

Example 25

2-[2-(Dimethylamino)ethyl]-2,3-dihydro-4-methylnaphth[2,3-f][1,4]-oxazepine-5(4H)thione oxalate [1:1] hemihydrate

To a solution of 15 g (0.05 mole) of 2-(2-chloroethyl)-2,3-dihydro-4-methylnaphth[2,3-f][1,4]-oxazepine-5(4H)-thione in 50 ml of absolute ethanol was added 10 ml of a 45% aqueous solution of dimethylamine. The solution was heated in a steel bomb for 16 hr. The ethanol was evaporated under reduced pressure and the residue partitioned between chloroform and 15% aqueous sodium hydroxide. The chloroform layer was separated and extracted with 3N aqueous hydrochloric acid. The acid layer was basified with 50% aqueous sodium hydroxide and extracted with chloroform. The chloroform solution was concentrated under reduced pressure and the residue was dissolved in isopropyl alcohol and reacted with oxalic acid. The salt was recrystallized from isopropyl alcohol and water to give the title compound, m.p. 115—118°C.

Analysis:

| | | | |
|---|---|---|---|
| Calculated for $C_{40}H_{50}N_4O_{11}S_2$: | C, 58.09; | H, 6.09; | N, 6.77 |
| Found: | C, 58.42; | H, 5.85; | N, 6.70 |

Example 26

2-[2-(Dimethylamino)ethyl]-2,3-dihydro-7,9-diiodo-4-methyl-1,4-benzoxazepin-5(4H)-one

Utilizing the procedures of Example 1 and 2 and substituting 2 - (2 - chloroethyl) - 7,9 - diiodo - 4 -

methyl - 2,3 - dihydro - 4 - methyl - 1,4 - benzoxazepin - 5(4H) - one for 2 - (chloroethyl) - 4 - methyl - 2,3 - dihydro - 1,4 - benzoxazepin - 5(4H) - one, the title compound is obtained.

Example 27

7-Chloro-2-[2-(dimethylamino)ethyl]-2,3-dihydro-4-methyl-1,4-benzoxazepin-5(4H)-one oxalate [1:1]

To a solution of 9.0 g (0.033 mole) of 7-chloro-2-(2-chloroethyl)-2,3-dihydro-4-methyl-1,4-benzoxazepin-5(4H)-one in 50 ml absolute ethanol was added 7 g (0.066 mole) of a 45% aqueous solution of dimethylamine. The solution was heated in a stainless steel bomb at 100°C for 14 hr. The reaction mixture was concentrated under reduced pressure and the residue was partitioned between chloroform and 15% aqueous sodium hydroxide. The chloroform layer was separated and evaporated under reduced pressure to give a viscous brown oil. The oil was dissolved in isopropyl alcohol and oxalic acid added. Recrystallization from isopropyl alcohol/water gave 7.0 g (57%) oxalate salt, m.p. 199—200°C.

Analysis:

| | | | |
|---|---|---|---|
| Calculated for $C_{16}H_{21}N_2O_6Cl$: | C, 51.55; | H, 5.68; | N, 7.51 |
| Found: | C, 51.52; | H, 5.72; | N, 7.44 |

Example 28

2-(Dimethylamino)methyl-2,3-dihydro-4-methylpyrido[3,2-f][1,4]-oxazepin-5(4H)-one

When in the procedure of Example 10, 2-chloromethyl-2,3-dihydro-4-methylpyrido[3,2-f][1,4]-oxazepin-5(4H)-one is substituted for 2-(2-chloroethyl)-2,3-dihydro-4-methylpyrido[3,2-f][1,4]-oxazepin-5(4H)-one, the title compound is prepared and is isolated if desired as a pharmaceutically acceptable salt.

Example 29

2-[2-(Dimethylamino)ethyl]-2,3-dihydro-4-methylpyrido[3,2-f][1,4]-oxazepin-5(4H)-thione methiodide

2-[2-(Dimethylamino)ethyl]-2,3-dihydro-4-methylpyrido[3,2-f][1,4]-oxazepine-5(4H)-thione fumarate [1:1], ethanol [2:1], 3.8 g (0.01 mole) was partitioned between chloroform and dilute sodium hydroxide. The chloroform extract was dried over sodium sulphate and concentrated. The residue was dissolved in 15 ml of methyl isobutyl ketone was added to a solution of 1.4 g (0.01 mole) of methyl iodide in 15 ml of isobutyl ketone. Recrystallization from 50% ethanol — 50% methyl isobutyl ketone gave 2.5 g (78%) of the product, m.p. 221—225°C.

Analysis:

| | | | |
|---|---|---|---|
| Calculated for $C_{14}H_{22}N_3OSI$: | C, 41.28; | H, 5.44; | N, 10.31 |
| Found: | C, 41.29; | H, 5.51; | N, 10.30 |

Example 30

7-Chloro-2-[2-(dimethylamino)ethyl]-2,3-dihydro-4-methyl-1,4-benzoxazepine-5(4H)-thione oxalate [1:1] hemihydrate

To a solution of 8.0 g (0.027 mole) of 7-chloro-2-(2-chloroethyl)-2,3-dihydro-4-methyl-1,4-benzoxazepine-5(4H)-thione in 50 ml of absolute ethanol was added 6 ml (0.054 mole) of 40% aqueous solution of dimethylamine. The solution was heated in a steel bomb at 90°C for 14 hr. The ethanol was removed under reduced pressure and the residue was partitioned between chloroform and aqueous sodium hydroxide. The chloroform layer was concentrated to give a viscous yellow oil. The oil was dissolved in isopropyl alcohol and reacted with oxalic acid. The oxalate salt immediately precipitated. The mixture was heated and a small amount of water was added to dissolve the salt. A white crystalline powder was obtained, m.p. 150—151°C.

Analysis:

| | | | |
|---|---|---|---|
| Calculated for $C_{32}H_{44}N_4Cl_2O_{11}S_2$: | C, 48.30; | H, 5,57; | N, 7.04 |
| Found: | C, 48.74; | H, 5.34; | N, 6.95 |

Example 31

2-[2-(Dimethylamino)ethyl]-2,3-dihydro-4-methylnaphth[2,1-f][1,4]-oxazepine-5(4H)-thione hydrochloride [1:1]

To a solution of 15.0 g (0.05 mole) of 2-(2-chloroethyl)-2,3-dihydro-4-methylnaphth[2,1-f][1,4]-oxazepine-5(4H)-thione in 50 ml absolute ethanol was added 10 g of a 40% aqueous solution of dimethylamine. The resulting solution was heated in a steel bomb at 100°C for 40 hr and concentrated under reduced pressure. The residue was partitioned between 15% aqueous sodium hydroxide and chloroform. The chloroform layer was evaporated and the residue partitioned between 3N hydrochloric acid and chloroform. The aqueous layer was made alkaline with 50% sodium hydroxide and extracted with chloroform. The chloroform extract was concentrated and the residue dissolved in isopropyl alcohol. Ethereal hydrogen chloride was added. Recrystallization of the precipitate from isopropyl alcohol/water gave 3.0 g (20%) of the product, m.p. 238—240°C.

Analysis:

| | | | |
|---|---|---|---|
| Calculated for $C_{18}H_{23}N_2ClOS$: | C, 61.61; | H, 6.61; | N, 7,98 |
| Found: | C, 61.80; | H, 6.61; | N, 7.91 |

Example 32

When in the procedure of Example 11 equal molar amounts of the following are substituted for 2-(2-chloroethyl)-2,3-dihydro-4-methylpyrido[3,2-f][1,4]-oxazepine-5(4H)-thione:

2-(2-chloroethyl)-2,3-dihydro-4-methylpyrido[3,4-f][1,4]-oxazepine-5(4H)-thione, and

2-(2-chloroethyl)-2,3-dihydro-4-methylpyrido[2,3-f][1,4]-oxazepine-5(4H)-thione,

there are obtained:

a) 2-[2-(dimethylamino)ethyl]-2,3-dihydro-4-methylpyrido[3,4-f][1,4]-oxazepine-5(4H)-thione fumarate, and

b) 2-[2-(dimethylamino)ethyl]-2,3-dihydro-4-methylpyrido[2,3-f][1,4]-oxazepine-5(4H)-thione fumarate.

Example 33

2-[2-(Dimethylamino)ethyl]-2,3-dihydro-7-methoxy-4-methyl-1,4-benzoxazepin-5(4H)-one oxalate [1:1] hemihydrate

To a solution of 3.0 g (0.011 mole) of 2-(2-chloroethyl)-2,3-dihydro-7-methoxy-4-methyl-1,4-benzoxazepin-5(4H)-one in 50 ml of absolute ethanol was added 3.0 g of a 40% aqueous solution of dimethylamine. The reaction mixture was heated in a stainless steel bomb at 100°C for 16 hr, cooled and evaporated under reduced pressure. The residue was partitioned between chloroform and 15% sodium hydroxide solution. The chloroform layer was concentrated and the residue, the free base, was dissolved in isopropyl alcohol and reacted with oxalic acid. The resulting oxalate salt was recrystallized from isopropyl alcohol/$H_2O$ to give 1.9 g (45%) of the title salt, m.p. 176—178°C.

Analysis:

| | | | |
|---|---|---|---|
| Calculated for $C_{34}H_{50}N_4O_{15}$: | C, 54,10; | H, 6.67; | N, 7.42 |
| Found: | C, 54.29; | H, 6.59; | N, 7.53 |

Example 34

7-Bromo-2-[2-(dimethylamino)ethyl]-2,3-dihydro-4-methyl-1,4-benzoxazepine-5(4H)-thione oxalate [1:1] monohydrate

To a solution of 13 g (0.04 mole) of 7-bromo-2-(2-chloroethyl)-2,3-dihydro-4-methyl-1,4-benzoxazepine-5(4H)-thione in 50 ml of absolute ethanol was added 8 ml of a 45% aqueous solution of dimethylamine. The solution was heated at 100°C in a steel bomb for 16 hr. The ethanol was evaporated under reduced pressure and the residue partitioned between ethyl acetate and 3N aqueous hydrochloric acid. The aqueous extract was basified with 50% aqueous sodium hydroxide and extracted with chloroform. The chloroform was concentrated under reduced pressure. The residue, the free base of the title compound, was dissolved in isopropyl alcohol and reacted with oxalic acid. The oxalate salt was recrystallized from 95% ethanol to give the title salt, m.p. 155—157°C.

Analysis:

| | | | |
|---|---|---|---|
| Calculated for $C_{32}H_{40}N_4Br_2O_{12}S_2$: | C, 42,58; | H, 5.14; | N, 6.12 |
| Found: | C, 42.93; | H, 4.79; | N, 6.19 |

Example 35a to h

When in the procedure of Example 27, equal molar amounts of the following are substituted for 7-chloro-2-(2-chloroethyl)-2,3-dihydro-4-methyl-1,4-benzoxazepin-5(4H)-one,

2-(2-chloroethyl)-4-cyclohexyl-2,3-dihydro-1,4-benzoxazepin-5(4H)-one,

2-(2-chloroethyl)-2,3-dihydro-4-ethyl-1,4-benzoxazepin-5(4H)-one,

2-(2-chloroethyl)-2,3-dihydro-4-isopropyl-1,4-benzoxazepin-5(4H)-one,

2-(2-chloroethyl)-4-(4-chlorobenzyl)-2,3-dihydro-1,4-benzoxazepin-5(4H)-one,

2-(2-chloroethyl)-2,3-dihydro-4-(4-methylbenzyl)-1,4-benzoxazepin-5(4H)-one,

2-(3-chloroethyl)-2,3-dihydro-4-(3,5-dimethoxybenzyl)-1,4-benzoxazepin-5(4H)-one,

2-(2-chloroethyl)-2,3-dihydro-4-(3-trifluoromethylbenzyl)-1,4-benzoxazepin-5(4H)-one, and

2-(2-chloroethyl)-2,3-dihydro-4-(4-nitrobenzyl)-1,4-benzoxazepin-5(4H)-one,

there are obtained:

a) 4-cyclohexyl-2-[2-(dimethylamino)ethyl]-2,3-dihydro-1,4-benzoxazepin-5(4H)-one oxalate,

b) 2-[2-(dimethylamino)ethyl]-2,3-dihydro-4-ethyl-1,4-benzoxazepin-5(4H)-one oxalate,

c) 2-[2-(dimethylamino)ethyl]-2,3-dihydro-4-isopropyl-1,4-benzoxazepin-5(4H)-one oxalate,

d) 4-(4-chlorobenzyl)-2-[2-(dimethylamino)ethyl]-2,3-dihydro-1,4-benzoxazepin-5(4H)-one oxalate,

e) 2-[2-(dimethylamino)ethyl]-2,3-dihydro-4-(4-methylbenzyl)-1,4-benzoxazepin-5(4H)-one oxalate,

f) 2-[2-(dimethylamino)ethyl]-2,3-dihydro-4-(3,5-dimethoxybenzyl)-1,4-benzoxazepin-5(4H)-one oxalate,

g) 2-[2-(dimethylamino)ethyl]-2,3-dihydro-4-[(3-(trifluoromethyl)benzyl]-1,4-benzoxazepin-5(4H)-one oxalate, and

h) 2-[2-(dimethylamino)ethyl]-2,3-dihydro-4-(4-nitrobenzyl)-1,4-benzoxazepin-5(4H)-one oxalate.

Example 36a to h

When in the procedure of Example 10, equal molar amounts of the following are substituted for 2-(2-chloroethyl)-2,3-dihydro-4-methylpyrido[3,2-f][1,4]-oxazepin-5(4H)-one hydrochloride,

2-(2-chloroethyl)-4-cyclohexyl-2,3-dihydropyrido[3,2-f][1,4]-oxazepin-5(4H)-one hydrochloride,
2-(2-chloroethyl)-2,3-dihydro-4-ethylpyrido[3,2-f][1,4]-oxazepin-5(4H)-one hydrochloride,
2-(2-chloroethyl)-2,3-dihydro-4-isopropylpyrido[3,2-f][1,4]-oxazepin-5(4H)-one hydrochloride,
2-(2-chloroethyl)-4-(4-chlorobenzyl)-2,3-dihydropyrido[3,2-f][1,4]-oxazepin-5(4H)-one hydrochloride,
2-(2-chloroethyl)-2,3-dihydro-4-(4-methylbenzyl)pyrido[3,2-f][1,4]-oxazepin-5(4H)-one hydrochloride,
2-(2-chloroethyl)-2,3-dihydro-4-(4-methoxybenzyl)pyrido[3,2-f][1,4]-oxazepin-5(4H)-one hydrochloride,
2-(2-chloroethyl)-2,3-dihydro-4-(3-trifluoromethylbenzyl)pyrido[3,2-f][1,4]-oxazepin-5(4H)-one hydrochloride, and
2-(2-chloroethyl)-2,3-dihydro-4-(4-nitrobenzyl)-pyrido[3,2-f][1,4]-oxazepin-5(4H)-one hydrochloride,

there are obtained:

a) 4-cyclohexyl-2-[2-(dimethylamino)ethyl]-2,3-dihydropyrido[3,2-f][1,4]-oxazepin-5(4H)-one fumarate,
b) 2-[2-(dimethylamino)ethyl]-2,3-dihydro-4-ethylpyrido[3,2-f][1,4]-oxazepin-5(4H)-one fumarate,
c) 2-[2-(dimethylamino)ethyl]-2,3-dihydro-4-isopropylpyrido[3,2-f][1,4]-oxazepin-5(4H)-one fumarate,
d) 4-(4-chlorobenzyl)-2-[2-(dimethylamino)ethyl]-2,3-dihydro-pyrido[3,2-f][1,4]-oxazepin-5(4H)-one fumarate,
e) 2-[2-(dimethylamino)ethyl]-2,3-dihydro-4-(4-methylbenzyl)-pyrido[3,2-f][1,4]-oxazepin-5(4H)-one fumarate,
f) 2-[2-(dimethylamino)ethyl]-2,3-dihydro-4-(4-methoxybenzyl)-pyrido[3,2-f][1,4]-oxazepin-5(4H)-one fumarate,
g) 2-[2-(dimethylamino)ethyl]-2,3-dihydro-4-(3-trifluoromethylbenzyl)-pyrido[3,2-f][1,4]-oxazepin-5(4H)-one fumarate, and
h) 2-[2-(dimethylamino)ethyl]-2,3-dihydro-4-(4-nitrobenzyl)-pyrido[3,2-f][1,4]-oxazepin-5(4H)-one fumarate.

## Example 37a to d

When in the procedure of Example 3, equal molar amounts of the following are substituted for morpholine:

pyrrolidine,
piperidine,
piperazine, and
4-methyl-piperazine,

there are obtained:

a) 2,3-dihydro-4-methyl-2-[2-(1-pyrrolidino)ethyl]-1,4-benzoxazepin-5(4H)-one fumarate,
b) 2,3-dihydro-4-methyl-2-[2-(1-piperidino)ethyl]-1,4-benzoxazepin-5(4H)-one fumarate,
c) 2,3-dihydro-4-methyl-2-[2-(1-piperazino)ethyl]-1,4-benzoxazepin-4(4H)-one fumarate, and
d) 2,3-dihydro-4-methyl-2-[2-(4-methylpiperazin-1-yl)ethyl]-1,4-benzoxazepin-5(4H)-one fumarate.

## Example 38

2-[2-(Dimethylamino)ethyl]-2,3-dihydro-4-methylpyrido[3,2-f][1,4]-thiazepin-5(4H)-one dihydrochloride

A solution of 1.5 g (0.0058 mole) of 2-(2-chloroethyl)-2,3-dihydro-4-methylpyrido[3,2-f][1,4]-thiazepin-5(4H)-one in 20 ml of dimethylamine was stirred at 25°C in a sealed container for 72 hr. The excess dimethylamine was allowed to evaporate and the residue was partitioned between chloroform and dilute sodium hydroxide. The chloroform layer was concentrated and the residue, the free base of the title compound, was dissolved in isopropyl alcohol and reacted with hydrogen chloride. The resulting hydrochloride salt weighed 1.5 g (77%), m.p.>250°C.

Analysis:

| | | | |
|---|---|---|---|
| Calculated for $C_{13}H_{21}N_3OSCl_2$: | C, 46.16; | H, 6.27; | N, 12.42 |
| Found: | C, 45.68; | H, 6.18; | N, 12.35 |

## Example 39

2-[2-(Dimethylamino)ethyl]-2,3-dihydro-4-methylpyrido[3,2-f][1,4]-thiazepine-5(4H)-thione oxalate

A solution of 1.5 g (0.005 mole) of 2-(2-chloroethyl)-2,3-dihydro-4-methylpyrido[3,2-f][1,4]-thiazepine-5(4H)-thione in 40 ml of dimethylamine was stirred at 25°C in a sealed container for 96 hr. The dimethylamine was allowed to evaporate and the residue was partitioned between methylene chloride and dilute sodium hydroxide. The chloroform layer was concentrated and the residue, the free base of the title compound, was reacted with 0.4 g oxalic acid in a solution of 30 ml of 90—100 isopropyl alcohol water. The resulting crystals were recrystallized from the same solvent to give 1 g of the product, m.p. 191—193°C.

Analysis:

| | | | |
|---|---|---|---|
| Calculated for $C_{15}H_{21}N_3S_2O_4$: | C, 48.50; | H, 5.70; | N, 11.33 |
| Found: | C, 48.49; | H, 5.84; | N, 10.99 |

## Example 40

2-[2-(Dimethylamino)ethyl]-2,3-dihydro-4-methylpyrido[3,4-f][1,4]oxazepin-5(4H)-one oxalate (1:2) hemihydrate

A solution of 5 g (0.02 mole) of 2-(2-chloroethyl)-2,3-dihydro-4-methylpyrido[3,4-f][1,4]oxazepin-5(4H)-one, in 25 ml of dimethylamine was placed in a sealed vessel and stirred for 72 hr. The vessel was opened

and the excess dimethylamine allowed to evaporate. The residue was dissolved in chloroform and the solvent was stripped off in vacuo to remove excess dimethylamine. The residue was partitioned between dilute sodium hydroxide and ethyl acetate. The ethyl acetate solution was concentrated and the residue was treated with 3 g (0.033 mole) of oxalic acid in 50 ml of isopropyl alcohol and enough water to dissolve the salt while boiling. The resulting crystals were recrystallized from the same solvent. Yield of product was 5.3 g (60%), m.p. 179—181°C.

Analysis:

| | | | |
|---|---|---|---|
| Calculated for $C_{34}H_{48}N_6O_{21}$: | C, 46.48; | H, 5.52; | N, 9.58 |
| Found: | C, 46.58; | H, 5.70; | N, 9.61 |

### Example 41

2-[2-(Dimethylamino)ethyl]-2,3-dihydro-4-methylpyrido[3,4-f][1,4]oxazepine-5(4H)-thione oxalate (1:2)

A 4 g (0.009 mole) sample of 2-[2-(dimethylamino)ethyl]-2,3-dihydro-4-methylpyrido[3,4-f][1,4]-oxazepin-5(4H)-one oxalate (1:2) hemihydrate was partitioned between dilute sodium hydroxide and chloroform. The aqueous layer was extracted three times and the combined chloroform extracts were dried over sodium sulphate and concentrated. The residue was dissolved in 200 ml of dry toluene and again concentrated in vacuo to effect drying. The residue was dissolved in dry pyridine (10 ml) and treated with 2.8 g (0.01 mole) of phosphorus pentasulphide. The mixture was stirred at reflux for 20 hr. The cooled mixture was partitioned between dilute sodium hydroxide and chloroform. The aqueous layer was extracted three times with chloroform. The combined chloroform extracts were dried over sodium sulphate and concentrated. One gram of the residue was treated with 0.6 g of oxalic acid in isopropyl alcohol/10% water. The resulting crystals were collected by filtration. Yield of oxalate salt was 0.37 g, m.p. 111—114°C.

Analysis:

| | | | |
|---|---|---|---|
| Calculated for $C_{17}H_{23}N_3SO_9$: | C, 45.84; | H, 5,20; | N, 9.43 |
| Found: | C, 45.46; | H, 5.38; | N, 9.28 |

### Example 42

2-(2-Aminopropyl)-2,3-dihydro-4-methyl-pyrido[3,2-f][1,4]-oxazepin-5(4H)-one oxalate [1:1]

2,3-Dihydro-4-methyl-5(4H)-oxopyrido[3,2-f][1,4]-oxazepine-2-propanenitrile, 5 g (0.22 mole), in 150 ml of ethanol, was treated with about 1.5 g of wet Raney nickel. The mixture was hydrogenated in a Parr apparatus at 60°C. and 40 psi. The mixture was cooled and filtered and the filtrate concentrated. The residue was treated with 3.9 g of oxalic acid in 130 ml of boiling isopropyl alcohol containing 2 ml of water. The hot solution was filtered and allowed to cool. The resulting solid was recrystallized from ethanol. Yield of oxalate hemihydrate was 3 g (43%), m.p. 126—134°C.

Analysis:

| | | | |
|---|---|---|---|
| Calculated for $C_{28}H_{40}N_6H_7$: | C, 50,30; | H, 6.03; | H, 12.57 |
| Found: | C, 50.46; | H, 5.71; | N, 12.21 |

### Example 43

2,3-Dihydro-4-methyl-2-[2-(4-morpholinyl)ethyl]pyrido[3,2-f][1,4]oxazepine-5(4H)-one maleate [1:1]

2-(2-Chloroethyl)-2,3-dihydro-4-methylpyrido[3,2-f][1,4]oxazepin-5(4H)-one hydrochloride, 16 g (.058 mole) was dissolved in morpholine (30 ml) and stirred overnight at room temperature. To the solution was added dilute sodium hydroxide solution (50 ml) and the resulting mixture extracted with chloroform (3 × 30 ml). The chloroform was removed on the rotary evaporator with aspiration. The residual morpholine was removed in vacuo at 50°C (rotary evaporator). To the residual free base (15.5 g, .053 mole) was added isopropyl alcohol (1 liter) and maleic acid (9.24 g, .080 mole). The mixture was heated to boiling and the clear solution cooled at 20°C for several hours. The resulting crystals, at 16 g (68.1%), were recrystallized from isopropyl alcohol, m.p. 163—165°C.

Analysis:

| | | | |
|---|---|---|---|
| Calculated for $C_{19}H_{25}N_3O_7$: | C, 56.01; | H, 6.18; | N, 10.31 |
| Found: | C, 55.71; | H, 6.21; | N, 10.18 |

### Example 44

2,3-Dihydro-4-methyl-2-[2-(1-pyrrolidinyl)ethyl]pyrido[3,2-f][1,4]-oxazepin-5(4H)-one fumarate [1:1]

A sample of 2-(2-chloroethyl)-2,3-dihydro-4-methylpyrido[3,2-f][1,4]-oxazepine-5(4H)-one hydrochloride, 16 g (0.058 mole) was dissolved in 65 ml of pyrrolidine. The stirred solution was heated to 80°C for 3 hr. The solution was cooled to room temperature and dilute sodium hydroxide solution (50 ml) was added. The resulting solution was extracted with chloroform (3 × 30 ml) and concentrated in vacuo. The residue taken up in boiling isopropyl alcohol (500 ml/and fumaric acid (9.2g, .079 mole) was added. The solution was filtered hot and the filtrate cooled to 20°C for several hours. The resulting crystals, 14 g (47.8%) were collected and recrystallized from isopropyl alcohol, m.p. 147—149°C.

Analysis:

| | | | |
|---|---|---|---|
| Calculated for $C_{23}O_{10}N_3H_{29}$: | C, 54.43; | H, 5.76; | N, 8.28 |
| Found: | C, 54.38; | H, 5.83; | N, 8.27 |

Example 45

2-[2-(Dibutylamino)ethyl]-2,3-dihydro-4-methyl-pyrido[3,2-f][1,4]oxazepin-5(4H)-one maleate [1:1]

2-(2-Chloroethyl)-2,3-dihydro-4-methylpyrido[3,2-f][1,4]oxazepine-5(4H)-one hydrochloride, 16 g (0.058 mole) was dissolved in dimethylformamide (30 ml) and di-n-butylamine (30 ml). The solution was stirred at 90°C for 3 hr at 100°C for 2.5 hr. The solution was cooled and to it was added 50 ml of dilute sodium hydroxide solution. The resulting mixture was extracted with chloroform (3 × 50 ml). The chloroform was removed on the rotary evaporator with water aspiration at 50°C. Residual dimethylformamide and di-n-butylamine were removed at low vacuum and 50°C (rotary evaporator). To the residual free base, 13.8 g (0.041 mole) was added isopropyl alcohol (900 ml) and oxalic acid, 5.6 g (0.062 mole) and the solution heated to boiling. The clear solution was cooled overnight at 20°C and filtered to give 13.6 g (56.5%) of crystals which were recrystallized from isopropyl alcohol, m.p. 195—196°C.

Analysis:

| | | | |
|---|---|---|---|
| Calculated for $C_{21}H_{33}N_3O_6$: | C, 59.59; | H, 7.85; | N, 9.72 |
| Found: | C, 59.37; | H, 7.91; | N, 9.86 |

Example 46

2-[2-(Diethylamino)ethyl]-2,3-dihydro-4-methylpyrido[3,2-f][1,4]oxazepin-5(4H)-one oxalate [1:1]

2-(2-Chloroethyl)-2,3-dihydro-4-methylpyrido[3,2-f][1,4]oxazepin-5(4H)-one hydrochloride, 16 g (0.058 mole) was suspended in diethylamine (30 ml). The suspension was stirred for 72 hr at room temperature. The mass spectrum indicated that the reaction had progressed 3% at this point. The mixture was then heated to reflux for 6 hr. Diethylamine was removed by rotary evaporation (70°C water aspirator). The residue was taken up in chloroform (100 ml) and washed with dilute aqueous sodium hydroxide (2 × 30 ml). The organic layer was concentrated by rotary evaporation (70°C, water aspirator). The residue was dissolved in boiling isopropyl alcohol and treated with oxalic acid. Upon cooling, 18.6 g (87.7%) of light brown crystals were collected (m.p. 150°C—155°C.). A sample was recrystallized three more times from isopropyl alcohol, m.p. 156—157°C.

Analysis:

| | | | |
|---|---|---|---|
| Calculated for $C_{17}H_{25}N_3O_6$: | C, 55.57; | H, 6.86; | H, 11.43 |
| Found: | C, 55.28; | H, 6.85; | N, 11.27 |

Example 47

2,3-Dihydro-4-methyl-2-[2-(1-piperidinyl)ethyl]pyrido[3,2-f][1,4]oxazepin-5(4H)-one oxalate [1:1]

2-(2-Chloroethyl)-2,3-dihydro-4-methylpyrido[3,2-f][1,4]oxazepin-5(4H)-one hydrochloride, 4 g (0.015 mole) was dissolved in piperidine (30 ml) and heated to 80°C with stirring for 20 minutes. The piperidine was removed by rotary evaporation (85°C, vacuum pump) and the residue taken up in chloroform (50 ml). The organic layer was washed with dilute aqueous sodium hydroxide (2 × 20 ml) and concentrated by rotary evaporation (80°C), water aspirator). The resulting oil was taken up in hot isopropyl alcohol and treated with oxalic acid. Upon cooling, crystals of the oxalate salt were collected and recrystallized from isopropyl alcohol, to give 3.4 g (62%) of pale brown crystals, m.p. 133—136°C.

Analysis:

| | | | |
|---|---|---|---|
| Calculated for $C_{18}H_{25}N_3O_6$: | C, 56.98; | H, 6.64; | N, 11.07 |
| Found: | C, 56.95; | H, 6.87; | N, 10.79 |

Example 48

2,3-Dihydro-4-methyl-2-[2-[methyl(phenylmethyl)amino]ethyl]pyrido[3,2-f][1,4]oxazepin-5(4H)-one maleate [1:1]

2-(2-Chloroethyl)-2,3-dihydro-4-methylpyrido[3,2-f][1,4]oxazepin-5(4H)-one hydrochloride, 4 g (0.015 mole) was dissolved in methyl benzyl amine (30 ml) and heated to 80°C with stirring. After three hours, the excess amine was removed by rotary evaporation (90°C, vacuum pump). The residual oil was taken up in chloroform (40 ml) and washed with dilute aqueous sodium hydroxide (30 ml). The chloroform layer was concentrated by rotary evaporation (90°C, water aspirator). The residual oil was dissolved in hot isopropyl alcohol and treated with maleic acid. Upon cooling, 4.23 g (66%) of pale brown crystals were collected, m.p. 167—169°C.

Analysis:

| | | | |
|---|---|---|---|
| Calculated for $C_{23}H_{27}N_3O_6$: | C, 62.57; | H, 6.16; | N, 9.52 |
| Found: | C, 62.28; | H, 6.16; | N, 9.24 |

Example 49

2,3-Dihydro-4-methyl-2-[2-(methylphenylamino)ethyl]pyrido[3,2-f][1,4]oxazepin-5(4H)-one

2-(2-Chloroethyl)-2,3-dihydro-4-methylpyrido[3,2-f][1,4]oxazepin-5(4H)-one hydrochloride, 4.00 g (0.015 mole) was dissolved in N-methylaniline (30 ml) and heated to 95°C with stirring for 2 days. Excess N-methylaniline was removed by rotary evaporation (95°C, vacuum pump). The residue was taken up in chloroform (80 ml) and washed with dilute aqueous sodium hydroxide (30 ml). The chloroform layer was decolourised with activated carbon and dried over sodium sulphate, filtered and concentrated by rotary evaporation. The remaining residue was dissolved in ethyl acetate (50 ml) and purified by high pressure

liquid chromatography using a silica gel column and ethyl acetate as the eluent. After purification, crystals formed from ethyl acetate. These crystals were recrystallized from ethyl acetate, giving 1.40 g (31%) of pale brown crystals.

Analysis:

| | | | |
|---|---|---|---|
| Calculated for $C_{18}H_{21}N_3O_2$: | C, 69.43; | H, 6.79; | N, 13.49 |
| Found: | C, 69,31; | H, 6.77; | N, 13.54 |

Example 50

2-[2-(2,5-Dimethyl-1-pyrrolidinyl)ethyl]-2,3-dihydro-4-methylpyrido[3,2-f][1,4]oxazepin-5(4H)-one fumarate [1:1]

2-(2-Chloroethyl)-2,3-dihydro-4-methylpyrido[3,2-f][1,4]oxazepin-5(4H)-one, 5.0 g (0.021 mole), was dissolved in 25 ml of absolute ethanol and 3 g (0.03 mole) of 2,5-dimethylpyrrolidine was added. The solution was heated to 75°C for 48 hrs with stirring. Because the reaction was incomplete at this time, an additional amount of 2,5-dimethylpyrrolidine (1.00 g, 0.01 mole) was added and the reaction continued. After 5 days, the reaction was still incomplete and more 2,5-dimethylpyrrolidine (1.00 g, 0.01 mole) was added. The reaction appeared complete 2 days later. Solvent was removed by rotary evaporation (80°C, water aspirator). Excess 2,5-dimethylpyrrolidine was removed by rotary evaporation (80°C, vacuum pump). The residue was taken up in chloroform (200 ml) and washed with dilute aqueous sodium hydroxide (2 × 75 ml). The organic layer was dried over sodium sulphate, filtered, and concentrated by rotary evaporation (70°C, water aspirator). The resulting oil was dissolved in hot isopropyl alcohol and treated with fumaric acid. Upon cooling, 2.38 g (27.4%) of pale brown crystals was collected, m.p. 161—162°C

Analysis:

| | | | |
|---|---|---|---|
| Calculated for $C_{21}H_{29}N_3O_6$: | C, 60.13; | H, 6.96; | N, 10.02 |
| Found: | C, 59.79; | H, 6.93; | N, 9.76 |

Example 51

2,3-Dihydro-4-methyl-2-[2-(2-methyl-1-pyrrolidinyl)ethyl]pyrido[3,2-f][1,4]oxazepin-5(4H)-one

To a solution of 3.5 g (0.0145 mole) of 2-(2-chloroethyl)-2,3-dihydro-4-methylpyrido[3,2-f][1,4]-oxazepine-5(4H)-one in ethanol (15 ml) was added 2-methyl pyrrolidine (5.0 g, 0.063 mole). The solution was heated to reflux for 3 hours with stirring. The ethanol was removed by rotary evaporation (water aspirator, 80°C). The residual oil was partitioned between dilute aqueous sodium hydroxide (50 ml) and chloroform (50 ml). The organic layer was saved and the aqueous layer extracted with chloroform (2 × 30 ml). All the chloroform layers were combined, dried over anhydrous sodium sulphate and concentrated by rotary evaporation (water aspirator, 70°C). The residual oil was then distilled at 200°C and low vacuum (vacuum pump) giving 1.5 g (35.7%) of a clear oil.

Analysis:

| | | | |
|---|---|---|---|
| Calculated for $C_{16}H_{23}N_3O_2$: | C, 66.41; | H, 8.01; | N, 14.52 |
| Found: | C, 65.83; | H, 8.06; | N, 14.39 |

Example 52

2,3-Dihydro-4-methyl-2-[2-(1H-pyrazol-1-yl)ethyl]pyrido[3,2-f][1,4]oxazepin-5(4H)-one

To a suspension of sodium hydride (1.2 g active, 0.05 mole) in dimethylformamide (15 ml) was added dropwise a solution of pyrazole (3.10 g, 0.045 mole) in dimethylformamide (15 ml). The resulting solution was then added to a solution of 2-(2-chloroethyl)-2,3-dihydro-4-methylpyrido[3,2-f][1,4]oxazepine-5(4H)-one (9.12 g, 0.038 mole) in 30 ml of dimethylformamide. The flask was sealed and stirred overnight. Because the reaction had not yet gone to completion at this point, pyrazole (3.12 g, 0.045 mole) was added to the reaction solution and stirred overnight. The reaction was still not complete and another suspension of sodium hydride (0.5 g active, 0.021 mole) and pyrazole (1.5 g, 0.022 mole) in dimethylformamide (10 ml) was added and the reaction stirred overnight. The reaction appeared to be complete. Dimethylformamide was removed by rotary-evaporation (80°C, vacuum pump), and the residue taken up in chloroform (100 ml) which was washed with dilute aqueous sodium hydroxide (1 × 50 ml), dried over anhydrous sodium sulphate and concentrated by rotary evaporation (70°C, water aspirator). The material was purified by high pressure liquid chromatography, 95:5 by volume ethanol:methanol on a silica gel column. The fractions containing the desired product were concentrated by rotary evaporation (70°C, water aspirator). Crystallization ensued upon cooling. The crystals were collected and recrystallized from ethanol. The yield was 1.5 (14.5%), m.p. 132—134°C.

Analysis:

| | | | |
|---|---|---|---|
| Calculated for $C_{14}H_{16}N_4O_2$: | C, 71.75; | H, 5.92; | N, 20.58 |
| Found: | C, 61.35; | H, 5.89; | N, 20.67 |

Example 53

2,3-Dihydro-2-[2-(1H-imidazol-1-yl)ethyl]-4-methylpyrido[3,2-f][1,4]oxazepin-5(4H)-one

To a solution of 2-(2-chloroethyl)-2,3-dihydro-4-methylpyrido[3,2-f][1,4]-oxazepine-5(4H)-one, 9.12 g (0.038 mole) in dimethylformamide (30 ml) was added imidazole, 5.66 g (0.083 mole). The solution was heated to 130°C for 18 hr. Dimethylformamide was removed by rotary evaporation (80°C, vacuum pump)

and the residue taken up in chloroform (100 ml). The chloroform was washed with dilute aqueous sodium hydroxide (30 ml), dried over sodium sulphate and concentrated by rotary evaporation (70°C, water aspirator) to an oil. Crystallization was induced with ethanol. White crystals, 1.5 g (14.5%) were collected, m.p. 150—152°C.

Analysis:

| | | | |
|---|---|---|---|
| Calculated for $C_{14}H_{16}N_4O_2$: | C, 61.75; | H, 5.92; | N, 20.58 |
| Found: | C, 61.36; | H, 5.92; | N, 20.60 |

Example 54

2-[2-(Dimethylamino)ethyl]-4-ethyl-2,3-dihydropyrido[3,2-f][1,4]oxazepin-5(4H)-one oxalate [1:1]

To 30 ml of dimethylamine collected at 0°C was added 6 g (0.021 mole) 2-(2-chloroethyl)-4-ethyl-2,3-dihydropyrido[3,2-f][1,4]oxazepin-5(4H)-one, hydrochloride. The flask was sealed tightly and stirred 70 hr at room temperature. The solution was then cooled to 0°C and the stopper of the flask removed. Dimethylamine was allowed to evaporate. The residue was taken up in chloroform (1 × 150 ml) and washed with dilute aqueous sodium hydroxide (1 × 50 ml). The organic layer was dried over sodium sulphate, filtered and concentrated by rotary evaporation (70°C, water aspirator). The material was dissolved in hot isopropyl alcohol and treated with oxalic acid. Upon cooling, 4.5 (61.5%) was collected, m.p. 208°C.

Analysis:

| | | | |
|---|---|---|---|
| Calculated for $C_{16}H_{23}N_3O_6$: | C, 54.38; | H, 6.56; | N, 11.89 |
| Found: | C, 54.26; | H, 6.61; | N, 11.81 |

Example 55

2,3-Dihydro-4-ethyl-2-[2-(1-pyrrolidinyl)ethyl]pyrido[3,2-f][1,4]oxazepin-5(4H)-one oxalate [1:1]

2-(2-Chloroethyl)-4-ethyl-2,3-dihydropyrido[3,2-f][1,4]oxazepin-5(4H)-one hydrochloride, 3 g (0.01 mole) was dissolved in pyrrolidine (30 ml) and heated to 70°C for 30 minutes with stirring. After cooling, the contents of the reaction flask were diluted with dilute aqueous sodium hydroxide (40 ml) and extracted with chloroform (2 × 30 ml). The chloroform layer was dried over sodium sulphate filtered and concentrated to a viscous brown oil by rotary evaporation (70°C, water aspirator). The oil was taken up in hot isopropyl alcohol and treated with oxalic acid. Upon cooling, the resulting solid was recrystallized from isopropyl alcohol, giving pale brown crystals, 1.80 g (45.4%), m.p. 185—188°C.

Analysis:

| | | | |
|---|---|---|---|
| Calculated for $C_{18}H_{25}N_3O_6$: | C, 56.98; | H, 6.64; | N, 11.07 |
| Found: | C, 56.90; | H, 6.67; | N, 10.90 |

Example 56

2,3-Dihydro-4-methyl-2-[2-(4-morpholinyl)ethyl]pyrido[3,2-f][1,4]oxazepin-5(4H)-thione

2-(2-Chloroethyl)-2,3-dihydro-4-methylpyrido[3,2-f][1,4]oxazepine-5(4H)-thione, 4.5 g (0.018 mole) was dissolved in morpholine (30 ml). The solution was heated with stirring to 50°—60°C for 6 hr. The morpholine was then removed by rotary evaporation (90°C, vacuum pump). The residue was taken up in chloroform (100 ml) and washed with dilute aqueous sodium hydroxide (2 × 30 ml). The organic layer was concentrated by rotary evaporation (60°C, water aspirator). The residue was recrystallized from ethanol giving 3.26 g (60%) of light yellow crystals, m.p. 152—153°C.

Analysis:

| | | | |
|---|---|---|---|
| Calculated for $C_{15}H_{21}N_3O_2S$: | C, 58.61; | H, 6.89; | N, 13.66 |
| Found: | C, 58.48; | H, 6.92; | N, 13.62 |

Example 57

2-[2-(Dibutylamino)ethyl]-2,3-dihydro-4-methylpyrido[3,2-f][1,4]oxazepine-5(4H)-thione oxalate [1:1]

2-(2-Chloroethyl)-2,3-dihydro-4-methylpyrido[3,2-f][1,4]-oxazepine-5(4H)-thione, 4 g (0.016 mole) was suspended in di-n-butylamine (30 ml). Dimethylformamide (*ca.* 10 ml) was added to the stirred mixture until dissolution occurred. The solution was heated to 140°C for 3.5 hr with stirring. Di-n-butylamine and dimethylformamide were removed by rotary evaporation (80°C vacuum pump). The residue was then diluted with dilute aqueous sodium hydroxide (50 ml) and extracted with chloroform (3 × 40 ml). Chloroform was removed by rotary evaporation (70°C, water aspirator). The residue was dissolved in boiling isopropyl alcohol and treated with oxalic acid. Upon cooling, the resulting oxalate salt was filtered and recrystallized from isopropyl alcohol to give 3.2 g (47%) of yellow crystals, m.p. 208°C.

Analysis:

| | | | |
|---|---|---|---|
| Calculated for $C_{21}H_{33}N_3O_5S$: | C, 57.38; | H, 7.57; | N, 9.56 |
| Found: | C, 57.04; | H, 7.63; | N, 9.31 |

Example 58

2-[2-(Diethylamino)ethyl-2,3-dihydro-4-methylpyrido[3,2-f][1,4]oxazepine-5(4H)-thione oxalate [1:1]

2-(2-Chloroethyl)-2,3-dihydro-4-methyl[3,2-f][1,4]oxazepine-5(4H)-thione, 4 g (0.016 mole) was suspended in diethylamine (30 ml). Dimethylformamide was added to the stirred suspension until

dissolution occurred (10 ml). The stirred solution was heated to 65°C for 8 hr. Diethylamine was removed by rotary evaporation (70°C, water aspirator); the remaining dimethylformamide was removed at low pressure (vacuum pump) and 90°C. The residue was taken up in chloroform (100 ml) and washed with dilute aqueous sodium hydroxide (2 × 30 ml). The organic layer was concentrated by rotary evaporation (70°C, water aspirator). The residue was dissolved in boiling isopropyl alcohol and treated with oxalic acid. Upon cooling, the oxalate salt, 1.7 g (28.5%) was obtained, m.p. 142—144°C.

Analysis:

| | | | |
|---|---|---|---|
| Calculated for $C_{17}H_{25}N_3O_5S$: | C, 53.25; | H, 6.57; | N, 10.95 |
| Found: | C, 53.14; | H, 6.60; | N, 10.72 |

## Example 59

2,3-Dihydro-4-methyl-2-[2-(1-pyrrolidinyl)ethyl]pyrido[3,2-f][1,4]oxazepine-5(4H)-thione oxalate [1:1]

2-(2-Chloroethyl)-2,3-dihydro-4-methylpyrido[3,2-f][1,4]oxazepine-5(4H)-thione, 5 g (0.02 mole) was dissolved in 30 ml of pyrrolidine. The solution was heated to 60°—80°C for 35 minutes with stirring. After cooling to room temperature, the reaction mixture was diluted with dilute aqueous sodium hydroxide (50 ml) and extracted with chloroform (2 × 50 ml). The organic layer was concentrated by rotary evaporation (70°C, water aspirator). Residual pyrrolidine was removed at 90°C and vacuum pump. The residue was dissolved in hot ethanol and treated with oxalic acid. Upon cooling, the oxalate salt was collected and recrystallized twice from ethanol to give 3.35 g, (45%) of product, m.p. 141°C.

Analysis:

| | | | |
|---|---|---|---|
| Calculated for $C_{17}H_{23}N_2O_5S$: | C, 53.53; | H, 6.08; | N, 11.02 |
| Found: | C, 53.39; | H, 6.11; | N, 10.91 |

## Example 60

2,3-Dihydro-2-[2-(1H-imidazol-1-yl)ethyl]-4-methylpyrido-[3,2-f][1,4]oxazepine-5(4H)-thione oxalate [2:3]

To a solution of 2-(2-chloroethyl)-2,3-dihydro-4-methylpyrido[3,2-f][1,4]oxazepine-5(4H)-thione, 4.5 g (0.018 mole) in dimethylformamide (35 ml) was added imidazole (2.20 g, 0.038 mole). The resulting solution was heated to 130°C for 15 hrs. Dimethylformamide was removed by rotary evaporation (80°C, vacuum pump), and the residue diluted with dilute aqueous sodium hydroxide (50 ml). The aqueous solution was extracted with chloroform (1 × 50 ml), dried over anhydrous sodium sulphate and concentrated by rotary evaporation (water aspirator, 70°C). The resulting oil was treated with oxalic acid in ethanol. Four grams (54%) of pale yellow crystals were collected and recrystallized again with ethanol, m.p. 163—167°C.

Analysis:

| | | | |
|---|---|---|---|
| Calculated for $C_{17}H_{19}O_7N_4S$: | C, 48.22; | H, 4.52; | N, 13.23 |
| Found: | C, 48.04; | H, 4.62; | N, 13.18 |

## Example 61

2-[2-(Dimethylamino)ethyl]-4-ethyl-2,3-dihydropyrido[3,2-f][1,4]oxazepin-5(4H)-thione

2-(2-Chloroethyl)-4-ethyl-2,3-dihydropyrido[3,2-f][1,4]oxazepin-5(4H)-thione hydrochloride, 5.00 g (0.016 mole) was added to 20 ml of anhydrous dimethylamine. The reaction flask was sealed tightly and stirred at room temperature for 6 days. The flask was opened after cooling to 0°C and dimethylamine allowed to evaporate at room temperature. The residue was taken up in chloroform (100 ml) and washed with dilute aqueous sodium hydroxide (1 × 30 ml). The chloroform layer was dried over sodium-sulphate, filtered and concentrated by rotary evaporation. The residual oil was dissolved in hot cyclohexane. Upon cooling, 1.76 g (39.4%) of light yellow crystals were collected, m.p. 73°C.

Analysis:

| | | | |
|---|---|---|---|
| Calculated for $C_{14}H_{21}N_3OS$: | C, 60.18; | H, 7.58; | N, 15.03 |
| Found: | C, 60.32; | H, 7.70; | N, 15.13 |

## Example 62

2,3-Dihydro-4-methyl-2-[2-[methyl(phenylmethyl)amino]ethyl]pyrido[3,2-f][1,4]oxazepine-5(4H)-thione oxalate [1:1]

To a solution of 4 g (0.0155 mole) of 2-(2-chloroethyl)-2,3-dihydro-4-methylpyrido[3,2-f][1,4]oxazepine-5(4H)-thione in 70 ml of chloroform was added 10.0 g (0.086 mole) of benzylmethylamine. The solution was stirred at reflux for 24 hr. The reaction solution was washed with water (2 × 50 ml) and concentrated by rotary evaporation (~70°C, water aspirator). The residue was distilled on a molecular still at 165°C/0.1 mm. The residue was treated with oxalic acid in hot isopropyl alcohol. Upon cooling, two crops of crystals were collected. The purity of each crop was checked. The two crops were combined and recrystallized together in hot isopropyl alcohol. Upon cooling, 3.69 g (55%) of pale yellow crystals, m.p. 163—166°C were collected.

Analysis:

| | | | |
|---|---|---|---|
| Calculated for $C_{21}H_{25}N_3O_5S$: | C, 58,45; | H, 5.84; | N, 9.74 |
| Found | C, 58.24; | H, 5.92; | N, 9.61 |

Example 63

2,3-Dihydro-2-[2-(methylamino)ethyl)-4-methylpyrido[3,2-f][1,4]oxazepine-5(4H)-thione oxalate [1:1:5]

2-(2-Chloroethyl)-2,3-dihydro-4-methylpyrido[3,2-f][1,4]oxazepine-5(4H)-thione, 4.0 g (0.016 mole) was suspended in a 30% solution of methylamine in 70 ml of ethanol and allowed to stir for 56 hr at room temperature. Because of incomplete reaction, the reaction solution was heated slowly over a 2 hr period to 55°C. and stirred at that temperature for 24 hr. Methylamine was removed by water aspiration for 1.5 hr. The resulting solution was concentrated by rotary evaporation (70°C, water aspirator). The residual oil was taken up in chloroform (150 ml) and washed with 2 M aqueous potassium hydroxide (2 × 50 ml). The chloroform layer was dried over sodium sulphate and concentrated by rotary evaporation (70°C, water aspirator). The residue was dissolved in hot ethanol and treated with oxalic acid. Upon cooling, 2.0 g (37.5%) of yellow crystals were collected, m.p. 137—138°C.

Analysis:

| | | | |
|---|---|---|---|
| Calculated for: $C_{15}H_{20}N_3O_7S$: | C, 46.63; | H, 5.22; | N, 10.67 |
| Found: | C, 46.47; | H, 5.35; | N, 10.85 |

Example 64

7-Chloro-2,3-dihydro-4-methyl-2-[2-[1-pyrrolidino)ethyl]pyrido[3,2-f][1,4]oxazepin-5(4H)-one fumarate [1:2.5]

7-Chloro-2-(2-chloroethyl)-2,3-dihydro-4-methylpyrido[3,2-f][1,4]oxazepin-5(4H)-one (2.5 g, 0.009 mole) was dissolved in 50 ml pyrrolidine and the solution was heated to 80°C for 1 hr. The pyrrolidine was removed by rotary evaporation (80°C, water aspirator) and the residue dissolved in 100 ml of chloroform. The organic layer was washed with water (2 × 50 ml), dried over sodium sulphate and concentrated by rotary evaporation (~80°C, water aspirator). The residue was treated with fumaric acid and allowed to stand overnight. The resulting crystals were collected, 1.25 g (23.2%), m.p. 164—166°C.

Analysis:

| | | | |
|---|---|---|---|
| Calculated for: $C_{25}H_{30}N_3O_{12}Cl$: | C, 50.05; | H, 5.04; | N, 7.00 |
| Found: | C, 50.22; | H, 5.14; | N, 7.02 |

Example 65

7-Chloro-2-[2-(dimethylamino)ethyl]-2,3-dihydro-4-methylpyrido[3,2-f][1,4]oxazepin-5(4H)-one oxalate [1:1]

A 2.8 g (0.01 mole) sample of 7-chloro-2-(2-chloroethyl)-2,3-dihydro-4-methylpyrido[3,2-f][1,4]oxazepin-5(4H)-one was added to 25 ml of dimethylamine and stirred for 96 hr in a sealed flask. The excess amine was allowed to evaporate and the residue was partitioned between chloroform and dilute sodium hydroxide. The chloroform was dried over sodium sulphate and concentrated. The residue was treated with 0.7 g of oxalic acid in isopropyl alcohol. The resulting crystals were recrystallized from the same solvent. Yield was 1.5 g of oxalate salt (40%), m.p. 150—156°C.

Analysis:

| | | | |
|---|---|---|---|
| Calculated for: $C_{15}H_{20}N_3O_6Cl$: | C, 48.20; | H, 5.39; | N, 11.24 |
| Found: | C, 48.09; | H, 5.47; | N, 11.12 |

Example 66

4-Cyclohexyl-2-[(dimethylamino)methyl]-2,3-dihydropyrido[3,2-f][1,4]oxazepin-5(4H)-one oxalate

Utilizing the procedure of Example 10, 2-(2-chloromethyl)-4-cyclohexyl-2,3-dihydropyrido[3,2-f][1,4]oxazepin-5(4H)-one (Intermediate 35) is reacted with 40% aqueous dimethylamine and reacted with oxalic acid in isopropyl alcohol.

Example 67

2-[2-(Dimethylamino)ethyl]-2,3-dihydro-4-phenylmethylpyrido[3,2-f][1,4]oxazepin-5(4H)-one oxalate [1:1.5] hemihydrate

A solution containing 94.2 g (0.6 mole) of 2 chloronicotinic acid and 100 g (0.54 mole) of 1-benzyl-3-pyrrolidinol in 800 ml of dry tetrahydrofuran was added at a rapid drop to a stirred suspension of 52 g (1.3 mole) of 60% sodium hydride/mineral oil in 500 ml of dry tetrahydrofuran at reflux temperature (addition time was about 1 hr). The mixture was heated to reflux for an additional 1.5 hr and then cooled at room temperature. Approximately 1 liter of ethyl acetate was added and filtration attempted unsuccessfully. The mixture was allowed to stand overnight at room temperature and then was concentrated on the rotary evaporator at 100°C and 50 mm pressure. The residue was dissolved in 1 litre of chloroform and the pH of the solution was adjusted to 6.15 with hydrogen chloride gas. To the solution was added, with stirring, 383 g (1.0 mole) of triphenylphosphine and 383 g (2.48 mole) of carbon tetrachloride. The mixture was refluxed for 1 hr and 50 ml of ethanol was added. The solution was cooled to room temperature and extracted three times with 400 ml portions of dilute hydrochloric acid. The chloroform layer was extracted with dilute sodium hydroxide, dried over sodium sulphate and concentrated. The mass spectra indicated the presence of 2-(2-chloroethyl)-2,3-dihydro-4-(phenylmethyl)pyrido[3,2-f][1,4]oxazepin-5(4H)-one (mass 316), triphenylphosphine (mass 262) and triphenylphosphine oxide (mass 278). One-third of the residue was chromatographed on a high pressure liquid chromatograph in an unsuccessful attempt to purify the compound. The other 2/3 of the residue was dissolved in 30 ml of chloroform and added to a solution of

30 g of dimethyl amine in ethanol. The solution was heated to reflux for 4 hr and concentrated on the rotary evaporator. The residue was partitioned between chloroform and 1 N hydrochloric acid. The acid layer was made basic with sodium hydroxide and extracted with chloroform. The chloroform layer was dried over sodium sulphate and concentrated. The residue (10 g) was treated with an equivalent amount of oxalic acid in a mixture of isopropyl alcohol-ethanol-isopropyl ether. The resulting crystals in the amount of 9 g (5%) were recrystallized from the same solvent mixture, m.p. 95—98°C.

Analysis:

| | | | |
|---|---|---|---|
| Calculated for: $C_{44}H_{54}N_6O_{17}$: | C, 56.28; | H, 5.79; | N, 8.95 |
| Found: | C, 56.61; | H, 5.76; | N, 8.77 |

Example 68

2-[2-(Dimethylamino)ethyl]-2,3-dihydropyrido[3,2-f][1,4]oxazepin-5(4H)-one

A solution of 3.0 g (0.006 mole) of 2-[2-(dimethylamino ethyl]-2,3-dihydro-4-phenylmethylpyrido[3,2-f][1,4]oxazepin-5(4H)-one oxalate [1:1.5]-hemihydrate in about 50 ml of water was made basic with dilute aqueous sodium hydroxide solution and then extracted with three 50 ml portions of benzene. The combined benzene extract was dried over anhydrous sodium sulphate and concentrated on the rotary evaporator (steam bath/50 mm). The residue was dried further by azeotroping 2 times with about 50 ml of dry benzene, evaporating to dryness each time. The final residue was dissolved in 40 ml of liquid ammonia and small spheres of sodium were added with stirring to the solution until a blue color persisted for 20 minutes. (Addition time was about 1 hr). Three grams of ammonium chloride was added slowly and the ammonia was allowed to evaporate. The residue was suspended in chloroform and the mixture was filtered. The filtrate was concentrated and the residue chromatographed on preparative high pressure liquid chromatograph using a silica gel column and eluting with 75% ethyl acetate/25% dimethylformamide. The yield of product was 0.1 g (7%). The chemical ionization mass spectrophotometer gave a peak at 236 corresponding to a molecular weight of 235. The $^1H$ NMR spectrum of the subject compound was obtained in $CDCl_3$ containing 1% tetramethylsilane (TMS) and is consistent with the proposed structure and dimethylformamide (DMF) and mineral oil as minor impurities. The chemical shifts, multiplicities, and assignments are given below:

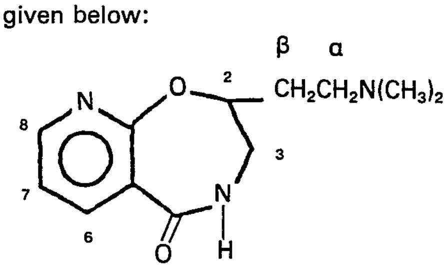

| Chemical Shifts (multiplicities) | Assignments |
|---|---|
| 8.45 (multiplet) | H(8) and H(6) |
| 8.00 (singlet) | C—H (DMF) |
| 7.85 (broad singlet) | N—H |
| 7.20 (doublet of doublets) | H(7) |
| 4.65 (pentet) | H(2) |
| 4.05 (broad singlet) | unknown impurity |
| 3.50 (triplet) | $H_2(3)$ |
| 2.95 (singlet) | $CH_3$(DMF) |
| 2.90 (singlet) | $CH_3$(DMF) |
| 2.60 (triplet) | $H_2$—α to amino nitrogen |
| 2.25 (singlet) | $N(CH_3)_2$ |
| 2.05 (multiplet) | $H_2$—β to amino nitrogen |
| 0.7—1.7 (multiplet) | mineral oil |

Example 69

2-[3-(Dimethylamino)propyl]-2,3-dihydro-4-methylpyrido[3,2-f][1,4]oxazepin-5(4H)-one fumarate [1:1.5] hemihydrate

To 5.0 g (0.021 mole) of 2-(3-aminopropyl)-2,3-dihydro-4-methylpyrido[3,2-f][1,4]-oxazepin-5(4H)-one was added, while cooling in a water bath, an 88% aqueous solution of formic acid, 20 g (0.38 mole). To the resulting solution was added a solution of 37% aqueous formaldehyde (inhibited with 13% methanol), 10.7 g (0.13 mole). The resulting solution was heated on a steam bath for 5.5 hr. The mixture was cooled and 100 ml of dilute aqueous hydrochloric acid was added. The solution was evaporated to dryness and the residue was dissolved in 50 ml of water. The solution was neutralized with dilute aqueous potassium hydroxide and extracted with four 50 ml portions of chloroform. The combined chloroform extracts was dried over sodium sulphate and concentrated by rotary evaporation. The residue was reacted with fumaric acid in hot isopropyl alcohol. The collected product, 3.0 g (31.8%) was recrystallized twice from isopropyl alcohol, m.p. 108—110°C.

Analysis:

| | | | |
|---|---|---|---|
| Calculated for: $C_{40}H_{56}N_6O_{17}$: | C, 53.81; | H, 6.32; | N, 9.41 |
| Found: | C, 53.69; | H, 6.33; | N, 9.41 |

Example 70

2-[3-(Dimethylamino)propyl]-2,3-dihydro-4-methylpyrido[3,2-f][1,4]oxazepine-5(4H)-thione oxalate [1:2]

To a solution of 11.0 g (0.042 mole) of 2-[3-(dimethylamino)propyl]-2,3-dihydro-4-methylpyrido-[3,2-f][1,4]oxazepin-5(4H)-one in 125 ml of pyridine was added 9.25 g (0.042 mole) of phosphorus pentasulphide. The mixture was heated to reflux for 3.5 hr while stirring. After cooling to room temperature, the reaction solution was added to an equal volume of 2 molar potassium hydroxide. The mixture was extracted with 800 ml of methylene chloride in several portions. The organic phase was washed with three 100 ml portions of dilute potassium hydroxide, dried over sodium sulphate, filtered and concentrated by rotary evaporator (water-aspirator, 70°C). The residual oil was subjected to reduced pressure of the vacuum pump for 2 hr at 90°C and then cooled and reacted with oxalic acid in isopropyl alcohol. Two crops, 4.5 and 3.1 g were collected, combined and recrystallized from isopropyl alcohol to give 6.5 g (34%) of yellow crystals, m.p. 136—138°C.

Analysis:

| | | | |
|---|---|---|---|
| Calculated for: $C_{18}H_{25}N_3O_9S$: | C, 47.05; | H, 5.42; | N, 9.16 |
| Found: | C, 46.76; | H, 5.75; | N, 9.04 |

Example 71

7-Chloro-2-[2-(dimethylamino)ethyl]-2,3-dihydro-4-methylpyrido[3,2-f][1,4]oxazepine-5(4H)-thione fumarate [1:1] hemihydrate, hemiisopropyl alcoholate

To 55 ml of a methanolic solution containing 57% by volume dimethylamine was added 2.50 g (0.009 mole) of 7-chloro-2-(2-chloroethyl)-2,3-dihydro-4-methylpyrido[3,2-f][1,4]-oxazepine-5(4H)-thione. The reaction vessel was sealed and allowed to stand for 16 hr. Thin-layer chromatography indicated the reaction was about 60% complete. The solution was heated gradually to 45°C (heating time about 5 hr). Methanol and unreacted dimethylamine were removed by rotary evaporator (water aspirator, 60°C). The residue was taken up in 100 ml of chloroform and the solution was washed with two 40 ml portions of water. The organic layer was dried over sodium sulphate, filtered and concentrated by rotary evaporator. The residue was reacted with fumaric acid in isopropyl alcohol. The resulting crystals, 1.43 g (36.5%) were recrystallized from isopropyl alcohol and dried thoroughly in a drying pistol, m.p. 98—104°C.

Analysis:

| | | | |
|---|---|---|---|
| Calculated for: $C_{37}H_{54}N_6O_{12}Cl_2S_2$: | C, 48.84; | H, 5.98; | N, 9.23 |
| Found: | C, 48.82; | H, 5.80; | N, 9.37. |

TABLE 1

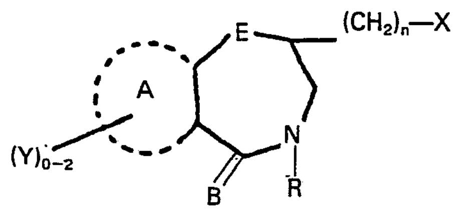

| Inter-mediate No. | A(Y)0—2 | B | R | E | X | n | Salt |
|---|---|---|---|---|---|---|---|
| 1 | benz | O | $—CH_3$ | O | Cl | 2 | |
| 2 | benz | O | $—CH_2—C_6H_5$ | O | Cl | 2 | |
| 3 | naphth[2,3-f] | O | $—CH_3$ | O | Cl | 2 | |
| 4 | pyrido[3,2-f] | O | $—CH_3$ | O | Cl | 2 | HCl |
| 5 | 8-Cl-benz | O | $—CH_3$ | O | Cl | 2 | — |
| 6 | 7-Br-benz | O | $—CH_3$ | O | Cl | 2 | — |
| 7 | 7-Cl-benz | O | $—CH_3$ | O | Cl | 2 | — |
| 8 | naphth[2,1-f] | O | $—CH_3$ | O | Cl | 2 | — |
| 9 | 7—$OCH_3$-benz | O | $—CH_3$ | O | Cl | 2 | — |
| 10 | benz | S | $—CH_3$ | O | Cl | 2 | — |
| 11 | pyrido[3,2-f] | S | $—CH_3$ | O | Cl | 2 | — |
| 12 | naphth[2,3-f] | S | $—CH_3$ | O | Cl | 2 | — |
| 13 | 8-Cl-benz | S | $—CH_3$ | O | Cl | 2 | — |
| 14 | 7-Br-benz | S | $—CH_3$ | O | Cl | 2 | — |
| 15 | naphth[2,1-f] | S | $—CH_3$ | O | Cl | 2 | — |
| 16 | pyrido[4,3-f] | O | $—CH_3$ | O | Cl | 2 | HCl |
| 17 | pyrido[3,4-f] | O | $—CH_3$ | O | Cl | 2 | HCl |
| 18 | pyrido[2,3-f] | O | $—CH_3$ | O | Cl | 2 | HCl |
| 19 | 7-Cl-benz | S | $—CH_3$ | O | Cl | 2 | — |
| 20 | 7,9-diiodo-benz | O | $—CH_3$ | O | Cl | 2 | — |
| 21 | pyrido[3,2-f] | S | $—CH_3$ | O | Cl | 1 | HCl |
| 22 | pyrido[4,3-f] | S | $—CH_3$ | O | Cl | 2 | — |
| 23 | 7—$OCH_3$-benz | S | $—CH_3$ | O | Cl | 2 | — |
| 24a) | benz | O | $—C_6H_{11}$ | O | Cl | 2 | — |
| b) | benz | O | $—C_2H_5$ | O | Cl | 2 | — |
| c) | benz | O | $—CH(CH_3)_2$ | O | Cl | 2 | — |

TABLE I Continued

| Intermediate No. | A(Y)0—2 | B | R | E | X | n | Salt |
|---|---|---|---|---|---|---|---|
| d) | benz | O | 4-Cl—$C_6H_4$—$CH_2$— | O | Cl | 2 | — |
| e) | benz | O | 4—$CH_3$—$C_6H_4$—$CH_2$— | O | Cl | 2 | — |
| f) | benz | O | 3,5—$(OCH_3)_2$—<br>$C_6H$—$CH_2$— | O | Cl | 2 | — |
| g) | benz | O | 3—$CF_3$—$C_6H_4$—$CH_2$— | O | Cl | 2 | — |
| h) | benz | O | 4—$NO_2$—$C_6H_4$—$CH_2$— | O | Cl | 2 | — |
| 25a) | pyrido[3,2-f] | O | —$C_6H_{11}$ | O | Cl | 2 | HCl |
| b) | pyrido[3,2-f] | O | —$C_2H_5$ | O | Cl | 2 | HCl |
| c) | pyrido[3,2-f] | O | —$CH(CH_3)_2$ | O | Cl | 2 | HCl |
| d) | pyrido[3,2-f] | O | 4—Cl—$C_6H_4$—$CH_2$— | O | Cl | 2 | HCl |
| e) | pyrido[3,2-f] | O | 4—$CH_3$—$C_6H_4$—$CH_2$— | O | Cl | 2 | HCl |
| f) | pyrido[3,2-f] | O | 3,5—$(OCH_3)_2$—<br>$C_6H$—$CH_2$— | O | Cl | 2 | HCl |
| g) | pyrido[3,2-f] | O | 3—$CF_3$—$C_6H_4$—$CH_2$ | O | Cl | 2 | HCl |
| h) | pyrido[3,2-f] | O | 4—$NO_2$—$C_6H_4$—$CH_2$— | O | Cl | 2 | HCl |
| 26 | pyrido[3,2-f] | O | —$CH_3$ | S | Cl | 2 | — |
| 27 | pyrido[3,2-f] | S | —$CH_3$ | S | Cl | 2 | — |
| 28 | pyrido[3,4-f] | O | —$CH_3$ | O | Cl | 2 | — |
| 29 | pyrido[3,4-f] | S | —$CH_3$ | O | Cl | 2 | HCl |
| 30 | pyrido[3,2-f] | O | —$CH_3$ | O | —CN | 2 | — |
| 31 | pyrido[3,2-f] | O | —$C_2H_5$ | O | Cl | 2 | HCl |
| 32 | pyrido[3,2-f] | S | —$C_2H_5$ | O | Cl | 2 | HCl |
| 33 | 7-Cl-pyrido[3,2-f] | O | —$CH_3$ | O | Cl | 2 | — |
| 34 | 7-Cl-pyrido[3,2-f] | S | —$CH_3$ | O | Cl | 2 | — |
| 35 | pyrido[3,2-f] | O | —$C_6H_{11}$ | O | Cl | 1 | — |
| 36 | pyrido[3,2-f] | O | —$CH_2C_6H_5$ | O | Cl | 2 | — |

TABLE 2

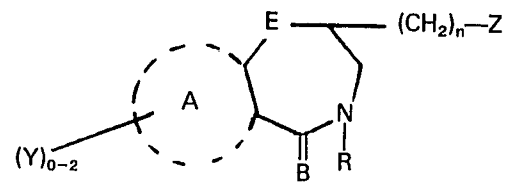

| Ex No. | A(Y)0—2 | B | R | E | Z | n | Salt |
|---|---|---|---|---|---|---|---|
| 1 | benz | O | —$CH_3$ | O | —$N(CH_3)_2$ | 2 | HCl |
| 2 | benz | O | —$CH_3$ | O | —$N(CH_3)_2$ | 2 | — |
| 3 | benz | O | —$CH_3$ | O | 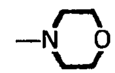 | 2 | fumarate |
| 4 | benz | O | —$CH_2$Ø | O | 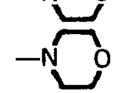 | 2 | — |
| 5 | benz | O | —$CH_2$Ø | O | —$NHCH_3$ | 2 | fumarate |
| 6 | benz | S | —$CH_3$ | O | —$N(CH_3)_2$ | 2 | HCl |
| 7 | benz | O | —$CH_2$Ø | O | —$N(CH_3)_2$ | 2 | $H_2O$ |
| 8 | benz | S | —$CH_3$ | O | 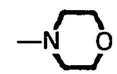 | 2 | HCl |
| 9 | naphth[2,3-f] | O | —$CH_3$ | O | —$N(CH_3)_2$ | 2 | oxalate |
| 10 | pyrido[3,2-f] | O | —$CH_3$ | O | —$N(CH_3)_2$ | 2 | 1.5 fumarate |
| 11 | pyrido[3,2-f] | S | —$CH_3$ | O | —$N(CH_3)_2$ | 2 | fumarate, 0.5 ethanol |
| 12 | pyrido[3,2-f] | S | —$CH_2$ | | —$N(CH_3)_2$ | 2 | fumarate |
| 13 | benz | S | —$CH_2$Ø | O | 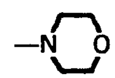 | 2 | — |
| 14 | benz | O | —$CH_3$ | O | —$NHCH_3$ | 2 | fumarate |
| 15 | benz | O | —$CH_3$ | O | —$NHCH_3$ | 2 | — |
| 16 | benz | S | —$CH_3$ | O | 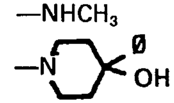 | 2 | — |

TABLE 2 cont.

| Ex. No. | A(Y)0—2 | B | R | E | Z | n | Salt |
|---|---|---|---|---|---|---|---|
| 17 | benz | S | —$CH_3$ | O | 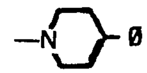 | 2 | — |
| 18 | 8-Cl-benz | S | —$CH_3$ | O | —$N(CH_3)_2$ | 2 | HCl |
| 19 | 8-Cl-benz | O | —$CH_3$ | O | —$N(CH_3)_2$ | 2 | oxalate |
| 20 | 7-Br-benz | O | —$CH_3$ | O | —$N(CH_3)_2$ | 2 | oxalate |
| 21 | naph[2,1-f] | O | —$CH_3$ | O | —$N(CH_3)_2$ | 2 | oxalate |
| 22a) | pyrido[4,3-f] | O | —$CH_3$ | O | —$N(CH_3)_2$ | 2 | fumarate |
| b) | pyrido[3,4-f] | O | —$CH_3$ | O | —$N(CH_3)_2$ | 2 | fumarate |
| c) | pyrido[2,3-f] | O | —$CH_3$ | O | —$N(CH_3)_2$ | 2 | fumarate |
| 23 | pyrido[4,3-f] | S | —$CH_3$ | O | —$N(CH_3)_2$ | 2 | 1.5 HCl |
| 24 | pyrido[3,2-f] | S | —$CH_3$ | O | —$N(C_2H_3)_2$ | 2 | — |
| 25 | naphth[2,3-f] | S | —$CH_3$ | O | —$N(CH_3)_2$ | 2 | oxalate, 1/2 $H_2O$ |
| 26 | 7,9-diiodobenz | O | —$CH_3$ | O | —$N(CH_3)_2$ | 2 | — |
| 27 | 7-Cl-benz | O | —$CH_3$ | O | —$N(CH_3)_2$ | 2 | oxalate |
| 28 | pyrido[3,2-f] | O | —$CH_3$ | O | —$N(CH_3)_2$ | 1 | — |
| 29 | pyrido[3,2-f] | S | —$CH_3$ | O | —$N(CH_3)_2$ | 2 | methiodide |
| 30 | 7-Cl-benz | S | —$CH_3$ | O | —$N(CH_3)_2$ | 2 | oxalate· 1/2 $H_2O$ |
| 31 | naphth[2,1-f] | S | —$CH_3$ | O | —$N(CH_3)_2$ | 2 | HCl |
| 32a) | pyrido[3,4-f] | S | —$CH_3$ | O | —$N(CH_3)_2$ | 2 | fumarate |
| b) | pyrido[2,3-f] | S | —$CH_3$ | O | —$N(CH_3)_2$ | 2 | fumarate |

TABLE 2 cont.

| Ex. No. | A(Y)0—2 | B | R | E | Z | n | Salt |
|---|---|---|---|---|---|---|---|
| 33 | 7-$OCH_2$benz | O | —$CH_3$ | O | —$N(CH_3)_2$ | 2 | oxalate· 1/2 $H_2O$ |
| 34 | 7-Br-benz | S | —$CH_3$ | O | —$N(CH_3)_2$ | 2 | oxalate· $H_2O$ |
| 35a) | benz | O | —$C_6H_{11}$ | O | —$N(CH_3)_2$ | 2 | oxalate |
| b) | benz | O | —$C_2H_5$ | O | —$N(CH_3)_2$ | 2 | oxalate |
| c) | benz | O | —$CH(CH_3)_2$ | O | —$N(CH_3)_2$ | 2 | oxalate |
| d) | benz | O | 4-Cl—$C_6H_4CH_2$— | O | —$N(CH_3)_2$ | 2 | oxalate |
| e) | benz | O | 4—$CH_2$—$C_6H_4CH_2$— | O | —$N(CH_3)_2$ | 2 | oxalate |
| f) | benz | O | 3,5-$(OCH_3)_2$— $C_4H_5CH_2$ | O | —$N(CH_3)_2$ | 2 | oxalate |
| g) | benz | O | 3—$CF_3$—$C_6H_4CH_2$— | O | —$N(CH_3)_2$ | 2 | oxalate |
| h) | benz | O | 4—$NO_2$—$C_6H_4CH_2$— | O | —$N(CH_3)_2$ | 2 | oxalate |
| 36a) | pyrido[3,2-b] | O | —$C_6H_{11}$ | O | —$N(CH_3)_2$ | 2 | fumarate |
| b) | pyrido[3,2-f] | O | —$C_2H_5$ | O | —$N(CH_3)_2$ | 2 | fumarate |
| c) | pyrido[3,2-f] | O | —$CH(CH_3)_2$ | O | —$N(CH_3)_2$ | 2 | fumarate |
| d) | pyrido[3,2-f] | O | 4-Cl—$C_6H_4CH_2$— | O | —$N(CH_3)_2$ | 2 | fumarate |
| e) | pyrido[3,2-f] | O | 4—$CH_3$—$C_6H_4CH_2$— | O | —$N(CH_3)_2$ | 2 | fumarate |
| f) | pyrido[3,2-f] | O | 4—$OCH_3$—$C_6H_4CH_2$— | O | —$N(CH_3)_2$ | 2 | fumarate |
| g) | pyrido[3,2-f] | O | 3—$CF_3$—$C_6H_4CH_2$— | O | —$N(CH_3)_2$ | 2 | fumarate |
| h) | pyrido[3,2-f] | O | 4—$NO_2$—$C_6H_4CH_2$— | O | —$N(CH_3)_2$ | 2 | fumarate |

TABLE 2 cont.

| Ex. No. | A(Y)0—2 | B | R | E | Z | n | Salt |
|---|---|---|---|---|---|---|---|
| 37a) | benz | O | —$CH_3$ | O | 1-pyrrolidinyl | 2 | fumarate |
| b) | benz | O | —$CH_3$ | O | 1-piperidinyl | 2 | fumarate |
| c) | benz | O | —$CH_3$ | O | 1-piperazinyl | 2 | fumarate |
| d) | benz | O | —$CH_3$ | O | 4—$CH_3$—piperizinyl | 2 | fumarate |
| 38 | pyrido[3,2-f] | O | —$CH_3$ | S | —$N(CH_3)_2$ | 2 | 2·HCl |
| 39 | pyrido[3,2-f] | S | —$CH_3$ | S | —$N(CH_3)_2$ | 2 | oxalate |
| 40 | pyrido[3,4-f] | O | —$CH_3$ | O | —$N(CH_3)_2$ | 2 | 1/2 $H_2O$ 2 oxalate |
| 41 | pyrido[3,4-f] | S | —$CH_3$ | O | —$N(CH_3)_2$ | 2 | 2 oxalate |
| 42 | pyrido[3,2-f] | O | —$CH_3$ | O | —$NH_2$ | 3 | 1/2 $H_2O$ oxalate |
| 43 | pyrido[3,2-f] | O | —$CH_3$ | O | 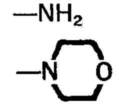 | 2 | maleate |
| 44 | pyrido[3,2-f] | O | —$CH_3$ | O | 1-pyrrolidinyl | 2 | 2 fumarate |
| 45 | pyrido[3,2-f] | O | —$CH_3$ | O | —$N(n\text{-butyl})_2$ | 2 | maleate |
| 46 | pyrido[3,2-f] | O | —$CH_3$ | O | —$N(C_2H_5)_2$ | 2 | oxalate |
| 47 | pyrido[3,2-f] | O | —$CH_3$ | O | 1-piperidinyl | 2 | oxalate |
| 48 | pyrido[3,2-f] | O | —$CH_3$ | O | —$N(CH_3)$(benzyl) | 2 | maleate |
| 49 | pyrido[3,2-f] | O | —$CH_3$ | O | —$N(CH_3)$—$C_6H_5$ | 2 | — |
| 50 | pyrido[3,2-f] | O | —$CH_3$ | O | 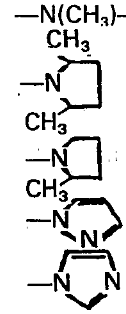 | 2 | fumarate |
| 51 | pyrido[3,2-f] | O | —$CH_3$ | O | | 2 | — |
| 52 | pyrido[3,2-f] | O | —$CH_3$ | O | | 2 | — |
| 53 | pyrido[3,2-f] | O | —$CH_3$ | O | | 2 | — |

TABLE 2 cont.

| Ex. No. | A(Y)0—2 | B | R | E | Z | n | Salt |
|---|---|---|---|---|---|---|---|
| 54 | pyrido[3,2-f] | O | $—C_2H_5$ | O | $—N(CH_3)_2$ | 2 | oxalate |
| 55 | pyrido[3,2-f] | O | $—C_2H_5$ | O | 1-pyrrolidinyl | 2 | oxalate |
| 56 | pyrido[3,2-f] | S | $—CH_3$ | O | 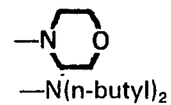 | 2 | — |
| 57 | pyrido[3,2-f] | S | $—CH_3$ | O | —N(n-butyl)$_2$ | 2 | oxalate |
| 58 | pyrido[3,2-f] | S | $—CH_3$ | O | $—N(C_2H_5)_2$ | 2 | oxalate |
| 59 | pyrido[3,2-f] | S | $—CH_3$ | O | 1-pyrrolidinyl | 2 | oxalate |
| 60 | pyrido[3,2-f] | S | $—CH_3$ | O | 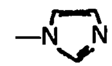 | 2 | 1.5 oxalate |
| 61 | pyrido[3,2-f] | S | $—C_2H_5$ | O | $—N(CH_3)_2$ | 2 | — |
| 62 | pyrido[3,2-f] | S | $—CH_3$ | O | $—N(CH_3)$(benzyl) | 2 | oxalate |
| 63 | pyrido[3,2-f] | S | $—CH_3$ | O | $—NHCH_3$ | 2 | 1.5 oxalate |
| 64 | 7-Cl-pyrido[3,2-f] | O | $—CH_3$ | O | -1-pyrrolidinyl | 2 | 2.5 fumarate |
| 65 | 7-Cl-pyrido[3,2-f] | O | $—CH_3$ | O | $—N(CH_3)_2$ | 2 | oxalate |
| 66 | pyrido[3,2-f] | O | $—C_6H_{11}$ | O | $—N(CH_3)_2$ | 1 | oxalate |
| 67 | pyrido[3,2-f] | O | $—CH_2C_6H_5$ | O | $—N(CH_3)_2$ | 2 | 1.5 oxalate $\frac{1}{2}$ $H_2O$ |
| 68 | pyrido[3,2-f] | O | H | O | $—N(CH_3)_2$ | 2 | — |
| 69 | pyrido[3,2-f] | O | $—CH_3$ | O | $—N(CH_3)_2$ | 3 | 1.5 fumarate $0.5H_2O$ |
| 70 | pyrido[3,2-f] | S | $—CH_3$ | O | $—N(CH_3)_2$ | 3 | 2 oxalate |
| 71 | 7-Cl-pyrido[3,2-f] | S | $—CH_3$ | O | $—N(CH_3)_2$ | 2 | 1/2 $H_2O$, 1/2 $(CH_3)_2HOH$ |

Pharmaceutical Compositions

The invention further provides pharmaceutical compositions for administration to a living animal body comprising, as active ingredients, at least one of the compounds of Formula I according to the invention in association with a pharmaceutical carrier or excipient. The compounds are thus presented in a therapeutic composition suitable for oral, rectal, parenteral, subcutaneous, intramuscular, intraperitoneal, intravenous, or intranasal administration. Thus, for example, compositions for oral administration can take the form of elixirs, capsules, tablets or coated tablets containing carriers conveniently used in the pharmaceutical art. Suitable tableting excipients include lactose, potato and maize starches, talc, gelatin and stearic and silicic acids, magnesium stearate and polyvinyl pyrrolidone.

For parenteral administration, the carrier or excipient can be comprised of a sterile parenterally acceptable liquid; e.g. water or arachis oil contained in ampoules.

In compositions for rectal administration, the carrier can be comprised of a suppository base; e.g., cocoa butter or a glyceride.

Application to the nose, throat or bronchial region can be in the form of gargle or an aerosol spray containing small particles of the agent of Formula I in a spray or dry powder form.

Advantageously, the compositions are formulated as dosage units, each unit being adapted to supply a fixed dose of active ingredients. Tablets, coated tablets, capsules, ampoules and suppositories are examples of preferred dosage forms according to the invention. It is only necessary that the active ingredient constitute an effective amount; i.e., such that a suitable effective dosage will be consistent with the dosage form employed. The exact individual dosages, as well as daily dosages, will of course be determined according to standard medical principles under the direction of a physician or veterinarian. Generally, the pharmacology tests on guinea pigs in comparison to certain other antihistaminic drugs suggests an effective dose for an adult will be in the range of 2 to 8 mg for the more active compounds.

Based on the animal data, unit dosages containing an amount of compound equivalent to about 0.03 to 0.10 mg of active drug per kilogram of body weight are contemplated. Daily dosages of about 0.2 to 0.6 mg/kg of body weight are contemplated for humans and obviously several small unit dosage forms may be administered at one time. However the scope of the invention is not to be limited by these contemplations due to uncertainty in transposing from animal data to humans.

Examples of unit dosage compositions are as follows:

Capsules:

| Ingredients | Per capsule |
|---|---|
| 1. Active ingredient | 4 mg. |
| 2. Lactose | 150 mg. |
| 3. Magnesium stearate | 4 mg. |

Tablets:

| Ingredients | |
|---|---|
| 1. Active ingredient | 4 mg. |
| 2. Corn starch | 20 mg. |
| 3. Kelacid | 20 mg. |
| 4. Keltose | 20 mg. |
| 5. Magnesium stearate | 1.3 mg. |

Procedure for tablets:

1. Blend 1, 2, 3, 4 in larger amounts.
2. Add sufficient water portionwise to blend to the blend from step 1 with careful stirring after each addition. Such additions of water and stirring continue until the mass is of a constituency to permit its conversion to wet granules.
3. The wet mass is converted to granules by passing it through the oscillating granulator using 8 mesh screen.
4. The wet granules are then dried in an oven at 140°F (60°C).
5. The dry granules are lubricated with the magnesium stearate.
6. The lubricated granules are compressed on a suitable tablet press.

Intramuscular Injection

| | Per ml. |
|---|---|
| 1. Active ingredients | 10.0 mg. |
| 2. Isotonic buffer solution 4.0 q.s. to | 1.0 ml. |

Procedure:
1. Dissolve the active ingredient in the buffer solution.
2. Aseptically filter the solution from step 1.
3. The sterile solution is now aseptically filled into sterile ampoules.
4. The ampoules are sealed under aseptic condition.

Suppositories:

| Ingredients | Per Supp. |
|---|---|
| 1. Active ingredient | 10.0 mg. |
| 2. Polyethylene Glycol 1000 | 1350.0 mg. |
| 3. Polyethylene Glycol 4000 | 450.0 mg. |

Procedure:
1. Melt 2 and 3 together and stir until uniform.
2. Dissolve No. 1 in the molten mass from step 1 and stir until uniform.
3. Pour the molten mass from step 2 into suppository moulds and chill.
4. Remove the suppositories from moulds and wrap.

Therapeutic compositions for combatting histamine in unit dosage form, comprising a pharmaceutical carrier and an effective amount of a compound of Formula I or a pharmaceutically acceptable acid addition salt thereof are therefore an embodiment of this invention.

Various modifications and equivalents will be apparent to one skilled in the art and may be made in the compounds, methods, processes and pharmaceutical compositions of the present invention without departing from the spirit and scope thereof, and it is therefore to be understood that the invention is to be limited only by the scope of the appended claims.

**Claims for the Contracting States: BE CH DE FR GB IT LI LU NL SE**

1. A compound having the formula:

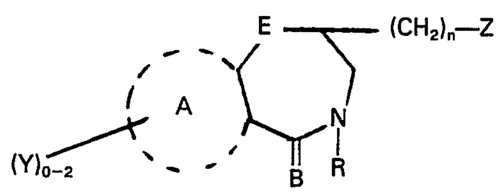

wherein:

A represents an aromatic ring system having two of its carbon atoms held mutually with the oxazepine or thiazepine moiety selected from benzene, naphthalene, and pyridine in any of its four positions, any of the rings being optionally substituted by one or two Y radicals selected from halo, loweralkyl, loweralkoxy, nitro and trifluoromethyl;

B and E are selected from oxygen and sulphur and may be the same or different;

R is selected from hydrogen, loweralkyl, $C_3$—$C_9$ cycloalkyl or phenyl-loweralkyl of which phenyl is optionally substituted by one or two radicals selected from halo, loweralkyl, loweralkoxy, nitro and trifluoromethyl;

n is 1, 2 or 3;

Z is selected from —$NR^1R^2$; 1H-pyrazol-1-yl and 1H-imidazol-1-yl;

each of $R^1$ and $R^2$ is selected from hydrogen, loweralkyl, $C_3$—$C_9$ cycloalkyl and phenyl-loweralkyl of which phenyl is optionally substituted by 1 or 2 radicals selected from halo, loweralkyl, loweralkoxy, nitro, trifluoromethyl and cyano, or $R^1$ and $R^2$ taken together with the adjacent nitrogen atom form a heterocyclic residue selected from 1-pyrrolidinyl, 2,5-dimethylpyrrolidin-1-yl, 2-methylpyrrolidin-1-yl, 1-piperidinyl, 4-substituted piperidine-1-yl, 4-morpholinyl, 1-piperazinyl, 4-substituted piperazin-1-yl and 1,2,3,6-tetrahydropyridin-1-yl and the pharmaceutically acceptable salts thereof with the proviso that when R = H, Z is never a primary or secondary amine and a further proviso that when n = 3, Z is not 1H-pyrazol-1-yl, 1H-imidazol-1-yl, 2,5-dimethylpyrrolidin-1-yl, 2-methylpyrrolidin-1-yl or 1,2,3,6-tetrahydropyridin-1-yl; where the term 'loweralkyl' refers to a straight or branched $C_{1-8}$ alkyl group, and 'loweralkoxy' means —O—loweralkyl.

2. A compound as claimed in Claim 1, wherein n is 2.

3. A compound as claimed in Claim 1 or 2, wherein A represents a benzene ring.

4. A compound as claimed in Claim 1 or 2, wherein A represents a naphthalene ring system.

5. A compound as claimed in Claim 1 or 2, wherein A represents a pyridine ring.

6. A compound as claimed in any one of Claims 1 to 5, wherein the ring represented by A is substituted by one or more chlorine or bromine atoms or by a methoxy group.

7. A compound as claimed in any one of Claims 1 to 6 wherein R represents a methyl, ethyl, benzyl or hexyl group.

8. A compound as claimed in any one of Claims 1 to 7, wherein Z represents a dimethylamino group.

9. 2 - [2 - (Dimethylamino)ethyl] - 2,3 - dihydro - 4 - methyl - 1,4 - benzoxazepin - 5(4H) - one, 2,3 - dihydro - 4 - methyl - 2 - [2 - (4 - morpholino)ethyl] - 1,4 - benzoxazepin - 5(4H)one, 4 - benzyl - 2,3 - dihydro - 2 - [2 - (4 - morpholino)ethyl] - 1,4 - benzoxazepin - 5(4H) one, 4 - benzyl - 2,3 - dihydro - 2 - [2 - (methylamino)ethyl] - 1,4 - benzoaxazepin - 5(4H) - one, 2 - [2 - (dimethylamino)ethyl] - 2,3 - dihydro - 4 - methyl - 1,4 - benzoaxazepine - 5(4H) - thione, 4 - benzyl - 2 - [2 - (dimethylamino)ethyl] - 2,3 - dihydro - 1,4 - benzoxazepin - 5(4H) - one, 2,3 - dihydro - 4 - methyl - 2 - [2 - (4 - morpholino)-ethyl] - 1,4 - benzoxazepine - 5(4H) - thione, 2 - [2 - (dimethylamino)ethyl] - 2,3 - dihydro - 4 - methylnaphth[2,3-f][1,4]oxazepin - 5(4H) - one, 2 - [2 - (dimethylamino)ethyl] - 2,3 - dihydro - 4 - methyl-pyrido[3,2 - f][1,4]oxazepin - 5(4H) - one, 2 - [2 - (dimethylamino)ethyl] - 2,3 - dihydro - 4 - methyl-pyrido[3,2 - f][1,4]oxazepine - 5(4H) - thione, 4 - benzyl - 2,3 - dihydro - 2 - [2 - (4 - morpholino)ethyl] - 1,4 - benzoxazepine - 5(4H) - thione, 2,3 - dihydro - 4 - methyl - 2[2 - (methylamino)ethyl] - 1,4 - benzoxazepin - 5(4H) - one, 2,3 - dihydro - 4 - methyl - 2[2 - (methylamino)ethyl] - 1,4 - benzoxazepin - 5(4H) - one fumarate, 2,3 - dihydro - 2 - [2 - (4 - hydroxy - 4 - phenyl) - piperidinylethyl] - 4 - methyl - 1,4 - benzoxazepine - 5(4H) - thione, 2,3 - dihydro - 4 - methyl - 2 - [2 - (4 - phenyl - 1,2,3,6 - tetrahydro)pyridinylethyl] - 1,4 - benzoxazepine - 5(4H) - thione, 8 - chloro - 2 - [2 - (dimethylamino)ethyl] - 2,3 - dihydro - 4 - methyl - 1,4 - benzoxazepine - 5(4H) - thione, 8 - chloro - 2 - [2 - (dimethylamino)-ethyl] - 2,3 - dihydro - 4 - methyl - 1,4 - benzoxazepin - 5(4H) - one, 7 - bromo - 2 - [2 - (dimethylamino)ethyl] - 2,3 - dihydro - 4 - methyl - 1,4 - benzoxazepin - 5(4H) - thione, 2 - [2 - (dimethylamino)-ethyl] - 2,3 - dihydro - 4 - methylnaphth[2,1 - f][1,4]oxazepin - 5(4H) - one, 2 - [2 - (dimethylamino)-ethyl] - 2,3 - dihydro - 4 - methylpyrido[4,3 - f][1,4]oxazepine - 5(4H) - thione, 2 - [2 - (dimethylamino)-ethyl] - 2,3 - dihydro - 4 - methylnaphth[2,3 - f][1,4]oxazepine - 5(4H) - thione, 7 - chloro - 2 - [2 - (dimethylamino)ethyl] - 2,3 - dihydro - 4 - methyl - 1,4 - benzoxazepin - 5(4H) - one, 2 - [2 - (dimethylamino)ethyl] - 2,3 - dihydro - 4 - methylpyrido[3,2 - f][1,4] - oxazepine - 5(4H) - thione methiodide, 7 - chloro - 2 - [2 - (dimethylamino)ethyl] - 2,3 - dihydro - 4 - methyl - 1,4 - benzoxazepine - 5(4H) - thione, 2 - [2 - (dimethylamino)ethyl] - 2,3 - dihydro - 4 - methylnaphth[2,1 - f][1,4] - oxazepine - 5(4H) - thione, 2 - [2 - (dimethylamino)ethyl] - 2,3 - dihydro - 7 - methoxy - 4 - methyl - 1,4 - benzoxapin - 5(4H) - one, 7 - bromo - 2 - [2 - (dimethylamino)ethyl] - 2,3 - dihydro - 4 - methyl - 1,4 - benzoxazepine - 5(4H) - thione, 2 - [2 - (dimethylamino)ethyl] - 2,3 - dihydro - 4 - methylpyrido[3,2 - f][1,4] - thiazepin - 5(4H) - one, 2 - [2 - (dimethylamino)ethyl] - 2,3 - dihydro - 4 - methylpyrido[3,2 - f][1,4] - thiazepine - 5(4H) - thione, 2 - [2 - (dimethylamino)ethyl] - 2,3 - dihydro - 4 - methylpyrido-[3,4 - f][1,4] - oxazepin - 5(4H) - one, 2 - [2 - (dimethylamino)ethyl] - 2,3 - dihydro - 4 - methylpyrido-[3,4 - f]oxazepin - 5(4H) - thione, 2 - (3 - aminopropyl) - 2,3 - dihydro - 4 - methyl - pyrido[3,2 - f][1,4] - oxazepin - 5(4H) - one, 2,3 - dihydro - 4 - methyl - 2 - [2 - (4 - morpholinyl)ethyl] - pyrido[3,2 - f][1,4] - oxazepin - 5(4H) - one, 2,3 - dihydro - 4 - methyl - 2 - [2 - (1 - pyrrolidinyl)ethyl] - pyrido - [3,2 - f][1,4] - oxazepin - 5(4H) - one, 2 - [2 - (dibutylamino)ethyl] - 2,3 - dihydro - 4 - methyl - pyrido[3,2 - f][1,4] - oxazepin - 5(4H) - one, 2 - [2 - (diethylamino)ethyl] - 2,3 - dihydro - 4 - methylpyrido - [3,2 - f][1,4] - oxaxepin - 5(4H) - one, 2,3 - dihydro - 4 - methyl - 2 - [2 - (1 - piperidinyl)ethyl pyrido[3,2 - f][1,4] - oxazepin - 5(4H) - one, 2,3 - dihydro - 4 - methyl - 2 - [2 - (methyl(phenylmethyl)amino)ethyl]-pyrido - [3,2 - f][1,4]oxazepin - 5(4H) - one, 2,3 - dihydro - 4 - methyl - 2 - [2 - (methylphenylamino)-ethyl]pyrido[3,2 - f][1,4] - oxazepin - 5(4H) - one, 2 - [2 - (2,5 - dimethyl - 1 - pyrrolidinyl)ethyl - 2,3 - dihydro - 4 - methyl - pyrido[3,2 - f][1,4] - oxazepin - 5(4H) - one, 2,3 - dihydro - 4 - methyl - 2 - [2 - (2 - methyl - 1 - pyrrolidinyl)ethyl]pyrido - [3,2 - f][1,4] - oxazepin - 5(4H) - one, 2,3 - dihydro - 4 - methyl - 2 - [2 - (1H - pyrazol - 1 - yl)ethyl] - pyrido[3,2 - f][1,4] - oxazepin - 5(4H) - one, 2,3 - dihydro - 2 - [2 - (1H - imidazol - 1 - yl)ethyl - 4 - methylpyrido[3,2 - f][1,4]oxazepin - 5(4H) - one, 2 - [2 - (dimethylamino)ethyl] - 4 - ethyl - 2,3 - dihydropyrido[3,2 - f][1,4] - oxazepin - 5(4H) - one, 2,3 - dihydro - 4 - ethyl - 2 - [2 - (1 - pyrrolidinyl)ethyl]pyrido[3,2 - f][1,4]oxazepin - 5(4H) - one, 2,3 - dihydro - 4 - methyl - 2 - [2 - (4 - morpholinyl)ethyl]pyrido[3,2 - f][1,4]oxazepin - 5(4H) - thione, 2 - [2 - (dibutylamino)ethyl] - 2,3 - dihydro - 4 - methylpyrido[3,2 - f][1,4] - oxazepin - 5(4H) - thione, 2 - [2 - (diethylamino)ethyl] - 2,3 - dihydro - 4 - methylpyrido[3,2 - f][1,4] - oxazepine - 5(4H) - thione, 2,3 - dihydro - 4 - methyl - 2 - [2 - (1 - pyrrolidinyl)ethyl]pyrido - [3,2 - f][1,4]oxazepine - 5(4H) - thione, 2,3 - dihydro - 2 - [2 - (1H - imidazol - 1 - yl)ethyl] - 4 - methylpyrido[3,2 - f][1,4] - oxaepine - 5(4H) - thione,

2 - [2 - (dimethylamino)ethyl] - 4 - ethyl - 2,3 - dihydropyrido[3,2 - f][1,4] - oxazepin - 5(4H) - thione, 2,3 - dihydro - 4 - methyl - 2 - [2 - [methyl(phenylmethyl)amino]ethyl]pyrido[3,2 - f][1,4] - oxazepine - 5(4H) - thione, 2,3 - dihydro - 2 - [(methylamino)ethyl] - 4 - methylpyrido[3,2 - f][1,4] - oxazepine - 5(4H) - thione, 7 - chloro - 2,3 - dihydro - 4 - methyl - 2 - [2 - (1 - pyrrolidino)ethyl]pyrido[3,2 - f][1,4] - oxazepin - 5(4H) - one, 7 - chloro - 2 - [2 - (dimethylamino)ethyl] - 2,3 - dihydro - 4 - methylpyrido-[3,2 - f][1,4] - oxazepin - 5(4H) - one, 2 - [2 - (dimethylamino)ethyl] - 2,3 - dihydro - 4 - phenylmethylpyrido[3,2 - f][1,4] - oxazepin - 5(4H) - one, 2 - [2 - (dimethylamino)ethyl] - 2,3 - dihydropyrido[3,2 - f][1,4] - oxazepin - 5(4H) - one, 2 - [3 - (dimethylamino)propyl] - 2,3 - dihydro - 4 - methylpyrido[3,2 - f][1,4] - oxazepin - 5(4H) - one, 2 - [3 - (dimethylamino)propyl] - 2,3 - dihydro - 4 - methylpyrido[3,2 - f][1,4] - oxazepine - 5(4H) - thione or 7 - chloro - 2 - [2 - (dimethylamino)ethyl] - 2,3 - dihydro - 4 - methylpyrido[3,2 - f]oxazepine - 5(4H) - thione or, where appropriate, a pharmaceutically acceptable salt thereof.

10. A compound having the formula:

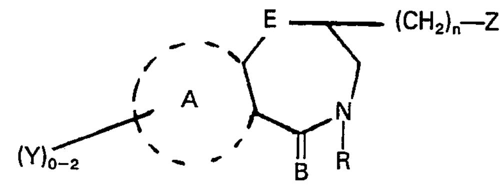

wherein:

A represents an aromatic ring system having two of its carbon atoms held mutually with the oxazepine or thiazepine moiety selected from benzene, naphthalene, and pyridine in any of its four positions, any of the rings being optionally substituted by one or two Y radicals selected from halo, loweralkyl, loweralkoxy, nitro and trifluoromethyl;

B and E are selected from oxygen and sulphur and may be the same or different;

R is selected from hydrogen, loweralkyl, cycloalkyl or phenyl-loweralkyl of which phenyl is optionally substituted by one or two radicals selected from halo, loweralkyl, loweralkoxy, nitro and trifluoromethyl;

n is 1, 2 or 3;

Z is selected from —$NR^1R^2$; 1H-pyrazol-1-yl and 1H-imidazol-1-yl;

each of $R^1$ and $R^2$ is selected from hydrogen, loweralkyl, cycloalkyl and phenyl-loweralkyl of which phenyl is optionally substituted by 1 or 2 radicals selected from halo, loweralkyl, loweralkoxy, nitro, trifluoromethyl and cyano, or $R^1$ and $R^2$ taken together with the adjacent nitrogen atom form a heterocyclic residue selected from 1-pyrrolidinyl, 2,5-dimethylpyrrolidin-1-yl, 2-methylpyrrolidin-1-yl, 1-piperidinyl, 4-substituted piperidine-1-yl, 4-morpholinyl, 1-piperazinyl, 4-substituted piperazin-1-yl and 1,2,3,6-tetrahydro-pyridin-1-yl and the pharmaceutically acceptable salts thereof with the proviso that when R = H, Z is never a primary or secondary amine and a further proviso that when n = 3, Z is not 1H-pyrazol-1-yl, 1H-imidazol-1-yl, 2,5-dimethylpyrrolidin-1-yl, 2-methylpyrrolidin-1-yl; where the term 'loweralkyl' refers to a straight or branched $C_{1-8}$ alkyl group, and 'loweralkoxy' means —O— lower alkyl; for use in medicine.

11. A compound as claimed in any one of Claims 2 to 9 for use in medicine.

12. A process for the preparation of a compound of the formula

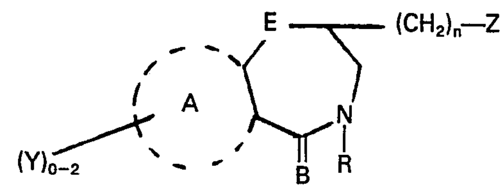

wherein:

A represents an aromatic ring system having two of its carbon atoms held mutually with the oxazepine or thiazepine moiety selected from benzene, naphthalene or a pyridine in any of its four positions, any of the rings being optionally substituted by one or two Y radicals selected from halo, lower alkyl, loweralkoxy, nitro and trifluoromethyl;

B and E are selected from oxygen and sulphur and may be the same or different;

R is selected from hydrogen, loweralkyl, cycloalkyl or phenyl-loweralkyl of which phenyl is optionally substituted by one or two radicals selected from halo, loweralkyl, loweralkoxy, nitro and trifluoromethyl;

n is 1, 2 or 3;

Z is selected from —$NR^1R^2$, 1H-pyrazol-1-yl or 1H-imidazol-1-yl;

$R^1$ and $R^2$ are selected from hydrogen, loweralkyl, cycloalkyl and phenyl-loweralkyl of which phenyl is optionally substituted by 1 or 2 radicals selected from halo, loweralkyl, loweralkoxy, nitro, trifluoromethyl and cyano, or $R^1$ and $R^2$ taken together with the adjacent nitrogen atom form a heterocyclic residue selected from 1-pyrrolidinyl, 2,5-dimethylpyrrolidin-1-yl, 2-methylpyrrolidin-1-yl, 1-piperidinyl, 4-substituted piperidine-1-yl, 4-morpholinyl, 1-piperazinyl, 4-substituted piperazin-1-yl and 1,2,3,6-tetrahydropyridin-1-yl and the pharmaceutically acceptable salts thereof with the proviso that when R is hydrogen, Z is never a

primary or secondary amine and a further proviso that when n = 3, Z is not 1H-pyrazol-1-yl, 1H-imidazol-1-yl, 2,5-dimethylpyrrolidin-1-yl, 2-methylpyrrolidin-1-yl or 1,2,3,6-tetrahydropyridin-1-yl, the process comprising either

i) reacting a compound of the formula

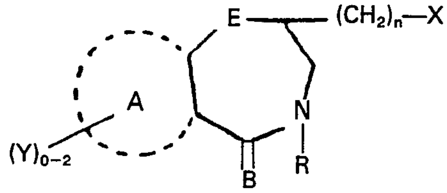

wherein A, B, E, R, Y and n are as defined for formula I and X represents chlorine, bromine or cyano with a compound of the formula

ZH

wherein Z is as defined for formula I; or

ii) reducing a compound of the formula

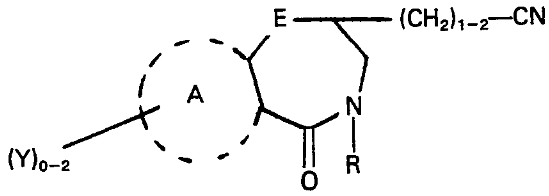

to give a compound of formula I in which B represents oxygen, Z represents —$NH_2$ and n is 2 or 3, and if necessary

iii) converting a compound of formula I so formed into another compound of formula I; and optionally

iv) preparing a pharmaceutically acceptable salt of a compound so formed; where the term 'loweralkyl' refers to a straight or branched $C_{1-8}$ alkyl group, and 'loweralkoxy' means —O— loweralkyl.

13. A process as claimed in Claim 12 for preparing a compound as claimed in any one of Claims 2 to 9.

14. A compound of the formula:

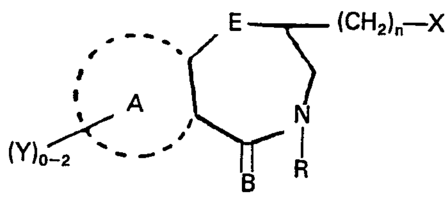

wherein:

A represents an aromatic ring system having two of its carbon atoms held mutually with the oxazepine or thiazepine moiety selected from benzene, a naphthalene and a pyridine in any of its four positions, any of which ring systems are optionally substituted by one or two Y radicals selected from halo, loweralkyl, loweralkoxy, nitro or trifluoromethyl;

B and E are selected from oxygen and sulphur and may be the same or different;

R is selected from loweralkyl, cycloalkyl or phenyl-loweralkyl, wherein phenyl is optionally substituted by one or two radicals selected from halo, loweralkyl, loweralkoxy, nitro and trifluoromethyl;

n is 1 or 2;

X is chlorine, bromine or cyano;

where the term 'loweralkyl' refers to a straight or branched $C_{1-8}$ alkyl group, and 'loweralkoxy' means —O— loweralkyl.

15. A compound as claimed in Claim 14, wherein n is 2.

16. A compound as claimed in Claim 14 or 15, wherein A represents a benzene ring.

17. A compound as claimed in Claim 14 or 15, wherein A represents a naphthalene ring system.

18. A compound as claimed in Claim 13 or 14, wherein A represents a pyridine ring.

19. A compound as claimed in any one of claims 13 to 17, wherein the ring represented by A is substituted by one or more chlorine or bromine atoms or by a methoxy group.

20. A compound as claimed in any one of Claims 13 to 18, wherein R represents a methyl, ethyl, benzyl or hexyl group.

21. 2 - (2 - Chloroethyl) - 2,3 - dihydro - 4 - methyl - 1,4 - benzoxazepin - 5(4H) - one, 4 - benzyl - 2 - (2 - chloroethyl) - 2,3 - dihydro - 1,4 - benzoxazepin - 5(4H) - one, 2 - (2 - chloroethyl) - 2,3 - dihydro - 4 - methylnaphth - [2,3 - f][1,4] oxazepin - 5(4H) - one, 2 - (2 - chloroethyl) - 2,3 - dihydro - 4 - methylpyrido[3,2 - f][1,4] - oxazepin - 5(4H) - one, 8 - chloro - 2 - (2 - chloroethyl) - 2,3 - dihydro - 4 - methyl - 1,4 - benzoxapin - 5(4H) - one, 7 - bromo - 2 - (2 - chloroethyl) - 2,3 - dihydro - 4 - methyl - 1,4 - benzoxazepin - 5(4H) - one, 7 - chloro - 2 - (2 - chloroethyl) - 2,3 - dihydro - 4 - methyl -

1,4 - benzoxazepin - 5(4H) - one, 2 - (2 - chloroethyl) - 2,3 - dihydro - 4 - methylnaphth[2,1 - f][1,4] - oxazepin - 5(4H) - one, 2 - (2 - chloroethyl) - 2,3 - dihydro - 7 - methoxy - 4 - methyl - 1,4 - benzoxazepin - 5(4H) - one, 2 - (2 - chloroethyl) - 2,3 - dihydro - 4 - methyl - 1,4 - benzoxazepine - 5(4H) - thione, 2 - (2 - chloroethyl) - 2,3 - dihydro - 4 - methylpyrido[3,2 - f][1,4] - oxazepin - 5(4H) - thione, 2 - (2 - chloroethyl) - 2,3 - dihydro - 4 - methylnaphth[2,3 - f][1,4] - oxazepine - 5(4H) - thione, 8 - chloro - 2 - (2 - chloroethyl) - 2,3 - dihydro - 4 - methyl - 1,4 - benzoxazepine - 5(4H) - thione, 7 - bromo - 2 - (2 - chloroethyl) - 2,3 - dihydro - 4 - methyl - 1,4 - benzoxazepine - 5(4H) - thione, 2 - (2 - chloroethyl) - 2,3 - dihydro - 4 - methylnaphth[2,1 - f][1,4] - oxazepine - 5(4H) - thione, 2 - (2 - chloroethyl) - 2,3 - dihydro - 4 - methyl - pyrido[4,3 - f][1,4] - oxazepine - 5(4H) - one, 7 - chloro - 2 - (2 - chloroethyl) - 2,3 - dihydro - 4 - methyl - 1,4 - benzoxazepine- 5(4H) - thione, 2 - (2 - chloroethyl) - 2,3 - dihydro - 4 - methyl - pyrido[4,3 - f][1,4]oxazepine - 5(4H) - thione, 2 - (2 - chloroethyl) - 2,3 - dihydro - 7 - methoxy - 4 - methyl - 1,4 - benzoxazepine - 5(4H) - thione, 2 - (2 - chloroethyl) - 2,3 - dihydro - 4 - methylpyrido[3,2 - f][1,4] - thiazepin - 5(4H) - one, 2 - (2 - chloroethyl) - 2,3 - dihydro - 4 - methylpyrido[3,2 - f][1,4] - thiazepine - 5(4H) - thione, 2 - (2 - chloroethyl) - 2,3 - dihydro - 4 - methylpyrido[3,4 - f][1,4]oxazepin - 5(4H) - one, 2 - (2 - chloroethyl) - 2,3 - dihydro - 4 - methylpyrido[3,4 - f][1,4]oxazepine - 5(4H) - thione, 2,3,4,5 - tetrahydro - 4 - methyl - 5 - oxopyrido[3,2 - f][1,4]oxazepine - 2 - propanenitrile, 2 - (2 - chloroethyl) - 4 - ethyl - 2,3 - dihydropyrido[3,2 - f][1,4] - oxazepin - 5(4H) - one, 2 - (2 - chloroethyl) - 4 - ethyl - 2,3 - dihydropyrido[3,2 - f][1,4]oxazepin - 5(4H) - thione, 7 - chloro - 2 - (2 - chloroethyl) - 2,3 - dihydro - 4 - methylpyrido[3,2 - f][1,4] - oxazepin - 5(4H) - one, 7 - chloro - 2 - (2 - chloroethyl) - 2,3 - dihydro - 4 - methylpyrido[3,2 - f][1,4] - oxazepine - 5(4H) - thione or 2 - (chloromethyl) - 4 - cyclohexyl - 2,3 - dihydropyrido[3,2 - f][1,4]oxazepin - 5(4H) - one or an acid addition salt thereof.

22. A process for the preparation of a compound of the formula:

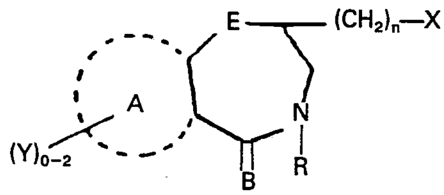

wherein:

A selected from an aromatic ring system having two of its carbon atoms held mutually with the oxazepine or thiazepine moiety selected from benzene, a naphthalene, a pyridine in any of its four positions, any of which ring system is optionally substituted by one or two Y radicals selected from halo, loweralkyl, loweralkoxy, nitro or trifluoromethyl;

B and E are selected from oxygen and sulphur and may be the same or different;

R is selected from loweralkyl, cycloalkyl or phenyl-loweralkyl wherein phenyl is optionally substituted by loweralkoxy, nitro or trifluoromethyl;

n is 1 or 2;

X is chlorine, bromine or cyano or an acid addition salt thereof, the process comprising

i) a) fusing a compound having the formula

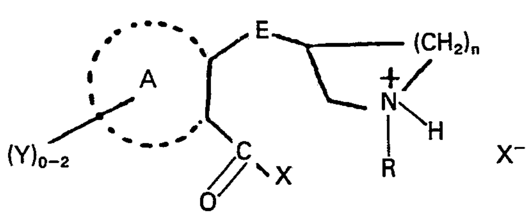

wherein x represents chlorine or bromine and A, E, n, R and Y are as defined above;

b) and when required reacting the compound so formed with a sulphurizing agent to obtain an oxazepinethione or a thiazepinethione; and

c) optionally reacting a compound obtained in step i)a) or i)b) with an alkali metal cyanide to obtain the corresponding compound in which X represents CN; or

ii) halogenating a compound obtained in step i)a), i)b) or i)c) having the formula

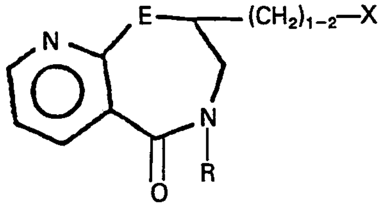

wherein E and R are as defined above and X is chlorine, bromine or cyano with sulphuryl chloride in a suitable solvent to obtain a compound having the formula

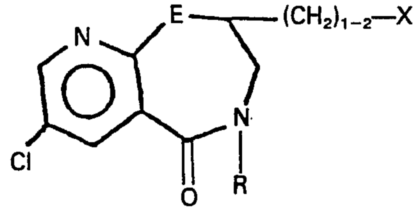

wherein E and R are as defined above and X is chlorine, bromine or cyano; where the term 'loweralkyl' refers to a straight or branched $C_{1-8}$ alkyl group, and 'loweralkoxy' means —O— loweralkyl.

23. A process as claimed in Claim 22 for preparing a compound as claimed in any one of Claims 15 to 21.

24. A compound selected from the group having the formula:

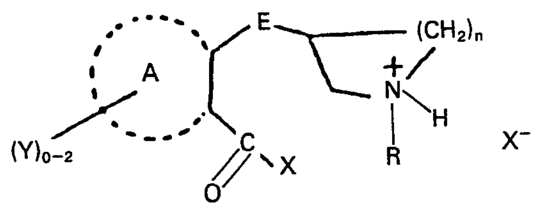

wherein:

A represents an aromatic ring system selected from benzene, a naphthalene or a pyridine in any of its four positions, any of which ring systems is optionally substituted by one or two Y radicals selected from halo, loweralkyl, loweralkoxy, nitro or trifluoromethyl;

R is selected from loweralkyl, cycloalkyl or phenyl-loweralkoxy, nitro and trifluoromethyl;

n is 1 or 2;

X is selected from chlorine, bromine and fluorine and E is selected from sulphur and oxygen; and where the term 'loweralkyl' refers to a straight or branched $C_{1-8}$ alkyl group, and 'loweralkoxy' means —O— loweralkyl.

25. A compound having the formula:

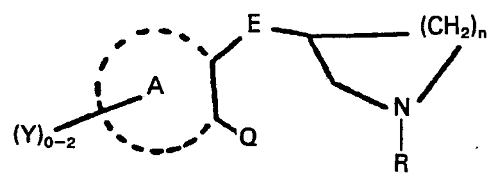

wherein:

A represents an aromatic ring system selected from a naphthalene or a pyridine in any of its four positions, any of which ring systems is optionally substituted by one or two radicals selected from the group consisting of halo, loweralkyl, loweralkoxy, nitro or trifluoromethyl;

R is selected from loweralkyl, cycloalkyl, and phenyl-loweralkyl wherein phenyl is optionally substituted by one or two radicals selected from halo, loweralkyl, loweralkoxy, nitro and trifluoromethyl;

n is 1 or 2;

Q is selected from

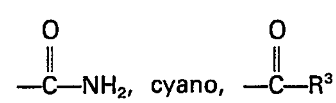

cyano,

wherein $R^3$ is H, an acid neutralizing ion or an esterifying radical, and E is oxygen or sulphur; where the term 'loweralkyl' refers to a straight or branched $C_{1-8}$ alkyl group, and 'loweralkoxy' means —O— loweralkyl.

26. 3-[(1-Methyl-3-pyrrolidinyl)oxy]-2-naphthalenecarboxamide, 3-[(1-methyl-3-pyrrolidinyl)oxy]-2-naphthalenecarboxylic acid, its oxalate salt, sodium 2-[(1-methyl-3-pyrrolidinyl)oxy]-3-pyridinecarboxylate, the free base thereof, 1-[(1-methyl-3-pyrrolidinyl)oxy]-2-naphthalenecarboxamide, 3-[(1-methyl-3-pyrrolidinyl)oxy]-4-pyridinecarbonitrile, its fumarate salt, or 1-[(1-methyl-3-pyrrolidinyl)oxy]-2-naphthalenecarbonitrile or its oxalate salt.

27. A pharmaceutical composition comprising

a) a compound having the formula:

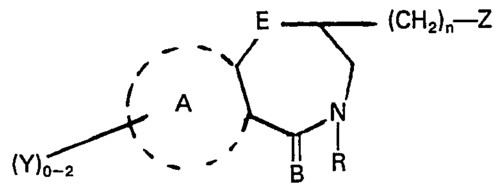

wherein:

A represents an aromatic system, having two of its carbon atoms held mutually with the oxazepine or thiazepine moiety, selected from benzene, a naphthalene or a pyridine in any of its four positions, any of which ring systems is optionally substituted by one or two Y radicals selected from halo, loweralkyl, loweralkoxy, nitro and trifluoromethyl;

B and E are selected from oxygen and sulphur and may be the same or different;

R is selected from hydrogen, loweralkyl, cycloalkyl or phenyl-loweralkyl or which phenyl may be optionally substituted by one or two radicals selected from halo, loweralkyl, loweralkoxy, nitro, trifluoromethyl and cyano;.

n is one, two or three;

Z is selected from —$NR^1R^2$, 1H-pyrazol-1-yl, or 1H-imidazol-1-yl;

each of $R^1$ and $R^2$ is selected from hydrogen, loweralkyl, cycloalkyl and phenyl-loweralkyl of which phenyl is optionally substituted by 1 or 2 radicals selected from halo, loweralkyl, loweralkoxy, nitro, trifluoromethyl and cyano, or $R^1$ and $R^2$ taken together with the adjacent nitrogen atom form a heterocyclic residue selected from 1-pyrrolidinyl, 2,5-dimethylpyrrolidin-1-yl, 2-methyl-pyrrolidin-1-yl, 1-piperidinyl, 4-substituted piperidine-1-yl, 4-morpholinyl, 1-piperazinyl, 4-substituted piperazin-1-yl and 1,2,3,6-tetrahydropyridin-1-yl and the pharmaceutically acceptable salts thereof, with the proviso that when R = H, Z is never a primary or secondary amine and a further proviso that when n = 3, Z is not 1H-pyrazol-1-yl, 1H-imidazol-1-yl, 2,5-dimethylpyrrolidin-1-yl, 2-methylpyrrolidin-1-yl or 1,2,3,6-tetrahydropyridin-1-yl; where the term 'loweralkyl' refers to a straight or branched $C_{1-8}$ alkyl group, and 'loweralkoxy' means —O—loweralkyl; and

b) a pharmaceutical carrier therefor.

28. A pharmaceutical composition comprising a) a compound as claimed in any one of Claims 2 to 9; and b) a pharmaceutical carrier therefor.

**Claims for the Contracting State: AT**

1. A process for the preparation of a compound having the formula:

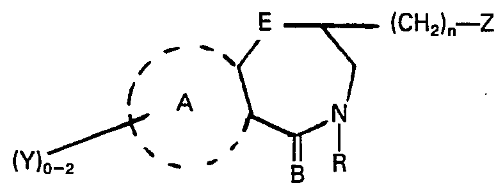

wherein:

A represents an aromatic ring system having two of its carbon atoms held mutually with the oxazepine or thiazepine moiety selected from benzene, naphthalene or a pyridine in any of its four positions, any of the rings being optionally substituted by one or two Y radicals selected from halo, loweralkyl, loweralkoxy, nitro and trifluoromethyl;

B and E are selected from oxygen and sulphur and may be the same or different;

R is selected from hydrogen, lower-alkyl, $C_3$—$C_9$ cycloalkyl or phenyl-loweralkyl, of which phenyl is optionally substituted by one or two radicals selected from halo, loweralkyl, loweralkoxy, nitro or trifluoromethyl;

n is 1, 2 or 3;

Z is selected from —$NR^1R^2$, 1H-pyrazol-1-yl and 1H-imidazol-1-yl;

each of $R^1$ and $R^2$ is selected from hydrogen, loweralkyl, $C_3$—$C_9$ cycloalkyl and phenyl-loweralkyl of which phenyl is optionally substituted by 1 to 2 radicals selected from halo, loweralkyl, loweralkoxy, nitro, trifluoromethyl and cyano, or $R^1$ and $R^2$ taken together with the adjacent nitrogen atom form a heterocyclic residue selected from 1-pyrrolidinyl, 2,5-dimethylpyrrolidin-1-yl, 2-methylpyrrolidin-1-yl, 1-piperidinyl, 4-substituted piperidine-1-yl, 4-morpholinyl, 1-piperazinyl, 4-substituted piperazin-1-yl and 1,2,3,6-tetrahydropyridin-1-yl and the pharmaceutically acceptable salts thereof with the proviso that when R = H, Z is never a primary or secondary amine and a further proviso that when n = 3, Z is not 1H-pyrazol-1-yl, 1H-imidazol-1-yl, 2,5-dimethylpyrrolidin-1-yl, 2-methylpyrrolidin-1-yl or 1,2,3,6-tetrahydropyridin-1-yl; where the term 'lower alkyl' refers to a straight or branched $C_{1-8}$ alkyl group, and 'lower alkoxy' means —O—loweralkyl; the process comprising either

i) reacting a compound of the formula

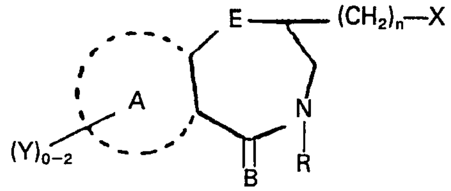

wherein A, B, E, R, Y and n are as defined for formula I and X represents chlorine, bromine or cyano with a compound of the formula

ZH

wherein Z is as defined for formula I; or

ii) reducing a compound of the formula

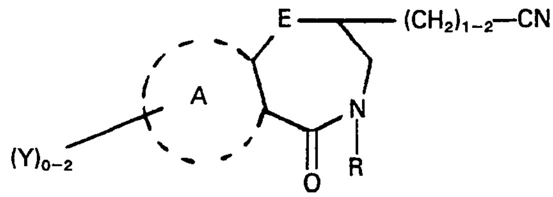

to give a compound of formula I in which B represents oxygen, Z represents $—NH_2$ and n is 2 or 3, and if necessary

iii) converting a compound of formula I so formed into another compound of formula I; and optionally

iv) preparing a pharmaceutically acceptable salt of a compound so formed; where the term 'lower alkyl' refers to a straight or branched $C_{1-8}$ alkyl group, and 'lower alkoxy' means —O— lower alkyl.

2. A process as claimed in Claim 1, wherein n is 2.

3. A process as claimed in Claim 1 or 2, wherein A represents a benzene ring.

4. A process as claimed in Claim 1 or 2, wherein A represents a naphthalene ring system.

5. A process as claimed in Claim 1 or 2, wherein A represents a pyridine ring.

6. A process as claimed in any one of Claims 1 to 5, wherein the ring represented by A is substituted by one or more chlorine or bromine atoms or by a methoxy group.

7. A process as claimed in any one of Claims 1 to 6 wherein R represents a methyl, ethyl, benzyl or hexyl group.

8. A process as claimed in any one of Claims 1 to 7, wherein Z represents a dimethylamino group.

9. A process as claimed in claim 1 for the preparation of 2 - [2 - (Dimethylamino)ethyl] - 2,3 - dihydro - 4 - methyl - 1,4 - benzoxazepin - 5(4H) - one, 2,3 - dihydro - 4 - methyl - 2 - [2 - (4 - morpholino)ethyl] - 1,4 - benzoxazepin - 5(4H)one, 4 - benzyl - 2,3 - dihydro - 2 - [2 - (4 - morpholino)-ethyl] - 1,4 - benzoxazepin - 5(4H) one, 4 - benzyl - 2,3 - dihydro - 2 - [2 - (methylamino)ethyl] - 1,4 - benzoaxazepin - 5(4H) - one, 2 - [2 - (dimethylamino)ethyl] - 2,3 - dihydro - 4 - methyl - 1,4 - benzoaxazepine - 5(4H) - thione, 4 - benzyl - 2 - [2 - (dimethylamino)ethyl] - 2,3 - dihydro - 1,4 - benzoxazepin - 5(4H) - one, 2,3 - dihydro - 4 - methyl - 2 - [2 - (4 - morpholino)ethyl] - 1,4 - benzoxazepine - 5(4H) - thione, 2 - [2 - (dimethylamino)ethyl] - 2,3 - dihydro - 4 - methylnaphth[2,3-f][1,4]oxazepin - 5(4H) - one, 2 - [2 - (dimethylamino)ethyl] - 2,3 - dihydro - 4 - methylpyrido[3,2 - f][1,4]oxazepin - 5(4H) - one, 2 - [2 - (dimethylamino)ethyl] - 2,3 - dihydro - 4 - methylpyrido[3,2 - f][1,4]oxazepine - 5(4H) - thione, 4 - benzyl - 2,3 - dihydro - 2 - [2 - (4 - morpholino)ethyl] - 1,4 - benzoxazepine - 5(4) - thione, 2,3 - dihydro - 4 - methyl - 2[2 - (methylamino)ethyl] - 1,4 - benzoxazepin - 5(4H) - one, 2,3 - dihydro - 4 - methyl - 2[2 - (methylamino)ethyl] - 1,4 - benzoxazepin - 5(4H) - one fumarate, 2,3 - dihydro - 2 - [2 - (4 - hydroxy - 4 - phenyl) - piperidinylethyl] - 4 - methyl - 1,4 - benzoxazepine - 5(4H) - thione, 2,3 - dihydro - 4 - methyl - 2 - [2 - (4 - phenyl - 1,2,3,6 - tetrahydro)pyridinylethyl] - 1,4 - benzoxazepine - 5(4H) - thione, 8 - chloro - 2 - [2 - (dimethylamino)ethyl] - 2,3 - dihydro - 4 - methyl - 1,4 - benzoxazepine - 5(4H) - thione, 8 - chloro - 2 - [2 - (dimethylamino)-ethyl] - 2,3 - dihydro - 4 - methyl - 1,4 - benzoxazepin - 5(4H) - one, 7 - bromo - 2 - [2 - (dimethylamino)ethyl - 2,3 - dihydro - 4 - methyl - 1,4 - benzoxazepin - 5(4H) - thione, 2 - [2 - (dimethylamino)-ethyl] - 2,3 - dihydro - 4 - methylnaphth[2,1 - f][1,4]oxazepin - 5(4H) - one, 2 - [2 - (dimethylamino)-ethyl] - 2,3 - dihydro - 4 - methylpyrido[4,3 - f][1,4]oxazepine - 5(4H) - thione, 2 - [2 - (dimethylamino)-ethyl] - 2,3 - dihydro - 4 - methylnaphth[2,3 - f][1,4]oxazepine - 5(4H) - thione - 7 - chloro - 2 - [2 - (dimethylamino)ethyl] - 2,3 - dihydro - 4 - methyl - 1,4 - benzoxazepin - 5(4H) - one, 2 - [2 - (dimethylamino)ethyl] - 2,3 - dihydro - 4 - methylpyrido(3,2 - f][1,4] - oxazepine - 5(4H) - thione methiodide, 7 - chloro - 2 - [2 - (dimethylamino)ethyl] - 2,3 - dihydro - 4 - methyl - 1,4 - benzoxazepine - 5(4H) - thione, 2 - [2 - (dimethylamino)ethyl] - 2,3 - dihydro - 4 - methylnaphth[2,1 - f][1,4] - oxazepine - 5(4H) - thione, 2 - 2 - (dimethylamino)ethyl - 2,3 - dihydro - 7 - methoxy - 4 - methyl - 1,4 - benzoxapin - 5(4H) - one, 7 - bromo - 2 - [2 - (dimethylamino)ethyl] - 2,3 - dihydro - 4 - methyl - 1,4 -

benzoxazepine - 5(4H) - thione, 2 - [2 - (dimethylamino)ethyl] - 2,3 - dihydro - 4 - methylpyrido[3,2 - f][1,4] - thiazepin - 5(4H) - one, 2 - [2 - (dimethylamino)ethyl] - 2,3 - dihydro - 4 - methylpyrido[3,2 - f][1,4] - thiazepine - 5(4H) - thione, 2 - [2 - (dimethylamino)ethyl] - 2,3 - dihydro - 4 - methylpyrido-[3,4 - f][1,4] - oxazepin - 5(4H) - one, 2 - [2 - (dimethylamino)ethyl] - 2,3 - dihydro - 4 - methylpyrido-[3,4 - f]oxazepin - 5(4H) - thione, 2 - (3 - aminopropyl) - 2,3 - dihydro - 4 - methyl - pyrido[3,2 - f][1,4] - oxazepin - 5(4H) - one, 2,3 - dihydro - 4 - methyl - 2 - [2 - (4 - morpholinyl)ethyl] - pyrido[3,2 - f][1,4] - oxazepin - 5(4H) - one, 2,3 - dihydro - 4 - methyl - 2 - [2 - (1 - pyrroldinyl)ethyl] - pyrido - [3,2 - f][1,4] - oxazepin - 5(4H) - one, 2 - [2 - (dibutylamino)ethyl] - 2,3 - dihydro - 4 - methyl - pyrido[3,2 - f][1,4] - oxazepin - 5(4H) - one, 2 - [2 - (diethylamino)ethyl] - 2,3 - dihydro - 4 - methylpyrido - [3,2 - f][1,4] - oxaxepin - 5(4H) - one, 2,3 - dihydro - 4 - methyl - 2 - [2 - (l - piperidinyl)ethyl pyrido[3,2 - f][1,4] - oxazepin - 5(4H) - one, 2,3 - dihydro - 4 - methyl - 2 - [2 - (methyl(phenylmethyl)amino)ethyl]-pyrido - [3,2 - f][1,4] - oxazepin - 5(4H) - one, 2,3 - dihydro - 4 - methyl - 2 - [2 - (methylphenylamino)-ethyl]pyrido[3,2 - f][1,4] - oxazepin - 5(4H) - one, 2 - [2 - (2,5 - dimethyl - 1 - pyrrolidinyl)ethyl - 2,3 - dihydro - 4 - methyl - pyrido[3,2 - f][1,4] - oxazepin - 5(4H) - one, 2,3 - dihydro - 4 - methyl - 2 - [2 - (2 - methyl - 1 - pyrrolidinyl)ethyl]pyrido - [3,2 - f][1,4] - oxazepin - 5(4H) - one, 2,3 - dihydro - 4 - methyl - 2 - [2 - (1H - pyrazol - 1 - yl)ethyl] - pyrido[3,2 - f][1,4] - oxazepin - 5(4H) - one, 2,3 - dihydro - 2 - [2 - (1H - imidazol - 1 - yl)ethyl] - 4 - methylpyrido[3,2 - f][1,4] - oxazepin - 5(4H) - one, 2 - [2 - (dimethylamino)ethyl] - 4 - ethyl - 2,3 - dihydropyrido[3,2 - f][1,4] - oxazepin - 5(4H) - one, 2,3 - dihydro - 4 - ethyl - 2 - [2 - (1 - pyrrolidinyl)ethyl]pyrido[3,2 - f][1,4] - oxazepin - 5(4H) - one, 2,3 - dihydro - 4 - methyl - 2 - [2 - (4 - morpholinyl)ethyl]pyrido[3,2 - f][1,4] - oxazepin - 5(4H) - thione, 2 - [2 - (dibutylamino)ethyl] - 2,3 - dihydro - 4 - methylpyrido[3,2 - f][1,4] - oxazepin - 5(4H) - thione, 2 - [2 - (diethylamino)ethyl] - 2,3 - dihydro - 4 - methylpyrido[3,2 - f][1,4] - oxazepine - 5(4H) - thione, 2,3 - dihydro - 4 - methyl - 2 - [2 - (1 - pyrrolidinyl)ethyl]pyrido - [3,2 - f][1,4]oxazepine - 5(4H) - thione, 2,3 - dihydro - 2 - [2 - (1H - imidazol - 1 - yl)ethyl] - 4 - methylpyrido[3,2 - f][1,4] - oxazepine - 5(4H) - thione, 2 - [2 - (dimethylamino)ethyl] - 4 - ethyl 2,3 - dihydropyrido[3,2 - f][1,4] - oxazepin - 5(4H) - thione, 2,3 - dihydro - 4 - methyl - 2 - [2 - [methyl(phenylmethyl)amino]ethyl]pyrido[3,2 - f][1,4] - oxazepine - 5(4H) - thione, 2,3 - dihydro - 2 - [methylamino)ethyl] - 4 - methylpyrido[3,2 - f][1,4] - oxazepine - 5(4H) - thione, 7 - chloro - 2,3 - dihydro - 4 - methyl - 2 - [2 - (1 - pyrrolidino)ethyl]pyrido-[3,2 - f][1,4] - oxazepin - 5(4H) - one, 7 - chloro - 2 [2 - (dimethylamino)ethyl] - 2,3 - dihydro - 4 - methylpyrido[3,2 - f][1,4] - oxazepin - 5(4H) - one, 2 - [2 - (dimethylamino)ethyl] - 2,3 - dihydro - 4 - phenylmethylpyrido[3,2 - f][1,4] - oxazepin - 5(4H) - one, 2 - [2 - (dimethylamino)ethyl] - 2,3 - dihydro-pyrido[3,2 - f][1,4] - oxazepin - 5(4H) - one, 2 - [3 - (dimethylamino)propyl] - 2,3 - dihydro - 4 - methyl-pyrido[3,2 - f][1,4] - oxazepin - 5(4H) - one, 2 - [3 - (dimethylamino)propyl] - 2,3 - dihydro - 4 - methyl-pyrido[3,2 - f][1,4] - oxazepine - 5(4H) - thione or 7 - chloro - 2 - [2 - (dimethylamino)ethyl] - 2,3 - dihydro - 4 - methylpyrido[3,2 - f]oxazepine - 5(4H) - thione or, where appropriate, a pharmaceutically acceptable salt thereof.

10. A process for the preparation of a compound of the formula:

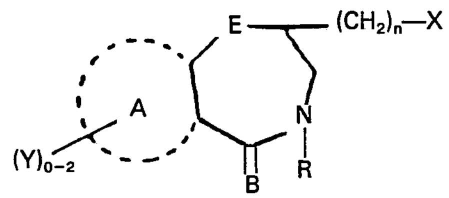

wherein:

A represents an aromatic ring system having two of its carbon atoms held mutually with the oxazepine or thiazepine moiety selected from benzene, a naphthalene and a pyridine in any of its four positions, any of which ring systems is optionally substituted by one or two Y radicals selected from halo, loweralkyl, loweralkoxy, nitro or trifluoromethyl;

B and E are selected from oxygen and sulphur and may be the same or different;

R is selected from loweralkyl, cycloalkyl or phenyl-loweralkyl wherein phenyl is optionally substituted by one or two radicals selected from halo, loweralkyl, loweralkoxy, nitro or trifluoromethyl;

n is 1 or 2;

X is chlorine, bromine or cyano;

where the term 'lower alkyl' refers to a straight or branched $C_{1-8}$ alkyl group, and 'lower alkoxy' means —O— lower alkyl; the process comprising

i) a) fusing a compound having the formula

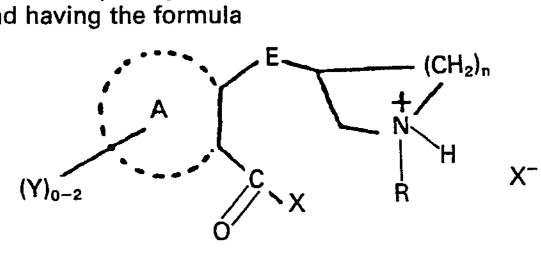

wherein x represents chlorine or bromine and A, E, n, R and Y are as defined above;

b) and when required reacting the compound so formed with a sulphurizing agent to obtain an oxazepinethione or a thiazepinethione; and

c) optionally reacting a compound obtained in step i)a) or i)b) with an alkali metal cyanide to obtain the corresponding compound in which X represents CN; or

ii) halogenating a compound obtained in step i)a), i)b) or i)c) having the formula

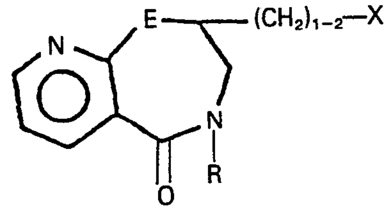

wherein E and R are as defined above and X is chlorine, bromine or cyano with sulphuryl chloride in a suitable solvent to obtain a compound having the formula

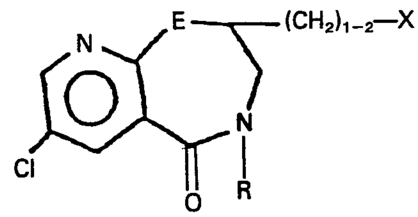

wherein E and R are as defined above and X is chlorine, bromine or cyano; where the term 'lower alkyl' refers to a straight or branched $C_{1-8}$ alkyl group, and 'lower alkoxy' means —O— loweralkyl.

11. A process as claimed in Claim 10, wherein n is 2.

12. A process as claimed in Claim 10 or 11, wherein A represents a benzene ring.

13. A process as claimed in Claim 10 or 11, wherein A represents a naphthalene ring system.

14. A process as claimed in Claim 10 or 11, wherein A represents a pyridine ring.

15. A process as claimed in any one of claims 10 to 14, wherein the ring represented by A is substituted by one or more chlorine or bromine atoms or by a methoxy group.

16. A process as claimed in any one of Claims 10 to 15, wherein R represents a methyl, ethyl, benzyl or hexyl group.

17. A process as claimed in Claim 10 for the preparation of 2 - (2-Chloroethyl) - 2,3 - dihydro - 4 - methyl - 1,4 - benzoxazepin - 5 - (4H) - one, 4 - benzyl - 2 - (2 - chloroethyl) - 2,3 - dihydro - 1,4 - benzoxazepin - 5(4H) - one, 2 - (2 - chloroethyl) - 2,3 - dihydro - 4 - methylnaphth - [2,3 - f][1,4] oxazepin - 5(4H) - one, 2 - (2 - chloroethyl) - 2,3 - dihydro - 4 - methylpyrido[3,2 - f][1,4] - oxazepin - 5(4H) - one, 8 - chloro - 2 - (2 - chloroethyl) - 2,3 - dihydro - 4 - methyl 1,4 - benzoxapin - 5(4H) - one, 7 - bromo - 2 - (2 - chloroethyl) - 2,3 - dihydro - 4 - methyl - 1,4 - benzoxazepin - 5(4H) - one, 7 - chloro - 2 - (2 - chloroethyl) - 2,3 - dihydro - 4 - methyl - 1,4 - benzoxazepin - 5(4H) - one, 2 - (2 - chloroethyl) - 2,3 - dihydro - 4 - methylnaphth[2,1 - f][1,4] - oxazepin - 5(4H) - one, 2 - (2 - chloroethyl) - 2,3 - dihydro - 7 - methoxy - 4 - methyl - 1,4 - benzoxazepin - 5(4H) - one, 2 - (2 - chloroethyl - 2,3 - dihydro - 4 - methyl - 1,4 - benzoaxazepine - 5(4H) - thione. 2 - (2 - chloroethyl) - 2,3 - dihydro - 4 - methylpyrido[3,2 - f][1,4] - oxazepin - 5(4H) - thione, 2 - (2 - chloroethyl) - 2,3 - dihydro - 4 - methylnaphth[2,3 - f][1,4] - oxazepine - 5(4H) - thione, 8 - chloro - 2 - (2 - chloroethyl) - 2,3 - dihydro - 4 - methyl - 1,4 - benzoxazepine - 5(4H) - thione, 7 - bromo - 2 - (2 - chloroethyl) - 2,3 - dihydro - 4 - methyl - 1,4 - benzoxazepine - 5(4H) - thione, 2 - (2 - chloroethyl) - 2,3 - dihydro - 4 - methylnaphth[2,1 - f][1,4] - oxazepine - 5(4H) - thione, 2 - (2 - chloroethyl) - 2,3 - dihydro - 4 - methyl - pyrido[4,3 - f][1,4] - oxazepine - 5(4H) - one, 7 - chloro - 2 - (2 - chloroethyl) - 2,3 - dihydro - 4 - methyl - 1,4 - benzoxazepine- 5 (4H) - thione, 2 - (2 - chloroethyl) - 2,3 - dihydro - 4 - methyl - pyrido[4,3 - f][1,4] - oxazepine - 5(4H) - thione, 2 - (2 - chloroethyl) - 2,3 - dihydro - 7 - methoxy - 4 - methyl - 1,4 - benzoxazepine - 5(4H) - thione, 2 - (2 - chloroethyl) - 2,3 - dihydro - 4 - methylpyrido[3,2 - f][1,4] - thiazepin - 5(4H) - one, 2 - (2 - chloroethyl) - 2,3 - dihydro - 4 - methylpyrido[3,2 - f][1,4] - thiazepine - 5(4H) - thione, 2 - (2 - chloroethyl) - 2,3 - dihydro - 4 - methylpyrido[3,4 - f][1,4] - oxazepin - 5(4H) - one, 2 - (2 - chloroethyl) - 2,3 - dihydro - 4 - methylpyrido[3,4 - f][1,4] - oxazepine - 5(4H) - thione, 2,3,4,5 - tetrahydro - 4 - methyl - 5 - oxopyrido[3,2 - f][1,4] - oxazepine - 2 - propanenitrile, 2 - (2 - chloroethyl) - 4 - ethyl - 2,3 - dihydropyrido[3,2 - f][1,4] - oxazepin - 5(4H) - one, 2 - (2 - chloroethyl) - 4 - ethyl - 2,3 - dihydropyrido[3,2 - f][1,4] - oxazepin - 5(4H) - thione, 7 - chloro - 2 - (2 - chloroethyl) - 2,3 - dihydro - 4 - methylpyrido[3,2 - f][1,4] - oxazepin - 5(4H) - one, 7 - chloro - 2 - (2 - chloroethyl) - 2,3 - dihydro - 4 - methylpyrido[3,2 - f][1,4] - oxazepine - 5(4H) - thione or 2 - (chloromethyl) - 4 - cyclohexyl - 2,3 - dihydropyrido[3,2 - f][1,4] - oxazepin - 5(4H) - one or an acid addition salt thereof.

18. A process for the preparation of a compound having the formula:

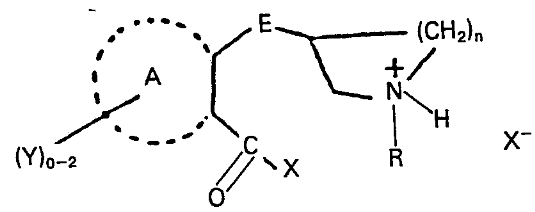

wherein:

A represents an aromatic ring system having selected from benzene, a naphthalene or a pyridine in any of its four positions, any of which rings systems is optionally substituted by one or two Y radicals selected from halo, loweralkyl, loweralkoxy, nitro and trifluoromethyl;

R is selected from loweralkyl, cycloalkyl or phenyl-loweralkyl, nitro and trifluoromethyl;

n is 1 or 2;

X is selected from chlorine, bromine and fluorine and E is selected from sulphur and oxygen; and where the term 'lower alkyl' refers to a straight or branched $C_{1-8}$ alkyl group, and 'lower alkoxy' means —O— lower alkyl, the process comprising halogenating a compound of the formula IVb

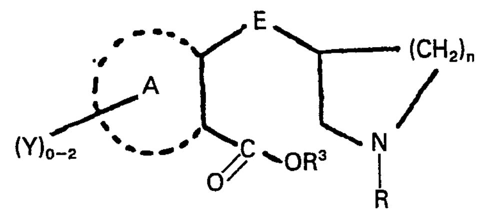

IVb

wherein A, E, R, Y and n are as given above and $R^3$ is hydrogen or an acid neutralizing ion.

19. A process for the preparation of a compound having the formula:

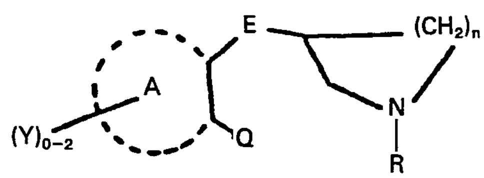

wherein:

A represents an aromatic ring system selected from a naphthalene or a pyridine in any of its four positions, any of which ring systems is optionally substituted by one or two radicals selected from the group consisting of halo, loweralkyl, loweralkoxy, nitro or trifluoromethyl;

R is selected from loweralkyl, cycloalkyl and phenyl-loweralkyl wherein phenyl is optionally substituted by one or two radicals selected from halo, loweralkyl, loweralkoxy, nitro and trifluoromethyl;

n is 1 or 2;

Q is selected from

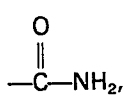, cyano, 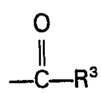

wherein $R^3$ is H, an acid neutralizing ion or an esterifying radical, and E is oxygen or sulphur; where the term 'lower alkyl' refers to a straight or branched $C_{1-8}$ alkyl group, and 'lower alkoxy' means —O— lower alkyl, the process comprising either

(i) reacting together a compound of each of the two following general formulae:

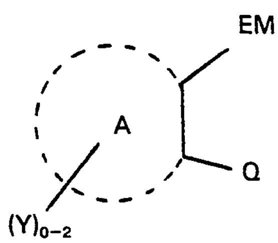

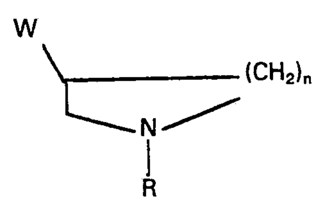

wherein

A, E, Q, R, Y and n are as defined above,

M is an acid neutralizing ion and
W is an aryl or alkyl sulphonate or halogen; or
(ii) reacting together a compound of each of the two following general formulae:

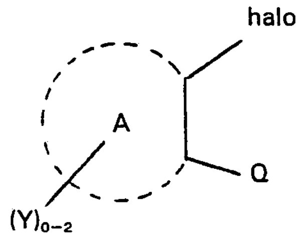

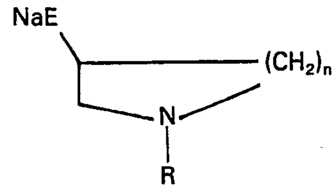

wherein A, E, Q, R, Y and n are as defined above and halo represents a halogen atom.

20. A process as claimed in Claim 19 for the preparation of 3-[(1-Methyl-3-pyrrolidinyl)oxy]-2-naphthalenecarboxamide, 3-[(1-methyl-3-pyrrolidinyl)oxy]-2-naphthalenecarboxylic acid, its oxalate salt, sodium 2-[(1-methyl-3-pyrrolidinyl)oxy]-3-pyridinecarboxylate, the free base thereof, 1-[(1-methyl-3-pyrrolidinyl)oxy]-2-naphthalenecarboxamide, 3-[(1-methyl-3-pyrrolidinyl)oxy]-4-pyridinecarbonitrile, its fumarate salt, or 1-[(1-methyl-3-pyrrolidinyl)oxy]-2-naphthalenecarbonitrile or its oxalate salt.

21. A process for the preparation of a pharmaceutical composition, the process comprising admixing

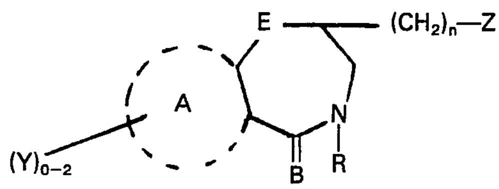

wherein

A represents an aromatic system having two of its carbon atoms held mutually with the oxazepine or thiazepine moiety selected from benzene, naphthalene or a pyridine in any of its four positions, any of which ring systems is optionally substituted by one or two Y radicals selected from halo, lower alkyl, loweralkoxy, nitro and trifluoromethyl;

B and E are selected from oxygen and sulphur and may be the same or different;

R is selected from hydrogen, loweralkyl, cycloalkyl or phenylloweralkyl or which phenyl may be optionally substituted by one or two radicals selected from halo, loweralkyl, loweralkoxy, nitro, trifluoromethyl and cyano;

n is one, two or three;

Z is selected from $—NR^1R^2$, 1H-pyrazol-1-yl or 1H-imidazol-1-yl;

each of $R^1$ and $R^2$ is selected from hydrogen, loweralkyl, cycloalkyl and phenylloweralkyl of which phenyl is optionally substituted by 1 or 2 radicals selected from halo, loweralkyl, loweralkoxy, nitro, trifluoromethyl and cyano, or $R^1$ and $R^2$ taken together with the adjacent nitrogen atom from a heterocyclic residue selected from 1-pyrrolidinyl, 2,5-dimethylpyrrolidin-1-yl, 2-methyl-pyrrolidin-1-yl, 1-piperidinyl, 4-substituted piperidine-1-yl, 4-morpholinyl, 1-piperazinyl, 4-substituted piperazin-1-yl and 1,2,3,6-tetrahydropyridin-1-yl and the pharmaceutically acceptable salts thereof, with the proviso that when R = H, Z is never a primary or secondary amine and a further proviso that when n = 3, Z is not 1H-pyrazol-1-yl, 1H-imidazol-1-yl, 2,5-dimethylpyrrolidin-1-yl, 2-methylpyrrolidin-1-yl or 1,2,3,6-tetrahydropyridin-1-yl; where the term 'lower alkyl' refers to a straight or branched $C_{1-8}$ alkyl group, and 'lower alkoxy' means —O— lower alkyl; and

b) a pharmaceutical carrier therefor.

22. A process for the preparation of a pharmaceutical composition, the process comprising admixing a) a compound preparable by a process as claimed in any one of Claims 2 to 9; and b) a pharmaceutical carrier therefor.

**Patentansprüche für die Vertragsstaaten: BE CH DE FR GB IT LI LU NL SE**

1. Verbindung mit der Formel

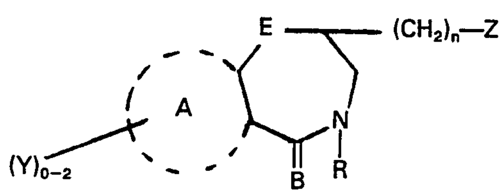

wobei

A ein aromatisches Ringsystem darstellt, von dem zwei der Kohlenstoffatome gemeinsam gebunden sind an den Oxazepin- oder Thiazepin-Teil, der ausgewählt ist aus Benzen, Naphthalen und Pyridin in jeder beliebigen der vier Positionen, jeder Ring wahlweise substituiert sein kann durch ein oder zwei Y-Radikale, die ausgewählt sind aus Halo, niedrigem Alkyl, niedrigem Alkoxy, Nitro und Trifluormethyl;

B und E aus Sauerstoff und Schwefel ausgewählt sind und gleich oder verschieden sein können;

R ausgewählt ist aus Wasserstoff, niedrigem Alkyl, $C_3$—$C_9$ Cycloalkyl oder niedrigem Alkylphenyl, wobei Phenyl wahlweise substituiert ist durch ein oder zwei Radikale, die aus Halo, niedrigem Alkyl, niedrigem Alkoxy, Nitro und Trifluormethyl ausgewählt sind;

n 1, 2 oder 3 ist;

Z aus —$NR^1R^2$, 1H-Pyrazol-1-yl und 1H-Imidazol-1-yl ausgewählt ist;

$R^1$ und $R^2$ ausgewählt sind aus Wasserstoff, niedrigem Alkyl, $C_3$—$C_9$ Cycloalkyl und niedrigem Alkylphenyl, wobei Phenyl wahlweise substituiert ist durch 1 oder 2 Radikale, die aus Halo, niedrigem Alkyl, niedrigem Alkoxy, Nitro, Trifluormethyl und Cyano ausgewählt sind, oder $R^1$ und $R^2$ zusammen mit dem benachbarten Stickstoffatom einen heterocyclischen Rest bilden, der ausgewählt ist aus 1-Pyrrolidinyl, 2,5-Dimethylpyrrolidin-1-yl, 2-Methylpyrrolidin-1-yl, 1-Piperidinyl, 4-substituiertem Piperidin-1-yl, 4-Morpholinyl, 1-Piperazinyl, 4-substituiertem Piperazin-1-yl und 1,2,3,6-Tetrahydropyridin-1-yl, und pharmazeutisch verträgliche Salze davon unter der Voraussetzung, daß Z kein primäres oder sekundäres Amin ist, wenn R = H ist, und weiters unter der Voraussetzung, daß Z kein 1H-Pyrazol-1-yl, 1H-Imidazol-1-yl, 2,5-Dimethylpyrrolidin-1-yl, 2-Methylpyrrolidin-1-yl oder 1,2,3,6-Tetrahydropyridin-1-yl ist, wenn n 3 ist; wobei die Begriffe "niedriges Alkyl" eine gerade oder verzweigte $C_{1-8}$ Alkylgruppe, und "niedriges Alkoxy" niedriges —O—Alkyl bedeuten.

2. Verbindung nach Anspruch 1, worin n 2 ist.

3. Verbindung nach Anspruch 1 oder 2, worin A einen Benzenring darstellt.

4. Verbindung nach Anspruch 1 oder 2, worin A ein Naphthalenringsystem darstellt.

5. Verbindung nach Anspruch 1 oder 2, worin A einen Pyridinring darstellt.

6. Verbindung nach einem der Ansprüche 1 bis 5, worin der durch A dargestellte Ring durch ein oder mehrere Chlor- oder Bromatome oder durch eine Methoxygruppe substituiert ist.

7. Verbindung nach einem der Ansprüche 1 bis 6, worin R eine Methyl-, Ethyl-, Benzyl- oder Hexylgruppe darstellt.

8. Verbindung nach einem der Ansprüche 1 bis 7, worin Z eine Dimethylaminogruppe darstellt.

9. 2 - [2 - (Dimethylamino)ethyl] - 2,3 - dihydro - 4 - methyl - 1,4-benzoxazepin - 5(4H) - on, 2,3 - Dihydro - 4 - methyl - 2 - [2 - (4 - morpholino)ethyl] - 1,4 - benzoxazepin - 5(4H) - on, 4-Benzyl - 2,3 - dihydro - 2 - [2-(4 - morpholino)ethyl] - 1,4 - benzoxazepin - 5(4H) - on, 4 - Benzyl - 2,3 - dihydro - 2 - [2 - (methylamino)ethyl] - 1,4 - benzoxazepin - 5(4H) - on, 2 - [2 - (Dimethylamino)ethyl] - 2,3 - dihydro - 4 - methyl - 1,4 - benzoxazepin - 5(4H) - thion, 4 - Benzyl - 2 - [2 - (dimethylamino)ethyl] - 2,3 - dihydro - 1,4 - benzoxazepin - 5(4H) - on, 2,3 - Dihydro - 4 - methyl - 2 - [2 - (4 - morpholino)ethyl] - 1,4 - benzoxazepin - 5(4H) - thion, 2 - [2 - (Dimethylamino)ethyl] - 2,3 - dihydro - 4 - methylnaphth[2,3-f][1,4] - oxazepin - 5(4H) - on, 2 - [2 - (Dimethylamino)ethyl] - 2,3 - dihydro - 4 - methylpyrido[3,2-f](1,4] - oxazepin - 5(4H) - on, 2 - [2 - (Dimethylamino)ethyl] - 2,3 - dihydro - 4 - methylpyrido[3,2-f][1,4] - oxazepin - 5(4H) - thion, 4 - Benzyl - 2,3 - dihydro - 2 - [2 - (4-morpholino)-ethyl] - 1,4 - benzoxazepin - 5(4H) - thion, 2,3 - Dihydro - 4 - methyl - 2 - [2 - (methylamino)ethyl] - 1,4 - benzoxazepin - 5(4H) - on, 2,3 - Dihydro - 4 - methyl - 2 - [2 - (methylamino)ethyl] - 1,4 - benzoxazepin - 5(4H) - on - fumarat, 2,3 - Dihydro - 2 - [2 - (4 - hydroxy - 4 - phenyl) - piperidinylethyl] - 4 - methyl - 1,4 - benzoxazepin - 5(4H) - thion, 2,3 - Dihydro - 4 - methyl - 2 - 2 - [(4 - phenyl - 1,2,3,6 - tetrahydro)pyridinyl - ethyl] - 1,4 - benzoxazepin - 5(4H) - thion, 8 - Chloro - 2 - [2 - (dimethylamino)ethyl] - 2,3 - dihydro - 4 - methyl - 1,4 - benzoxazepin - 5(4H) - thion, 8 - Chloro - 2 - [2 - (dimethylamino)ethyl] - 2,3 - dihydro - 4 - methyl - 1,4 - benzoxazepin - 5(4H) - on, 7 - Bromo - 2 - [2 - (dimethylamino)ethyl] - 2,3 - dihydro - 4 - methyl - 1,4 - benzoxazepin - 5(4H) - thion, 2 - [2 - (Dimethylamino)ethyl] - 2,3 - dihydro - 4 - methylnaphth[2,1-f][1,4] - oxazepin - 5(4H) - on, 2 - [2 - (Dimethylamino)ethyl] - 2,3 - dihydro - 4 - methylpyrido[4,3-f][1,4] - oxazepin - 5(4H) - thion, 2 - [2 - (Dimethylamino)ethyl] - 2,3 - dihydro - 4 - methylnaphth[2,3-f][1,4] - oxazepin - 5(4H) - thion, 7 - Chloro - 2 - [2 - (dimethylamino)ethyl] - 2,3 - dihydro - 4 - methyl - 1,4 - benzoxazepin - 5(4H) - on, 2 - [2 - (Dimethylamino)ethyl] - 2,3 - dihydro - 4 - methylpyrido[3,2-f][1,4] - oxazepin - 5(4H) - thion - methiodid, 7 - Chloro - 2 - [2 - (dimethylamino)ethyl] - 2,3 - dihydro - 4 - methyl - 1,4 - benzoxazepin - 5(4H) - thion, 2 - [2 - (Dimethylamino)ethyl] - 2,3 - dihydro - 4 - methylnaphth[2,1-f][1,4] - oxazepin - 5(4H) - thion, 2 - 2 - (Dimethylamino)ethyl - 2,3 - dihydro - 7 - methoxy - 4 - methyl - 1,4 - benzoxazepin - 5(4H) - on, 7 - Bromo - 2 - [2 - (dimethylamino)ethyl] - 2,3 - dihydro - 4 - methyl - 1,4 - benzoxazepin - 5(4H) - thion, 2 - [2 - (Dimethylamino)ethyl] - 2,3 - dihydro - 4 - methylpyrido[3,2-f][1,4] - thiazepin - 5(4H) - on, 2 - [2 - (Dimethylamino)ethyl] - 2,3 - dihydro - 4 - methyl - pyrido[3,2-f][1,4] - thiazepin - 5(4H) - thion, 2 - [2 - (Dimethylamino)ethyl] - 2,3 - dihydro - 4 - methylpyrido[3,4-f][1,4] - oxazepin - 5(4H) - on, 2 - [2 - (Dimethylamino)ethyl] - 2,3 - dihydro - 4 - methylpyrido[3,4-f] - oxazepin - 5(4H) - thion, 2 - (3 - Aminopropyl)-2,3 - dihydro - 4 - methyl - pyrido[3,2-f][1,4] - oxazepin - 5(4H) - on, 2,3 - Dihydro - 4 - methyl - 2 - [2 - (4 - morpholinyl)ethyl] - pyrido[3,2-f][1,4] - oxazepin - 5(4H) - on, 2,3 - Dihydro - 4 - methyl - 2 - [2 - (1 - pyrrolidinyl)ethyl] - pyrido[3,2-f][1,4] - oxazepin -

5(4H) - on, 2 - [2 - (Dibutylamino)ethyl] - 2,3 - dihydro - 4 - methyl - pyrido[3,2-f][1,4] - oxazepin - 5(4H) - on, 2 - [2 - (Diethylamino)ethyl] - 2,3 - dihydro - 4 - methylpyrido[3,2-f][1,4] - oxazepin - 5(4H) - on, 2,3 - Dihydro - 4 - methyl - 2 - [2 - (1 - piperidinyl)ethylpyrido[3,2-f][1,4] - oxazepin - 5(4H) - on, 2,3 - Dihydro - 4 - methyl - 2 - [2 - (methyl(phenylmethyl)amino)ethyl]pyrido[3,2-f][1,4] - oxazepin - 5(4H) - on, 2,3 - Dihydro - 4 - methyl - 2 - [2 - (methylphenylamino)ethyl]pyrido[3,2-f][1,4] - oxazepin - 5(4H) - on, 2 - [2 - (2,5 - Dimethyl - 1 - pyrrolidinyl)ethyl - 2,3 - dihydro - 4 - methyl - pyrido[3,2-f][1,4] - oxazepin - 5(4H) - on, 2,3 - Dihydro - 4 - methyl - 2 - [2 - (2 - methyl - 1 - pyrrolidinyl)ethyl]-pyrido[3,2-f][1,4] - oxazepin - 5(4H) - on, 2,3 - Dihydro - 4 - methyl - 2 - [2 - (1H - pyrazol - 1 - yl)ethyl] - pyrido[3,2-f][1,4] - oxazepin - 5(4H) - on, 2,3 - Dihydro - 2 - [2 - (1H - imidazol - 1 - yl)ethyl - 4 - methylpyrido[3,2-f][1,4] - oxazepin - 5(4H) - on, 2 - [2 - Dimethylamino)ethyl] - 4 - ethyl - 2,3 - dihydropyrido[3,2-f][1,4] - oxazepin - 5(4H) - on, 2,3 - Dihydro - 4 - ethyl - 2 - [2 - (1 - pyrrolidinyl)ethyl]-pyrido[3,2-f][1,4] - oxazepin - 5(4H) - on, 2,3 - Dihydro - 4 - methyl - 2 - [2 - (4 - morpholinyl)ethyl]-pyrido[3,2-f][1,4] - oxazepin - 5(4H) - thion, 2 - [2 - (Dibutylamino)ethyl] - 2,3 - dihydro - 4 - methyl-pyrido[3,2-f][1,4] - oxazepin - 5(4H) - thion, 2 - [2 - (Diethylamino)ethyl] - 2,3 - dihydro - 4 - methyl-pyrido[3,2-f][1,4] - oxazepin - 5(4H) - thion, 2,3 - Dihydro - 4 - methyl - 2 - [2 - (1 - pyrrolidinyl)-ethyl]pyrido-[3,2-f][1,4] - oxazepin - 5(4H) - thion, 2,3 - Dihydro - 2 - [2 - (1H - imidazol - 1 - yl)ethyl] - 4 - methyl - pyrido[3,2-f][1,4] - oxazepin - 5(4H) - thion, 2 - [2 - (Dimethylamino)ethyl] - 4 - ethyl - 2,3 - dihydropyrido[3,2-f][1,4] - oxazepin - 5(4H) - thion, 2,3 - Dihydro - 4 - methyl - 2 - [2 - [methyl(phenyl-methyl)amino]ethyl] - pyrido[3,2-f][1,4] - oxazepin - 5(4H) - thion, 2,3 - Dihydro - 2 - [methylamino)-ethyl] - 4 - methylpyrido[3,2-f][1,4] - oxazepin - 5(4H) - thion, 7 - Chloro - 2,3 - dihydro - 4 - methyl - 2 - [2 - (1 - pyrrolidino)ethyl]pyrido[3,2-f][1,4] - oxazepin - 5(4H) - on, 7 - Chloro - 2 - [2 - (dimethylamino)ethyl] - 2,3 - dihydro - 4 - methylpyrido[3,2-f][1,4] - oxazepin - 5(4H) - on, 2 - [2 - (Dimethylamino)ethyl] - 2,3 - dihydro - 4 - phenylmethylpyrido[3,2-f][1,4] - oxazepin - 5(4H) - on, 2 - [2 - (Dimethylamino)ethyl] - 2,3 - dihydropyrido-[3,2-f][1,4] - oxazepin - 5(4H) - on, 2 - [3 - (Dimethylamino)propyl] - 2,3 - dihydro - 4 - methylpyrido[3,2-f][1,4] - oxazepin - 5(4H) - on, 2 - [3 - (Dimethylamino)propyl] - 2,3 - dihydro - 4 - methylpyrido[3,2-f][1,4] - oxazepin - 5(4H) - thion oder 7 - Chloro - 2 - [2 - (dimethylamino)ethyl] - 2,3 - dihydro - 4 - methylpyrido - [3,2-f] - oxazepin - 5(4H) - thion oder, wenn geeignet, ein pharmazeutisch verträgliches Salz davon.

10. Verbindung mit der Formel

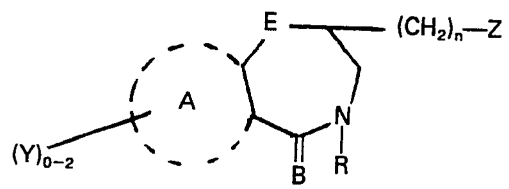

worin

A ein aromatisches Ringsystem darstellt, von dem zwei der Kohlenstoffatome gemeinsam gebunden sind an den Oxazepin- oder Thiazepin-Teil, der ausgewählt ist aus Benzen, Naphthalen und Pyridin in jeder beliebigen der vier Positionen, jeder der Ringe wahlweise substituiert sein kann durch ein oder zwei Y-Radikale, die ausgewählt sind aus Halo, niedrigem Alkyl, niedrigem Alkoxy, Nitro und Trifluormethyl;

B und E aus Sauerstoff und Schwefel ausgewählt sind und gleich oder verschieden sein können;

R ausgewählt ist aus Wasserstoff, niedrigem Alkyl, Cycloalkyl oder niedrigem Alkylphenyl, wobei Phenyl wahlweise substituiert ist durch ein oder zwei Radikale, die aus Halo, niedrigem Alkyl, niedrigem Alkoxy, Nitro und Trifluormethyl ausgewählt sind;

n 1, 2 oder 3 ist;

Z aus —$NR^1R^2$; 1H-Pyrazol-1-yl und 1H-Imidazol-1-yl ausgewählt ist;

$R^1$ und $R^2$ ausgewählt sind aus Wasserstoff, niedrigem Alkyl, Cycloalkyl und niedrigem Alkylphenyl, wobei Phenyl wahlweise substituiert ist durch 1 oder 2 Radikale, die aus Halo, niedrigem Alkyl, niedrigem Alkoxy, Nitro, Trifluormethyl und Cyano ausgewählt sind, oder $R^1$ und $R^2$ zusammen mit dem benachbarten Stickstoffatom einen heterocyclischen Rest bilden, der ausgewählt ist aus 1-Pyrrolidinyl, 2,5-Dimethylpyrrolidin-1-yl, 2-Methylpyrrolidin-1-yl, 1-Piperidinyl, 4-substituiertem Piperidin-1-yl, 4-Morpholinyl, 1-Piperazinyl, 4-substituiertem Piperazin-1-yl und 1,2,3,6-Tetrahydropyridin-1-yl, und pharmazeutisch verträgliche Salze davon unter der Voraussetzung, daß Z kein primäres oder sekundäres Amin ist, wenn R = H ist, und der weiteren Voraussetzung , daß Z kein 1H-Pyrazol-1-yl, 1H-Imidazol-1-yl, 2,5-Dimethylpyrrolidin-1-yl, 2-Methylpyrrolidin-1-yl ist, wenn n 3 ist;

wobei die Begriffe "niedriges Alkyl" eine gerade oder verzweigte $C_{1-8}$ Alkylgruppe, und "niedriges Alkoxy" niedriges —O—Alkyl bedeuten; zur Verwendung in der Medizin.

11. Verbindung nach einem der Ansprüche 2 bis 9 zur Verwendung in der Medizin.

12. Verfahren zur Herstellung einer Verbindung mit der Formel

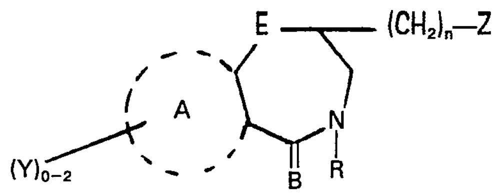

worin

A eine aromatisches Ringsystem darstellt, von dem zwei der Kohlenstoffatome geminsam gebunden sind an den Oxazepin- oder Thiazepin-Teil, der ausgewählt ist aus Benzen, Naphthalen oder einem Pyridin in jeder beliebigen der vier Positionen, jeder der Ringe wahlweise substituiert sein kann durch ein oder zwei Y-Radikale, die ausgewählt sind aus Halo, niedrigem Alkyl, niedrigem Alkoxy, Nitro und Trifluormethyl;

B und E aus Sauerstoff und Schwefel ausgewählt sind und gleich oder verschieden sein können;

R ausgewählt ist aus Wasserstoff, niedrigem Alkyl, Cycloalkyl oder niedrigem Alkylphenyl, wobei Phenyl wahlweise substituiert ist durch ein oder zwei Radikale, die aus Halo, niedrigem Alkyl, niedrigem Alkoxy, Nitro und Trifluormethyl ausgewählt sind;

n 1, 2 oder 3 ist;

Z aus —$NR^1R^2$, 1H-Pyrazol-1-yl oder 1H-Imidazol-1-yl ausgewählt ist;

$R^1$ und $R^2$ ausgewählt sind aus Wasserstoff, niedrigem Alkyl, Cycloalkyl und niedrigem Alkylphenyl, wobei Phenyl wahlweise substituiert ist durch 1 oder 2 Radikale, die aus Halo, niedrigem Alkyl, niedrigem Alkoxy, Nitro, Trifluormethyl und Cyano ausgewählt sind, oder $R^1$ und $R^2$ zusammen mit dem benachbarten Stickstoffatom einen heterocyclischen Rest bilden, der ausgewählt ist aus 1-Pyrrolidinyl, 2,5-Dimethylpyrrolidin-1-yl, 2-Methylpyrrolidin-1-yl, 1-Piperidinyl, 4-substituiertem Piperidin-1-yl, 4-Morpholinyl, 1-Piperazinyl, 4-substituiertem Piperazin-1-yl und 1,2,3,6-Tetrahydropyridin-1-yl, und pharmazeutisch verträgliche Salze davon unter der Voraussetzung, daß Z kein primäres oder sekundäres Amin ist, wenn R Wasserstoff ist, und der weiteren Voraussetzung, daß Z kein 1H-Pyrazol-1-yl, 1H-Imidazol-1-yl, 2,5-Dimethypyrrolidin-1-yl, 2-Methylpyrrolidin-1-yl oder 1,2,3,6-Tetrahydropyridin-1-yl ist, wenn n 3 ist;

wobei sich das Verfahren zusammensetzt aus

(i) einer Reaktion zwischen einer Verbindung mit der Formel

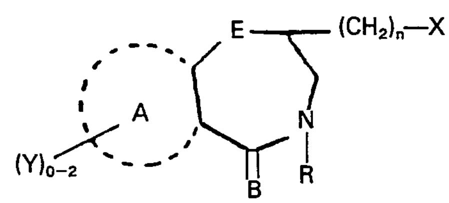

worin A, B, E, R, Y und n das gleiche sind wie bereits bei Formel I definiert, und X Chlor, Brom oder Cyano darstellt, und einer Verbindung mit der Formel

ZH

worin Z bereits bei Formel I definiert ist;

oder

(ii) einer Reduktion der Verbindung mit der Formel

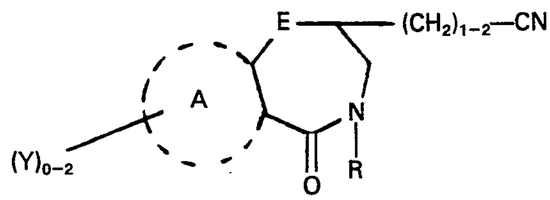

um eine Verbindung mit der Formel I zu erhalten, in der B Sauerstoff darstellt, Z —$NH^2$ darstellt und n 2 oder 3 ist, und wenn notwendig

(iii) einer Umwandlung eine so gebildeten Verbindung mit der Formel I in eine andere Verbindung mit der Formel I;

und wahlweise

(iv) einer Herstellung eines pharmazeutisch verträglichen Salzes einer so gebildeten Verbindung; wobei die Begriffe "niedriges Alkyl" eine gerade oder verzweigte $C_{1-8}$ Alkylgruppe, und, "niedriges Alkoxy" niedriges —O—Alkyl bedeuten.

13. Verfahren nach Anspruch 12 zur Herstellung einer Verbindung nach einem der Ansprüche 2 bis 9.

14. Verbindung mit der Formel

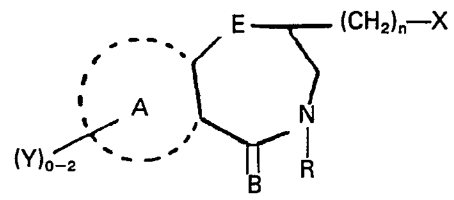

worin

A ein aromatisches Ringsystem darstellt, von dem zwei der Kohlenstoffatome gemeinsam gebunden sind an den Oxazepin- oder Thiazepin-Teil, der ausgewählt ist aus Benzen, Naphthalen und einem Pyridin in jeder beliebigen der vier Positionen, jedes der Ringsysteme wahlweise substituiert sein kann durch ein oder zwei Y-Radikale, die ausgewählt sind aus Halo, niedrigem Alkyl, niedrigem Alkoxy, Nitro und Trifluormethyl;

B und E aus Sauerstoff und Schwefel ausgewählt sind und gleich oder verschieden sein können;

R ausgewählt ist aus niedrigem Alkyl, Cycloalkyl oder niedrigem Alkylphenyl, wobei Phenyl wahlweise substituiert ist durch ein oder zwei Radiakle, die aus Halo, niedrigem Alkyl, niedrigem Alkoxy, Nitro und Trifluormethyl ausgewählt sind;

n 1 oder 2 ist;

X Chlor, Brom oder Cyano ist;

wobei die Begriffe "niedriges Alkyl" eine gerade oder verzweigte $C_{1-8}$ Alkylgruppe, und "niedriges Alkoxy" niedriges —O—Alkyl bedeuten.

15. Verbindung nach Anspruch 14, worin n 2 ist.

16. Verbindung nach Anspruch 14 oder 15, worin A einen Benzenring darstellt.

17. Verbindung nach Anspruch 14 oder 15, worin A ein Naphthalenringsystem darstellt.

18. Verbindung nach Anspruch 13 oder 14, worin A einen Pyridinring darstellt.

19. Verbindung nach einem der Ansprüche 13 bis 17, worin der durch A dargestellte Ring durch ein oder mehrere Chlor- oder Bromatome oder durch eine Methoxygruppe substituiert ist.

20. Verbindung nach einem der Ansprüche 13 bis 18, worin R eine Methyl-, Ethyl-, Benzyl- oder Hexylgruppe darstellt.

21. 2 - (2 - Chlorethyl) - 2,3 - dihydro - 4 - methyl - 1,4 - benzoxazepin - 5(4H) - on, 4 - Benzyl - 2 - (2 - chloroethyl) - 2,3 - dihydro - 1,4 - benzoxazepin - 5(4H) - on, 2 - (2 - Chloroethyl) - 2,3 - dihydro - 4 - methylnaphth - [2,3-f][1,4] - oxazepin - 5(4H) - on, 2 - (2 - Chloroethyl) - 2,3 - dihydro - 4 - methyl - pyrido[3,2-f][1,4]-oxazepin - 5(4H) - on, 8 - Chloro - 2 - (2 - chloroethyl) - 2,3 - dihydro - 4 - methyl - 1,4 - benzoxazepin - 5(4H) - on, 7 - Bromo - 2 - (2 - chloroethyl) - 2,3 - dihydro - 4 - methyl - 1,4 - benzoxazepin - 5(4H) - on, 7 - Chloro - 2 - (2 - Chloroethyl) - 2,3 - dihydro - 4 - methyl - 1,4 - benzoxazepin - 5(4H) - on, 2 - (2 - Chloroethyl) - 2,3 - dihydro - 4 - naphth [2,1-f][1,4] - oxazepin - 5(4H) - on, 2 - (2 - Chloroethyl - 2,3 - dihydro - 7 - methoxy - 4 - methyl - 1,4 - benzoxazepin - 5(4H) - on, 2 - (2 - Chloroethyl) - 2,3 - dihydro - 4 - methyl - 1,4 - benzoxazepin - 5(4H) - thion, 2 - (2 - Chloroethyl) - 2,3 - dihydro - 4 - methylpyrido[3,2-f][1,4] - oxazepin - 5(4H) - thion, 2 - (2 - Chloroethyl) - 2,3 - dihydro - 4 - methylnaphth[2,3-f][1,4] - oxazepin - 5(4H) - thion, 8 - Chloro - 2 - (2 - chloroethyl) - 2,3 - dihydro - 4 - methyl - 1,4 - benzoxazepin - 5(4H) - thion, 7 - Bromo - 2 - (2 - chloroethyl) - 2,3 - dihydro - 4 - methyl - 1,4 - benzoxazepin - 5(4H) - thion, 2 - (2 - Chloroethyl) - 2,3 - dihydro - 4 - methylnaphth[2,1-f][1,4]-oxazepin - 5(4H) - thion, 2 - (2 - Chloroethyl) - 2,3 - dihydro - 4 - methyl - pyrido[4,3-f][1,4] - oxazepin - 5(4H) - on, 7 - Chloro - 2 - (2 - chloroethyl) - 2,3 - dihydro - 4 - methyl - 1,4 - benzoxazepin - 5(4H) - thion, 2 - [(2 - Chloroethyl) - 2,3 - dihydro - 4 - methyl - pyrido[4,3-f][1,4] - oxazepin - 5(4H) - thion, 2 - (2 - Chloroethyl - 2,3 - dihydro - 7 - methoxy - 4 - methyl - 1,4 - benzoxazepin - 5(4H) - thion, 2 - (2 - Chloroethyl) - 2,3 - dihydro - 4 - methylpyrido[3,2-f][1,4] - thiazepin - 5(4H) - on, 2 - (2 - Chloroethyl) - 2,3 - dihydro - 4 - methylpyrido[3,2-f][1,4] - thiazepin - 5(4H) - thion, 2 - (2 - Chloroethyl) - 2,3 - dihydro - 4 - methylpyrido[3,4-f][1,4] - oxazepin - 5(4H) - on, 2 - (2 - Chloroethyl) - 2,3 - dihydro - 4 - methylpyrido[3,4-f][1,4] - oxazepin - 5(4H) - thion, 2,3,4,5 - Tetrahydro - 4 - methyl - 5 - oxopyrido[3,2-f][1,4] - oxazepin - 2 - propannitril, 2 - (2 - Chloroethyl) - 4 - ethyl - 2,3 - dihydropyrido[3,2-f][1,4] - oxazepin - 5(4H) - on, 2 - (2 - Chloroethyl) - 4 - ethyl - 2,3 - dihydropyrido[3,2-f][1,4]-oxazepin - 5(4H) - thion, 7 - Chloro - 2 - (2 - chloroethyl) - 2,3 - dihydro - 4 - methylpyrido[3,2-f][1,4] - oxazepin - 5(4H) - on, 7 - Chloro - 2 - (2 - chloroethyl) - 2,3 - dihydro - 4 - methylpyrido[3,2-f][1,4]oxazepin - 5(4H) - thion oder 2 - (Chloromethyl) - 4 - cyclohexyl - 2,3 - dihydropyrido[3,2-f][1,4] - oxazepin - 5(4H) - on oder ein Säureadditionssalz davon.

22. Verfahren zur Herstellung einer Verbindung mit der Formel

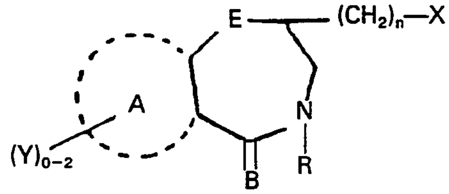

worin

A aus einem aromatischen Ringsystem ausgewählt ist, von dem zwei der Kohlenstoffatome gemeinsam gebunden sind an den Oxazepin- oder Thiazepin-Teil, der ausgewählt ist aus Benzen, einem Naphthalen und einem Pyridin in jeder beliebigen der vier Positionen, jeder der Ringe wahlweise substituiert sein kann durch ein oder zwei Y-Radikale, die ausgewählt sind aus Halo, niedrigem Alkyl, niedrigem Alkoxy, Nitro und Trifluormethyl;

B und E aus Sauerstoff und Schwefel ausgewählt sind und gleich oder verschieden sein können;

R ausgewählt ist aus niedrigem Alkyl, Cycloalkyl oder niedrigem Alkylphenyl, wobei Phenyl wahlweise substituiert ist durch niedriges Alkoxy, Nitro und Trifluormethyl;

n 1 oder 2 ist;

X Chlor, Brom oder Cyano oder ein Säureadditionssalz davon ist, und sich das Verfahren zusammensetzt aus

i)a) einer Kondensation einer Verbindung mit der Formel

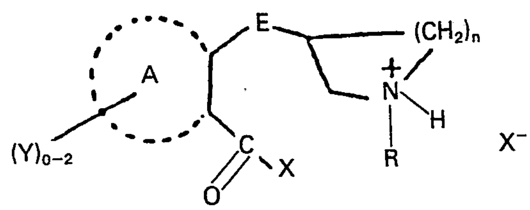

worin X Chlor oder Brom darstellt und A, E, n, R und Y das gleiche sind wie oben definiert;

(b) wenn erforderlich einer Reaktion zwischen einer so gebildeten Verbindung und einem Schwefelungsmittel, um ein Oxazepinthion oder ein Thiazepinthion zu erhalten;

und

(c) wahlweise einer Reaktion einer Verbindung, die durch Schritt i)a) oder i)b) erhalten wurde, mit einem Alkalimetallcyanid, um die entsprechende Verbindung zu erhalten, in welcher X CN darstellt; oder

(ii) einer Halogenierung einer Verbindung, die durch Schritt i)a), i)b) oder i)c) erhalten wurde, mit der Formel

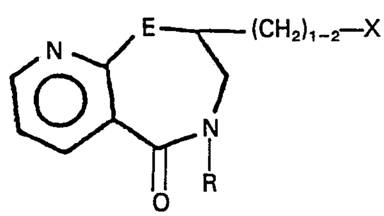

worin E und R das gleiche sind wie oben definiert, und X Chlor, Brom oder Cyano ist, mit Sulphurylchlorid in einem geeigneten Lösungsmittel, um eine Verbindung mit der Formel

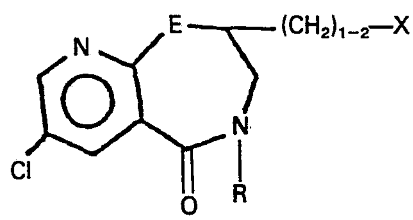

zur erhalten,

worin E und R das gleiche sind wie oben definiert, und X Chlor, Brom oder Cyano ist; wobei die Begriffe "niedriges Alkyl" eine gerade oder verzweigte $C_{1-8}$ Alkylgruppe, und "niedriges Alkoxy" niedriges —O—Alkyl bedeuten.

23. Verfahren nach Anspruch 22 zur Herstellung einer Verbindung nach einem der Ansprüche 15 bis 21.

24. Verbindung, ausgewählt aus der Gruppe mit der Formel

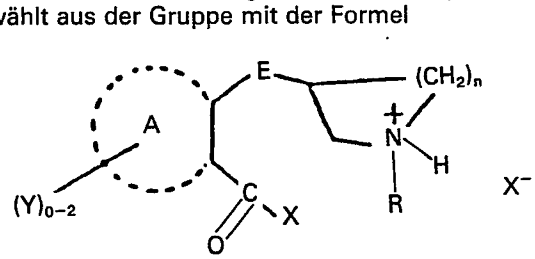

worin

A eine aromatisches Ringsystem darstellt, ausgewählt aus Benzen, einem Naphthalen oder einem

Pyridin in jeder beliebigen der vier Positionen, jeder der Ringsysteme wahlweise substituiert sein kann durch ein oder zwei Y-Radikale, die ausgewählt sind aus Halo, niedrigem Alkyl, niedrigem Alkoxy, Nitro und Trifluormethyl;

R ausgewählt ist aus niedrigem Alkyl, Cycloalkyl oder niedrigem Alkylphenyl, Nitro und Trifluormethyl;

n 1 oder 2 ist;

X ausgewählt ist aus Chlor, Brom und Fluor, und E ausgewählt ist aus Schwefel und Sauerstoff; und wobei die Begriffe "niedriges Alkyl" eine gerade oder verzweigte $C_{1-8}$ Alkylgruppe, und "niedriges Alkoxy" niedriges —O—Alkyl bedeuten.

25. Verbindung mit der Formel

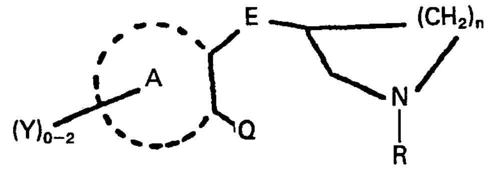

worin

A eine aromatisches Ringsystem darstellt, ausgewählt aus Naphthalen oder einem Pyridin in jeder beliebigen der vier Positionen, jeder der Ringsysteme wahlweise substituiert sein kann durch ein oder zwei Radikale, die ausgewählt sind aus der Gruppe Halo, niedriges Alkyl, niedriges Alkoxy, Nitro und Trifluormethyl;

R ausgewählt ist aus niedrigem Alkyl, Cycloalkyl oder niedrigem Alkylphenyl, wobei Phenyl wahlweise substituiert ist durch ein oder mehrere Radikale, ausgewählt aus Halo, niedrigem Alkyl, niedrigem Alkoxy, Nitro und Trifluormethyl;

n 1 oder 2 ist;

Q ausgewählt ist aus

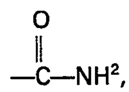, Cyano, 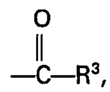,

worin $R^3$ H ist, einem Säureneutralisationsion oder einem Veresterungsradikal, und E Sauerstoff oder Schwefel ist; wobei die Begriffe "niedriges Alkyl" eine gerade oder verzweigte $C_{1-8}$ Alkylgruppe, und "niedriges Alkoxy" niedriges —O—Alkyl bedueten.

26. 3 - [(1 - Methyl - 3 - pyrrolidinyl)oxy] - 2 - naphthalen - carboxamid, 3 - [(1 - Methyl - 3 - pyrrolidinyl)oxy] - 2 - naphthalencarboxylsäure, deren Oxalatsalz, Natrium - 2 - [(1 - methyl - 3 - pyrrolidinyl)oxy] - 3 - pyridincarboxalat, die freie Base davon, 1 - [(1 - Methyl - 3 - pyrrolidinyl)oxy] - 2 - naphthalencarboxamid, 3 - [(1 - Methyl - 3 - pyrrolidinyl)oxy] - 4 - pyridincarbonitril, dessen Fumaratsalz, oder 1 - [(1 - Methyl - 3 - pyrrolidinyl)oxy] - 2 - naphthalencarbonitril oder dessen Oxalatsalz.

27. Pharmazeutische Zusammensetzung, bestehend aus (a) einer Verbindung mit der Formel

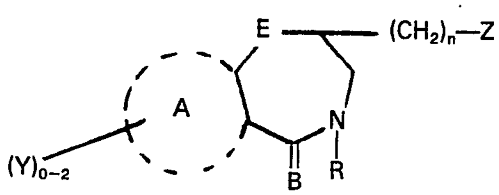

worin

A ein aromatisches Ringsystem darstellt, von dem zwei der Kohlenstoffatome gemeinsam gebunden sind an den Oxazepin- oder Thiazepin-Teil, der ausgewählt ist aus Benzen, einem Naphthalen oder einem Pyridin in jeder beliebigen der vier Positionen, jeder der Ringsysteme wahlweise substituiert sein kann durch ein oder zwei Y-Radikale, die ausgewählt sind aus Halo, niedrigem Alkyl, niedrigem Alkoxy, Nitro und Trifluormethyl;

B und E aus Sauerstoff und Schwefel ausgewählt sind und gleich oder verschieden sein können;

R ausgewählt ist aus Wasserstoff, niedrigem Alkyl, Cycloalkyl oder niedrigem Alkylphenyl, wobei Phenyl wahlweise substituiert ist durch ein oder zwei Radikale, die aus Halo, niedrigem Alkyl, niedrigem Alkoxy, Nitro, Trifluormethyl und Cyano ausgewählt sind;

n 1, 2 oder 3 ist;

Z aus $—NR^1R^2$, 1H-Pyrazol-1-yl und 1H-Imidazol-1-yl ausgewählt ist;

$R^1$ und $R^2$ ausgewählt sind aus Wasserstoff, niedrigem Alkyl, Cycloalkyl und niedrigem Alkylphenyl, wobei Phenyl wahlweise substituiert ist durch 1 oder 2 Radikale, die aus Halo, niedrigem Alkyl, niedrigem Alkoxy, Nitro, Trifluormethyl und Cyano ausgewählt sind, oder $R^1$ und $R^2$ zusammen mit dem benachbarten Stickstoffatom einen heterocyclischen Rest bilden, der ausgewählt ist aus 1-Pyrrolidinyl, 2,5-Dimethylpyrrolidin-1-yl, 2-Methylpyrrolidin-1-yl, 1-Piperidinyl, 4-substituiertem Piperidin-1-yl, 4-Morpholinyl, 1-Piperazinyl, 4-substituiertem Piperazin-1-yl und 1,2,3,6-Tetrahydropyridin-1-yl, und pharmazeutisch

verträgliche Salze davon unter der Voraussetzung, daß Z kein primäres oder sekundäres Amin ist, wenn R = H ist, und der weiteren Voraussetzung, daß Z kein 1H-Pyrazol-1-yl, 1H-Imidazol-1-yl, 2,5-Dimethylpyrrolidin-1-yl, 2-Methylpyrrolidin-1-yl oder 1,2,3,6-Tetrahydropyridin-1-yl ist, wenn n 3 ist; wobei die Begriffe "niedriges Alkyl" eine gerade oder verzweigte $C_{1-8}$ Alkylgruppe, und "niedriges Alkoxy" niedriges —O—Alkyl bedeuten, und (b) einem pharmazeutischen Träger dafür.

28. Pharmazeutische Zusammensetzung aus
(a) einer Verbindung nach einem der Ansprüche 2 bis 9, und
(b) einem pharmazeutischen Träger dafür.

**Patentansprüche für den Vertragsstaat: AT**

1. Verfahren zur Herstellung einer Verbindung mit der Formel

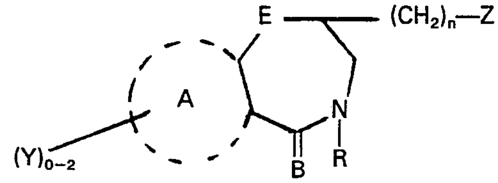

worin

A ein aromatisches Ringsystem darstellt, von dem zwei der Kohlenstoffatome gemeinsam gebunden sind an den Oxazepin- oder Thiazepin-Teil, der ausgewählt ist aus Benzen, Naphthalen und Pyridin in jeder beliebigen der vier Positionen, jeder der Ringe wahlweise substituiert sein kann durch ein oder zwei Y-Radikale, die ausgewählt sind aus Halo, niedrigem Alkyl, niedrigem Alkoxy, Nitro und Trifluormethyl;

B und E aus Sauerstoff und Schwefel ausgewählt sind und gleich oder verschieden sein können;

R ausgewählt ist aus Wasserstoff, niedrigem Alkyl, $C_3$—$C_9$ Cycloalkyl oder niedrigem Alkylphenyl, wobei Phenyl wahlweise substituiert ist durch ein oder zwei Radikale, die aus Halo, niedrigem Alkyl, niedrigem Alkoxy, Nitro und Trifluormethyl ausgewählt sind;

n 1, 2 oder 3 ist;

Z aus —$NR^1R^2$, 1H-Pyrazol-1-yl und 1H-Imidazol-1-yl ausgewählt ist;

$R^1$ und $R^2$ ausgewählt sind aus Wasserstoff, niedrigem Alkyl, $C_3$—$C_9$ Cycloalkyl und niedrigem Alkylphenyl, wobei Phenyl wahlweise substituiert ist durch 1 oder 2 Radikale, die aus Halo, niedrigem Alkyl, niedrigem Alkoxy, Nitro, Trifluormethyl und Cyano ausgewählt sind, oder $R^1$ und $R^2$ zusammen mit dem benachbarten Stickstoffatom einen heterocyclischen Rest bilden, der ausgewählt ist aus 1-Pyrrolidinyl, 2,5-Dimethylpyrrolidin-1-yl, 2-Methylpyrrolidin-1-yl, 1-Piperidinyl, 4-substituiertem Piperidin-1-yl, 4-Morpholinyl, 1-Piperazinyl, 4-substituiertem Piperazin-1-yl und 1,2,3,6-Tetrahydropyridin-1-yl, und pharmazeutisch verträgliche Salze davon unter der Voraussetzung, daß Z kein primäres oder sekundäres Amin ist, wenn R = H ist, und der weiteren Voraussetzung, daß Z kein 1H-Pyrazol-1-yl, 1H-Imidazol-1-yl, 2,5-Dimethylpyrrolidin-1-yl, 2-Methylpyrrolidin-1-yl oder 1,2,3,6-Tetrahydropyridin-1-yl ist, wenn n 3 ist; wobei die Begriffe "niedriges Alkyl" eine gerade oder verzweigte $C_{1-8}$ Alkylgruppe, und "niedriges Alkoxy" niedriges —O-Alkyl bedeuten; und sich das Verfahren zusammensetzt aus

(i) einer Reaktion zwischen einer Verbindung mit der Formel

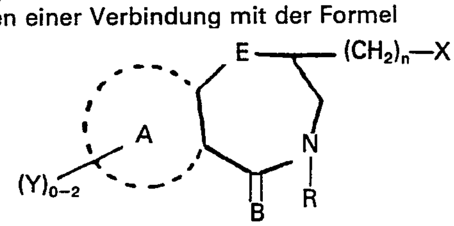

worin A, B, E, R, Y und n das gleiche sind wie bei Formel I definiert, und X Chlor, Brom oder Cyano darstellt, und einer Verbindung mit der Formel

ZH

wobei Z bei Formel I definiert ist; oder

(ii) einer Reduktion der Verbindung mit der Formel

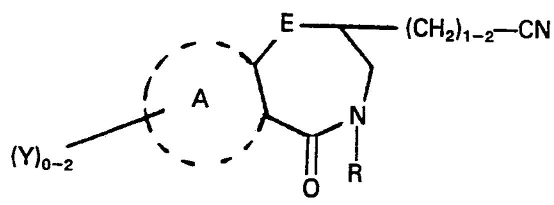

um eine Verbindung mit der Formel I zu erhalten, in der B Sauerstoff darstellt, Z —$NH^2$ darstellt und n 2 oder 3 ist, und wenn notwendig

(iii) einer Umwandlung einer so gebildeten Verbindung mit der Formel I in eine andere Verbindung mit der Formel I; und wahlweise

(iv) einer Herstellung eines pharmazeutisch verträglichen Salzes einer so gebildeten Verbindung; wobei die Begriffe "niedriges Alkyl" eine gerade oder verzweigte $C_{1-8}$ Alkylgruppe, und "niedriges Alkoxy" niedriges —O-Alkyl bedeuten.

2. Verbindung nach Anspruch 1, worin n 2 ist.

3. Verbindung nach Anspruch 1 oder 2, worin A einen Benzenring darstellt.

4. Verbindung nach Anspruch 1 oder 2, worin A ein Naphthalenringsystem darstellt.

5. Verbindung nach Anspruch 1 oder 2, worin A einen Pyridinring darstellt.

6. Verbindung nach einem der Ansprüche 1 bis 5, worin der durch A dargestellte Ring durch ein oder mehrere Chlor- oder Bromatome oder durch eine Methoxygruppe substituiert ist.

7. Verbindung nach einem der Ansprüche 1 bis 6, worin R eine Methyl-, Ethyl-, Benzyl- oder Hexylgruppe darstellt.

8. Verbindung nach einem der Ansprüche 1 bis 7, worin Z eine Dimethylaminogruppe darstellt.

9. Verfahren nach Anspruch 1 zur Herstellung von 2 - [2 - (Dimethylamino)ethyl] - 2,3 - dihydro - 4 - methyl - 1,4 - benzoxazepin - 5(4H) - on, 2,3 - Dihydro - 4 - methyl - 2 - [2 - (4 - morpholino)ethyl] - 1,4 - benzoxazepin - 5(4H) - on, 4 - Benzyl - 2,3 - dihydro - 2 - [2 - (4 - morpholino)ethyl] - 1,4 - benzoxazepin - 5(4H) - on, 4 - Benzyl - 2,3 - dihydro - 2 - [2 - (methylamino)ethyl] - 1,4 - benzoxazepin - 5(4H) - on, 2 - [2 - (Dimethylamino)ethyl] - 2,3 - dihydro - 4 - methyl - 1,4 - benzoxazepin - 5(4H) - thio, 4 - Benzyl - 2 - [2 - (dimethylamino)ethyl] - 2,3 - dihydro - 1,4 - benzoxazepin - 5(4H) - on, 2,3 - Dihydro - 4 - methyl - 2 - [2 - (4 - morpholino)ethyl] - 1,4 - benzoxazepin - 5(4H) - thion, 2 - [2 - (Dimethylamino)ethyl] - 2,3 - dihydro - 4 - methylnapth[2,3 - f][1,4] - oxazepin - 5(4H) - on, 2 - [2 - (Dimethylamino)ethyl] - 2,3 - dihydro - 4 - methylpyrido[3,2 - f][1,4] - oxazepin - 5(4H) - on, 2 - [2 - (Dimethylamino)ethyl] - 2,3 - dihydro - 4 - methylpyrido[3,2 - f][1,4] - oxazepin - 5(4H) - thion, 4 - Benzyl - 2,3 - dihydro - 2 - [2 - (4 - morpholino)ethyl] - 1,4 - benzoxazepin - 5(4H) - thion, 2,3 - Dihydro - 4 - methyl - 2 - [2 - (methylamino)ethyl] - 1,4 - benzoxazepin - 5(4H) - on, 2,3 - Dihydro - 4 - methyl - 2 - [2 - (methylamino)ethyl] - 1,4 - benzoxazepin - 5(4H) - on - fumarat, 2,3 - Dihydro - 2 - [2 - (4 - hydroxy - 4 - phenyl) - piperidinylethyl] - 4 - methyl - 1,4 - benzoxazepin - 5(4H) - thion, 2,3 - Dihydro - 4 - methyl - 2 - [2 - (4 - phenyl - 1,2,3,6 - tetrahydro)pyridinyl - ethyl] - 1,4 - benzoxazepin - 5(4H) - thion, 8 - Chloro - 2 - [2 - (dimethylamino)ethyl] - 2,3 - dihydro - 4 - methyl - 1,4 - benzoxazepin - 5(4H) - thion, 8 - Chloro - 2 - [2 - (dimethylamino)ethyl] - 2,3 - dihydro - 4 - methyl - 1,4 - benzoxazepin - 5(4H) - on, 7 - Bromo - 2 - [2 - (dimethylamino)ethyl] - 2,3 - dihydro - 4 - methyl - 1,4 - benzoxazepin - 5(4H) - thion, 2 - [2 - (Dimethylamino)ethyl] - 2,3 - dihydro - 4 - methylnaphth - [2,1 - f][1,4] - oxazepin - 5(4H) - on, 2 - [2 - (Dimethylamino)ethyl] - 2,3 - dihydro - 4 - methylpyrido[4,3 - f][1,4] - oxazepin - 5(4H) - thion, 2 - [2 - (Dimethylamino)ethyl] - 2,3 - dihydro - 4 - methylnaphth[2,3 - f][1,4] - oxazepin - 5(4H) - thion, 7 - Chloro - 2 - [2 - (dimethylamino)ethyl] - 2,3 - dihydro - 4 - methyl - 1,4 - benzoxazepin - 5(4H) - on, 2 - [2 - (Dimethylamino)ethyl] - 2,3 - dihydro - 4 - methylpyrido[3,2 - f][1,4] - oxazepin - 5(4H) - thion - methiodid, 7 - Chloro - 2 - [2 - (dimethylamino)ethyl] - 2,3 - dihydro - 4 - methyl - 1,4 - benzoxazepin - 5(4H) - thion, 2 - [2 - (Dimethylamino)ethyl] - 2,3 - dihydro - 4 - methylnaphth[2,1 - f][1,4] - oxazepin - 5(4H) - thion, 2,2 - (Dimethylamino)ethyl - 2,3 - dihydro - 7 - methoxy - 4 - methyl - 1,4 - benzoxazepin - 5(4H) - on, 7 - Bromo - 2 - [2 - (Dimethylamino)ethyl] - 2,3 - dihydro - 4 - methyl - 1,4 - benzoxazepin - 5(4H) - thion, 2 - [2 - (Dimethylamino)ethyl] - 2,3 - dihydro - 4 - methylpyrido[3,2 - f][1,4] - thiazepin - 5(4H) - on, 2 - [2 - (Dimethylamino)ethyl] - 2,3 - dihydro - 4 - methylpyrido[3,2 - f][1,4] - thiazepin - 5(4H) - thion, 2 - [2 - (Dimethylamino)ethyl] - 2,3 - dihydro - 4 - methylpyrido[3,4 - f][1,4] - oxazepin - 5(4H) - on, 2 - [2 - (Dimethylamino)ethyl] - 2,3 - dihydro - 4 - methylpyrido[3,4 - f] - oxazepin - 5(4H) - thion, 2 - (3 - Aminopropyl) - 2,3 - dihydro - 4 - methyl - pyrido[3,2 - f][1,4] - oxazepin - 5(4H) - on, 2,3 - Dihydro - 4 - methyl - 2 - [2 - (4 - morpholinyl)ethyl] - pyrido[3,2 - f][1,4] - oxazepin - 5(4H) - on, 2,3 - Dihydro - 4 - methyl - 2 - [2 - (1 - pyrrolidinyl)ethyl] - pyrido[3,2 - f][1,4] - oxazepin - 5(4H) - on, 2 - [2 - (Dibutylamino)ethyl] - 2,3 - dihydro - 4 - methyl - pyrido[3,2 - f][1,4] - oxazepin - 5(4H) - on, 2 - [2 - (Diethylamino)ethyl] - 2,3 - dihydro - 4 - methylpyrido[3,2 - f][1,4] - oxazepin - 5(4H) - on, 2,3 - Dihydro - 4 - methyl - 2 - [2 - (1 - piperidinyl)ethyl] - pyrido[3,2 - f][1,4] - oxazepin - 5(4H) - on, 2,3 - Dihydro - 4 - methyl - 2 - [2 - (methyl(phenylmethyl)amino)ethyl]pyrido[3,2 - f][1,4] - oxazepin - 5(4H) - on, 2,3 - Dihydro - 4 - methyl - 2 - [2 - (methyl(phenylamino)ethyl]pyrido[3,2 - f][1,4] - oxazepin - 5(4H) - on, 2 - [2 - (2,5 - Dimethyl - 1 - pyrrolidinyl)ethyl - 2,3 - dihydro - 4 - methyl - pyrido[3,2 - f][1,4] - oxazepin - 5(4H) - on, 2,3 - Dihydro - 4 - methyl - 2 - [2 - (2 - methyl - 1 - pyrrolidinyl)ethyl]pyrido[3,2 - f][1,4] - oxazepin - 5(4H) - on, 2,3 - Dihydro - 4 - methyl - 2 - [2 - (1H - pyrazol - 1 - yl)ethyl] - pyrido[3,2 - f][1,4] - oxazepin - 5 - (4H) - on, 2,3 - Dihydro - 2 - [2 - (1H - imidazol - 1 - yl)ethyl] - 4 - methylpyrido[3,2 - f][1,4] - oxazepin - 5(4H) - on, 2 - [2 - Dimethylamino)ethyl] - 4 - ethyl - 2,3 - dihydropyrido[3,2 - f][1,4] - oxazepin - 5(4H) - on, 2,3 - Dihydro - 4 - ethyl - 2 - [2 - (1 - pyrrolidinyl)ethyl]pyrido[3,2 - f][1,4] - oxazepin - 5(4H) - on, 2,3 - Dihydro - 4 - methyl - 2 - [2 - (4 - morpholinyl)ethyl]pyrido[3,2 - f][1,4] - oxazepin - 5(4H) - thion, 2 - [2 - (Dibutylamino)ethyl] - 2,3 - dihydro - 4 - methylpyrido[3,2 - f][1,4] - oxazepin - 5(4H) - thion, 2 - [2 - (Diethylamino)ethyl] - 2,3 - dihydro - 4 - methylpyrido[3,2 - f][1,4] - oxazepin - 5(4H) - thion, 2,3 - Dihydro - 4 - methyl - 2 - [2 - (1 - pyrrolidinyl)ethyl]pyrido[3,2 - f][1,4] - oxazepin - 5(4H) - thion, 2,3 - Dihydro - 2 - [2 - (1H - imidazol - 1 - yl)ethyl] - 4 - methylpyrido[3,2 - f][1,4] - oxazepin - 5(4H) - thion, 2 - [2 - (Dimethylamino)ethyl] - 4 - ethyl - 2,3 - dihydropyrido[3,2 - f][1,4] - oxazepin - 5(4H) - thion, 2,3 - Dihydro - 4 - methyl - 2 - [2 - [methyl(phenylmethyl)amino]ethyl]-

pyrido[3,2 - f][1,4] - oxazepin - 5(4H) - thion, 2,3 - Dihydro - 2 - [methyl(amino)ethyl] - 4 - methylpyrido[3,2 - f][1,4] - oxazepin - 5(4H) - thion, 7 - Chloro - 2,3 - dihydro - 4 - methyl - 2 - [2 - (1 - pyrrolidino) - ethyl]pyrido[3,2 - f][1,4] - oxazepin - 5(4H) - on, 7 - Chloro - 2 - [2 - (dimethylamino)ethyl] - 2,3 - dihydro - 4 - methylpyrido[3,2 - f][1,4] - oxazepin - 5(4H) - on, 2 - [2 - (Dimethylamino)ethyl] - 2,3 - dihydro - 4 - phenylmethylpyrido[3,2 - f][1,4] - oxazepin - 5(4H) - on, 2 - (Dimethylamino)ethyl] - 2,3 - dihydropyrido[3,2 - f][1,4] - oxazepin - 5(4H) - on, 2 - [3 - (Dimethylamino)propyl] - 2,3 - dihydro - 4 - methylpyrido[3,2 - f][1,4] - oxazepin - 5(4H) - on, 2 - [3 - (Dimethylamino)propyl] - 2,3 - dihydro - 4 - methylpyrido[3,2 - f][1,4] - oxazepin - 5(4H) - thion oder 7 - Chloro - 2 - [2 - (dimethylamino)ethyl] - 2,3 - dihydro - 4 - methylpyrido - [3,2 - f] - oxazepin - 5(4H) - thion oder, wenn geeignet, ein pharmazeutisch verträgliches Salz davon.

10. Verfahren zur Herstellung einer Verbindung mit der Formel

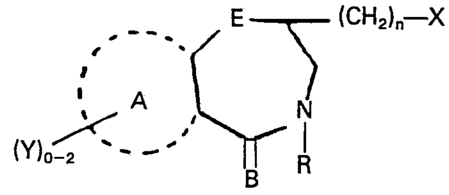

worin

A ein aromatisches Ringsystem darstellt, von dem zwei der Kohlenstoffatome gemeinsam gebunden sind an den Oxazepin- oder Thiazepin-Teil, der ausgewählt ist aus Benzen, einem Naphthalen und einem Pyridin in jeder beliebigen der vier Positionen, jeder der Ringe wahlweise substituiert sein kann durch ein oder zwei Y-Radikale, die ausgewählt sind aus Halo, niedrigem Alkyl, niedrigem Alkoxy, Nitro und Trifluormethyl;

B und E aus Sauerstoff und Schwefel ausgewählt sind und gleich oder verschieden sein können;

R ausgewählt ist aus niedrigem Alkyl, Cycloalkyl oder niedrigem Alkylphenyl, wobei Phenyl wahlweise substituiert ist durch ein oder zwei Radikale, die aus Halo, niedrigem Alkyl, niedrigem Alkoxy, Nitro und Trifluormethyl ausgewählt sind;

n 1 oder 2 ist;

X Chlor, Brom oder Cyano ist;

wobei die Begriffe "niedriges Alkyl" eine gerade oder verzweigte $C_{1-8}$ Alkylgruppe, und "niedriges Alkoxy" niedriges —O-Alkyl bedeuten, und soch das Verfahren zusammensetzt aus

i)a) einer Kondensation einer Verbindung mit der Formel

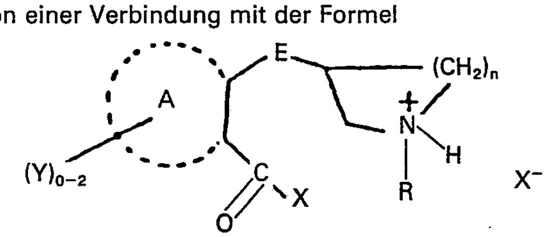

worin X Chlor oder Brom darstellt und A, E, n, R und Y das gleiche sind wie oben definiert;

b) wenn erforderlich einer Reaktion zwischen einer so gebildeten Verbindung und einem Schwefelungsmittel, um ein Oxazepinthion oder ein Thiazepinthion zu erhalten; und

c) wahlweise einer Reaktion einer Verbindung, die durch Schritt i)a) oder i)b) erhalten wurde, mit einem Alkalimetallcyanid, um die entsprechende Verbindung zu erhalten, in welcher X CN darstellt; oder

(ii) einer Halogenierung einer Verbindung, die durch Schritt i)a), i)b) oder i)c) erhalten wurde, mit der Formel

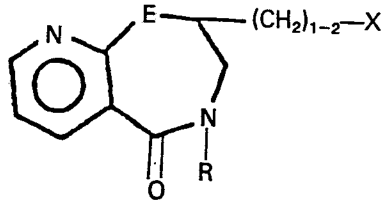

worin E und R das gleiche sind wie oben definiert, und X Chlor, Brom oder Cyano ist, mit Sulphurylchlorid in einem geeigneten Lösungsmittel, um eine Verbindung mit der Formel

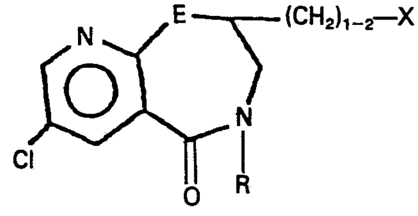

zu erhalten, worin E und R das gleiche sind wie oben definiert, und X Chlor, Brom oder Cyano ist; wobei die Begriffe "niedriges Alkyl" eine gerade oder verzweigte $C_{1-8}$ Alkylgruppe, und "niedriges Alkoxy" niedriges —O-Alkyl bedeuten.

11. Verbindung nach Anspruch 10, worin n 2 ist.

12. Verbindung nach Anspruch 10 oder 11, worin A einen Benzenring darstellt.

13. Verbindung nach Anspruch 10 oder 11, worin A ein Naphthalenringsystem darstellt.

14. Verbindung nach Anspruch 10 oder 11, worin A einen Pyridinring darstellt.

15. Verbindung nach einem der Ansprüche 10 bis 14, worin der durch A dargestellte Ring durch ein oder mehrere Chlor- oder Bromatome oder durch eine Methoxygruppe substituiert ist.

16. Verbindung nach einem der Ansprüche 10 bis 15, worin R eine Methyl-, Ethyl-, Benzyl oder Hexylgruppe darstellt.

17. Verfahren nach Anspruch 10 zur Herstellung von 2 - (2 - Chlorethyl) - 2,3 - dihydro - 4 - methyl - 1,4 - benzoxazepin - 5(4H) - on, 4 - Benzyl - 2 - (2 - chloroethyl) - 2,3 - dihydro - 1,4 - benzoxazepin - 5(4H) - on, 2 - (2 - Chloroethyl) - 2,3 - dihydro - 4 - methylnaphth[2,3 - f][1,4] - oxazepin - 5(4H) - on, 2 - (2 - Chloroethyl) - 2,3 - dihydro - 4 - methylpyrido[3,2 - f][1,4] - oxazepin - 5(4H) - on, 8 - Chloro - 2 - (2 - chloroethyl) - 2,3 - dihydro - 4 - methyl - 1,4 - benzoxazepin - 5(4H) - on, 7 - Bromo - 2 - (2 - chloroethyl) - 2,3 - dihydro - 4 - methyl - 1,4 - benzoxazepin - 5(4H) - on, 7 - Chloro - 2 - (2 - chloroethyl) - 2,3 - dihydro - 4 - methyl - 1,4 - benzoxazepin - 5(4H) - on, 2 - (2 - Chloroethyl) - 2,3 - dihydro - 4 - methylnaphth[2,1 - f][1,4] - oxazepin - 5(4H) - on, 2 - (2 - Chloroethyl - 2,3 - dihydro - 7 - methoxy - 4 - methyl - 1,4 - benzoxazepin - 5(4H) - on, 2 - (2 - Chloroethyl) - 2,3 - dihydro - 4 - methyl - 1,4 - benzoxazepin - 5(4H) - thion, 2 - (2 - Chloroethyl) - 2,3 - dihydro - 4 - methylpyrido[3,2 - f][1,4] - oxazepin - 5(4H) - thion, 2 - (2 - Chloroethyl) - 2,3 - dihydro - 4 - methylnaphth[2,3 - f][1,4] - oxazepin - 5(4H) - thion, 8 - Chloro - 2 - (2 - chloroethyl) - 2,3 - dihydro - 4 - methyl - 1,4 - benzoxazepin - 5(4H) - thion, 7 - Bromo - 2 - (2 - chloroethyl) - 2,3 - dihydro - 4 - methyl - 1,4 - benzoxazepin - 5(4H) - thion, 2 - (2 - Chloroethyl) - 2,3 - dihydro - 4 - methylnaphth[2,1 - f][1,4] - oxazepin - 5(4H) - thion, 2 - (2 - Chloroethyl) - 2,3 - dihydro - 4 - methylpyrido[4,3 - f][1,4] - oxazepin - 5(4H) - on, 7 - Chloro - 2 - (2 - chloroethyl) - 2,3 - dihydro - 4 - methyl - 1,4 - benzoxazepin - 5(4H) - thion, 2 - (2 - Chloroethyl) - 2,3 - dihydro - 4 - methyl - pyrido[4,3 - f][1,4] - oxazepin - 5(4H) - thion, 2 - (2 - Chloroethyl - 2,3 - dihydro - 7 - methoxy - 4 - methyl - 1,4 - benzoxazepin - 5(4H) - thion, 2 - (2 - Chloroethyl) - 2,3 - dihydro - 4 - methylpyrido[3,2 - f][1,4] - thiazepin - 5(4H) - on, 2 - (2 - Chloroethyl) - 2,3 - dihydro - 4 - methylpyrido[3,2 - f][1,4] - thiazepin - 5(4H) - thion, 2 - (2 - Chloroethyl) - 2,3 - dihydro - 4 - methylpyrido[3,4 - f][1,4] - oxazepin - 5(4H) - on, 2 - (2 - Chloroethyl) - 2,3 - dihydro - 4 - methylpyrido[3,4 - f][1,4] - oxazepin - 5(4H) - thion, 2,3,4,5 - Tetrahydro - 4 - methyl - 5 - oxopyrido[3,2 - f][1,4] - oxazepin - 2 - propannitril, 2 - (2 - Chloroethyl) - 4 - ethyl - 2,3 - dihydropyrido[3,2 - f][1,4] - oxazepin - 5(4H) - on, 2 - (2 - Chloroethyl) - 4 - ethyl - 2,3 - dihydropyrido[3,2 - f][1,4] - oxazepin - 5(4H) - thion, 7 - Chloro - 2 - (2 - chloroethyl) - 2,3 - dihydro - 4 - methylpyrido[3,2 - f][1,4] - oxazepin - 5(4H) - on, 7 - Chloro - 2 - (2 - chloroethyl) - 2,3 - dihydro - 4 - methylpyrido[3,2 - f][1,4] - oxazepin - 5(4H) - thion oder 2 - (Chloromethyl) - 4 - cyclohexyl - 2,3 - dihydropyrido[3,2 - f][1,4] - oxazepin - 5(4H) - on oder ein Säureadditionssalz davon.

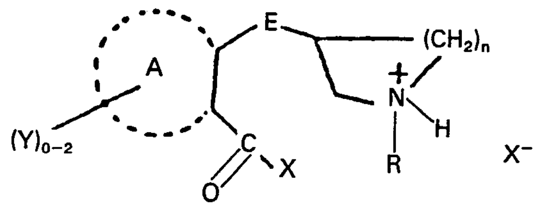

worin

A ein aromatisches Ringsystem darstellt, ausgewählt aus Benzen, einem Naphthalen oder einem Pyridin in jeder beliebigen der vier Positionen, jedes der Ringsysteme wahlweise substituiert sein kann durch ein oder zwei Y-Radikale, die ausgewählt sind aus Halo, niedrigem Alkyl, niedrigem Alkoxy, Nitro und Trifluormethyl;

R ausgewählt ist aus niedrigem Alkyl, Cycloalkyl oder niedrigem Alkylphenyl, Nitro und Trifluormethyl;

n 1 oder 2 ist;

X ausgewählt ist aus Chlor, Brom und Fluor, und E ausgewählt ist aus Schwefel und Sauerstoff; wobei die Begriffe "niedriges Alkyl" eine gerade oder verzweigte $C_{1-8}$ Alkylgruppe, und "niedriges Alkoxy" niedriges —O-Alkyl bedeuten, und das Verfahren aus einer Halogenierung der Verbindung mit der Formel IVb besteht

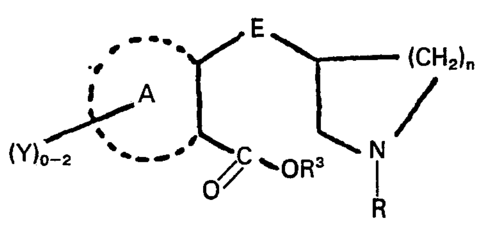

IVb

worin A, E, R, Y und n das gleiche sind wie oben definiert, und $R_3$ Wasserstoff oder ein Säureneutralisationsion ist.

19. Verfahren zur Herstellung einer Verbindung mit der Formel

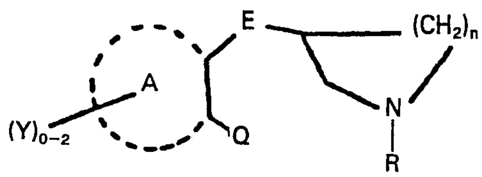

worin

A ein aromatisches Ringsystem darstellt, ausgewählt aus einem Naphthalen oder einem Pyridin in jeder beliebigen der vier Positionen, jedes der Ringsysteme wahlweise substituiert sein kann durch ein oder zwei Radikale, die ausgewählt sind aus der Gruppe Halo, niedriges Alkyl, niedriges Alkoxy, Nitro und Trifluormethyl;

R ausgewählt ist aus niedrigem Alkyl, Cycloalkyl oder niedrigem Alkylphenyl, wobei Phenyl wahlweise substituiert ist durch ein oder mehrere Radikale, ausgewählt aus Halo, niedrigem Alkyl, niedrigem Alkoxy, Nitro und Trifluormethyl;

n 1 oder 2 ist;

Q ausgewählt ist aus

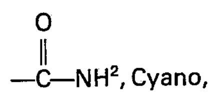 Cyano, 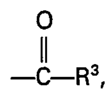

worin $R^3$ H ist, einem Säureneutralisationsion oder einem Veresterungsradikal, und E Sauerstoff oder Schwefel ist; wobei die Begriffe "niedriges Alkyl" eine gerade oder verzweigte $C_{1-8}$ Alkylgruppe, und "niedriges Alkoxy" niedriges —O-Alkyl bedeuten, und das Verfahren sich zusammensetzt entweder aus

(i) einer Reaktion zwischen Verbindungen jeder der beiden folgenden allgemeinen Formeln

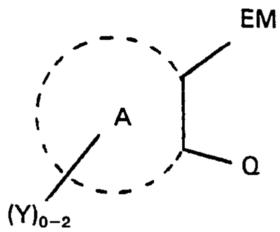

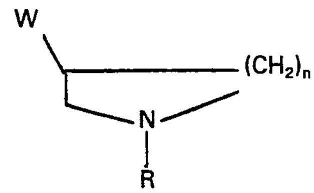

worin A, E, Q, R, Y und n das gleiche bedeuten wie oben definiert,

M ein Säureneutralisation ist und

W ein Aryl- oder Alkyllsulphonat oder Halogen ist; oder

(ii) einer Reaktion zwischen Verbindungen jeder der beiden folgenden allgemeinen Formeln

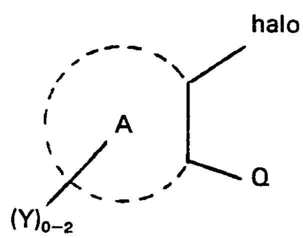

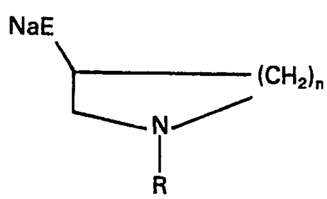

worin A, E, Q, R, Y und n das gleiche bedeuten wie oben definiert und Halo ein Halogenatom darstellt.

20. Verfahren nach Anspruch 19 zur Herstellung von 3-[(1-Methyl-3-pyrrolidinyl)oxy]-2-naphthalencarboxamid, 3-[(1-Methyl-3-pyrrolidinyl)oxy]-2-naphthalencarboxylsäure, deren Oxalatsalz, Natrium-2-[(1-methyl-3-pyrrolidinyl)oxy]-3-pyridincarboxalat, die freie Base davon, 1-[(1-Methyl-3-pyrrolidinyl)oxy]-2-naphthalencarboxamid, 3-[(1-Methyl-3-pyrrolidinyl)oxy]-4-pyridincarbonitril, dessen Fumaratsalz, oder 1-[(1-Methyl-3-pyrrolidinyl)oxy]-2-naphthalencarbonitril oder dessen Oxalatsalz.

21. Verfahren zur Herstellung einer pharmazeutischen Zusammensetzung durch Beimischen einer Verbindung mit der Formel

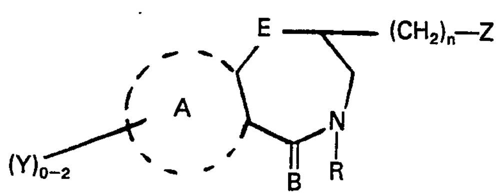

worin

A ein aromatisches Ringsystem darstellt, von dem zwei der Kohlenstoffatome gemeinsam gebunden sind an den Oxazepin- oder Thiazepin-Teil, der ausgewählt ist aus Benzen, einem Naphthalen oder einem Pyridin in jeder beliebigen der vier Positionen, jedes der Ringsysteme wahlweise substituiert sein kann durch ein oder zwei Y-Radikale, die ausgewählt sind aus Halo, niedrigem Alkyl, niedrigem Alkoxy, Nitro und Trifluormethyl;

B und E aus Sauerstoff und Schwefel ausgewählt sind und gleich oder verschieden sein können;

R ausgewählt ist aus Wasserstoff, niedrigem Alkyl, Cycloalkyl oder niedrigem Alkylphenyl, wobei Phenyl wahlweise substituiert ist durch ein oder zwei Radikale, die aus Halo, niedrigem Alkyl, niedrigem Alkoxy, Nitro, Trifluormethyl und Cyano ausgewählt sind;

n 1, 2 oder 3 ist;

Z aus —$NR^1R^2$, 1H-Pyrazol-1-yl oder 1H-Imidazol-1-yl ausgewählt ist;

$R^1$ und $R^2$ ausgewählt sind aus Wasserstoff, niedrigem Alkyl, Cycloalkyl und niedrigem Alkylphenyl, wobei Phenyl wahlweise substituiert ist durch 1 oder 2 Radikale, die aus Halo, niedrigem Alkyl, niedrigem Alkoxy, Nitro, Trifluormethyl und Cyano ausgewählt sind, oder $R^1$ und $R^2$ zusammen mit dem benachbarten Stickstoffatom einen heterocyclischen Rest bilden, der ausgewählt ist aus 1-Pyrrolidinyl, 2,5-Dimethylpyrrolidin-1-yl, 2-Methylpyrrolidin-1-yl, 1-Piperidinyl, 4-substituiertem Piperidin-1-yl, 4-Morpholinyl, 1-Piperazinyl, 4-substituiertem Piperazin-1-yl und 1,2,3,6-Tetrahydropyridin-1-yl, und pharmazeutisch verträgliche Salze davon unter der Voraussetzung, daß Z kein primäres oder sekundäres Amin ist, wenn R = H ist, und weiters unter der Voraussetzung, daß Z kein 1H-Pyrazol-1-yl, 1H-Imidazol-1-yl, 2,5-Dimethylpyrrolidon-1-yl, 2-Methylpyrrolidin-1-yl oder 1,2,3,6-Tetrahydropyridin-1-yl ist, wenn n 3 ist; wobei die Begriffe "niedriges Alkyl" eine gerade oder verzweigte $C_{1-8}$ Alkylgruppe, und "niedriges Alkoxy" niedriges —O-Alkyl bedeuten, und (b) einem pharmazeutischen Träger dafür.

22. Verfahren zur Herstellung einer pharmazeutischen Zusammensetzung durch Zusammenmischen von (a) einer Verbindung, die durch ein Verfahren nach einem der Ansprüche 2 bis 9 herstellbar ist, mit (b) einem pharmazeutischen Träger dafür.

**Revendications pour les Etats contractants: BE CH DE FR GB IT LI LU NL SE**

1. Composé de formule:

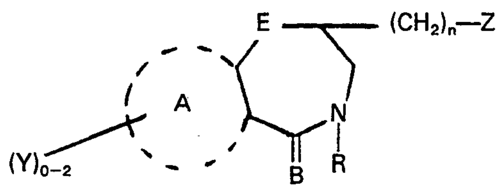

où:

A représente un système cyclique aromatique ayant deux de ses atomes de carbone mutuellement maintenus avec la partie oxazépine ou thiazépine, choisi entre le benzène, le naphtalène et la pyridine en l'une quelconque de ses quatre positions, l'un quelconque des cycles étant éventuellement substitué par un ou deux radicaux Y choisis entre halo, alcoyle inférieur, alcoxy inférieur, nitro et trifluorométhyle;

B et E sont choisis entre l'oxygène et le soufre et peuvent être identiques ou différents;

R est choisi entre hydrogène, alcoyle inférieur, $C_3$—$C_9$-cycloalcoyle et phényl-alcoyle inférieur où phényle est éventuellement substitué par un ou deux radicaux choisis entre halo, alcoyle inférieur, alcoxy inférieur, nitro et trifluorométhyle;

n est 1, 2 ou 3;

Z est choisi entre —$NR^1R^2$, 1H-pyrazol-1-yle et 1H-imidazol-1-yle;

chacun d'entre $R^1$ et $R^2$ est choisi parmi hydrogène, alcoyle inférieur, $C_3$—$C_9$-cycloalcoyle et phényl-alcoyle inférieur, où phényle est éventuellement substitué par un ou deux radicaux choisis parmi halo, alcoyle inférieur, alcoxy inférieur, nitro, trifluorométhyle et cyano, ou bien $R^1$ ou $R^2$, pris ensemble avec l'atome d'azote adjacent, forment un résidu hétérocyclique choisi parmi 1-pyrrolidinyle, 2,5-diméthylpyrrolidin-1-yle, 2-méthylpyrrolidin-1-yle, 1-pipéridinyle, pipéridin-1-yle 4-substitué, 4-morpholinyle, 1-pipérazinyle, pipérazin-1-yle 4-substitué et 1,2,3,6-tétrahydropyridin-1-yle, et les sels pharmaceutiquement acceptables correspondants, avec la restriction que lorsque R=H, Z n'est jamais une amine primaire ou secondaire et avec la restriction supplémentaire que lorsque n=3, Z n'est pas 1H-pyrazol-1-yle, 1H-imidazol-1-yle, 2,5-diméthylpyrrolidin-1-yle, 2-méthylpyrrolidin-1-yle ou 1,2,3,6-tétrahydropyridin-1-yle;

où le terme "alcoyle inférieur" désigne un radical $C_{1-8}$-alcoyle droit ou ramifié et "alcoxy inférieur" désigne —O-alcoyle inférieur.

2. Composé suivant la revendication 1, où n est 2.

3. Composé suivant la revendication 1 ou 2, où A représente un cycle benzène.

4. Composé suivant la revendication 1 ou 2, où A représente un système cyclique naphtalène.

5. Composé suivant la revendication 1 ou 2, où A représente un cycle pyridine.

6. Composé suivant l'une quelconque des revendications 1 à 5, où le cycle représenté par A est substitué par un ou plusieurs atomes de chlore ou de brome ou par un radical méthoxy.

7. Composé suivant l'une quelconque des revendications 1 à 6, où R représente un radical méthyle, éthyle, benzyle ou hexyle.

8. Composé suivant l'une quelconque des revendications 1 à 7, où Z représente un radical diméthylamino.

9. La 2-[2-(diméthylamino)éthyl]-2,3-dihydro-4-méthyl-1,4-benzoxazépin-5(4H)-one,
la 2,3-dihydro-4-méthyl-2-[2-(4-morpholino)éthyl]-1,4-benzoxazépin-5(4H)-one,
la 4-benzyl-2,3-dihydro-2-[2-(4-morpholino)éthyl]-1,4-benzoxazépin-5(4H)-one,
la 4-benzyl-2,3-dihydro-2-[2-(méthylamino)éthyl]-1,4-benzoxazépin-5(4H)-one,
la 2-[2-(diméthylamino)éthyl]-2,3-dihydro-4-méthyl-1,4-benzoxazépine-5(4H)-thione,
la 4-benzyl-2-[2-(diméthylamino)éthyl]-2,3-dihydro-1,4-benzoxazépin-5(4H)-thione,
la 2,3-dihydro-4-méthyl-2-[2-(4-morpholino)éthyl]-1,4-benzoxazépine-5(4H)-thione,
la 2-[2-(diméthylamino)éthyl]-2,3-dihydro-4-méthylnapht[2,3-f][1,4]oxazépin-5(4H)-one,
la 2-[2-(diméthylamino)éthyl]-2,3-dihydro-4-méthylpyrido[3,2-f][1,4]oxazépine-5(4H)-one,
la 2-[2-(diméthylamino)éthyl]-2,3-dihydro-4-méthylpyrido[3,2-f][1,4]oxazépine-5(4H)-thione,
la 4-benzyl-2,3-dihydro-2-[2-(4-morpholino)éthyl]-1,4-benzoxazépine-5(4H)-thione,
la 2,3-dihydro-4-méthyl-2-[2-(méthylamino)éthyl]-1,4-benzoxazépine-5(4H)-one,
le fumarate de 2,3-dihydro-4-méthyl-2-[2-(méthylamino)éthyl]-1,4-benzoxazépin-5(4H)-one,
la 2,3-dihydro-2-[2-(4-hydroxy-4-phényl)-pipéridinyléthyl]-4-méthyl-1,4-benzoxazépin-5(4H)-thione,
la 2,3-dihydro-4-méthyl-2-[2-(4-phényl-1,2,3,6-tétrahydropyridinyl)éthyl]-1,4-benzoxazépine-5(4H)-thione,
la 8-chloro-2-[2-(diméthylamino)éthyl]-2,3-dihydro-4-méthyl-1,4-benzoxazépine-5(4H)-thione,
le 8-chloro-2-[2-(diméthylamino)éthyl]-2,3-dihydro-4-méthyl-1,4-benzoxazépin-5(4H)-one,
la 7-bromo-2-[2-(diméthylamino)éthyl]-2,3-dihydro-4-méthyl-1,4-benzoxazépine-5(4H)-thione,
la 2-[2-(diméthylamino)éthyl]-2,3-dihydro-4-méthylnapht[2,1-f][1,4]oxazépin-5(4H)-one,
la 2-[2-(diméthylamino)éthyl]-2,3-dihydro-4-méthylpyrido[4,3-f][1,4]oxazépine-5(4H)-thione,
la 2-[2-(diméthylamino)éthyl]-2,3-dihydro-4-méthylnapht[2,3-f][1,4]oxazépine-5(4H)-thione,
la 7-chloro-2-[2-(diméthylamino)éthyl]-2,3-dihydro-4-méthyl-1,4-benzoxazépin-5(4H)-one,
le méthiodure de 2-[2-(diméthylamino)éthyl]-2,3-dihydro-4-méthylpyrido[3,2-f][1,4]oxazépine-5(4H)-thione,
la 7-chloro-2-[2-(diméthylamino)éthyl]-2,3-dihydro-4-méthyl-1,4-benzoxazépine-5(4H)-thione,
la 2-[2-(diméthylamino)éthyl]-2,3-dihydro-4-méthylnapht[2,1-f][1,4]-oxazépine-5(4H)-thione,
la 2-[2-(diméthylamino)éthyl]-2,3-dihydro-7-méthoxy-4-méthyl-1,4-benzoxazépin-5(4H)-one,
la 7-bromo-2-[2-(diméthylamino)éthyl]-2,3-dihydro-4-méthyl-1,4-benzoxazépine-5(4H)-thione,
la 2-[2-(diméthylamino)éthyl]-2,3-dihydro-4-méthylpyrido[3,2-f][1,4]thiazépin-5(4H)-one,
la 2-[2-(diméthylamino)éthyl]-2,3-dihydro-4-méthylpyrido[3,2-f][1,4]thiazépine-5(4H)-thione,
la 2-[2-(diméthylamino)éthyl]-2,3-dihydro-4-méthylpyrido[3,4-f][1,4]oxazépin-5(4H)-one,
la 2-[2-(diméthylamino)éthyl]-2,3-dihydro-4-méthylpyrido[3,4-f][1,4]oxazépine-5(4H)-thione,
la 2-(3-aminopropyl)-2,3-dihydro-4-méthylpyrido[3,2-f][1,4]oxazépin-5(4H)-one,
la 2,3-dihydro-4-méthyl-2-[2-(4-morpholinyl)éthyl]pyrido[3,2-f][1,4]oxazépin-5(4H)-one,
la 2,3-dihydro-4-méthyl-2-[2-(1-pyrrolidinyl)éthyl]pyrido[3,2-f][1,4]oxazépin-5(4H)-one,
la 2-[2-(dibutylamino)éthyl]-2,3-dihydro-4-méthylpyrido[3,2-f][1,4]oxazépin-5(4H)-one,
la 2-[2-(diéthylamino)éthyl]-2,3-dihydro-4-méthylpyrido[3,2-f][1,4]oxazépin-5(4H)-one,
la 2,3-dihydro-4-méthyl-2-[2-(1-pipéridinyl)éthyl]pyrido[3,2-f][1,4]oxazépin-5(4H)-one,
la 2,3-dihydro-4-méthyl-2-[2-(méthyl(phénylméthyl)amino)éthyl]pyrido[3,2-f][1,4]oxazépin-5(4H)-one,
la 2,3-dihydro-4-méthyl-2-[2-(méthylphénylamino)éthyl]pyrido[3,2-f][1,4]oxazépin-5(4H)-one,
la 2-[2-(2,5-diméthyl-1-pyrrolidinyl)éthyl]-2,3-dihydro-4-méthylpyrido[3,2-f][1,4]oxazépin-5(4H)-one,
la 2,3-dihydro-4-méthyl-2-[2-(2-méthyl-1-pyrrolidinyl)éthyl]pyrido[3,2-f][1,4]oxazépin-5(4H)-one,
la 2,3-dihydro-4-méthyl-2-[2-(1H-pyrazol-1-yl)éthyl]pyrido[3,2-f][1,4]oxazépin-5-(4H)-one,
la 2,3-dihydro-2-[2-(1H-imidazol-1-yl)éthyl]-4-méthylpyrido[3,2-f][1,4]oxazépin-5(4H)-one,
la 2-[2-(diméthylamino)éthyl]-4-éthyl-2,3-dihydropyrido[3,2-f][1,4]oxazépin-5(4H)-one,
la 2,3-dihydro-4-éthyl-2-[2-(1-pyrrolidinyl)éthyl]pyrido[3,2-f][1,4]oxazépin-5(4H)-one,
la 2,3-dihydro-4-méthyl-2-[2-(4-morpholinyl)éthyl]pyrido[3,2-f][1,4]oxazépine-5(4H)-thione,
la 2-[2-(dibutylamino)éthyl]-2,3-dihydro-4-méthylpyrido[3,2-f][1,4]oxazépine-5(4H)-thione,
la 2-[2-(diéthylamino)éthyl]-2,3-dihydro-4-méthylpyrido[3,2-f][1,4]oxazépine-5[4H)-thione,
la 2,3-dihydro-4-méthyl-2-[2-(1-pyrrolidinyl)éthyl]pyrido[3,2-f][1,4]oxazépine-5(4H)-thione,
la 2,3-dihydro-2-[2-(1H-imidazol-1-yl)éthyl]-4-méthylpyrido[3,2-f][1,4]oxazépine-5[4H]-thione,
la 2-[2-(diméthylamino)éthyl]-4-éthyl-2,3-dihydropyrido[3,2-f][1,4]oxazépine-5(4H)-thione,
la 2,3-dihydro-4-méthyl-2-[2-(méthyl(phénylméthyl)amino)éthyl]pyrido[3,2-f][1,4]oxazépine-5(4H)-thione,
la 2,3-dihydro-2-[2-(méthylamino)éthyl]-4-méthylpyrido[3,2-f][1,4]oxazépine-5(4H)-thione,
la 7-chloro-2,3-dihydro-4-méthyl-2-[2-(1-pyrrolidino)éthyl]pyrido[3,2-f][1,4]oxazépin-5(4H)-one,
la 7-chloro-2-[2-(diméthylamino)éthyl]-2,3-dihydro-4-méthylpyrido[3,2-f][1,4]oxazépin-5(4H)-one,
la 2-[2-(diméthylamino)éthyl]-2,3-dihydro-4-phénylméthylpyrido[3,2-f][1,4]oxazépin-5-(4H)-one,

la 2-[2-(diméthylamino)éthyl]-2,3-dihydropyrido[3,2-f][1,4]oxazépin-5(4H)-one,
la 2-[3-(diméthylamino)propyl]-2,3-dihydro-4-méthylpyrido[3,2-f][1,4]oxazépin-5(4H)-one,
la 2-[3-(diméthylamino)propyl]-2,3-dihydro-4-méthylpyrido[3,2-f][1,4]oxazépine-5(4H)-thione, ou
la 7-chloro-2-[2-(diméthylamino)éthyl]-2,3-dihydro-4-méthylpyrido[3,2-f]oxazépine-5(4H)-thione, ou bien, lorsqu'il y a lieu, un sel pharmaceutiquement acceptable correspondant.

10. Composé de formule:

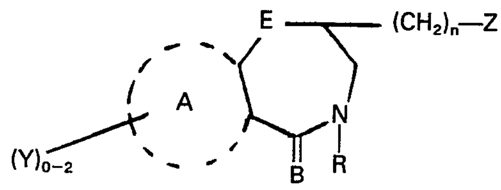

où:

A représente un système cyclique aromatique ayant deux de ses atomes de carbone mutuellement maintenus avec la partie oxazépine ou thiazépine, choisi entre le benzène, le naphtalène et la pyridine en l'une quelconque de ses quatre positions, l'un quelconque des cycles étant éventuellement substitué par un ou deux radicaux Y choisis entre halo, alcoyle inférieur, alcoxy inférieur, nitro et trifluorométhyle;

B et E sont choisis entre l'oxygène et le soufre et peuvent être identiques ou différents;

R est choisi entre hydrogène, alcoyle inférieur, $C_3$—$C_9$-cycloalcoyle et phényl-alcoyle inférieur où phényle est éventuellement substitué par un ou deux radicaux choisis entre halo, alcoyle inférieur, alcoxy inférieur, nitro et trifluorométhyle;

n est 1, 2 ou 3;

Z est choisi entre —$NR^1R^2$, 1H-pyrazol-1-yle et 1H-imidazol-1-yle;

chacun d'entre $R^1$ et $R^2$ est choisi parmi hydrogène, alcoyle inférieur, $C_3$—$C_9$-cycloalcoyle et phénylalcoyle inférieur, où phényle est éventuellement substitué par un ou deux radicaux choisis parmi halo, alcoyle inférieur, alcoxy inférieur, nitro, trifluorométhyle et cyano, ou bien $R^1$ ou $R^2$, pris ensemble avec l'atome d'azote adjacent, forment un résidu hétérocyclique choisi parmi 1-pyrrolidinyle, 2,5-diméthylpyrrolidin-1-yle, 2-méthylpyrrolidin-1-yle, 1-pipéridinyle, pipéridin-1-yle 4-substitué, 4-morpholinyle, 1-pipérazinyle, pipérazin-1-yle 4-substitué et 1,2,3,6-tétrahydropyridin-1-yle, et les sels pharmaceutiquement acceptables correspondants, avec la restriction que lorsque R=H, Z n'est jamais une amine primaire ou secondaire et avec la restriction supplémentaire que lorsque n=3, Z n'est pas 1H-pyrazol-1-yle, 1H-imidazol-1-yle, 2,5-diméthylpyrrolidin-1-yle ou 2-méthylpyrrolidin-1-yle;
où le terme "alcoyle inférieur" désigne un radical $C_{1-8}$-alcoyle droit ou ramifié et "alcoxy inférieur" désigne —O-alcoyle inférieur; à utiliser en médecine.

11. Composé suivant l'une quelconque des revendications 2 à 9, à utiliser en médecine.

12. Procédé pour préparer un composé de formule:

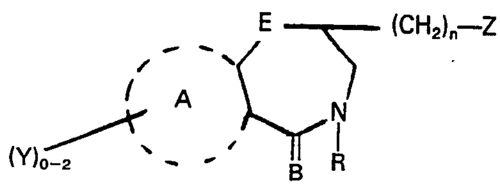

où:

A représente un système cyclique aromatique ayant deux de ses atomes de carbone mutuellement maintenus avec la partie oxazépine ou thiazépine, choisi entre le benzène, le naphtalène et la pyridine en l'une quelconque de ses quatre positions, l'un quelconque des cycles étant éventuellement substitué par un ou deux radicaux Y choisis entre halo, alcoyle inférieur, alcoxy inférieur, nitro et trifluorométhyle;

B et E sont choisis entre l'oxygène et le soufre et peuvent être identiques ou différents;

R est choisi entre hydrogène, alcoyle inférieur, cycloalcoyle et phényl-alcoyle inférieur où phényle est éventuellement substitué par un ou deux radicaux choisis entre halo, alcoyle inférieur, alcoxy inférieur, nitro et trifluorométhyle;

n est 1, 2 ou 3;

Z est choisi entre —$NR^1R^2$, 1H-pyrazol-1-yle et 1H-imidazol-1-yle;

$R^1$ et $R^2$ sont choisis parmi hydrogène, alcoyle inférieur, cycloalcoyle et phényl-alcoyle inférieur, où phényle est éventuellement substitué par un ou deux radicaux choisis parmi halo, alcoyle inférieur, alcoxy inférieur, nitro, trifluorométhyle et cyano, ou bien $R^1$ ou $R^2$, pris ensemble avec l'atome d'azote adjacent, forment un résidu hétérocyclique choisi parmi 1-pyrrolidinyle, 2,5-diméthylpyrrolidin-1-yle, 2-méthylpyrrolidin-1-yle, 1-pipéridinyle, pipéridin-1-yle 4-substitué, 4-morpholinyle, 1-pipérazinyle, pipérazin-1-yle 4-substitué et 1,2,3,6-tétrahydropyridin-1-yle, et les sels pharmaceutiquement acceptables correspondants, avec la restriction que lorsque R=H, Z n'est jamais une amine primaire ou secondaire et avec la restriction supplémentaire que lorsque n=3, Z n'est pas 1H-pyrazol-1-yle, 1H-imidazol-1-yle, 2,5-diméthylpyrrolidin-1-yle, 2-méthylpyrrolidin-1-yle ou 1,2,3,6-tétrahydropyridin-1-yle; ce procédé comprenant:

i) la réaction d'un composé de formule:

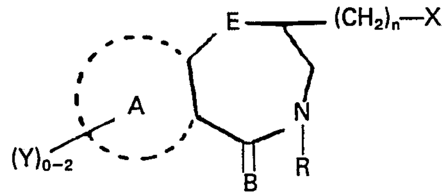

où A, B, E, R, Y et n sont tels que définis à propos de la formule I et X représente chlore, brome ou cyano, avec un composé de formule:

ZH

où Z est tel que défini à propos de la formule I:

ii) la réduction d'un composé de formule:

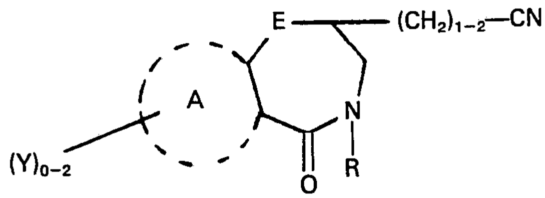

pour former un composé de formule I, où B représente oxygène, Z représenté $—NH_2$ et n est 2 ou 3; et, si nécessaire:

iii) la conversion d'un composé de formule I ainsi obtenu en un autre composé de formule I; et facultativement:

iv) la préparation d'un sel pharmaceutiquement acceptable d'un composé ainsi obtenu;

où le terme "alcoyle inférieur" désigne un radical $C_{1-8}$-alcoyle droit ou ramifié et "alcoxy inférieur" désigne —O-alcoyle inférieur.

13. Procédé suivant la revendication 12, pour préparer un composé suivant l'une quelconque des revendications 2 à 9.

14. Composé de formule:

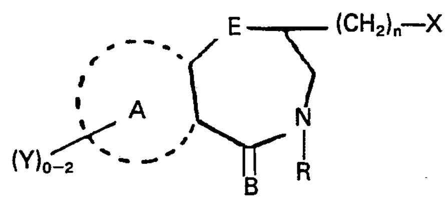

où:

A représente un système cyclique aromatique ayant deux de ses atomes de carbone mutuellement maintenus avec la partie oxazépine ou thiazépine, choisi entre le benzène, le naphtalène et la pyridine en l'une quelconque de ses quatre positions, l'un quelconque des cycles étant éventuellement substitué par un ou deux radicaux Y choisis entre halo, alcoyle inférieur, alcoxy inférieur, nitro et trifluorométhyle;

B et E sont choisis entre l'oxygène et le soufre et peuvent être identiques ou différents;

R est choisi entre alcoyle inférieur, cylcoalcoyle et phényl-alcoyle inférieur où phényle est éventuellement substitué par un ou deux radicaux choisis entre halo, alcoyle inférieur, alcoxy inférieur, nitro et trifluorométhyle;

n est 1 ou 2;

X est chlore, brome ou cyano;

où le terme "alcoyle inférieur" désigne un radical $C_{1-8}$-alcoyle droit ou ramifié et "alcoxy inférieur" désigne —O-alcoyle inférieur.

15. Composé suivant la revendication 14, où n est 2.

16. Composé suivant la revendication 14 ou 15, où A représente un cycle benzène.

17. Composé suivant la revendication 14 ou 15, où A représente un système cyclique naphtalène.

18. Composé suivant la revendication 14 ou 15, où A représente un cycle pyridine.

19. Composé suivant l'une quelconque des revendications 14 à 18, où le cycle représenté par A est substitué par un ou plusieurs atomes de chlore ou de brome ou par un radical méthoxy.

20. Composé suivant l'une quelconque des revendications 13 à 18, où R représente un radical méthyle, éthyle, benzyle ou hexyle.

21. La 2-(2-chloroéthyl)-2,3-dihydro-4-méthyl-1,4-benzoxazépin-5-(4H)-one,

la 4-benzyl-2-(2-chloroéthyl)-2,3-dihydro-1,4-benzoxazépin-5(4H)-one,

la 2-(2-chloroéthyl)-2,3-dihydro-4-méthylnapht[2,3-f][1,4]oxazépin-5(4H)-one,

la 2-(2-chloroéthyl)-2,3-dihydro-4-méthylpyrido[3,2-f][1,4]oxazépin-5(4H)-one,
la 8-chloro-2-(2-chloroéthyl)-2,3-dihydro-4-méthyl-1,4-benzoxazépin-5(4H)-one,
la 7-bromo-2-(2-chloroéthyl)-2,3-dihydro-4-méthyl-1,4-benzoxazépin-5(4H)-one,
la 7-chloro-2-(2-chloroéthyl)-2,3-dihydro-4-méthyl-1,4-benzoxazépin-5(4H)-one,
la 2-(2-chloroéthyl)-2,3-dihydro-4-méthylnapht[2,1-f][1,4]oxazépin-5(4H)-one,
la 2-(2-chloroéthyl)-2,3-dihydro-7-méthoxy-4-méthyl-1,4-benzoxazépin-5(4H)-one,
la 2-(2-chloroéthyl)-2,3-dihydro-4-méthyl-1,4-benzoxazépine-5(4H)-thione,
la 2-(2-chloroéthyl)-2,3-dihydro-4-méthylpyrido[3,2-f][1,4]oxazépine-5(4H)-thione,
la 2-(2-chloroéthyl)-2,3-dihydro-4-méthylnapht[2,3-f][1,4]oxazépine-5(4H)-thione,
la 8-chloro-2-(2-chloroéthyl)-2,3-dihydro-4-méthyl-1,4-benzoxazépine-5(4H)-thione,
la 7-bromo-2-(2-chloroéthyl)-2,3-dihydro-4-méthyl-1,4-benzoxazépine-5(4H)-thione,
la 2-(2-chloroéthyl)-2,3-dihydro-4-méthylnapht[2,1-f][1,4]oxazépine-5(4H)-thione,
la 2-(2-chloroéthyl)-2,3-dihydro-4-méthylpyrido[4,3-f][1,4]oxazépin-5(4H)-one,
la 7-chloro-2-(2-chloroéthyl)-2,3-dihydro-4-méthyl-1,4-benzoxazépine-5(4H)-one,
la 2-(2-chloroéthyl)-2,3-dihydro-4-méthylpyrido[4,3-f][1,4]oxazépine-5(4H)-one,
la 2-(2-chloroéthyl)-2,3-dihydro-7-méthoxy-4-méthyl-1,4-benzoxazépine-5(4H)-one,
la 2-(2-chloroéthyl)-2,3-dihydro-4-méthylpyrido[3,2-f][1,4]thiazépin-5(4H)-one,
la 2-(2-chloroéthyl)-2,3-dihydro-4-méthylpyrido[3,2-f][1,4]thiazépine-5(4H)-one,
la 2-(2-chloroéthyl)-2,3-dihydro-4-méthylpyrido[3,4-f][1,4]oxazépin-5(4H)-one,
la 2-(2-chloroéthyl)-2,3-dihydro-4-méthylpyrido[3,4-f][1,4]oxazépine-5(4H)-one,
le 2,3,4,5-tétrahydro-4-méthyl-5-oxopyrido[3,2-f][1,4]oxazépine-2-propanenitrile,
la 2-(2-chloroéthyl)-4-éthyl-2,3-dihydropyrido[3,2-f][1,4]oxazépin-5(4H)-one,
la 2-(2-chloroéthyl)-4-éthyl-2,3-dihydropyrido[3,2-f][1,4]oxazépine-5(4H)-thione,
la 7-chloro-2-(2-chloroéthyl)-2,3-dihydro-4-méthylpyrido[3,2-f][1,4]oxazépin-5(4H)-one,
la 7-chloro-2-(2-chloroéthyl)-2,3-dihydro-4-méthyl-pyrido[3,2-f][1,4]oxazépine-5(4H)-thione, ou
la 2-(chlorométhyl)-4-cyclohexyl-2,3-dihydropyrido[3,2-f][1,4]oxazépin-5(4H)-one ou un sel d'addition d'acide correspondant.

22. Procédé pour préparer un composé de formule:

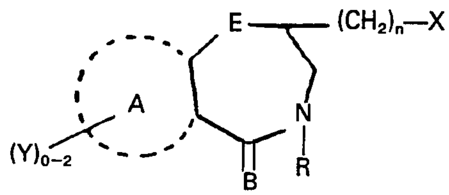

où:

A représente un système cyclique aromatique ayant deux de ses atomes de carbone mutuellement maintenus avec la partie oxazépine ou thiazépine, choisi entre le benzène, le naphtalène et la pyridine en l'une quelconque de ses quatre positions, l'un quelconque des cycles étant éventuellement substitué par un ou deux radicaux Y choisis entre halo, alcoyle inférieur, alcoxy inférieur, nitro et trifluorométhyle;

B et E sont choisis entre l'oxygène et le soufre et peuvent être identiques ou différents;

R est choisi entre alcoyle inférieur, cycloalcoyle et phényl-alcoyle inférieur ou phényle est éventuellement substitué par alcoxy inférieur, nitro ou trifluorométhyle;

n est 1 ou 2;

X est chlore, brome ou cyano, ou un sel d'addition d'acide correspondant, lequel composé comprend:

i) a) la fusion d'un composé de formule:

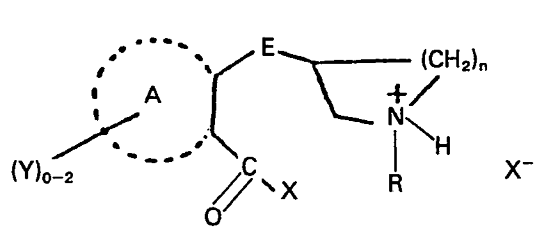

où X représente chlore ou brome et A, E, n, R et Y sont tels que définis ci-dessus;

b) lorsque la chose est requise, la réaction du composé résultant avec un agent de sulfuration pour former une oxazépinethione ou une thiazépinethione, et

c) éventuellement, la réaction d'un composé obtenu au stade i)a) ou i)b) avec un cyanure de métal alcalin pour obtenir le composé correspondant où X représente CN, ou

ii) l'halogénation d'un composé obtenu au stade i)a), i)b) i)c) de formule:

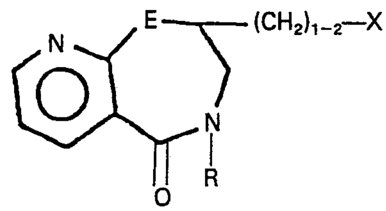

où E et R sont tels que définis ci-dessus et X est chlore, brome ou cyano, avec du chlorure de sulfuryle dans un solvant approprié pour obtenir un composé de formule:

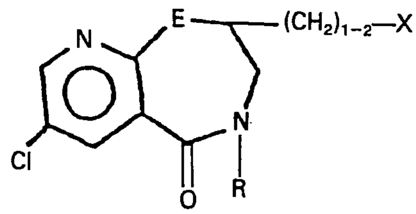

où E et R sont tels que définis ci-dessus et X est chlore, brome ou cyano; où le terme "alcoyle inférieur" désigne un radical $C_{1-8}$-alcoyle droit ou ramifié et "alcoxy inférieur" désigne —O-alcoyle inférieur.

23. Procédé suivant la revendication 22, pour préparer un composé suivant l'une quelconque des revendications 15 à 21.

24. Composés choisi dans la classe de formule:

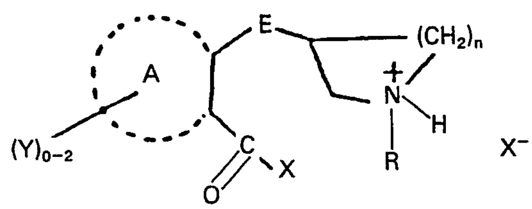

où:

A représente un système cyclique aromatique choisi entre le benzène, le naphtalène et la pyridine en l'une quelconque de ses quatre positions, l'un quelconque des cycles étant éventuellement substitué par un ou deux radicaux Y choisis entre halo, alcoyle inférieur, alcoxy inférieur, nitro et trifluorométhyle;

R est choisi entre alcoyle inférieur, cycloalcoyle, phényl-alcoxy inférieur, nitro et trifluorométhyle;

n est 1 ou 2;

X est choisi entre chlore, brome et fluor, et

E est choisi entre soufre et oxygène, où le terme "alcoyle inférieur" désigne un radical $C_{1-8}$-alcoyle droit ou ramifié et "alcoxy inférieur" désigne —O-alcoyle inférieur.

25. Composé de formule:

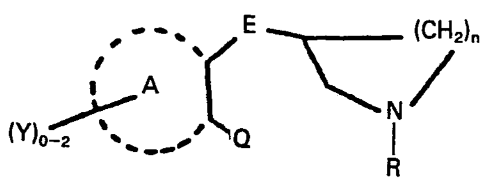

où:

A représente un system cyclique aromatique choisi entre le naphtalène et la pyridine dans l'une quelconque de ses quatre positions, l'un quelconque de systèmes cycliques étant facultativement substitué par un ou deux radicaux choisis dans la classe formée par halo, alcoyle inférieur, alcoxy inférieur, nitro et trifluorométhyle;

R est choisi parmi alcoyle inférieur, cycloalcoyle, phényl-alcoyle inférieur, où phényle est éventuellement substitué par un ou deux radicaux choisis parmi halo, alcoyle inférieur, alcoxy inférieur, nitro et trifluorométhyle;

n est 1 ou 2;

Q est choisi entre —C(O)—$NH_2$, cyano et —C(O)—$R^3$, où $R^3$ est H, un ion neutralisant les acides ou un radical estérifiant, et

E est oxygène ou soufre; où le terme "alcoyle inférieur" désigne un radical $C_{1-8}$-alcoyle droit ou ramifié et "alcoxy inférieur" désigne —O—alcoyle inférieur.

26. Le 3-[(1-méthyl-3-pyrrolidinyl)oxy]-2-naphtalènecarboxamide, l'acide 3-[(1-méthyl-3-pyrrolidinyl)oxy]-2-naphtalènecarboxylique, son sel oxalique, le 2-[(1-méthyl-3-pyrrolidinyl)oxy]-3-pyridinecarboxylate de sodium, sa base libre, le 1-[(1-méthyl-3-pyrrolidinyl)oxy]-2-naphtalènecarboxamide, le 3-[(1-méthyl-3-pyrrolidinyl)oxy]-4-pyridinecarbonitrile, son sel fumarique ou le 1-[(1-méthyl-3-pyrrolidinyl)oxy]-2-naphtalènecarbonitrile ou son sel oxalique.

27. Composition pharmaceutique comprenant:

a) un composé de formule:

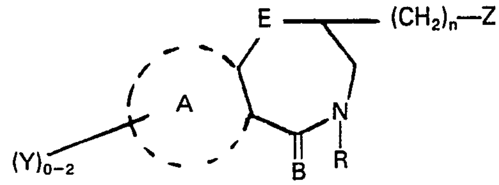

A représente un système cyclique aromatique ayant deux de ses atomes de carbone mutuellement maintenus avec la partie oxazépine ou thiazépine, choisi entre le benzène, le naphtalène et la pyridine en l'une quelconque de ses quatre positions, l'un quelconque des cycles étant éventuellement substitué par un ou deux radicaux Y choisis entre halo, alcoyle inférieur, alcoxy inférieur, nitro et trifluorométhyle;

B et E sont choisis entre l'oxygène et le soufre et peuvent être identiques ou différents;

R est choisi entre hydrogène, alcoyle inférieur, cycloalcoyle et phényl-alcoyle inférieur où phényle est éventuellement substitué par un ou deux radicaux choisis entre halo, alcoyle inférieur, alcoxy inférieur, nitro et trifluorométhyle;

n est 1, 2 ou 3;

Z est choisi entre —$NR^1R^2$, 1H-pyrazol-1-yle et 1H-imidazol-1-yle;

chacun d'entre $R^1$ et $R^2$ est choisi parmi hydrogène, alcoyle inférieur, cycloalcoyle et phényl-alcoyle inférieur, où phényle est éventuellement substitué par un ou deux radicaux choisis parmi halo, alcoyle inférieur, alcoxy inférieur, nitro, trifluorométhyle et cyano, ou bien $R^1$ ou $R^2$, pris ensemble avec l'atome d'azote adjacent, forment un résidu hétérocyclique choisi parmi 1-pyrrolidinyle, 2,5-diméthylpyrrolidin-1-yle, 2-méthylpyrrolidin-1-yle, 1-pipéridinyle, pipéridin-1-yle 4-substitué, 4-morpholinyle, 1-pipérazinyle, pipérazin-1-yle 4-substitué et 1,2,3,6-tétrahydropyridin-1-yle, et les sels pharmaceutiquement acceptables correspondants, avec la restriction que lorsque R=H, Z n'est jamais une amine primaire ou secondaire et avec la restriction supplémentaire que lorsque n=3, Z n'est pas 1H-pyrazol-1-yle, 1H-imidazol-1-yle, 2,5-diméthylpyrrolidin-1-yle, 2-méthylpyrrolidin-1-yle ou 1,2,3,6-tétrahydropyridin-1-yle;

où le terme "alcoyle inférieur" désigne un radical $C_{1-8}$-alcoyle droit ou ramifié et "alcoxy inférieur" désigne —O-alcoyle inférieur, et

b) un excipient pharmaceutique.

28. Composition pharmaceutique comprenant:

a) un composé suivant l'une quelconque des revendications 2 à 9, et

b) un excipient pharmaceutique.

**Revendications pour l'Etat contractant: AT**

1. Procédé pour prépare un composé de formule:

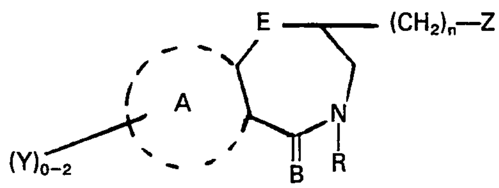

où:

A représente un système cyclique aromatique ayant deux de ses atomes de carbone mutuellement maintenus avec la partie oxazépine ou thiazépine, choisi entre le benzène, le naphtalène et la pyridine en l'une quelconque de ses quatre positions, l'un quelconque des cycles étant éventuellement substitué par un ou deux radicaux Y choisis entre halo, alcoyle inférieur, alcoxy inférieur, nitro et trifluorométhyle;

B et E sont choisis entre l'oxygène et le soufre et peuvent être identiques ou différents;

R est choisi entre hydrogène, alcoyle inférieur, $C_3$—$C_9$-cycloalcoyle et phényl-alcoyle inférieur où phényle est éventuellement substitué par un ou deux radicaux choisis entre halo, alcoyle inférieur, alcoxy inférieur, nitro et trifluorométhyle;

n est 1, 2 ou 3;

Z est choisi entre —$NR^1R^2$, 1H-pyrazol-1-yle et 1H-imidazol-1-yle;

chacun d'entre $R^1$ et $R^2$ est choisi parmi hydrogène, alcoyle inférieur, $C_3$—$C_9$-cycloalcoyle et phényl-alcoyle inférieur, où phényle est éventuellement substitué par un ou deux radicaux choisis parmi halo, alcoyle inférieur, alcoxy inférieur, nitro, trifluorométhyle et cyano, ou bien $R^1$ ou $R^2$, pris ensemble avec l'atome d'azote adjacent, forment un résidu hétérocyclique choisi parmi 1-pyrrolidinyle, 2,5-diméthylpyrrolidin-1-yle, 2-méthylpyrrolidin-1-yle, 1-pipéridinyle, pipéridin-1-yle 4-substitué, 4-morpholinyle, 1-pipérazinyle, pipérazin-1-yle 4-substitué et 1,2,3,6-tétrahydropyridin-1-yle, et les sels pharmaceutiquement acceptables correspondants, avec la restriction que lorsque R=H, Z n'est jamais une amine primaire ou secondaire et avec la restriction supplémentaire que lorsque n=3, Z n'est pas 1H-pyrazol-1-yle, 1H-imidazol-1-yle, 2,5-diméthylpyrrolidin-1-yle, 2-méthylpyrrolidin-1-yle ou 1,2,3,6-tétrahydropyridin-1-yle;
où le terme "alcoyle inférieur" désigne un radical $C_{1-8}$-alcoyle droit ou ramifié et "alcoxy inférieur" désigne —O-alcoyle inférieur; ce procédé comprenant:

i) la réaction d'un composé de formule:

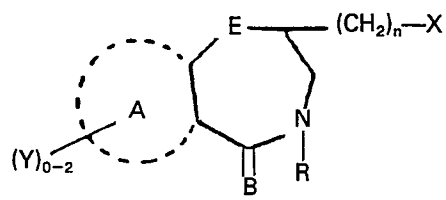

où A, B, E, R, Y et n sont tels que définis à propos de la formule I et X représente chlore, brome ou cyano, avec un composé de formule:

ZH

où Z est tel que défini à propos de la formule I:

ii) la réduction d'un composé de formule:

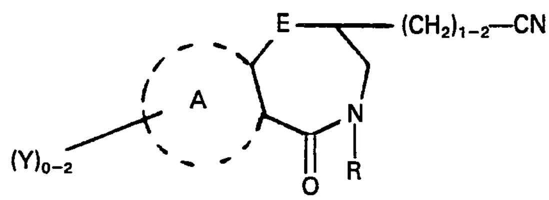

pour former un composé de formule I, où B représente oxygène, Z représenté $—NH_2$ et n est 2 ou 3; et, si nécessaire:

iii) la conversion d'un composé de formule I ainsi obtenu en un autre composé de formule I; et facultativement:

iv) la préparation d'un sel pharmaceutiquement acceptable d'un composé ainsi obtenu;

où le terme "alcoyle inférieur" désigne un radical $C_{1-8}$-alcoyle droit ou ramifié et "alcoxy inférieur" désigne —O—alcoyle inférieur.

2. Procédé suivant la revendication 1, où n est 2.

3. Composé suivant la revendication 1 ou 2, où A représente un cycle benzène.

4. Composé suivant la revendication 1 ou 2, où A représente un système cyclique naphtalène.

5. Composé suivant la revendication 1 ou 2, où A représente un cycle pyridine.

6. Procédé suivant l'une quelconque des revendications 1 à 5, où le cycle représenté par A est substitué par un ou plusieurs atomes de chlore ou de brome ou par un radical méthoxy.

7. Procédé suivant l'une quelconque des revendications 1 à 6, où R représente un radical méthyle, éthyle, benzyle ou hexyle.

8. Procédé suivant l'une quelconque des revendications 1 à 7, où Z représente un radical diméthylamino.

9. Procédé suivant la revendication 1, pour préparer la 2-[2-(diméthylamino)éthyl]-2,3-dihydro-4-méthyl-1,4-benzoxazépin-5(4H)-one,
la 2,3-dihydro-4-méthyl-2-[2-(4-morpholino)éthyl]-1,4-benzoxazépin-5(4H)-one,
la 4-benzyl-2,3-dihydro-2-[2-(4-morpholino)éthyl]-1,4-benzoxazépin-5(4H)-one,
la 4-benzyl-2,3-dihydro-2-[2-(méthylamino)éthyl]-1,4-benzoxazépin-5(4H)-one,
la 2-[2-(diméthylamino)éthyl]-2,3-dihydro-4-méthyl-1,4-benzoxazépine-5(4H)-thione,
la 4-benzyl-2-[2-(diméthylamino)éthyl]-2,3-dihydro-1,4-benzoxazépin-5(4H)-thione,
la 2,3-dihydro-4-méthyl-2-[2-(4-morpholino)éthyl]-1,4-benzoxazépine-5(4H)-thione,
la 2-[2-(diméthylamino)éthyl]-2,3-dihydro-4-méthylnapht[2,3-f][1,4]oxazépin-5(4H)-one,
la 2-[2-(diméthylamino)éthyl]-2,3-dihydro-4-méthylpyrido[3,2-f][1,4]oxazépin-5(4H)-one,
la 2-[2-(diméthylamino)éthyl]-2,3-dihydro-4-méthylpyrido[3,2-f][1,4]oxazépine-5(4H)-thione,
la 4-benzyl-2,3-dihydro-2-[2-(4-morpholino)éthyl]-1,4-benzoxazépine-5(4H)-thione,
la 2,3-dihydro-4-méthyl-2-[2-(méthylamino)éthyl]-1,4-benzoxazépin-5(4H)-one,
le fumarate de 2,3-dihydro-4-méthyl-2-[2-(méthylamino)éthyl]-1,4-benzoxazépin-5(4H)-one,
la 2,3-dihydro-2-[2-(4-hydroxy-4-phényl)-pipéridinyléthyl]-4-méthyl-1,4-benzoxazépine-5(4H)-thione,
la 2,3-dihydro-4-méthyl-2-[2-(4-phényl-1,2,3,6-tétrahydropyridinyl)éthyl]-1,4-benzoxazépine-5(4H)-thione,

la 8-chloro-2-[2-(diméthylamino)éthyl]-2,3-dihydro-4-méthyl-1,4-benzoxazépine-5(4H)-thione,
le 8-chloro-2-[2-(diméthylamino)éthyl]-2,3-dihydro-4-méthyl-1,4-benzoxazépin-5(4H)-one,
la 7-bromo-2-[2-(diméthylamino)éthyl]-2,3-dihydro-4-méthyl-1,4-benzoxazépine-5(4H)-thione,
la 2-[2-(diméthylamino)éthyl]-2,3-dihydro-4-méthylnapht[2,1-f][1,4]oxazépin-5(4H)-one,
la 2-[2-(diméthylamino)éthyl]-2,3-dihydro-4-méthylpyrido[4,3-f][1,4]oxazépine-5(4H)-thione,
la 2-[2-(diméthylamino)éthyl]-2,3-dihydro-4-méthylnapht[2,3-f][1,4]oxazépine-5(4H)-thione,
la 7-chloro-2-[2-(diméthylamino)éthyl]-2,3-dihydro-4-méthyl-1,4-benzoxazépin-5(4H)-one,
le méthiodure de 2-[2-(diméthylamino)éthyl]-2,3-dihydro-4-méthylpyrido[3,2-f][1,4]oxazépine-5(4H)-thione,
la 7-chloro-2-[2-(diméthylamino)éthyl]-2,3-dihydro-4-méthyl-1,4-benzoxazépine-5(4H)-thione,
la 2-[2-(diméthylamino)éthyl]-2,3-dihydro-4-méthylnapht[2,1-f][1,4]-oxazépine-5(4H)-thione,
la 2-[2-(diméthylamino)éthyl]-2,3-dihydro-7-méthoxy-4-méthyl-1,4-benzoxazépin-5(4H)-one,
la 7-bromo-2-[2-(diméthylamino)éthyl]-2,3-dihydro-4-méthyl-1,4-benzoxazépine-5(4H)-thione,
la 2-[2-(diméthylamino)éthyl]-2,3-dihydro-4-méthylpyrido[3,2-f][1,4]thiazépin-5(4H)-one,
la 2-[2-(diméthylamino)éthyl]-2,3-dihydro-4-méthylpyrido[3,2-f][1,4]thiazépine-5(4H)-thione,
la 2-[2-(diméthylamino)éthyl]-2,3-dihydro-4-méthylpyrido[3,4-f][1,4]oxazépin-5(4H)-one,
la 2-[2-(diméthylamino)éthyl]-2,3-dihydro-4-méthylpyrido[3,4-f][1,4]oxazépine-5(4H)-thione,
la 2-(3-aminopropyl)-2,3-dihydro-4-méthylpyrido[3,2-f][1,4]oxazépin-5(4H)-one,
la 2,3-dihydro-4-méthyl-2-[2-(4-morpholinyl)éthyl]pyrido[3,2-f][1,4]oxazépin-5(4H)-one,
la 2,3-dihydro-4-méthyl-2-[2-(1-pyrrolidinyl)éthyl]pyrido[3,2-f][1,4]oxazépin-5(4H)-one,
la 2-[2-(dibutylamino)éthyl]-2,3-dihydro-4-méthylpyrido[3,2-f][1,4]oxazépin-5(4H)-one,
la 2-[2-(diéthylamino)éthyl]-2,3-dihydro-4-méthylpyrido[3,2-f][1,4]oxazépin-5(4H)-one,
la 2,3-dihydro-4-méthyl-2-[2-(1-pipéridinyl)éthyl]pyrido[3,2-f][1,4]oxazépin-5(4H)-one,
la 2,3-dihydro-4-méthyl-2-[2-(méthyl(phénylméthyl)amino)éthyl]pyrido[3,2-f][1,4]oxazépin-5(4H)-one,
la 2,3-dihydro-4-méthyl-2-[2-(méthylphénylamino)éthyl]pyrido[3,2-f][1,4]oxazépin-5(4H)-one,
la 2-[2-(2,5-diméthyl-1-pyrrolidinyl)éthyl]-2,3-dihydro-4-méthylpyrido[3,2-f][1,4]oxazépin-5(4H)-one,
la 2,3-dihydro-4-méthyl-2-[2-(2-méthyl-1-pyrrolidinyl)éthyl]pyrido[3,2-f][1,4]oxazépin-5(4H)-one,
la 2,3-dihydro-4-méthyl-2-[2-(1H-pyrazol-1-yl)éthyl]pyrido[3,2-f][1,4]oxazépin-5-(4H)-one,
la 2,3-dihydro-2-[2-(1H-imidazol-1-yl)éthyl]-4-méthylpyrido[3,2-f][1,4]oxazépin-5(4H)-one,
la 2-[2-(diméthylamino)éthyl]-4-éthyl-2,3-dihydropyrido[3,2-f][1,4]oxazépin-5(4H)-one,
la 2,3-dihydro-4-éthyl-2-[2-(1-pyrrolidinyl)éthyl]pyrido[3,2-f][1,4]oxazépin-5(4H)-one,
la 2,3-dihydro-4-méthyl-2-[2-(4-morpholinyl)éthyl]pyrido[3,2-f][1,4]oxazépine-5(4H)-thione,
la 2-[2-(dibutylamino)éthyl]-2,3-dihydro-4-méthylpyrido[3,2-f][1,4]oxazépine-5(4H)-thione,
la 2-[2-(diéthylamino)éthyl]-2,3-dihydro-4-méthylpyrido[3,2-f][1,4]oxazépine-5[4H)-thione,
la 2,3-dihydro-4-méthyl-2-[2-(1-pyrrolidinyl)éthyl]pyrido[3,2-f][1,4]oxazépine-5(4H)-thione,
la 2,3-dihydro-2-[2-(1H-imidazol-1-yl)éthyl]-4-méthylpyrido[3,2-f][1,4]oxazépine-5[4H]-thione,
la 2-[2-(diméthylamino)éthyl]-4-éthyl-2,3-dihydropyrido[3,2-f][1,4]oxazépine-5(4H)-thione,
la 2,3-dihydro-4-méthyl-2-[2-(méthyl(phénylméthyl)amino)éthyl]pyrido[3,2-f][1,4]oxazépine-5(4H)-thione,
la 2,3-dihydro-2-[2-(méthylamino)éthyl]-4-méthylpyrido[3,2-f][1,4]oxazépine-5(4H)-thione,
la 7-chloro-2,3-dihydro-4-méthyl-2-[2-(1-pyrrolidino)éthyl]pyrido[3,2-f][1,4]oxazépin-5(4H)-one,
la 7-chloro-2-[2-(diméthylamino)éthyl]-2,3-dihydro-4-méthylpyrido[3,2-f][1,4]oxazépin-5(4H)-one,
la 2-[2-(diméthylamino)éthyl]-2,3-dihydro-4-phénylméthylpyrido[3,2-f][1,4]oxazépin-5-(4H)-one,
la 2-[2-(diméthylamino)éthyl]-2,3-dihydropyrido[3,2-f][1,4]oxazépin-5(4H)-one,
la 2-[3-(diméthylamino)propyl]-2,3-dihydro-4-méthylpyrido[3,2-f][1,4]oxazépin-5(4H)-one,
la 2-[3-(diméthylamino)propyl]-2,3-dihydro-4-méthylpyrido[3,2-f][1,4]oxazépine-5(4H)-thione, ou
la 7-chloro-2-[2-(diméthylamino)éthyl]-2,3-dihydro-4-méthylpyrido[3,2-f]oxazépine-5(4H)-thione, ou bien, lorsqu'il y a lieu, un sel pharmaceutiquement acceptable correspondant.

10. Procédé pour préparer un composé de formule:

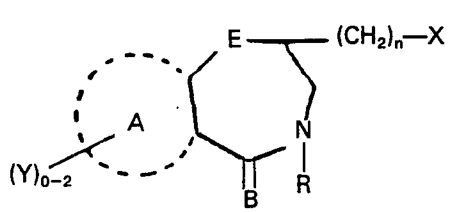

où:

A représente un système cyclique aromatique ayant deux de ses atomes de carbone mutuellement maintenus avec la partie oxazépine ou thiazépine, choisi entre le benzène, le naphtalène et la pyridine en l'une quelconque de ses quatre positions, l'un quelconque des cycles étant éventuellement substitué par un ou deux radicaux Y choisis entre halo, alcoyle inférieur, alcoxy inférieur, nitro et trifluorométhyle;

B et E sont choisis entre l'oxygène et le soufre et peuvent être identiques ou différents;

R est choisi entre alcoyle inférieur, cycloalcoyle et phényl-alcoyle inférieur où phényle est éventuellement substitué par un ou deux radicaux choisis entre halo, alcoyle inférieur, alcoxy inférieur, nitro et trifluorométhyle;

n est 1, 2 ou 3;

X est chlore, brome ou cyano; où le terme "alcoyle inférieur" désigne un radical $C_{1-8}$-alcoyle droit ou ramifié et "alcoxy inférieur" désigne —O-alcoyle inférieur;

i) a) la fusion d'un composé de formule:

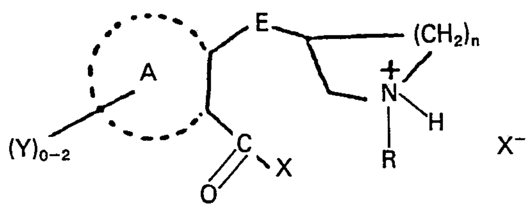

où X représente chlore ou brome et A, E, n, R et Y sont tels que définis ci-dessus;

b) lorsque la chose est requise, la réaction du composé résultant avec un agent de sulfuration pour former une oxazépinethione ou une thiazépinethione, et

c) éventuellement, la réaction d'un composé obtenu au stade i)a) ou i)b) avec un cyanure de métal alcalin pour obtenir le composé correspondant où X représente CN, ou

ii) l'halogénation d'un composé obtenu au stade i)a), i)b) ou i)c) de formule:

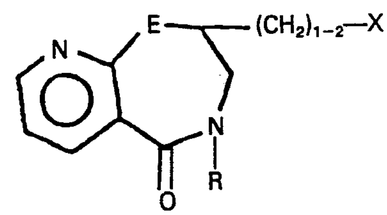

où E et R sont tels que définis ci-dessus et X est chlore, brome ou cyano, avec du chlorure de sulfuryle dans un solvant approprié pour obtenir un composé de formule:

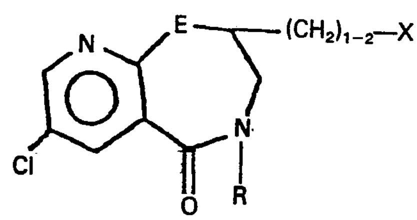

où E et R sont tels que définis ci-dessus et X est chlore, brome ou cyano;
où le terme "alcoyle inférieur" désigne un radical $C_{1-8}$-alcoyle droit ou ramifié et "alcoxy inférieur" désigne —O-alcoyle inférieur.

11. Procéde suivant la revendication 10, où n est 2.

12. Procédé suivant la revendication 10 ou 11, où A représente un cycle benzène.

13. Procédé suivant la revendication 10 ou 11, où A représente un système cyclique naphtalène.

14. Procédé suivant la revendication 10 ou 11, où A représente un cycle pyridine.

15. Procédé suivant l'une quelconque des revendications 10 à 14, où le cycle représenté par A est substitué par un ou plusieurs atomes de chlore ou de brome ou par un radical méthoxy.

16. Procédé suivant l'une quelconque des revendications 10 à 15, où R représente un radical méthyle, éthyle, benzyle ou hexyle.

17. Procédé suivant la revendication 10, pour préparer la 2-(2-chloroéthyl)-2,3-dihydro-4-méthyl-1,4-benzoxazépin-5-(4H)-one,
la 4-benzyl-2-(2-chloroéthyl)-2,3-dihydro-1,4-benzoxazépin-5(4H)-one,
la 2-(2-chloroéthyl)-2,3-dihydro-4-méthylnapht[2,3-f][1,4]oxazépin-5(4H)-one,
la 2-(2-chloroéthyl)-2,3-dihydro-4-méthylpyrido[3,2-f][1,4]oxazépin-5(4H)-one,
la 8-chloro-2-(2-chloroéthyl)-2,3-dihydro-4-méthyl-1,4-benzoxazépin-5(4H)-one,
la 7-bromo-2-(2-chloroéthyl)-2,3-dihydro-4-méthyl-1,4-benzoxazépin-5(4H)-one,
la 7-chloro-2-(2-chloroéthyl)-2,3-diohydro-4-méthyl-1,4-benzoxazépin-5(4H)-one,
la 2-(2-chloroéthyl)-2,3-dihydro-4-méthylnapht[2,1-f][1,4]oxazépin-5(4H)-one,
la 2-(2-chloroéthyl)-2,3-dihydro-7-méthoxy-4-méthyl-1,4-benzoxazépin-5(4H)-one,
la 2-(2-chloroéthyl)-2,3-dihydro-4-méthyl-1,4-benzoxazépine-5(4H)-thione,

la 2-(2-chloroéthyl)-2,3-dihydro-4-méthylpyrido[3,2-f][1,4]oxazépine-5(4H)-thione,
la 2-(2-chloroéthyl)-2,3-dihydro-4-méthylnapht[2,3-f][1,4]oxazépine-5(4H)-thione,
la 8-chloro-2-(2-chloroéthyl)-2,3-dihydro-4-méthyl-1,4-benzoxazépine-5(4H)-thione,
la 7-bromo-2-(2-chloroéthyl)-2,3-dihydro-4-méthyl-1,4-benzoxazépine-5(4H)-thione,
la 2-(2-chloroéthyl)-2,3-dihydro-4-méthylnapht[2,1-f][1,4]oxazépine-5(4H)-thione,
la 2-(2-chloroéthyl)-2,3-dihydro-4-méthylpyrido[4,3-f][1,4]oxazépin-5(4H)-one,
la 7-chloro-2-(2-chloroéthyl)-2,3-dihydro-4-méthyl-1,4-benzoxazépine-5(4H)-thione,
la 2-(2-chloroéthyl)-2,3-dihydro-4-méthylpyrido[4,3-f][1,4]oxazépine-5(4H)-thione,
la 2-(2-chloroéthyl)-2,3-dihydro-7-méthoxy-4-méthyl-1,4-benzoxazépine-5(4H)-thione,
la 2-(2-chloroéthyl)-2,3-dihydro-4-méthylpyrido[3,2-f][1,4]thiazépin-5(4H)-one,
la 2-(2-chloroéthyl)-2,3-dihydro-4-méthylpyrido[3,2-f][1,4]thiazépine-5(4H)-thione,
la 2-(2-chloroéthyl)-2,3-dihydro-4-méthylpyrido[3,4-f][1,4]oxazépin-5(4H)-thione,
la 2-(2-chloroéthyl)-2,3-dihydro-4-méthylpyrido[3,4-f][1,4]oxazépine-5(4H)-thione,
le 2,3,4,5-tétrahydro-4-méthyl-5-oxopyrido[3,2-f][1,4]oxazépine-2-propanenitrile,
la 2-(2-chloroéthyl)-4-éthyl-2,3-dihydropyrido[3,2-f][1,4]oxazépin-5(4H)-one,
la 2-(2-chloroéthyl)-4-éthyl-2,3-dihydropyrido[3,2-f][1,4]oxazépine-5(4H)-thione,
la 7-chloro-2-(2-chloroéthyl)-2,3-dihydro-4-méthylpyrido[3,2-f][1,4]oxazépin-5(4H)-one,
la 7-chloro-2-(2-chloroéthyl)-2,3-dihydro-4-méthyl-pyrido[3,2-f][1,4]oxazépine-5(4H)-thione, ou
la 2-(chlorométhyl)-4-cyclohexyl-2,3-dihydropyrido[3,2-f][1,4]oxazépin-5(4H)-one ou un sel d'addition d'acide correspondant.

18. Procédé pour préparer un composé de formule:

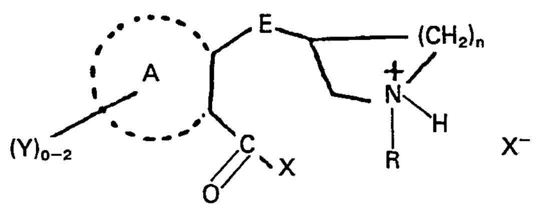

où:

A représente un système cyclique aromatique choisi entre le benzène, le naphtalène et la pyridine en l'une quelconque de ses quatre positions, l'un quelconque des cycles étant éventuellement substitué par un ou deux radicaux Y choisis entre halo, alcoyle inférieur, alcoxy inférieur, nitro et trifluorométhyle;

R est choisi entre alcoyle inférieur, cycloalcoyle, phényl-alcoxy inférieur, nitro et trifluorométhyle;

n est 1 ou 2;

X est choisi entre chlore, brome et fluor, et

E est choisi entre soufre et oxygène, où le terme "alcoyle inférieur" désigne un radical $C_{1-8}$-alcoyle droit ou ramifié et "alcoxy inférieur" désigne —O-alcoyle inférieur; lequel procédé comprend l'halogénation d'un composé de formule IVb:

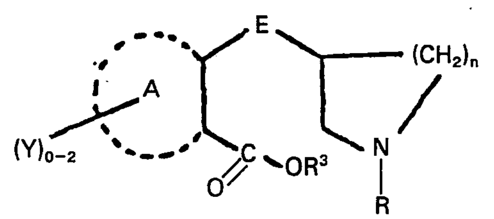

IVb

où A, E, R, Y et n sont tels que définis ci-dessus et $R^3$ est hydrogène ou un ion neutralisant les acides.

19. Procédé pour préparer un composé de formule:

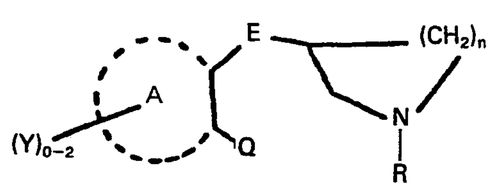

où:

A représente un système cyclique aromatique choisi entre le naphtalène et la pyridine dans l'une quelconque de ses quatre positions, l'un quelconque de systèmes cycliques étant facultativement substitué par un ou deux radicaux choisis dans la classe formée par halo, alcoyle inférieur, alcoxy inférieur, nitro et trifluorométhyle;

R est choisi parmi alcoyle inférieur, cycloalcoyle, phényl-alcoyle inférieur, où phényle est éventuellement substitué par un ou deux radicaux choisis parmi halo, alcoyle inférieur, alcoxy inférieur, nitro et trifluorométhyle;

n est 1 ou 2;

Q est choisi entre —C(O)—$NH_2$, cyano et —C(O)—$R^3$, où $R^3$ est H, un ion neutralisant les acides ou un radical estérifiant, et

E est oxygène ou soufre; où le terme "alcoyle inférieur" désigne un radical $C_{1-8}$-alcoyle droit ou ramifié et "alcoxy inférieur" désigne —O—alcoyle inférieur; lequel procédé comprend soit:

(i) la réaction mutuelle d'un composé de chacune des deux formules générales suivantes:

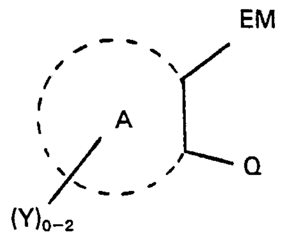

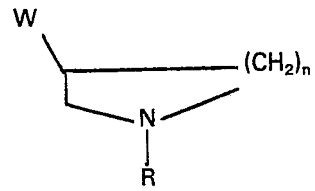

où A, E, Q, R, Y et N sont tels que définis ci-dessus,

M est un ion neutralisant les acides, et

W est un aryl- ou alcoyl-sulfonate ou un halogène; ou

(ii) la réaction mutuelle d'un composé de chacune des deux formules générales:

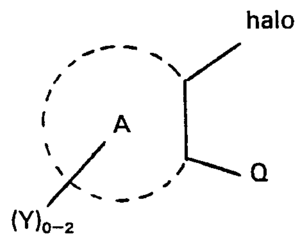

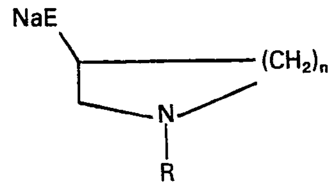

où A, E, Q, R, Y et n sont tels que définis ci-dessus et halo représente un atoms d'halogène.

20. Procédé suivant la revendication 19, pour préparer le 3-[(1-méthyl-3-pyrrolidinyl)oxy]-2-naphtalènecarboxamide, l'acide 3-[(1-méthyl-3-pyrrolidinyl)oxy]-2-naphtalènecarboxylique, son sel oxalique, le 2-[(1-méthyl-3-pyrrolidinyl)oxy]-3-pyridine-carboxylate de sodium, sa base libre, le 1-[(1-méthyl-3-pyrrolidinyl)oxy]-2-naphtalènecarboxamide, le 3-[(1-méthyl-3-pyrrolidinyl)oxy]-4-pyridinecarbonitrile, son sel fumarique ou le 1-[(1-méthyl-3-pyrrolidinyl)oxy]-2-naphtalènecarbonitrile ou son sel oxalique.

21. Procédé pour préparer une composition pharmaceutique, lequel procédé comprend le mélange

a) d'un composé de formule:

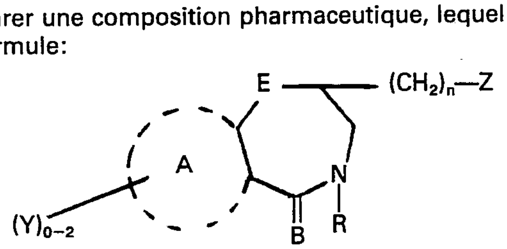

où:

A représente un système cyclique aromatique ayant deux de ses atomes de carbone mutuellement maintenus avec la partie oxazépine ou thiazépine, choisi entre le benzène, le naphtalène et la pyridine en l'une quelconque de ses quatre positions, l'un quelconque des cycles étant éventuellement substitué par un ou deux radicaux Y choisis entre halo, alcoyle inférieur, alcoxy inférieur, nitro et trifluorométhyle;

B et E sont choisis entre l'oxygène et le soufre et peuvent être identiques ou différents;

R est choisi entre hydrogène, alcoyle inférieur, cycloalcoyle et phényl-alcoyle inférieur ou phényle est éventuellement substitué par un ou deux radicaux choisis entre halo, alcoyle inférieur, alcoxy inférieur, nitro et trifluorométhyle;

n est 1, 2 ou 3;

Z est choisi entre —$NR^1R^2$, 1H-pyrazol-1-yle et 1H-imidazol-1-yle;

chacun d'entre $R^1$ et $R^2$ est choisi parmi hydrogène, alcoyle inférieur, cycloalcoyle et phényl-alcoyle inférieur, où phényle est éventuellement substitué par un ou deux radicaux choisis parmi halo, alcoyle inférieur, alcoxy inférieur, nitro, trifluorométhyle et cyano, ou bien $R^1$ ou $R^2$, pris ensemble avec l'atome d'azote adjacent, forment un résidu hétérocyclique choisi parmi 1-pyrrolidinyle, 2,5-diméthylpyrrolidin-1-yle, 2-méthylpyrrolidin-1-yle, 1-pipéridinyle, pipéridin-1-yle 4-substitué, 4-morpholinyle, 1-pipérazinyle, pipérazin-1-yle 4-substitué et 1,2,3,6-tétrahydropyridin-1-yle, et les sels pharmaceutiquement acceptables correspondants, avec la restriction que lorsque R=H, Z n'est jamais une amine primaire ou secondaire et

avec la restriction supplémentaire que lorsque n=3, Z n'est pas 1H-pyrazol-1-yle, 1H-imidazol-1-yle, 2,5-diméthylpyrrolidin-1-yle, 2-méthylpyrrolidin-1-yle ou 1,2,3,6-tétrahydropyridin-1-yle;
où le terme "alcoyle inférieur" désigne un radical $C_{1-8}$-alcoyle droit ou ramifié et "alcoxy inférieur" désigne —O-alcoyle inférieur, et

b) un excipient pharmaceutique.

22. Procédé de préparation d'une composition pharmaceutique, lequel procédé comprend le mélange:

a) d'un composé pouvant être préparé par un procédé suivant l'une quelconque des revendications 2 à 9, et

b) d'un excipient pharmaceutique.